# EUROPEAN PATENT SPECIFICATION

(11) **EP 4 562 021 B1**
(45) Date of publication and mention of the grant of the patent: **08.07.2026**
(21) Application number: 23754144.6
(22) Date of filing: 26.07.2023
(51) Int. Cl.: C07J 51/00, C07F 7/08, C07F 7/18

(54) **IONIZABLE CATIONIC LIPIDS INCORPORATING SILICON**
KATIONISCHE LIPIDE AUF SILIKONBASIS
LIPIDES CATIONIQUES À BASE DE SILICIUM

(30) Priority: 28.07.2022 EP 22462007
(43) Date of publication of application: 04.06.2025
(73) Proprietor: AldexChem Kft., 2030 Érd (HU)
(72) Inventor: RÉPÁSI, József, 2030 Érd (HU); SZILVÁGYI, Gábor, 2017 Pócsmegyer (HU)
(74) Representative: Danubia Patent & Law Office LLC
(86) International application number: PCT/EP2023/070748
(87) International publication number: WO 2024/023174

(56) References cited:
- WO-A1-2011/134675
- WO-A1-2021/055835
- WO-A1-2021/204175
- WO-A1-2022/129966
- WO-A1-2022/140239
- WO-A2-2022/152109

## Description

### TECHNICAL FIELD

The present invention belongs to the field of biomedicine and drug delivery.

The invention relates to a novel ionizable cationic lipid family incorporating silicon, which belongs to the trademark LipexSil^{™} 1^{st} generation lipids, wherein at least one of the two side chains contains silyl acetal linker. Lipids containing silyl acetal linker(s) so far are unprecedented in the art and are effective as ionizable cationic lipids in the formulation of empty or loaded lipid nanoparticles (LNPs). The novel linkers according to the invention are designed by means of borane catalysts [WO 2022/129966]. The invention describes the synthesis of the lipids of formula (I), formation and characterization of nanoparticles and biological experiments demonstrating that the lipid nanoparticles prepared with these novel lipids can efficiently deliver their cargo (e.g. RNA, DNA, mRNA, siRNA, pDNA, circular DNA, small biologically active molecules) into the cells.

### BACKGROUND

There are many challenges associated with the transfection of nucleic acids-based agents e.g. messenger RNA, antisense oligonucleotides, ribozymes, plasmids and of small molecules etc. to trigger a desired response in a biological system. There is a need to design and synthesize novel second generation lipids (biodegradable ionizable amine lipids) in order to improve the delivery of biologically active agents, for example DNA, RNA, mRNA and various small molecules to cells. In the field of medicine those treatments are the most effective which can selectively and directly target affected cells or tissues with the appropriate active pharmaceutical agents. With this in mind, the efficiency of treatment shall be increased, and undesirable toxic side effects shall be avoided or reduced. Loaded lipid nanoparticles (LNPs), which contain lipid components have been proven to be an effective carrier system which can be functionalized to protect and deliver the payload to its targeted site. It is to be emphasized that LNPs formed from ionizable cationic lipids also have proven to be outstanding lipid-based carriers in gene therapy.

LNPs also contain synthetic lipids which may be toxic to human cells and the cytotoxicity of synthetic lipids depends on certain motifs (e.g. type of headgroup, linker) in the lipids relating to their biodegradability and path of their metabolism in the human body.

The LNPs have some key parameters which have a great role in successful drug delivery applications such as particle size, polydispersity index (PDI), zeta potential and apparent pKa of LNP. Based on literature data the average particle size of LNPs is normally between 100 and 400 nm by accompanying narrow particle size distribution, <0.2 PDI (Advanced NanoBiomed Research, 2022, 2, 2100109). The optimal apparent pKa of LNPs are between 6 and 7 (Trends in Pharmacological Sciences, 2021, 42:6, 448-460).

In the art few examples can be found demonstrating that silicon atom containing lipids are also suitable components of LNPs [WO 2011134675, WO 2021055835] which are effective in the delivery of DNA, RNA, siRNA and nucleotides etc. Silicon is a carbon isostere in drug research and the incorporation of silicon atom could provide innovative solutions to medicinal chemistry problems. The incorporated silicon atom in the lipids can enhance the lipophilicity thereby improving the membrane permeability. Nevertheless, there is still growing demand in the art of efficient, biodegradable, less toxic delivery platforms, since in the next decade the gene therapy is going to become part of the routine treatments.

WO-A-2021/055835 and WO-A-2011/134675 disclose transfection agents containing silicon atoms in the hydrophobic part of the molecule, but where the silicon atoms in these compounds of these documents do not appear in the content of the ketal groups (a), (b) or (c) in which they appear in variable group T¹ in formula (I) of the present invention.

### DETAILED DESCRIPTION

The present invention provides a series of novel lipids incorporating silicon having the structure of formula (I), details of synthetic methods, details of LNPs' formation and characterisation as well as toxicity and transfection experiments of loaded LNPs.

In one embodiment, the invention provides an ionizable cationic lipid of formula (I) 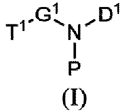 or its salt or stereoisomer,
wherein:
- G¹ is: unsubstituted C₂-C₉ alkylene,
- (CH₂)ₓ-CH=CH-(CH₂) _{y}-, wherein x is an integer selected from 1 to 6, y is an integer selected from 1 to 6, and the sum of x+y is an integer selected from 2 to 7, or
- (CH₂)_{w}-X-(CH₂) _{z}-, wherein w is an integer selected from 1 to 7, z is an integer selected from 2 to 7, the sum of w+z is an integer selected from 3 to 8, wherein-(CH₂)_{z}- is attached to N, and X is selected from O, S, SO and SO₂;
- T¹ is: 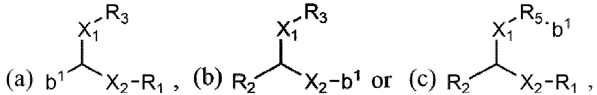 wherein
b¹ is a bond to G¹,
X₁ and X₂ are the same or different and each independently represents O or S,
R₁ is linear C₁-C₁₇ alkyl, non-linear C₃-C₁₇ alkyl, in both cases optionally containing one S, SO, SO₂, O, or Si(R^{a})₂, wherein R^{a} is C₁-C₆ alkyl, at any position in the carbon chain with the proviso that the heteroatom is not in the alpha or omega position of the carbon chain, C₃-C₁₇ alkenyl containing one double bond with the proviso that there is at least one -CH₂- group between the double bond and X₂, or polyunsaturated C₈-C₂₀ alkenyl with the proviso that there is at least one -CH₂- group between the first double bond and X₂, or
R₁ is 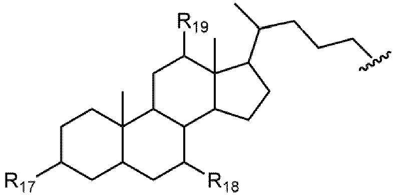 wherein R₁₇, R₁₈ and R₁₉ are the same or different and each independently can be H, OH, -C₁-C₆ alkoxy or fluorine;
R₂ is
linear C₁-C₁₇ alkyl, non-linear C₃-C₁₇ alkyl, in both cases optionally containing one S, SO, SO₂, O, or Si(R^{b})₂, wherein R^{b} is C₁-C₆ alkyl, at any position in the carbon chain with the proviso that the heteroatom is not in the alpha or omega position of the carbon chain , C₃-C₁₇ alkenyl containing one double bond with the proviso that there is at least one -CH₂- group between the double bond and the carbon linking X₁ and X₂, or polyunsaturated C₈-C₂₀ alkenyl with the proviso that there is at least one -CH₂- group between the first double bond and the carbon linking X₁ and X₂, or
R₂ is 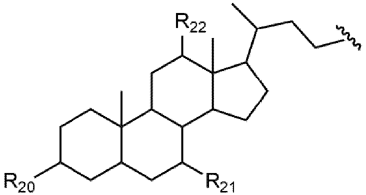 wherein R₂₀, R₂₁ and R₂₂ are the same or different and each independently can be H, OH, -C₁-C₆ alkoxy or fluorine,
with the proviso that R₁ and R₂ cannot be both at the same time, 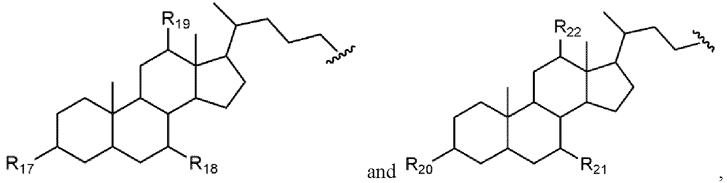 or
R₁ is connected to R₃ via alkylene or monounsaturated alkenyl forming a C₃-C₃₀-membered ring, or
R₂ is connected to R₃ via alkylene or monounsaturated alkenyl forming a C₃-C₃₀-membered ring, wherein the carbon atom of R₂ at the first or second position numbered from that carbon atom which is attached to the carbon linking X₁ and X₂, can be optionally replaced by O or S heteroatom, or
R₂ is connected to R₁ via alkylene or monounsaturated alkenyl forming a C₃-C₃₀-membered ring, wherein the carbon atom of R₂ at the first or second position numbered from that carbon atom which is attached to the carbon linking X₁ and X₂, can be optionally replaced by O or S heteroatom;
R₃ is 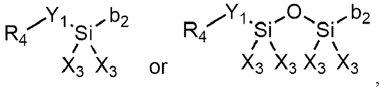
R₃-b¹ is 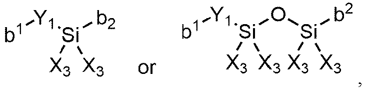
wherein
   b² is a link to X₁,
   Y₁ is -O- or -CH₂- or -O-CH₂-CH₂- wherein -CH₂- is attached to Si,
   each X₃ is independently selected from the group consisting of C₁-C₄ alkyl or C₁-C₄ alkoxy,
   R₄ is linear C₂-C₁₇ alkyl, or non-linear C₃-C₁₇ alkyl, in both cases optionally containing one S, SO, SO₂, O, or Si(R^{c})₂, wherein R^{c} is C₁-C₆ alkyl, at any position in the carbon chain with the proviso that the heteroatom is not in the alpha or omega position of the carbon chain, C₃-C₂₀ alkenyl containing one double bond with the proviso there is at least one -CH₂- group between the double bond and Y₁, or polyunsaturated C₈-C₂₀ alkenyl with the proviso there is at least one -CH₂- group between the first double bond and Y₁ or,
   R⁴ is 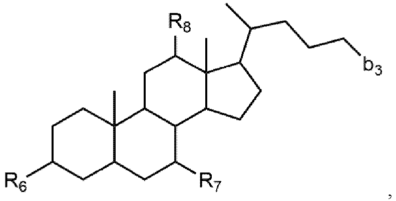
   wherein b³ is a bond to Y₁,
   R₆, R₇, R₈ are the same or different and each independently can be H, OH, -C₁-C₆ alkoxy or fluorine;
- D¹ is: selected from the group consisting of 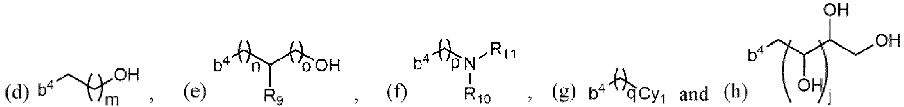 wherein
b⁴ is a bond to nitrogen,
R₉ can be C₁-C₆ alkyl, cyclopentyl, cyclohexyl, hydroxyl, hydroxymethyl, hydroxyethyl, phenyl, benzyl, 4-hydroxy benzyl,

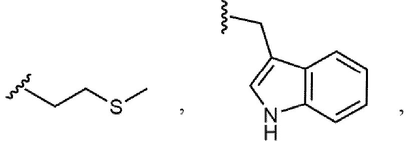

R₁₀ and R₁₁ are independently selected from H and C₁-C₆ alkyl,
m is an integer selected from 1 to 6,
n is an integer selected from 0 to 6,
o is an integer selected from 0 to 6,
p is an integer selected from 2 to 6,
q is an integer selected from 0 to 6,
j is an integer selected from 1 to 4,
Cy₁ is a C₃-C₆ cycloalkyl optionally substituted by one or more -OH, or
Cy₁ is a pyranose, furanose ring, which can be linked via any of its OH group, wherein the pyranose or furanose ring is optionally substituted by a -NH-CO-CH₃ group, adenine, guanine, uracil, cytosine, thymine, a mono- or oligosaccharide, or by a 4-, 5-, 6- or 7-membered heterocyclic ring containing 1, 2, 3 or 4 heteroatoms selected from N, O or S; or
Cy₁ is a 4-, 5-, 6- or 7-membered heterocyclic ring containing 1, 2, 3 or 4 heteroatoms selected from O, N or S, wherein the heterocyclic ring is optionally substituted by R₁₂,
wherein
   R₁₂ represents C₁-C₆ alkyl, an arginine containing peptide, a pyranose or furanose ring attached by any of their ring-carbon atoms to the 4-, 5-, 6- or 7-membered heterocyclic ring, wherein the pyranose or furanose ring is optionally substituted by a -NH-CO-CH₃ group, adenine, guanine, uracil, cytosine, thymine or a mono- or oligosaccharide,
   R₁₂ can be a group selected from (d), (e) or (f), wherein b⁴ is a bond to the heterocyclic ring, m, n, o, p, R₉, R₁₀ and R₁₁ are as defined above,
      or
   R₁₂ can be a 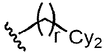 group wherein r is an integer selected from 1 to 4 and Cy₂ is a 4-, 5-, 6- or 7-membered heterocyclic ring containing 1, 2, 3 or 4 heteroatoms selected from O, N and S, optionally substituted by C₁-C₆ alkyl;
P is selected from
(i) G²-T²
   wherein
   G² is defined as G¹ above, where G¹ and G² may be identical or different;
   T² is defined as T¹ above, b¹ is a bond to G², and wherein T¹ and T² may be identical or different; or
(ii) G²-T³
   wherein
   G² is as defined above;
   T³ is 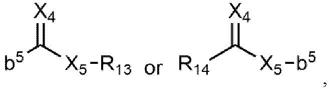
   wherein
   b⁵ is a bond to G²,
   X₄ and X₅ can be the same or different and each independently is O, S, or NR^{d} wherein R^{d} is H, C₁-C₆ alkyl, -OH or C₁-C₆ alkoxy,
   R₁₃ is linear C₂-C₁₇ alkyl, non-linear C₃-C₁₇ alkyl, both optionally containing one of S, SO, SO₂, O, Si(R^{e})₂, wherein R^{e} is C₁-C₆ alkyl at any position in the carbon chain with the proviso that the heteroatom is not in the alpha or omega position of the carbon chain, C₃-C₂₀ alkenyl containing one double bond with the proviso there is at least one -CH₂- group between the double bond and X₅ or polyunsaturated C₈-C₂₀ alkenyl with the proviso that there is at least one -CH₂-between the first double bond and X₅, or
   R₁₃ is 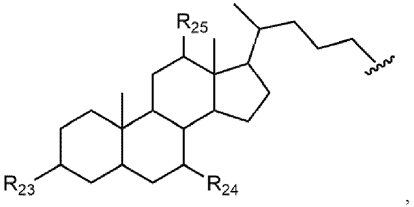 wherein R₂₃, R₂₄, R₂₅ are the same or different and each independently can be H, OH, - C₁-C₆ alkoxy or fluorine,
   R₁₄ is linear C₂-C₁₇ alkyl, non-linear C₃-C₁₇ alkyl, both optionally containing one of S, SO, SO₂, O, Si(R^{f})₂, wherein R^{f} is C₁-C₆ alkyl at any position in the carbon chain with the proviso that the heteroatom is not in the alpha or omega position of the carbon chain, C₃-C₂₀ alkenyl containing one double bond with the proviso there is at least one -CH₂- group between the double bond and the carbon linking X₄ and X₅ or polyunsaturated C₈-C₂₀ alkenyl with the proviso that there is at least one -CH₂- between the first double bond and the carbon linking X₄ and X₅; or
   R₁₄ is 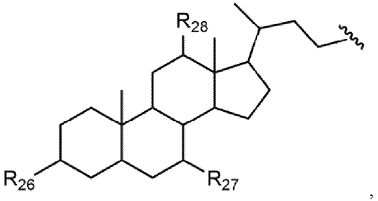 wherein R₂₆, R₂₇, R₂₈ are the same or different and each independently can be H, OH, - C₁-C₆ alkoxy or fluorine;
and (iii) T⁴, wherein
   T⁴ is 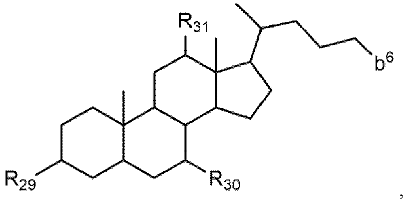 wherein, b⁶ is a bond to the nitrogen atom, R₂₉, R₃₀, R₃₁ can be the same or different and each independently can be H, OH, -C₁-C₆ alkoxy or fluorine;
   C₄-C₂₀ alkenyl containing one double bond, with the proviso that there is at least one -CH₂-between the double bond and N, or
   polyunsaturated C₈-C₂₀ alkenyl, with the proviso that there is at least one -CH₂- between the first double bond and N.

By unsubstituted C₂-C₉ alkylene we mean ethylene (-(CH₂)₂-), propylene (-(CH₂)₃-), butylene (-(CH₂)₄-), pentylene (-(CH₂)₅-), hexylene (-(CH₂)₆-), heptylene (-(CH₂)₇-), octylene (-(CH₂)₈-), or nonylene (-(CH₂)₉-).

By linear C₁-C₁₇ alkyl we mean methyl, ethyl, propyl, butyl, pentyl, hexyl, heptyl, octyl, nonyl, decyl, undecyl, dodecyl tridecyl, tetradecyl, pentadecyl, hexadecyl, or heptadecyl.

By non-linear C₃-C₁₇ alkyl we mean a saturated branched alkyl group containing 3 to 17 carbon atoms, e.g. isopropyl, isobutyl, 3-methylpentyl, 2-propylpentyl, 2-methylhexyl, 2-ethylhexyl, 2-ethyldecyl, 2-propyldecyl, 2-butyldecyl, 2-pentyldecyl, 2-hexyldecyl, or 2-heptyldecyl.

By C₁-C₆ alkyl group we mean a linear or non-linear alkyl group containing 1 to 6 carbon atoms, e.g. methyl, ethyl, propyl, butyl, pentyl, hexyl, isopropyl, isobutyl, *tert*-butyl, neopentyl, isopentyl, neohexyl or isohexyl, preferably methyl or ethyl.

By C₁-C₄ alkyl group we mean a linear or non-linear alkyl group containing 1 to 4 carbon atoms, preferably methyl.

By linear C₁-C₁₇ alkyl or non-linear C₃-C₁₇ alkyl both containing one S, SO, SO₂. O, or Si(R)₂, wherein R is C₁-C₆ alkyl as defined above, at any position in the carbon chain with the proviso that the heteroatom is not in the alpha or omega position of the carbon chain, we mean a linear C₁-C₁₇ alkyl or non-linear C₃-C₁₇ alkyl as defined above, wherein one -CH₂- of the alkyl chain, that is not in the terminal position at any side of the chain, is replaced by one of the following groups: -O- , -S-, -SO-, SO₂-, -Si(R)₂- .

By C₃-C₁₇ alkenyl containing one double bond or C₃-C₂₀ alkenyl containing one double bond we mean a linear or non-linear alkyl group as defined above containing 3 to 17 or 3 to 20 carbon atoms respectively, which contains one double bond in the chain, e.g. hex-3-en-1-yl, oct-3-en-1-yl, dec-3-en-1-yl, dec-2-en-1-yl, undec-3-en-2-yl, preferably hex-3-en-1-yl, oct-3-en-1-yl.

By C₁-C₆ alkoxy we mean -O-C₁-C₆ alkyl group wherein the C₁-C₆ alkyl group is as defined above.

By C₁-C₄ alkoxy we mean -O-C₁-C₄ alkyl group wherein the C₁-C₄ alkyl group is as defined above, like methoxy, ethoxy, propoxy, butoxy, isopropoxy, sec-butoxy or tert-butoxy.

By R₁ connected to R₃ via alkylene or monounsaturated alkenyl forming a C₃-C₃₀-membered ring we mean e.g.:

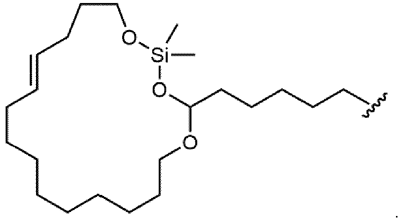

By R₂ connected to R₃ via alkylene or monounsaturated alkenyl forming a C₃-C₃₀-membered ring, wherein the carbon atom of R₂ at the first or second position numbered from that carbon atom which is attached to the carbon linking X₁ and X₂, can be optionally replaced by O or S heteroatom, we mean e.g.:

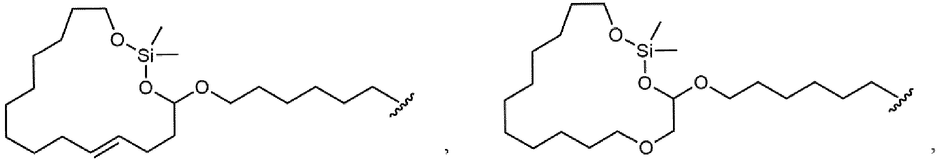

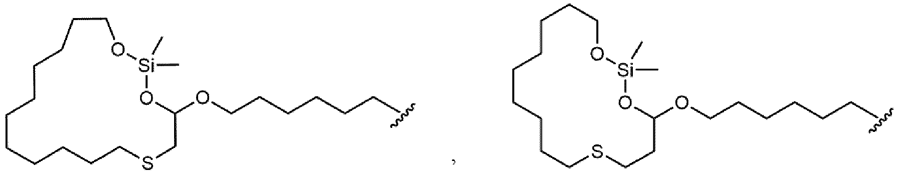

By R₂ connected to R₁ via alkylene or monounsaturated alkenyl forming a C₃-C₃₀-membered ring, wherein the carbon atom of R₂ at the first or second position numbered from that carbon atom which is attached to the carbon linking X₁ and X₂, can be optionally replaced by O or S heteroatom, we mean e.g.:

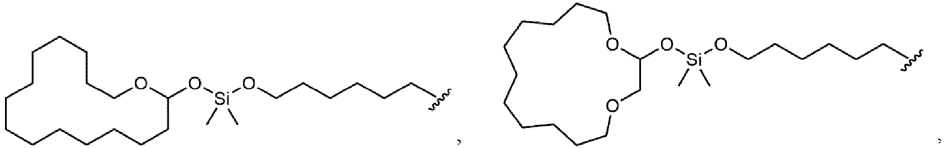

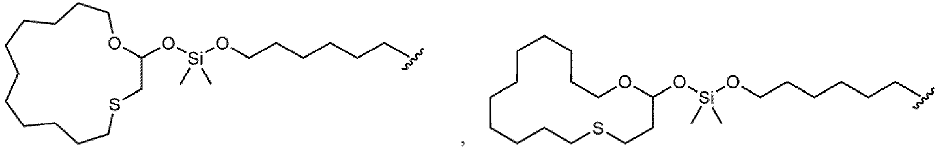

By polyunsaturated C₈-C₂₀ alkenyl we mean a linear alkyl chain containing 8 to 20 carbon atoms in which there are more than one isolated double bonds in the chain, preferably groups derived from omega-3 and omega-6 fatty acids, e.g. one of the following groups in T₄, R₁ or R₁₃: 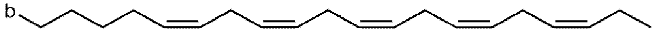 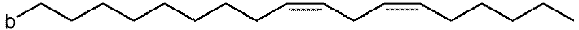 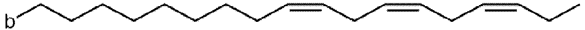 and one of the following groups in R₂ or R₁₄: 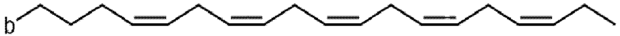 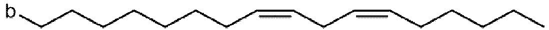 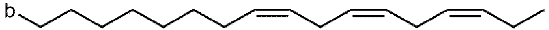 wherein the group is linked via b.

By C₃-C₆ cycloalkyl we mean, for example, cyclopropyl, cyclopentyl, or cyclohexyl.

By 4 or 5 or 6 or 7-membered heterocyclic ring containing 1, 2, 3 or 4 heteroatoms selected from N, O or S we mean an aromatic ring or an unsaturated, partly saturated or fully saturated heterocyclic ring, e.g. azetine, azetidine, pyrrolidine, oxazolidine, piperidine, piperazine, morpholine, pyrrole, imidazole, pyrazole, 1,2,3-triazole, 1,2,4-triazole, tetrazole, pyridine, pyrimidine, pyridazine, pyrazine, 1,2,4-triazine, 1,3,5-triazine, 1,2,4,5 tetrazine, oxazole, isoxazole, thiazole, isothiazole, 1,2-oxazine, 1,3-oxazine, 1,4-oxazine, 1,2-thiazine, 1,3-thiazine, 1,4-thiazine, azepane, azepine, 1,2-diazepane, 1,3-diazepane, 1,4-diazepane, 1,2-diazepine, 1,3-diazepine, 1,4-diazepine, 1,2-oxazepane, 1,3-oxazepane, 1,4-oxazepane, 1,2-oxazepine, 1,3-oxazepine, 1,4-oxazepine, 1,2-thiazepane, 1,3-thiazepane, 1,4-thiazepane, 1,2-thiazepine, 1,3-thiazepine, or 1,4-thiazepine ring, preferably pyrrolidine, imidazole, 1,2,3-triazol, piperidine, piperazine or morpholine.

By furanose we mean all stereoisomers of ribofuranose, deoxyribofuranose, xylofuranose, fructofuranose, glucofuranose, galactofuranose, mannofuranose, allofuranose, arabinofuranose, or altrofuranose which can be also and protected with e.g. acetyl, benzyl, benzoyl, isopropylidene or benzylidene groups. For example: 2,3,5,6-tetra-O-acetyl-galactofuranose.

By pyranose we mean all stereoisomers of glucopyranose, galactopyranose, mannopyranose, allopyranose, fructopyranose, arabinopyranose or altropyranose which can also be protected with e.g. acetyl, benzyl, benzoyl, isopropylidene, or benzylidene groups. For example: 2,3,4,6-tetra-O-acetyl-glucopyranose.

By mono-, oligosaccharide we mean monosaccharide as we defined above and we mean oligosaccharide e.g. sucrose, lactose, maltose, isomaltulose, cellobiose, trehalose.

By arginine containing peptide we mean peptide with 6 amino acids or less comprising at least one arginine residue. The peptide is linked by its C or N terminus.

By salts of the compounds of formula (I) we mean salts of the compounds of formula (I) with inorganic or organic acids. Preferred salts are those with pharmaceutically acceptable acids. The salts are e.g. chloride, sulfate, phosphate, formate, acetate, fumarate, maleate, oxalate, citrate or tartrate. The salts formed during purification or isolation are also subject of the invention.

By stereoisomers we mean optical and geometric isomers. The compounds of formula (I) may contain one or more asymmetric carbon atoms thus they can exist in the form of optical isomers, enantiomers or diastereomers. The compounds of formula (I) may contain double bounds and the groups attached to the double bond can have different (cis or trans) confirmations, e.g. cis or trans fatty acid moieties.

A specific group of compounds of formula (I) is wherein
G' is unsubstituted C₂-C₉ alkylene,
   - (CH₂)ₓ-CH=CH-(CH₂) _{y}-, wherein x is an integer selected from 1 to 6, y is an integer selected from 1 to 6, and the sum of x+y is an integer selected from 2 to 7, or
   - (CH₂)_{w}-X-(CH₂) _{z}-, wherein w is an integer selected from 1 to 7, z is an integer selected from 2 to 7, the sum of w+z is an integer selected from 3 to 8, wherein-(CH₂)_{z}- is attached to N, and X is selected from O, S, SO and SO₂;
T¹ is 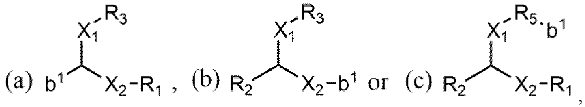 wherein
   b¹ is a bond to G¹,
   X₁ and X₂ are the same or different and each independently represents O or S,
   R₁ is linear C₂-C₁₇ alkyl, non-linear C₃-C₁₇ alkyl, in both cases optionally containing one S, SO, SO₂, O, or Si(R^{a})₂, wherein R^{a} is C₁-C₆ alkyl, at any position in the carbon chain with the proviso that the heteroatom is not in the alpha or omega position of the carbon chain, C₃-C₁₇ alkenyl containing one double bond with the proviso that there is at least one -CH₂- group between the double bond and X₂, or polyunsaturated C₈-C₂₀ alkenyl with the proviso that there is at least one -CH₂- group between the first double bond and X₂,
   R₂ is linear C₂-C₁₇ alkyl, non-linear C₃-C₁₇ alkyl, in both cases optionally containing one S, SO, SO₂, O, or Si(R^{b})₂, wherein R^{b} is C₁-C₆ alkyl, at any position in the carbon chain with the proviso that the heteroatom is not in the alpha or omega position of the carbon chain, C₃-C₁₇ alkenyl containing one double bond with the proviso that there is at least one -CH₂- group between the double bond and the carbon linking X₁ and X₂, or polyunsaturated C₈-C₂₀ alkenyl with the proviso that there is at least one -CH₂- group between the first double bond and the carbon linking X₁ and X₂,
   R₃ is 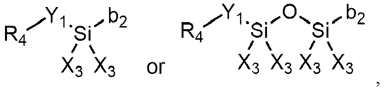
   R₃-b¹ is 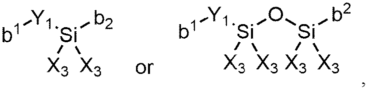 wherein
      b² is a link to X₁,
      Y₁ is -O- or -CH₂- or -O-CH₂-CH₂- wherein -CH₂- is attached to Si, each X₃ is independently selected from the group consisting of C₁-C₄ alkyl or C₁-C₄ alkoxy,
      R₄ is linear C₂-C₁₇ alkyl, or non-linear C₃-C₁₇ alkyl, in both cases optionally containing one S, SO, SO₂, O, or Si(R^{c})₂, wherein R^{c} is C₁-C₆ alkyl, at any position in the carbon chain with the proviso that the heteroatom is not in the alpha or omega position of the carbon chain, C₃-C₂₀ alkenyl containing one double bond with the proviso there is at least one -CH₂- group between the double bond and Y₁, or polyunsaturated C₈-C₂₀ alkenyl with the proviso there is at least one -CH₂- group between the first double bond and Y_{1 ,} or,
      R⁴ is 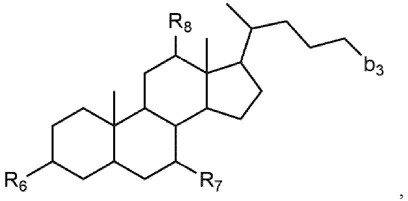 wherein b³ is a bond to Y₁, R₆, R₇, R₈ can be the same or different and each independently can be H, OH, -C₁-C₆ alkoxy or fluorine;
D¹ is selected from the group consisting of 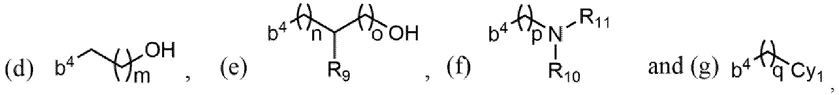
wherein
   b⁴ is a bond to nitrogen,
   R₉ can be C₁-C₆ alkyl, hydroxy, hydroxymethyl,
   m is an integer selected from 1 to 6,
   n is an integer selected from 0 to 6,
   o is an integer selected from 0 to 6,
   p is an integer selected from 2 to 6,
   R₁₀ and R₁₁ are independently selected from H and C₁-C₆ alkyl,
   q is an integer selected from 0 to 6,
   Cy₁ is a C₃-C₆ cycloalkyl optionally substituted by one or more -OH, or
   Cy₁ is a pyranose, furanose ring, which can be linked via any of its OH group, wherein the pyranose or furanose ring is optionally substituted by a -NH-CO-CH₃ group, adenine, guanine, uracil, cytosine, thymine, a mono- or oligosaccharide, or by a 4-, 5-, 6- or 7-membered heterocyclic ring containing 1, 2, 3 or 4 heteroatoms selected from N, O or S, or Cy₁ is a 4-, 5-, 6- or 7-membered heterocyclic ring containing 1, 2, 3 or 4 heteroatoms selected from O, N or S, wherein the heterocyclic ring is optionally substituted by R¹²,
   wherein
      R₁₂ represents C₁-C₆ alkyl, an arginine containing peptide, a pyranose or furanose ring attached by any of their ring-carbon atoms to the 4-, 5-, 6- or 7-membered heterocyclic ring, wherein the pyranose or furanose ring is optionally substituted by a -NH-CO-CH₃ group, adenine, guanine, uracil, cytosine, thymine or a mono- or oligosaccharide,
      R₁₂ can be a group selected from (d), (e) or (f), wherein b⁴ is a bond to the heterocyclic ring, m, n, o, p, R₉, R₁₀ and R₁₁ are as defined above,
         or
      R₁₂ can be a 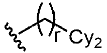 group wherein r is an integer selected from 1 to 4 and Cy₂ is a 4-, 5-, 6- or 7-membered heterocyclic ring containing 1, 2, 3 or 4 heteroatoms selected from O, N and S, optionally substituted by C₁-C₆ alkyl;
P is selected from
   (i) G²-T², wherein
      G² is defined as G¹ above, where G¹ and G² may be identical or different;
      T² is defined as T¹ above b¹ is a bond to G², and wherein T¹ and T² may be identical or different; or
   (ii) G²-T³, wherein
      G² is as defined above;
      T³ is 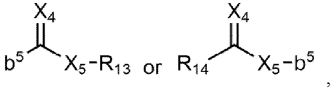 wherein
      b⁵ is a bond to G²,
      X₄ and X₅ can be the same or different and each independently is O, S, or NR^{d} wherein R^{d} is H, C₁-C₆ alkyl, -OH or C₁-C₆ alkoxy,
      R₁₃ is linear C₂-C₁₇ alkyl, non-linear C₃-C₁₇ alkyl, both optionally containing one of S, SO, SO₂, O, Si(R^{e})₂ -wherein R^{e} is C₁-C₆ alkyl- at any position in the carbon chain with the proviso that the heteroatom is not in the alpha or omega position of the carbon chain, C₃-C₂₀ alkenyl containing one double bond with the proviso there is at least one -CH₂- group between the double bond and X₅ or polyunsaturated C₈-C₂₀ alkenyl with the proviso that there is at least one -CH₂-between the first double bond and X₅;
      R₁₄ is linear C₂-C₁₇ alkyl, non-linear C₃-C₁₇ alkyl, both optionally containing one of S, SO, SO₂, O, Si(R^{f})₂ -wherein R^{f} is C₁-C₆ alkyl- at any position in the carbon chain with the proviso that the heteroatom is not in the alpha or omega position of the carbon chain, C₃-C₂₀ alkenyl containing one double bond with the proviso there is at least one -CH₂- group between the double bond and the carbon linking X₄ and X₅ or polyunsaturated C₈-C₂₀ alkenyl with the proviso that there is at least one -CH₂- between the first double bond and the carbon linking X₄ and X₅;
      and
   (iii) T⁴, wherein
      T⁴ is 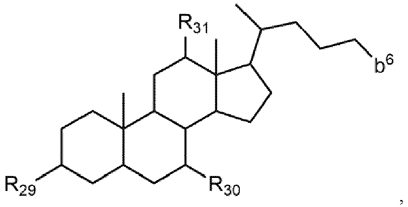 wherein, b⁶ is a bond to the nitrogen atom, R₂₉, R₃₀, R₃₁ can be the same or different and each independently can be H, OH, -C₁-C₆ alkoxy or fluorine; or
      C₄-C₂₀ alkenyl containing one double bond, with the proviso that there is at least one -CH₂-between the double bond and N, or
      polyunsaturated C₈-C₂₀ alkenyl, with the proviso that there is at least one -CH₂- between the first double bond and N.

A narrower group of compounds of formula (I) is, wherein G¹ and, if present, G² are the same or different and each is independently linear C₄-C₉ alkylene, preferably C₅-C₉ alkylene, or, alternatively, each is independently linear C₅, C₆, C₇, or C₉ alkylene.

Another group of compounds of formula (I) is, wherein R₁ is linear C₁, C₂, C₄, C₆, C₇, C₈, C₉, C₁₀, or C₁₂ alkyl, or R₁ is linear C₉ alkenyl, and/or R₂ is linear C₁, C₃, C₅, C₆, C₇, C₈, C₉, or C₁₁ alkyl, preferably R₁ is linear C₂, C₈, C₁₀ alkyl, or R₁ is linear C₉ alkenyl and/or R₂ is linear C₁, C₃, C₇,or C₉ alkyl.

A specific embodiment of this group is wherein T¹ is (c) and R₁ is linear C₂, C₄, C₆, C₇, C₈, C₉, C₁₀, or C₁₂ alkyl, or R₁ is linear C₉ alkenyl and R₂ is linear C₁, C₃, C₅, C₆, C₇, C₈, C₉, or C₁₁ alkyl, preferably R₁ is linear C₂, C₈, C₁₀ alkyl or R₁ is linear C₉ alkenyl, and R₂ is linear C₁, C₃, C₇, or C₉ alkyl.

Another group of compounds of formula (I) is, wherein R₄ is linear C₅, C₇, C₈ alkyl, or R₄ is non-linear C₁₆ alkyl.

A specific embodiment of this group is wherein T¹ is (b).

A further group of compounds of formula (I) is, wherein P is G²-T².

In a particular embodiment of that group, G¹-T¹ and G²-T² are identical.

Another group of compounds of formula (I) is, wherein P is G²-T³.

In a particular embodiment of that group, T¹-G¹ has one of the following structures

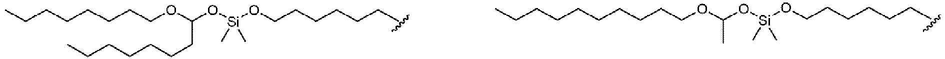

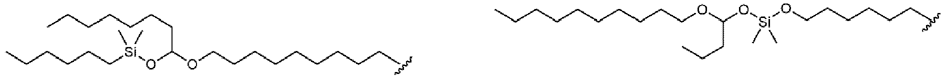

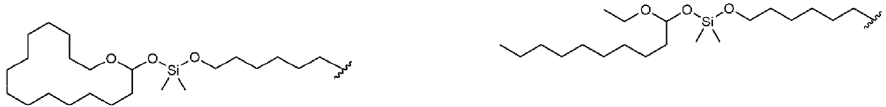

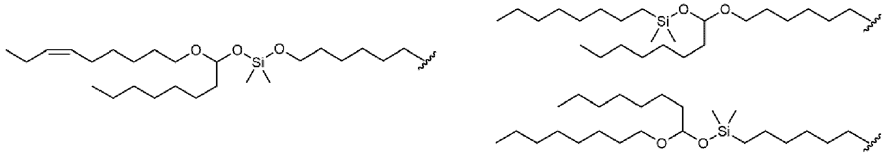

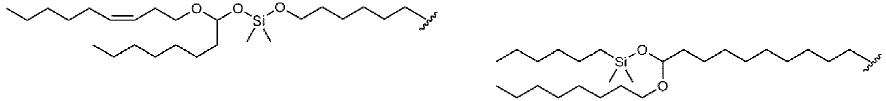

A narrower group of compounds of formula (I) is, wherein T³ has one of the following structures 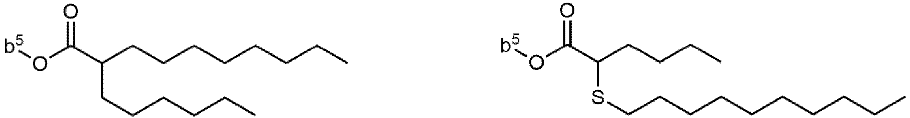 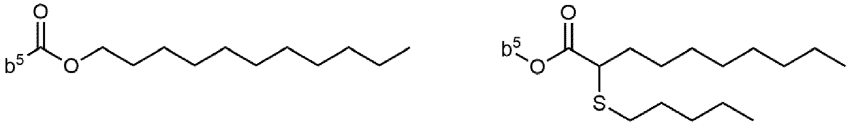 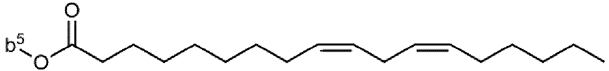 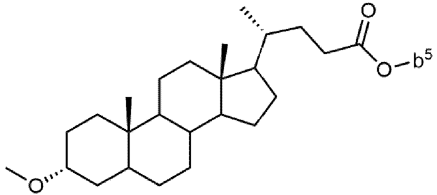 wherein b⁵ is the bond to G².

Another group of compounds of formula (I) is wherein P is T⁴.

In a particular embodiment of that group, T⁴ is 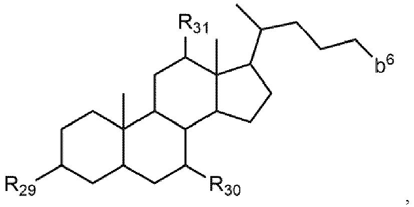 wherein, b⁶ is a bond to the nitrogen atom, R₂₉, R₃₀, R₃₁ can be the same or different and each independently can be H, OH, -C₁-C₆ alkoxy or fluorine;
or
polyunsaturated C₈-C₂₀ alkenyl, with the proviso that there is at least one -CH₂- between the first double bond and N.

In a further particular embodiment of that group T⁴ has the following structure 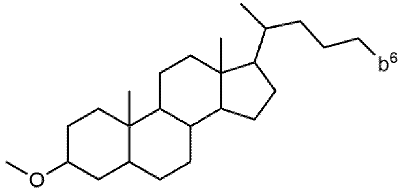 wherein b⁶ is a bond to the nitrogen atom.

Another group of the compounds of formula (I) is D¹ has one of the following structures:

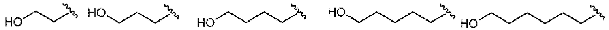

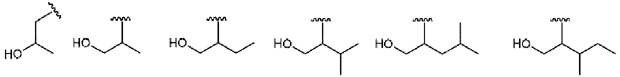

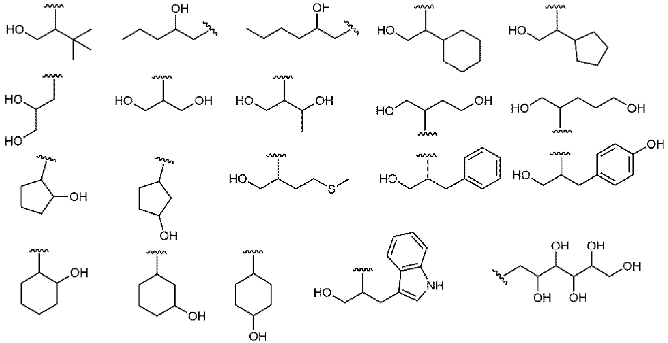

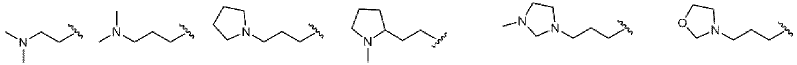

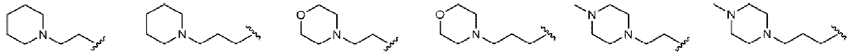

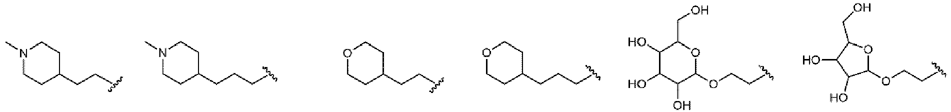

Another group of the compounds of formula (I) is wherein D¹ has one of the following structures: 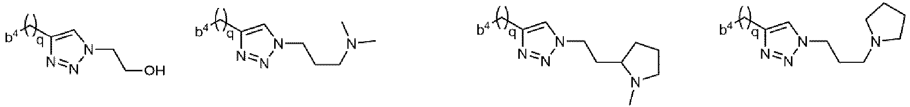 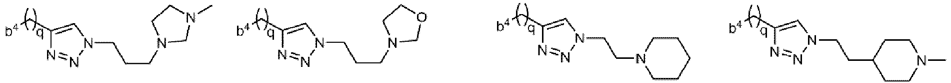 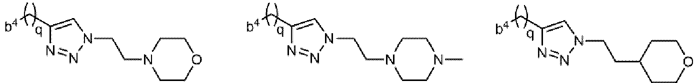 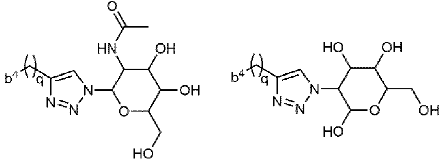 wherein q is an integer selected from 1 to 4 and b⁴ is a bond to the nitrogen.

In some embodiments D¹ has one of the following structures:

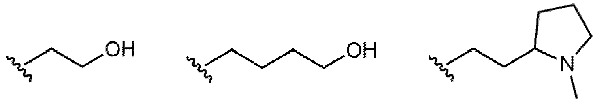

The invention also provides a lipid nanoparticle (LNP) comprising compound of formula (I) as defined above and a therapeutic agent (e.g. RNA, DNA, mRNA, siRNA, pDNA, circular DNA, or small biologically active molecules).

In a particular embodiment the invention provides a lipid nanoparticle comprising compound formula (I) and mRNA (e.g. Luciferase or green fluorescence protein (GFP)).

The invention also provides a method for delivering mRNA into a cell.

The present invention opens a new chemical space via silicon containing linkers that results in novel biodegradable lipids of formula (I). The mentioned linkers are very difficult to synthesize with the traditional chemical toolbox, but recently published borane catalysts [WO2022/129966] enabled their synthetic feasibility. The mixed acetal building blocks of the present invention were prepared via similar way as described in WO2022/129966. These novel mixed acetals are structurally different from that ones are described in WO2022/129966, moreover further difference is that the mixed acetal moiety is retained and incorporated into the lipids according to the present invention.

Another significant feature of present invention is the modularity. Thanks to the nature of the designed silicon-based linker a large number of compounds and diverse lipid library can be designed and synthesized from simple and readily available starting materials.

Additionally, the chemical stability (e.g. sensitivity to hydrolysis) of the silicon containing ionizable lipids can be fine-tuned with the chemical properties of the substituents and with the number of O and Si atoms on the mixed silyl acetal group (linker), which is manifested in the biodegradability of the lipids.

Another aspect of the invention is the preparation of compounds of formula (I) and the intermediates and building blocks used therein.

The compounds of the formula (I) can be prepared according to the following reaction schemes.

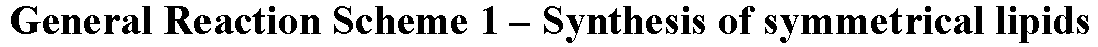

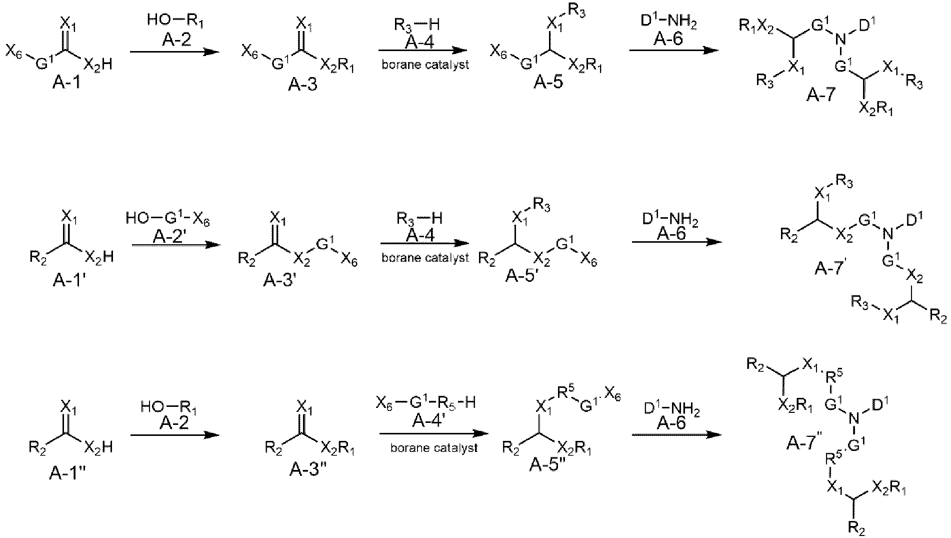

**General Reaction Scheme 1** illustrates methods to make symmetrical lipids of this invention having the structure of formula (I) wherein T¹-G¹ and P are identical, X₆ is halogen or pseudohalogen, G¹, X₁, X₂, R₁, R₂, R₃, D¹ are as defined above. Compounds of structure A-1, A-1', A-1", A-2, A-2', A-3, A-3', A-3" and A-4, A-4' and A-6 can be purchased or prepared according to methods known in the art. Reaction of carboxylic acid derivative A-1 or A-1' or A-1" with alcohol derivative A-2 or A-2' under appropriate esterification conditions (e.g. oxalyl chloride, Et₃N) yields ester A-3 or A-3' or A-3" which can be transformed to mixed silyl acetal A-5 or A-5' or A-5" with appropriate silane derivative A-4 or A-4' and borane catalyst [borane catalysts are disclosed in WO2022/129966]. The resulting mixed silyl acetal A-5 or A-5' or A-5" which correspond to the formula (II) that contains halogen or pseudohalogen atom is reacted with the amine derivative A-6 under appropriate alkylation conditions (e.g. KI, K₂CO₃ in CPME/MeCN) to yield a compound of A-7 or A-7' or A-7" respectively.

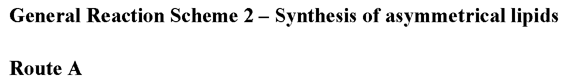

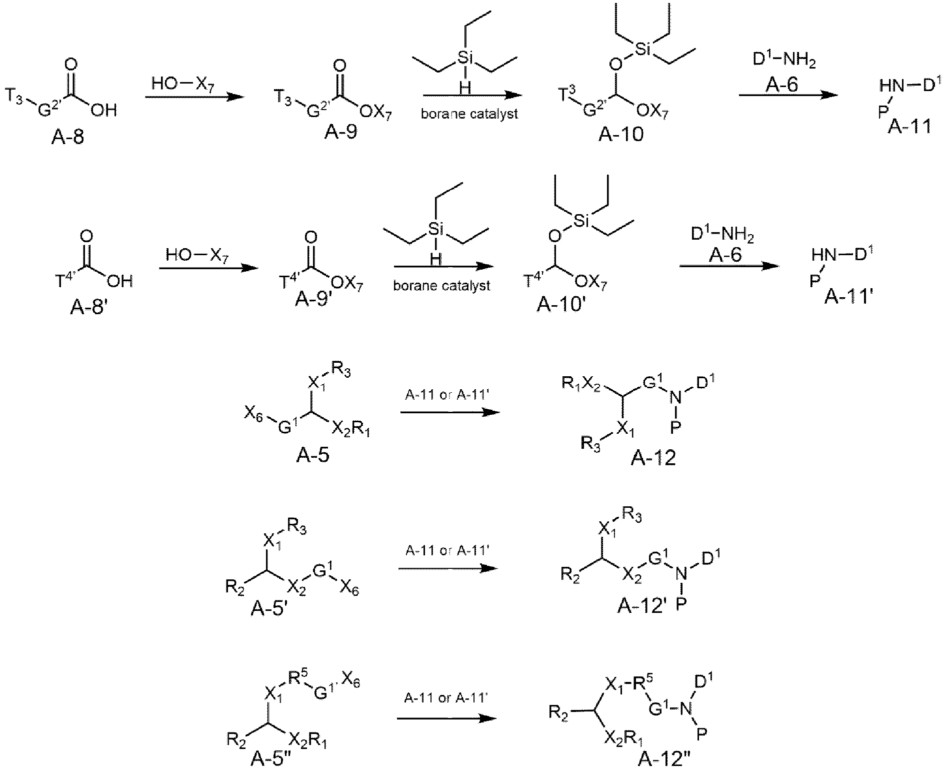

**Route A of General Reaction Scheme 2** illustrates methods to make asymmetrical lipids of this invention having the structure of formula (I) wherein T¹-G¹ and P are different and P is G²-T³ or T⁴, X₆ is halogen or pseudohalogen, G¹, X₁, X₂, R₁, R₂, R₃ , D¹ are as defined above. Compounds of structure A-8, A-8' and A-6 can be purchased or prepared according to methods known in the art. Reaction of carboxylic acid derivative A-8 or A-8' with methanol or ethanol (HO-X₇) under appropriate esterification conditions (e.g. oxalyl chloride, Et₃N) yields ester A-9 or A-9' which can be transformed to mixed silyl acetal A-10 or A-10' with triethyl silane and the appropriate borane catalyst [borane catalysts are disclosed in WO2022/129966]. The resulting mixed silyl acetal A-10 or A-10' is transformed to aldehyde (in advance or in situ) that is reacted with appropriate amine derivative A-6 under reductive amination conditions (e.g. NaHB(OAc)₃, AcOH) to yield a compound A-11 or A-11' as an intermediate. Reaction of intermediate A-11 or A-11' and mixed silyl acetal A-5 or A-5' or A-5" (compound of formula II) under appropriate alkylation conditions (e.g. KI, K₂CO₃ in CPME/MeCN) yields a compound of A-12 or A-12' or A-12".

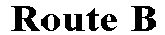

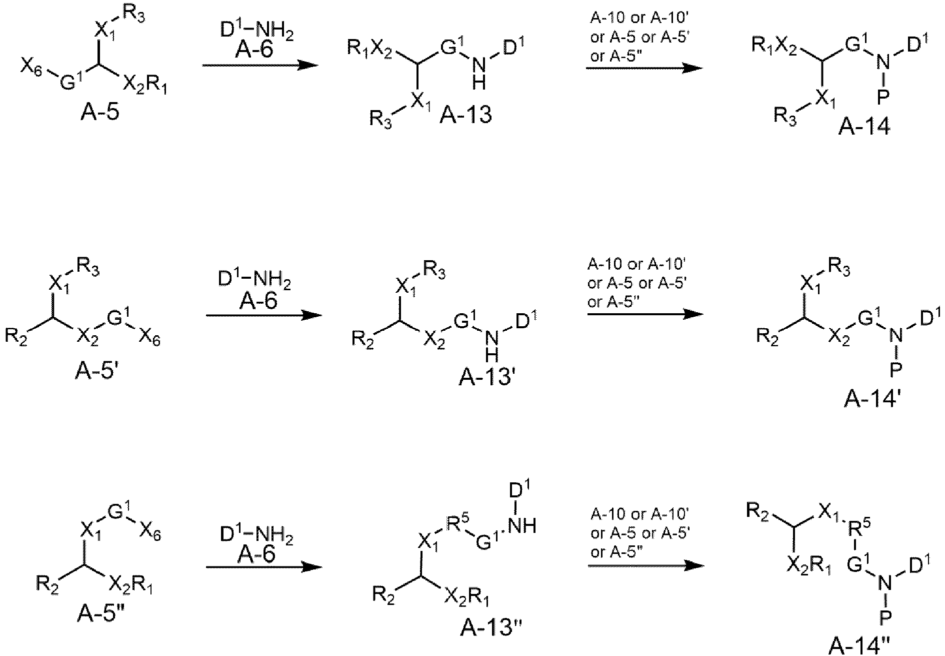

**Route B of General Reaction Scheme 2** illustrates methods to make asymmetrical lipids of this invention having the structure of formula (I) wherein T¹-G¹ and P are different and P is G²-T², G²-T³ or T⁴, X₆ is halogen or pseudohalogen, G¹, X₁, X₂, R₁, R₂, R₃ , D¹ are as defined above. Reaction of silyl acetal A-5 or A-5' or A-5" corresponding to formula (II) containing halogen or pseudohalogen is reacted with the amine derivative A-6 under appropriate alkylation conditions (e.g. KI, K₂CO₃ in CPME/MeCN) to yield A-13 or A-13' or A-13" as an intermediate having the structure of formula (Ia). The resulting intermediate A-13 or A-13' or A-13" is reacted with A-10 or A-10', which is transformed to aldehyde (in advance or in situ) under appropriate reductive amination conditions to yield A-14 or A-14' or A-14" or in other case A-13 or A-13' or A-13" is reacted with silyl acetal having halogen or pseudohalogen A-5 or A-5' or A5" to yield A-14 or A-14' or A-14".

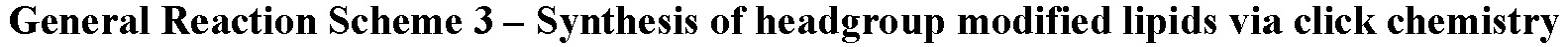

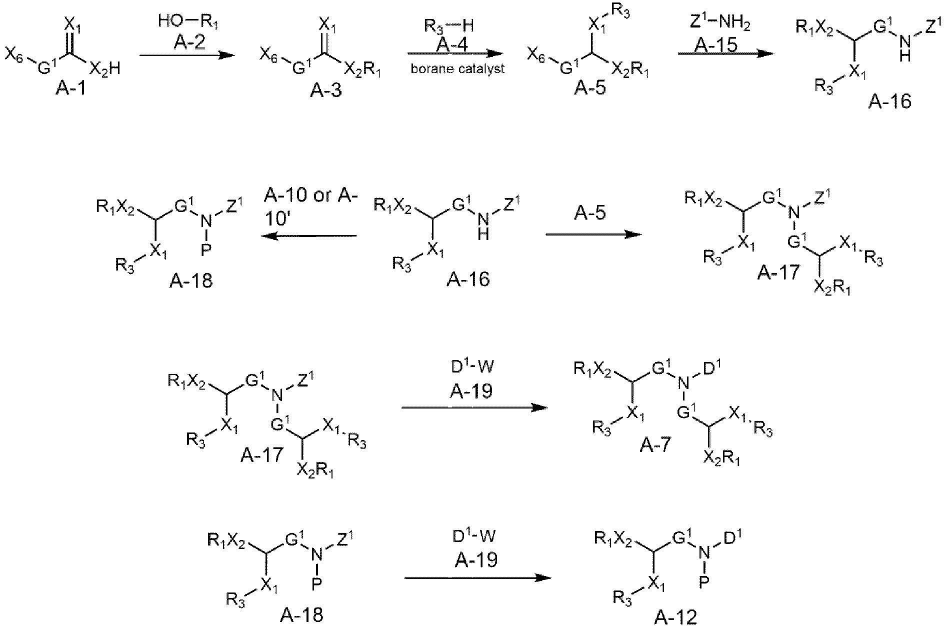

**General Reaction Scheme 3** illustrates methods to make headgroup modified lipids via click chemistry of this invention having the structure of formula (I) wherein T¹-G¹ and P are identical or different, X₆ is halogen or pseudohalogen, W is N₃ or ethynyl depending on the meaning of Z¹ and G¹, X₁, X₂, R₁, R₂, R₃ , D¹ Z ¹ are as defined above. Only one case is demonstrated in Scheme 3 but the other cases similarly can be derived based on General Reaction Scheme 1 and 2. Compounds of structure A-1, A-2, A-3, A-4, A-15 and A-19 can be purchased or prepared according to methods known in the art. Reaction of carboxylic acid derivative A-1 with alcohol derivative A-2 under appropriate esterification conditions (e.g. oxalyl chloride, Et₃N) yields ester A-3 which can be transformed to mixed silyl acetal A-5 with appropriate silane derivative A-4 and borane catalyst [borane catalysts are disclosed in WO2022/129966]. The resulting mixed silyl acetal A-5 that contains halogen or pseudohalogen is reacted with the amine derivative A-15 under appropriate alkylation conditions (e.g. KI, K₂CO₃ in CPME/MeCN) to yield a compound A-16 a compound of formula (Ib)).

From the resulting intermediate A-16 the compound A-17 (formula (Ic)) can be synthesized with further alkylation as described above or the compound A-18 (formula (Ic) ) can be synthesized under appropriate reductive amination conditions (e.g. NaHB(OAc)₃, AcOH). Reaction of A-17 or A-18 with A-19 under appropriate click chemistry condition (e.g. azidosugar, CuBr, PMDT in DMF) yields a compound of A-7 or A-12 wherein D¹ is (g) and Cy₁ is an optionally substituted 1,2,3-triazole ring.

### Brief Description of the Drawings

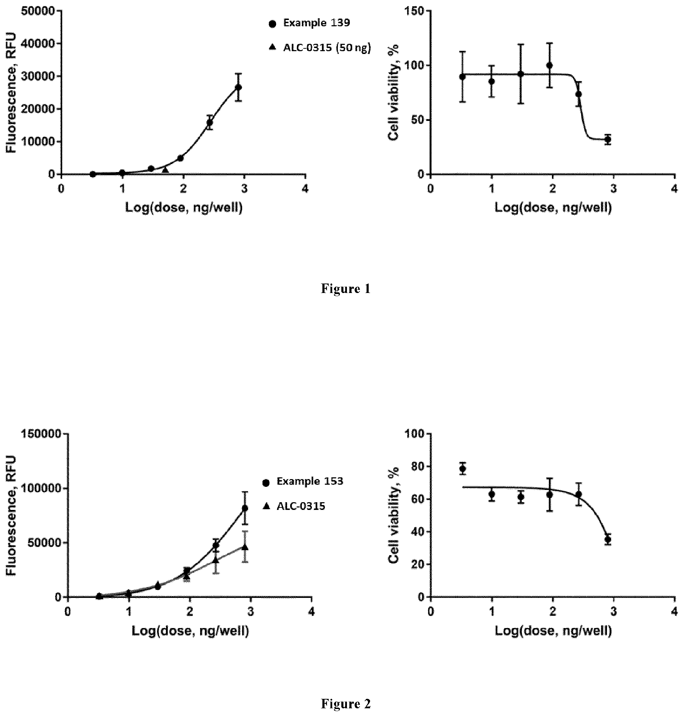

**Figure 1****:** Transfection efficacy and toxicity of **Example 139** at different doses of GFP mRNA (3.3-800 ng mRNA/well): (experiments based on Method B)
**Figure 2****:** Transfection efficacy and toxicity of **Example 153** at different doses of GFP mRNA (3.3-800 ng mRNA/well): (experiments based on Method B)

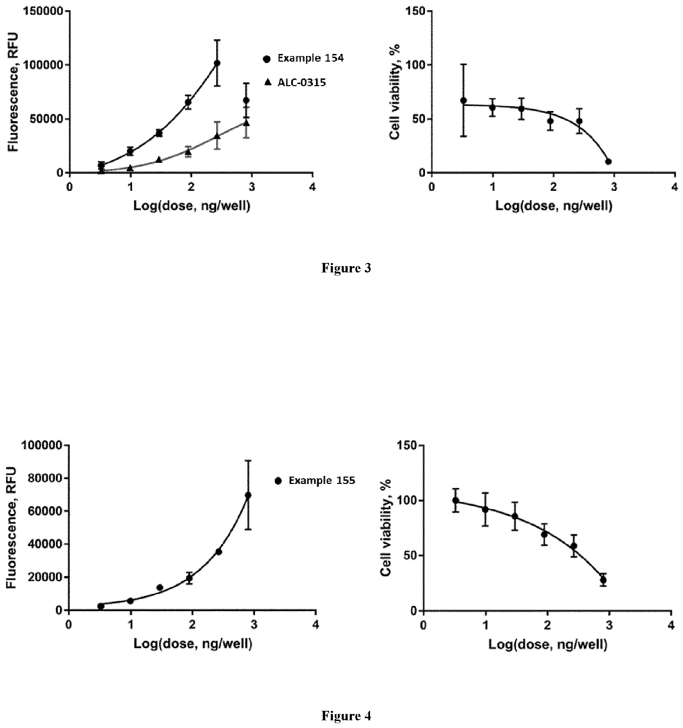

**Figure 3****:** Transfection efficacy and toxicity of **Example 154** at different doses of GFP mRNA (3.3-800 ng mRNA/well): (experiments based on Method B)
**Figure 4****:** Transfection efficacy and toxicity of **Example 155** at different doses of GFP mRNA (3.3-800 ng mRNA/well): (experiments based on Method B)

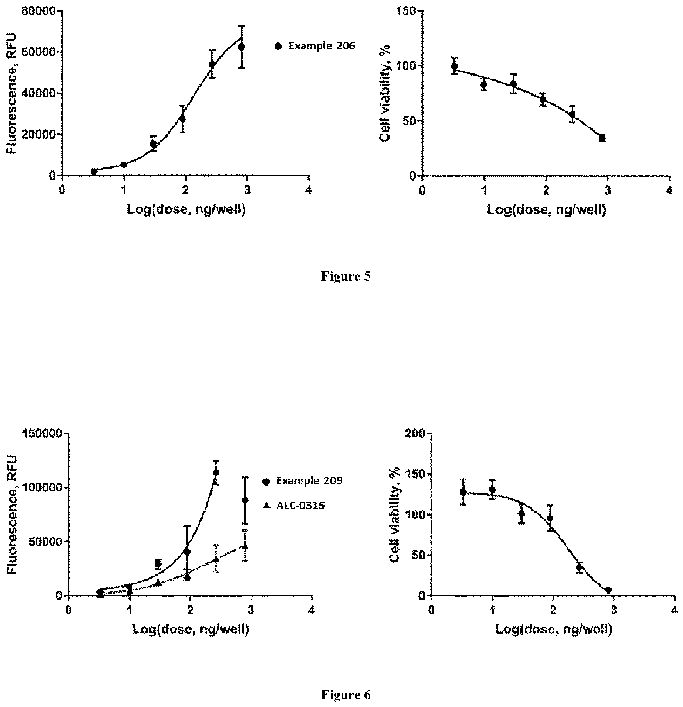

**Figure 5****:** Transfection efficacy and toxicity of **Example 206** at different doses of GFP mRNA (3.3-800 ng mRNA/well): (experiments based on Method B)
**Figure 6****:** Transfection efficacy and toxicity of **Example 209** at different doses of GFP mRNA (3.3-800 ng mRNA/well): (experiments based on Method B)

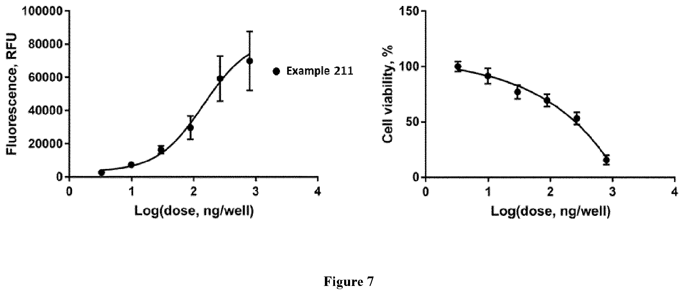

**Figure 7****:** Transfection efficacy and toxicity of **Example 211** at different doses of GFP mRNA (3.3-800 ng mRNA/well): (experiments based on Method B)

### EXAMPLES

The invention is illustrated by the following examples. These examples illustrate, but do not limit the invention. For all of the examples, standard work-up and purification methods known to those skilled in the art can be utilized.

### SYNTHESIS

### Ester Synthesis (general procedure A-D)

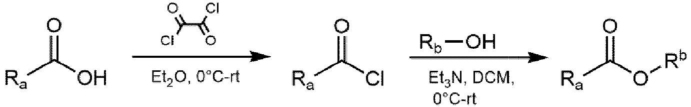

### General Procedure A

A three neck round bottom flask was equipped with dropping funnel, drying tube (with CaCl₂) and thermometer and charged with 1 eq. acid and diethyl ether (concentration: 1.0 mmol acid in 0.8 mL diethyl ether). The solution in the flask was cooled down to 0°C and 1.10 eq. oxalyl chloride was added dropwise. The reaction mixture was left to warm to room temperature and stirred for overnight. After this time, it was concentrated under reduced pressure to give an oil which was used in the next without further purification.

A three neck round bottom flask was equipped with dropping funnel, drying tube (with CaCl₂) and thermometer and charged with 1.0 eq. alcohol, 1.0 eq. triethylamine and dichloromethane (concentration: 1.0 mmol alcohol in 4.0 ml dichloromethane). The mixture in the flask was cooled down to 0°C and the dichloromethane solution of 1.0 eq. acyl chloride (concentration: 1.0 mmol acyl chloride in 1.0 ml dichloromethane) was added dropwise. The reaction mixture was left to warm to room temperature and stirred for overnight. The reaction was monitored by TLC (hexane/ethyl acetate 4/1, PMA visualization). After overnight the volatiles were removed under reduced pressure and filtered the triethylamine hydrochloride salt. The filtrate was evaporated onto silica gel under reduced pressure and it was purified by flash column chromatography.

### General Procedure B

A three neck round bottom flask was equipped with dropping funnel, drying tube (with CaCl₂) and thermometer and charged with 1.0 eq. acid and diethyl ether (concentration: 1.0 mmol acid in 0.8 mL diethyl ether). The solution in the flask was cooled down to 0°C and 1.1 eq. oxalyl chloride was added dropwise. The reaction mixture was left to warm to room temperature and stirred for overnight. After this time, it was concentrated under reduced pressure to give an oil which was used in next without further purification.

A three neck round bottom flask was equipped with dropping funnel, drying tube (with CaCl₂) and thermometer and charged with 1.0 eq. alcohol, 1.0 eq. triethylamine and dichloromethane (concentration: 1.0 mmol alcohol in 4 ml dichloromethane). The mixture in the flask was cooled down to 0°C and the dichloromethane solution of 1.0 eq. acyl chloride (concentration: 1.0 mmol acyl chloride in 1.0 ml dichloromethane) was added dropwise. The reaction mixture was left to warm to room temperature and stirred for overnight. The reaction was monitored by TLC (hexane/ethyl acetate 4/1, PMA visualization). After overnight the mixture was concentrated to half and the precipitated triethylamine hydrochloride salt was filtered off. The salt was washed with 20 ml of dichloromethane and 50 ml of hexane. The filtrate was evaporated. The residue was taken up in 150 ml hexane and 30 g silica gel was added and stirred for 10 minutes and filtered. The silica gel was rinsed with 2 × 100 ml hexane. The filtrate was concentrated under reduced pressure to give the product as an oil.

### General Procedure C

A three neck round bottom flask was equipped with dropping funnel, drying tube (with CaCl₂) and thermometer and charged with 1.25 eq. oxalyl chloride and abs. dichloromethane (concentration: 1.0 mmol oxalyl chloride in 0.41 mL abs. dichloromethane). The solution in the flask was cooled down to 0°C and 1.0 eq. acid was added dropwise. The reaction mixture was left to warm to room temperature and stirred for overnight. After this time, it was concentrated under reduced pressure to give an oil which was used in the ester synthesis without further purification.

A three neck round bottom flask was equipped with dropping funnel, drying tube (with CaCl₂) and thermometer and charged with 1.0 eq. alcohol, 1.0 eq. triethylamine and dichloromethane (concentration: 1.0 mmol alcohol in 4 ml dichloromethane). The mixture in the flask was cooled down to 0°C and the dichloromethane solution of 1.0 eq. acyl chloride (concentration: 1.0 mmol acyl chloride in 1.0 ml dichloromethane) was added dropwise. The reaction mixture was left to warm to room temperature and stirred for overnight. The reaction was monitored by TLC (hexane/ethyl acetate 9/1). After overnight the volatiles were removed under reduced pressure and filtered the triethylamine hydrochloride salt. The filtrate was evaporated onto silica gel under reduced pressure, and it was purified by flash column chromatography.

### General Procedure D

A three neck round bottom flask was equipped with dropping funnel, drying tube (with CaCl₂) and thermometer and charged with carboxylic acid (1.0 eq.), alcohol (1.0 eq.), DMAP (0.5 eq.) and abs. dichloromethane (concentration: 1.0 mmol carboxylic acid in 8.5 mL abs. dichloromethane). The solution in the flask was cooled down to 0°C and 2.5 eq. DCC in dichloromethane (concentration: 1.0 mmol DCC in 0.45 mL abs. dichloromethane) was added dropwise. After that, the reaction mixture was stirred for further 1 h at 0°C and then left to stir overnight at room temperature. Then reaction mixture was diluted with petroleum ether and filtrated through a celite pad. Celite was washed with petroleum ether and the filtrate was combined and concentrated under reduced pressure. The crude material was purified by flash column chromatography (0-15 % dichloromethane in petroleum ether).

### Examples of ester synthesis

### Example 1

### Synthesis of octyl 6-bromohexanoate

Octyl 6-bromohexanoate was prepared from 6-bromohexanoic acid and 1-octanol according to general procedure **A** and purified based on the following method: 250 g silica column, eluent: hexane/ethyl acetate 4/1. The product was obtained as a yellowish oil: 27.7 g, 90.15 mmol, 87 % yield.

¹H NMR (500 MHz, CDCl₃) δ 4.08 (t, 2H), δ 3.42 (t, 2H), δ 2.33 (t, 2H), δ 1.89 (p, 2H), δ 1.70-1.60 (m, 4H), δ 1.55-1.49 (m, 2H), δ 1.45-1.21 (m, 10H), δ 0.90 (t, 3H)

### Example 2

### Synthesis of decyl 6-bromohexanoate

Decyl 6-bromohexanoate was prepared from 6-bromohexanoic acid and 1-decanol according to general procedure **A** and purified based on the following method: 750 g silica column, eluent: 0-20% ethyl acetate in hexane. The product was obtained as a yellowish oil: 35.0 g, 104.37 mmol, 89 % yield.

¹H NMR (500 MHz, Benzene-d₆) δ 4.03 (t, 2H), δ 2.86 (t, 2H), δ 2.02 (t, 2H), δ 1.48 (p, 2H), δ 1.41-1.34 (m, 4H), δ 1.32-1.14 (m, 14H), δ 1.09 (m, 2H), δ 0.90 (t, 3H)

### Example 3

### Synthesis of octyl 10-bromodecanoate

Octyl 10-bromodecanoate was prepared from 10-bromodecanoic acid and 1-octanol according to general procedure **A** and purified based on the following method: 250 g silica column, eluent: 0-20% ethyl acetate in hexane. The product was obtained as a yellowish oil: 20.23 g, 55.67 mmol, 70 % yield.

¹H NMR (500 MHz, Benzene-d₆) δ 4.06 (t, 2H), δ 2.95 (t, 2H), δ 2.19 (t, 2H), δ 1.59 (p, 2H), δ 1.53-1.43 (m, 4H), δ 1.31-0.94 (m, 20H), δ 0.88 (t, 3H)

### Example 4

### Synthesis of 6-bromohexyl octanoate

6-bromohexyl octanoate was prepared from octanoic acid and 6-bromo-1-hexanol according to general procedure **B.** The product was obtained as a yellowish oil: 46.0 g, 149.70 mmol, 90 % yield.

¹H NMR (500 MHz, Benzene-d₆) δ 3.95 (t, 2H), δ 2.89 (t, 2H), δ 2.17 (t, 2H), δ 1.59 (p, 2H), δ 1.40 (p, 2H), δ 1.32 (p, 2H), δ 1.27-1.10 (m, 8H), δ 1.01 (m, 4H), δ 0.85 (t, 3H)

### Example 5

### Synthesis of 6-bromohexyl decanoate

6-bromohexyl decanoate was prepared from decanoic acid and 6-bromo-1-hexanol according to general procedure **B.** The product was obtained as a yellowish oil: 26.4 g, 78.73 mmol, 75 % yield.

¹H NMR (500 MHz, Benzene-d₆) δ 3.95 (t, 2H), δ 2.89 (t, 2H), δ 2.18 (t, 2H), δ 1.61 (p, 2H), δ 1.40 (p, 2H), δ 1.32 (p, 2H), δ 1.29-1.12 (m, 12H), δ 1.01 (m, 4H), δ 0.89 (t, 3H)

### Example 6

### Synthesis of 9-bromononyl octanoate

9-bromononyl octanoate was prepared from octanoic acid and 9-bromo-1-nonanol according to general procedure **B.** The product was obtained as a yellowish oil: 51.42 g, 147.18 mmol, 89 % yield.

¹H NMR (500 MHz, Benzene-d₆) δ 4.05 (t, 2H), δ 2.96 (t, 2H), δ 2.18 (t, 2H), δ 1.60 (p, 2H), δ 1.48 (m, 4H), δ 1.29-0.92 (m, 18H), δ 0.85 (t, 3H)

### Example 7

### Synthesis of octyl octanoate

Octyl octanoate was prepared from octanoic acid and 1-octanol according to general procedure **B.** The product was obtained as a yellowish oil: 37.40 g, 145.85 mmol, 89 % yield.

¹H NMR (500 MHz, Benzene-d₆) δ 4.05 (t, 2H), δ 2.17 (t, 2H), δ 1.59 (m, 2H), δ 1.48 (m, 2H), δ 1.33-1.07 (m, 18H), δ 0.86 (m, 6H)

### Example 8

### Synthesis of heptyl octanoate

Heptyl octanoate was prepared from octanoic acid and 1-heptanol according to general procedure **A.** The product was obtained as a yellowish oil: 2.18 g, 8.99 mmol, 77 % yield.

¹H NMR (500 MHz, Benzene-d₆) δ 3.99 (t, 2H), δ 2.22 (t, 2H), δ 1.57-1.49 (m, 4H), δ 1.35-1.10 (m, 16H), δ 0.83-0.79 (m, 6H)

### Example 9

### Synthesis of nonyl octanoate

Nonyl octanoate was prepared from octanoic acid and 1-nonanol according to general procedure **A.** The product was obtained as a yellowish oil: 2.03 g, 7.05 mmol mmol, 61 % yield.

¹H NMR (500 MHz, Benzene-d₆) δ 3.99 (t, 2H), δ 2.22 (t, 2H), δ 1.57-1.50 (m, 4H), δ 1.29-1.10 (m, 20H), δ 0.83-0.79 (m, 6H)

### Example 10

### Synthesis of octyl heptanoate

Octyl heptanoate was prepared from heptanoic acid and 1-octanol according to general procedure **A.** The product was obtained as a yellowish oil: 2.49 g, 10.27 mmol, 86 % yield.

¹H NMR (500 MHz, Benzene-d₆) δ 3.99 (t, 2H), δ 2.22 (t, 2H), δ 1.57-1.50 (m, 4H), δ 1.27-1.10 (m, 16H), δ 0.83-0.79 (m, 6H)

### Example 11

### Synthesis of octyl nonanoate

Octyl nonanoate was prepared from nonanoic acid and 1-octanol according to general procedure **A.** The product was obtained as a yellowish oil: 2.62 g, 9.69 mmol, 78 % yield.

¹H NMR (500 MHz, Benzene-d₆) δ 3.99 (t, 2H), δ 2.22 (t, 2H), δ 1.57-1.50 (m, 4H), δ 1.34-1.10 (m, 20H), δ 0.83-0.79 (m, 6H)

### Example 12

### Synthesis of hexyl decanoate

Hexyl decanoate was prepared from decanoic acid and 1-hexanol according to general procedure **A.** The product was obtained as a yellowish oil: 2.63 g, 10.26 mmol, 88 % yield.

¹H NMR (500 MHz, Benzene-d₆) δ 3.99 (t, 2H), δ 2.22 (t, 2H), δ 1.57-1.48 (m, 4H), δ 1.24-1.12 (m, 18H), δ 0.84-0.79 (m, 6H)

### Example 13

### Synthesis of decyl hexanoate

Decyl hexanoate was prepared from hexanoic acid and 1-decanol according to general procedure **A.** The product was obtained as a yellowish oil: 2.77 g, 10.8 mmol, 90 % yield.

¹H NMR (500 MHz, Benzene-d₆) δ 3.99 (t, 2H), δ 2.22 (t, 2H), δ 1.60-1.49 (m, 4H), δ 1.29-1.12 (m, 18H), δ 0.85-0.79 (m, 6H)

### Example 14

### Synthesis of Octyl octanethioate

Octyl octanethioate was prepared from octanoic acid and 1-octanethiol according to general procedure **C.** The product was obtained as a yellowish oil: 3.38 g, 13.08 mmol, 90 % yield.

¹H NMR (500 MHz, Benzene-d₆) δ 2.79 (t, 2H), δ 2.46 (t, 2H), δ 1.63-1.53 (m, 2H), δ 1.51-1.44 (m, 2H), δ 1.35-1.10 (m, 18H), δ 0.83-0.78 (m, 6H)

### Example 15

### Synthesis of Decyl butanoate

Decyl butanoate was prepared from butyryl chloride and 1-decanol according to general procedure **A.** The product was obtained as a yellowish oil: 3.05 g, 13.36 mmol, 93 % yield.

¹H NMR (500 MHz, Benzene-d₆) δ 4.05 (t, 2H), δ 2.27 (t, 2H), δ 1.72-1.57 (m, 4H), δ 1.38-1.20 (br. m, 14H), δ 0.94 (t, 3H), δ 0.87 (t, 3H)

### Example 16

### Synthesis of (Z)-non-6-en-1-yl octanoate

(Z)-non-6-en-1-yl octanoate was prepared from octanoic acid and (Z)-non-6-en-1-ol according to general procedure **C.** The product was obtained as a yellowish oil: 2.10 g, 7.82 mmol, 85 % yield.

¹H NMR (500 MHz, Benzene-d₆) δ 5.46-5.30 (m, 2H), δ 4.03 (t, 2H), δ 2.17 (t, 2H), δ 2.03-1.91 (m, 4H), δ 1.62-1.55 (m, 2H), δ 1.50-1.42 (m, 2H) δ 1.30-1.12 (m, 12H), δ 0.93 (t, 3H), δ 0.85 (t, 3H)

### Example 17

### Synthesis of (Z)-non-3-en-1-yl octanoate

(Z)-non-3-en-1-yl octanoate was prepared from octanoic acid and (Z)-non-3-en-1-ol according to general procedure **C.** The product was obtained as a yellowish oil: 2.17 g, 8.07 mmol, 88 % yield.

¹H NMR (500 MHz, Benzene-d₆) δ 5.44-5.35 (m, 2H), δ 5.27-5.18 (m, 2H), δ 3.95 (t, 2H), δ 2.31-2.13 (m, 4H), δ 1.98-1.87 (m, 2H), δ 1.58-1.44 (m, 2H) δ 1.32-1.08 (m, 12H), δ 0.82-0.73 (m, 6H)

### Example 18

### Synthesis of heptadecan-9-yl 8-bromooctanoate

Heptadecan-9-yl 8-bromooctanoate was prepared from 8-bromooctanoic acid and heptadecane-9-ol according to general procedure **C.** The product was obtained as a yellowish oil: 3.20 g, 6.93 mmol, 13 % yield.

¹H NMR (500 MHz, CDCl₃) δ 4.92-4.84 (m, 1H), δ 3.41 (t, 2H), δ 2.30 (t, 2H), δ 1.90-1.84 (m, 2H), δ 1.70-1.20 (br. m, 36H), δ 0.89 (m, 6H)

### Example 19

### Synthesis of 6-ethoxy-6-oxohexyl 2-hexyldecanoate

*Step a)* In a 500 ml 2-necked flask equipped with a stirring bar and reflux condenser, under inert conditions (nitrogen gas), 2-hexyldecanoic acid (44.6 g, 51.0 mL, 1 eq. 174 mmol) was dissolved in abs. toluene (150 mL). Next, under stirring, 5 drops of DMF and thionyl chloride (51.7 g, 31.7 mL, 2.5 eq. 435 mmol) were added. The reaction mixture was stirred at room temperature until the gas evolution slowed down (approx. 2 hours). Then the mixture was heated to 80-90°C and stirred for further 2 hours, until gas evolution ceased. Monitoring of the reaction was done by TLC.

*Step b)* Next, the solvent and residual thionyl chloride was removed in vacuo and the resulting crude product was used for the next reaction step without further purification. In a 2-necked 500 ml flask, equipped with a stirring bar, condenser and addition funnel, under inert conditions (nitrogen gas), ethyl 6-hydroxyhexanoate (26.4 g, 26.8 mL, 1 eq. 165 mmol) was dissolved in 100 ml abs. toluene. Next, triethylamine (25.0 g, 34.5 mL, 1.5 eq. 248 mmol) was added, followed by the dropwise addition of 2-hexyldecanoyl chloride (47.8 g, 1.05 eq. 174 mmol) dissolved in 60 ml abs. toluene. Immediately, the precipitation of the forming TEA-HCl began. The reaction was monitored by TLC and stirring was continued overnight at room temperature (16 h). Next, the reaction mixture was filtered through Celite and washed with additional toluene. The resulting clear solution was washed with 1M aqueous HCl, followed by washing with sat. NaHCO₃ (aq) and sat. NaCl (aq). The extracted organic phase was dried on MgSO₄, filtered and concentrated in vacuo, resulting in 73.2 g of crude product. The ester was purified using column chromatography on silica gel, with hexanes and ethyl acetate as eluent, affording 6-ethoxy-6-oxohexyl 2-hexyldecanoate (58.50 g, 147 mmol, 89 % cumulative yield for the 2 reaction steps)

¹H NMR (500 MHz, CDCl₃) δ 4.16-4.12 (q, 2H), δ 4.08 (t, 2H), δ 2.33-2.30 (m, 2H), δ 1.71-1.54 (m, 6H), δ 1.48-1.37 (m, 4H), δ 1.34-1.20 (br. m, 24H), δ 0.89 (t, 6H)

### Example 20

### Synthesis of 6-methoxy-6-oxohexyl 2-(decylthio)hexanoate

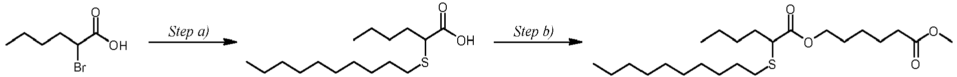

*Step a)* a flame dried 500 ml three neck round bottom flask was purged with the stream of argon. The flask was equipped with dropping funnel, thermometer and argon balloon and charged with 2-bromohexanoic acid (10.00 g 51.27 mmol) and 300 ml of abs. tetrahydrofuran. The reaction mixture was cooled down to 0°C. KOtBu (16.51 g, 147.13 mmol) was added portionwise maintaining the inner temperature at 0°C. After one hour stirring at 0°C 1-decanthiol (12.06 g, 69.21 mmol) was added dropwise maintaining the inner temperature at 0°C. The reaction mixture was stirred overnight at room temperature. The reaction was monitored by TLC (hexane/ethyl acetate 4:1, PMA visualization) and almost full conversion was detected after overnight stirring. Then 250 ml of 1 N HCl solution was added to the reaction mixture and extracted with 3x250 ml hexane. The combined organic phases were washed with 3×100 ml brine and dried over MgSO₄ and filtered and concentrated under reduced pressure. The crude product was purified by flash column chromatography with gradient elution (0-20 % EtOAc in n-hexane) to give yellowish oil 2-(decylthio)hexanoic acid (12.46 g, 43.19 mmol, 84 % yield).

¹H NMR (500 MHz, DMSO-d₆) δ 3.15 (t, 1H), δ 2.56 (m, 2H), δ 1.78-1.67 (m, 1H), δ 1.58-1.45 (m, 3H), δ 1.39-1.17 (m, 18H), δ 0.86 (t, 6H)

*Step b)* 6-methoxy-6-oxohexyl 2-(decylthio)hexanoate was prepared from 2-(decylthio)hexanoic acid and methyl 6-hydroxyhexanoate according to general procedure C and purified based on the following method: 250 g silica column, eluent: 0-10% ethyl acetate in hexane. The product was obtained as a brownish oil: 10.77 g, 25.85 mmol, 79 % yield.

¹H NMR (500 MHz, Benzene-d₆) δ 4.00 (m, 2H), δ 3.33 (s, 3H), δ 3.27 (t, 1H), δ 2.74-2.65 (m, 1H), δ 2.64-2.55 (m, 1H), δ 2.02 (t, 3H), δ 1.75 (m, 1H), δ 1.58 (m, 2H), δ 1.5-1.1 (m, 24H), δ 0.90 (t, 3H), δ 0.80 (t, 3H)

### Example 21

### Synthesis of 6-methoxy-6-oxohexyl 2-(pentylthio)decanoate

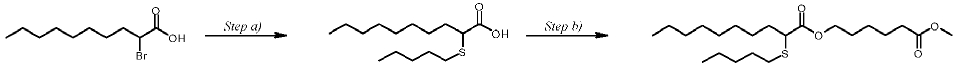

*Step a)* a flame dried 250 ml three neck round bottom flask was purged with the stream of argon. The flask was equipped with dropping funnel, thermometer and argon balloon and charged with 2-bromodecanoic acid (6.80 g, 27.07 mmol), 1-pentanethiol (3.81 g, 36.55 mmol) and 60 ml of abs. tetrahydrofuran. The reaction mixture was cooled down to 0°C. KOtBu (8.72 g, 77.7 mmol) in 80 ml of abs. THF was added dropwise meanwhile keeping the internal temperature at 0°C. The reaction mixture was stirred further for overnight at room temperature. The reaction was monitored by TLC (hexane/ethyl acetate 4:1, PMA visualization) and almost full conversion was detected after overnight. Then 150 ml of 1 N HCl solution was added to the reaction mixture and extracted with 3×150 ml hexane. The combined organic phases were washed with 2×150 ml brine and dried over MgSO₄ and filtered and concentrated under reduced pressure. The crude product was purified by flash column chromatography with gradient elution (0-20 % EtOAc in n-hexane) to give yellowish oil 2-(pentylthio)decanoic acid (6.20 g, 22.59 mmol, 83 % yield).

¹H NMR (500 MHz, DMSO-d₆) δ 3.16 (t, 1H), δ 2.56 (m, 2H), δ 1.77-1.66 (m, 1H), δ 1.58-1.44 (m, 3H), δ 1.41-1.14 (m, 16H), δ 0.85 (t, 6H)

*Step b)* 6-methoxy-6-oxohexyl 2-(pentylthio)decanoate was prepared from 2-(pentylthio)decanoic acid and methyl 6-hydroxyhexanoate according to general procedure **C** and purified based on the following method: 250 g silica column, eluent: 0-10% ethyl acetate in hexane. The product was obtained as a brownish oil: 9.37 g, 23.27 mmol, 75 % yield.

¹H NMR (500 MHz, Benzene-d₆) δ 4.00 (m, 2H), δ 3.33 (s, 3H), δ 3.30 (t, 1H), δ 2.71-2.64 (m, 1H), δ 2.61-2.53 (m, 1H), δ 2.02 (t, 3H), δ 1.78 (m, 1H), δ 1.55 (m, 2H), δ 1.48-1.32 (m, 6H), δ 1.32-1.07 (m, 16H), δ 0.88 (t, 3H), δ 0.81 (t, 3H)

### Example 22

### Synthesis of methyl (4R)-4-((3R,10S,13R)-3-methoxy-10,13-dimethylhexadecahydro-1H-cyclopenta[a]phenanthren-17-yl)pentanoate

Into a 1000 ml pressure flask - under argon stream - Lithocholic acid (5.00 g, 13.30 mmol), 240 ml of abs. acetonitrile, caesium carbonate (9.53 g, 29.26 mmol) and methyl iodide (8.30 g, 58.52 mmol) was charged. The flask was sealed, and the reaction mixture was heated up 110°C for 24 hours. Then the reaction mixture was cooled down and 20 ml of sodium sulphite (10 w/w%) solution and 200 ml of water was added. The mixture was extracted with 3×200 ml of ethyl acetate. The combined organic phases were washed with 1×100 ml of brine and dried over MgSO₄, filtered, and concentrated under reduced pressure. The residue was purified by flash column chromatography with gradient elution (5-20% ethyl acetate in hexane) to give the product as a white solid methyl (4R)-4-((3R,10S,13R)-3-methoxy-10,13-dimethylhexadecahydro-1H-cyclopenta[a]phenanthren-17-yl)pentanoate (1.30 g, 3.21 mmol, 24 % yield).

¹H NMR (500 MHz, Benzene-d6) δ 3.40 (s, 3H), δ 3.38 (t, 3H), δ 2.32-2.23 (m, 1H), δ 2.20-2.10 (m, 1H), δ 1.95-1.83 (m, 2H), δ 1.83-1.70 (m, 3H), δ 1.67-1.54 (m, 3H), δ 1.53-086 (m, 19H), δ 0.84 (d, 3H), δ 0.74 (s, 3H), δ 0.52 (s, 3H)

### Example 23

### Synthesis of undecyl 6-bromohexanoate

Undecyl 6-bromohexanoate was prepared from 6-bromooctanoic acid and 1-undecanol according to general procedure **C.** The product was obtained as a yellowish oil: 27.95 g, 80 mmol, 78 % yield.

¹H NMR (500 MHz, DMSO-d6) δ 4.01 (t, 2H), δ 3.52 (t, 2H), δ 3.52 (t, 2H), δ 2.30 (t, 2H), δ 1.85-1.78 (m, 2H), δ 1.58-1.52 (m, 4H), δ 1.42-1.38 (m, 2H), δ 1.33-1.20 (br. m, 14H), δ 0.87 (t, 3H)

### Example 24

### Synthesis of 4-chlorobutyl (9Z,12Z)-octadeca-9,12-dienoate

4-chlorobutyl (9Z,12Z)-octadeca-9, 12-dienoate was prepared from (9Z,12Z)-Octadeca-9,12-dienoic acid and 4-chlorobutan-1-ol according to general procedure **D.** The product was obtained as a yellowish oil: 1.20 g, 3.23 mmol, 45 % yield.

¹H NMR (500 MHz, Benzene-d6) δ 5.41-5.27 (m, 4H), δ 4.1 (t, 2H), δ 3.58 (t, 2H), δ 2.27 (t, 2H), δ 2.29 (t, 2H), δ 2.08-2.00 (m, 4H), δ 1.86-1.78 (m, 4H), δ 1.64-1.59 (m, 2H), δ 1.37-1.25 (m, 14H), δ 0.88 (t, 3H)

### Example 25

### Synthesis of 4-chlorobutyl (4R)-4-((3R,10S,13R)-3-methoxy-10,13-dimethylhexadecahydro-1H-cyclopenta[a]phenanthren-17-yl)pentanoate

4-Chlorobutyl (4R)-4-((3R,10S,13R)-3-methoxy-10,13-dimethylhexadecahydro-1H-cyclopenta[a]phenanthren-17-yl)pentanoate was prepared from (4R)-4-((3R,10S,13R)-3-methoxy-10,13-dimethylhexadecahydro-1H-cyclopenta[a]phenanthren-17-yl)pentanoic acid and 4-chlorobutan-1-ol according to general procedure **D.** The product was obtained as a colourless oil: 2.75 g, 5.72 mmol, 81 % yield.

¹H NMR (500 MHz, CDCl₃) δ 4.12-4.07 (m, 2H), δ 3.56 (t, 2H), δ 3.34 (s, 3H) δ 3.21-3.11 (m, 1H), δ 2.40-2.29 (m, 1H), δ 2.26-2.14 (m, 1H), δ 1.92-1.51 (br. m, 14H), δ 1.49-0.99 (br. m, 16H), δ 0.91-0.89 (m, 6H), δ 0.63 (s, 3H)

### Examples of silane synthesis

### Example 26

### Synthesis of dimethyl(octyl)silane

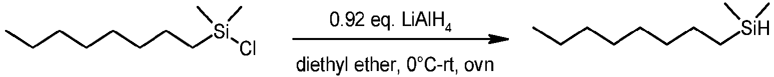

A flame dried 250 ml three neck round bottom flask was purged with the stream of argon. The flask was equipped with septum, thermometer and argon balloon and charged with chloro(dimethyl)octylsilane (20.00 g, 96.70 mmol) and 100 ml abs. diethyl ether. The reaction mixture was cooled down to 0°C and lithium aluminium hydride (3.38 g, 88.96 mmol) was added portionwise over 6 hours and maintained the inner temperature between 0-5°C. The reaction was stirred overnight at room temperature. Then the reaction mixture was cooled down again to 0°C and quenched with the mixture of 30 ml water and 50 ml of 5 w/w% NaOH solution maintained the inner temperature below 5°C, subsequently further portion of water (30 ml) was added, and left to warm up to room temperature. The phases were separated, and the organic phase was washed with 25 ml of water and dried over MgSO₄. After filtration of MgSO4 the volatiles were evaporated under reduced pressure to give the product as a colourless oil, dimethyl(octyl)silane (14.40 g, 83.53 mmol, 86% yield)

¹H NMR (500 MHz, CDCl₃) δ 3.85 (m, 1H), δ 1.25 (m, 12H), δ 0.90 (t, 3H), δ 0.59 (m, 2H), δ 0.08 (d, 6H)

### Example 27

### Synthesis of dimethyl(hexyl)silane

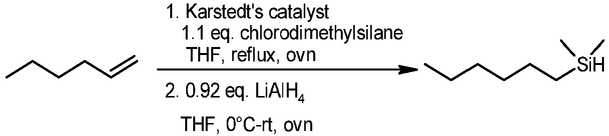

A flame dried 250 ml three neck round bottom flask was purged with the stream of argon. The flask was equipped with thermometer, condenser, argon balloon and charged with chlorodimethylsilane (10.00 g, 105.69 mmol), 1-hexene (8.09 g, 96.17 mmol), Karstedt's catalyst (0.111 ml of 2% Pt in xylene solution) and 100 ml abs. tetrahydrofuran. The reaction mixture was refluxed for 5 hours. The reaction was monitored by GC and after the five-hour reaction time full conversion was detected. The reaction mixture was cooled down to room temperature and concentrated under reduced pressure to give chloro(hexyl)dimethylsilane as a colorless oil (15.60 g, 87.26 mmol, 83 % yield).

A flame dried 250 ml three neck round bottom flask was purged with the stream of argon. The flask was equipped with septum, thermometer and argon balloon and charged with chloro(dimethyl)hexylsilane (15.60 g, 87.26 mmol) and 90 ml abs. diethyl ether. The reaction mixture was cooled down to 0°C and lithium aluminium hydride (3.05 g, 80.28 mmol) was added portionwise over 5 hours and maintained the inner temperature between 0-5°C. The reaction was stirred for further 1 hour at room temperature. Then the reaction mixture was cooled down again to 0°C and quenched with the mixture of 30 ml water and 50 ml of 5 w/w% NaOH solution maintained the inner temperature below 5°C, subsequently further portion of water (30 ml) was added, and left to warm up to room temperature. The phases were separated, and the organic phase was washed with 25 ml of water and dried over MgSO₄. After filtration of MgSO4 the volatiles were evaporated under reduced pressure. The residue was purified by atmospheric distillation to give the product as a colourless oil, dimethyl(hexyl)silane (10.30 g, 71.36 mmol, 82% yield).

¹H NMR (500 MHz, Benzene-d₆) δ 4.12 (m, 1H), δ 1.31 (m, 8H), δ 0.89 (t, 3H), δ 0.53 (m, 2H), δ 0.04 (d, 6H)

### Example 28

### Synthesis of 1,1,3,3-tetramethyl-1-octyldisiloxane

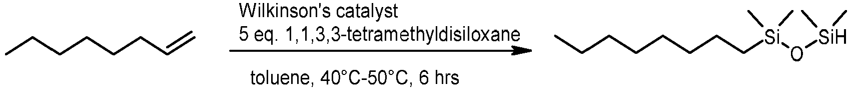

A flame dried 500 ml three neck round bottom flask was purged with the stream of argon. The flask was equipped with dropping funnel, thermometer, condenser, argon balloon and charged with 1,1,3,3-tetramethyldisiloxane (119.70 g, 891 mmol), Wilkinson's catalyst (5 mg, 0.0054 mmol) and 48.50 ml abs. toluene. The reaction mixture was heated up to 40°C and 1-octene (20.00 g, 178.22 mmol) was added dropwise to the reaction mixture. After addition of 1-octene the resultant mixture was stirred for further 5 hours at 50°C. After this reaction time full conversion was detected by ¹H NMR. The reaction mixture was concentrated under reduced pressure to give the product as a colourless oil, 1,1,3,3-tetramethyl-1-octyldisiloxane (46.00 g, 186.58 mmol, 98 % yield).

¹H NMR (500 MHz, CDCl₃) δ 4.69 (m, 1H), δ 1.28 (m, 12H), δ 0.90 (t, 3H), δ 0.55 (m, 2H), δ 0.18 (d, 6H), δ 0.07 (s, 6H)

### Example 29

### Synthesis of dimethyl(octyloxy)silane

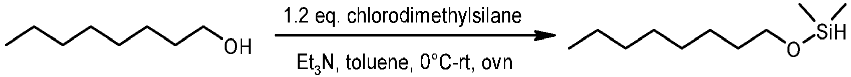

A 250 ml three neck round bottom flask was purged with the stream of argon. The flask was equipped with dropping funnel, thermometer, argon balloon and charged with 1-octanol (5.20 g, 40.00 mmol), triethylamine (6.07 g, 60.00 mmol) and 40.0 ml abs. toluene. The reaction mixture was cooled down to 0°C and chlorodimethylsilane (4.54 g, 48.00 mmol) was added dropwise maintaining the inner temperature between 0°C-5°C. After addition of the silane the reaction was stirred for overnight at room temperature. Then the reaction mixture was filtered through on a pad of celite and washed with 2×15 ml pentane and the precipitated triethylamine hydrochloride salt was filtered off. The filtrate was concentrated under reduced pressure. The crude product was purified by vacuum distillation at 0.02-0.05 mbar (46°C-51°C) to give the product as a colourless oil, dimethyl(octyloxy)silane (4.86 g, 25.80 mmol, 64 % yield).

¹H NMR (500 MHz, Benzene-d₆) δ 4.88 (m, 1H), δ 3.57 (t, 2H), δ 1.54 (p, 2H), δ 1.32 (m, 2H), δ 1.23 (m, 8H), δ 0.88 (t, 3H), δ 0.15 (d, 6H)

### Example 30

### Synthesis of ((2-hexyldecyl)oxy)dimethylsilane

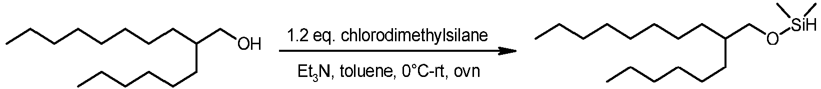

A 250 ml three neck round bottom flask was purged with the stream of argon. The flask was equipped with dropping funnel, thermometer, argon balloon and charged with 2-hexyldecan-1-ol (6.23 g, 22.87 mmol), triethylamine (3.47 g, 34.31 mmol) and 44.2 ml abs. toluene. The reaction mixture was cooled down to 0°C and chlorodimethylsilane (2.60 g, 27.45 mmol) was added dropwise maintained the inner temperature between 0°C-5°C. After addition of the silane the reaction was stirred for overnight at room temperature. Then the reaction mixture was filtered through on a pad of celite and washed with 2×15 ml pentane and the precipitated triethylamine hydrochloride salt was filtered off. The filtrate was concentrated under reduced pressure. The crude product was purified by vacuum distillation to give the product as a colourless oil, ((2-hexyldecyl)oxy)dimethylsilane (2.61 g, 8.68 mmol, 34 % yield).

¹H NMR (500 MHz, Benzene-d₆) δ 4.90 (p, 1H), δ 3.57 (d, 2H), δ 1.56 (m, 1H), δ 1.47 (m, 2H), δ 1.28 (m, 22H), δ 0.90 (m, 6H), δ 0.17 (d, 6H)

### Example 31

### Synthesis of 1,1,3,3-tetramethyl-1-(octyloxy)disiloxane

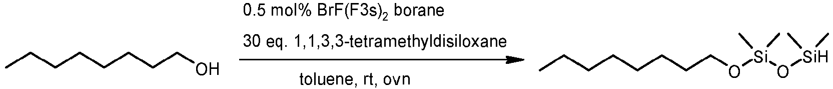

A flame dried 500 ml three neck round bottom flask was purged with the stream of argon. The flask was equipped with dropping funnel, thermometer, argon balloon and charged with 1.41 ml BrF(F3s)₂ borane catalyst solution (202 mg was dissolved in 4.00 ml abs. toluene) [borane catalysts are disclosed in WO2022/129966] and 1,1,3,3-tetramethyldisiloxane (128.42 g, 965 mmol). To the reaction mixture the toluene solution of 1-octanol (4.15 g, 31.87 mmol in 16 ml of abs. toluene) was added dropwise and the reaction mixture was stirred overnight at room temperature. Then the reaction mixture was concentrated under reduced pressure. The crude product was purified by vacuum distillation using Vigreux column at 0.02-0.05 mbar (52°C-60°C) to give the product as a colourless oil, 1,1,3,3-tetramethyl-1-(octyloxy)disiloxane (8.36 g, 21.86 mmol, 69 % yield).

¹H NMR (500 MHz, Benzene-d₆) δ 5.04 (m, 1H), δ 3.70 (t, 2H), δ 1.60 (p, 2H), δ 1.38 (m, 2H), δ 1.27 (m, 8H), δ 0.89 (t, 3H), δ 0.21 (d, 6H), δ 0.17 (s, 6H)

### Example 32

### Synthesis of (6-bromohexyl)dimethylsilane

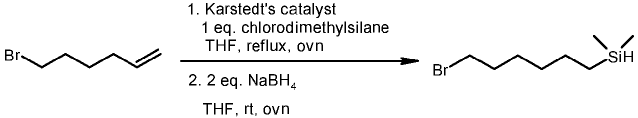

A flame dried 250 ml three neck round bottom flask was purged with the stream of argon. The flask was equipped with thermometer, condenser, argon balloon and charged with chlorodimethylsilane (6.38 g, 67.40 mmol), 6-bromo-1-hexene (10.00 g, 61.33 mmol), Karstedt's catalyst (69.6 µl of 2% Pt in xylene solution) and 60 ml abs. tetrahydrofuran. The reaction was monitored by GC and after overnight full conversion was detected. The reaction mixture was cooled down to room temperature and concentrated under reduced pressure to give (6-bromohexyl)chlorodimethylsilane as a yellow oil (16.65 g, 64.61 mmol, 96 % yield).

A flame dried 250 ml three neck round bottom flask was purged with the stream of argon. The flask was equipped with dropping funnel, thermometer, argon balloon and charged with the suspension of grounded NaBH₄ (4.89 g, 129.23 mmol) and 11 ml of abs. tetrahydrofuran. The mixture of (6-bromohexyl)chlorodimethylsilane (16.65 g, 64.61 mmol) and 50 ml abs. tetrahydrofuran was added dropwise to the suspension and stirred overnight at room temperature. The volatiles were removed under reduced pressure and 50 ml of hexane was added to the residue. The suspension was passed through a short pad of silica gel. The filtrate was concentrated under reduced pressure. The residue was further purified by vacuum distillation at 0.02-0.05 mbar (38-39°C) to give the product as a colourless oil, (6-bromohexyl)dimethylsilane (9.70 g, 43.45 mmol, 67% yield).

¹H NMR (500 MHz, Benzene-d₆) δ 4.09 (m, 1H), δ 2.95 (t, 2H), δ 1.48 (p, 2H), δ 1.20-1.04 (m, 6H), δ 0.48-0.4 (m, 2H) δ 0.02 (d, 6H)

### Example 33

### Synthesis of ((6-bromohexyl)oxy)dimethylsilane

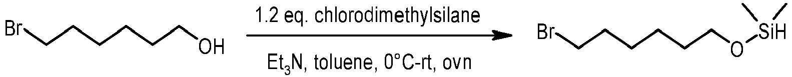

A 250 ml three neck round bottom flask was purged with the stream of argon. The flask was equipped with dropping funnel, thermometer, argon balloon and charged with 6-bromo-1-hexanol (8.00 g, 44.20 mmol), triethylamine (6.70 g, 66.30 mmol) and 44.2 ml abs. toluene. The reaction mixture was cooled down to 0°C and chlorodimethylsilane (5.02 g, 53.04 mmol) was added dropwise maintained the inner temperature between 0°C-5°C. After addition of the silane the reaction was stirred overnight at room temperature. Then the reaction mixture was filtered through on a pad of celite and washed with 2×15 ml pentane and the precipitated triethylamine hydrochloride salt was filtered off. The filtrate was concentrated under reduced pressure. The crude product was purified by vacuum distillation at 0.02-0.05 mbar (63°C-64°C) to give the product as a colourless oil, ((6-bromohexyl)oxy)dimethylsilane (7.27 g, 30.39 mmol, 63 % yield).

¹H NMR (500 MHz, Benzene-d₆) δ 4.85 (m, 1H), δ 3.47 (t, 2H), δ 2.90 (t, 2H), δ 1.45 (m, 2H), δ 1.37 (m, 2H), δ 1.11 (m, 4H) δ 0.14 (d, 6H)

### Example 34

### Synthesis of ((5-bromopentyl)oxy)dimethylsilane

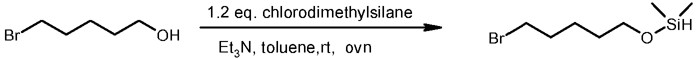

Triethylamine (8.39 g, 82.94 mmol, 1.50 eq.) was added to a stirred mixture of 5-bromopentan-1-ol (9.24 g, 55.29 mmol, 1.00 eq.) and 50 ml dry toluene under Ar atmosphere. Chlorodimethylsilane (6.28 g, 66.35 mmol, 1.20 eq.) was added, and the mixture became a white slurry mixture. It was shaken a couple of times before leaving to stir overnight. After 16 h the mixture was filtered (glass frit) and the precipitate was rinsed with toluene. The filtrate was evaporated. The residue was diluted with petroleum ether (~20 mL) resulting a white precipitate which was separated by centrifugation. The clear solution was concentrated. The remaining oil was purified by vacuum distillation at 1 mbar to give the product as a colourless oil, ((5-bromopentyl)oxy)dimethylsilane (9.23 g, 40.99 mmol, 74 % yield).

¹H NMR (500 MHz, Benzene-d₆) δ 4.84 (m, 1H), δ 3.42 (t, 2H), δ 2.92 (t, 2H), δ 1.50-1.43 (m, 2H), δ 1.35-1.18 (m, 4H), δ 0.13 (d, 6H)

### Example 35

### Synthesis of ((4-bromobutyl)oxy)dimethylsilane

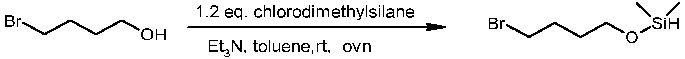

Triethylamine (7.93 g, 78.42 mmol, 1.50 eq.) was added to a stirred mixture of 4-bromobutan-1-ol (8.00 g, 52. 28 mmol, 1.00 eq.) and 50 ml dry toluene under Ar atmosphere. Chlorodimethylsilane (5.93 g, 62.74 mmol, 1.20 eq.) was added, and the mixture became a white slurry mixture. It was shaken a couple of times before leaving to stir overnight. After 16 h the mixture was filtered (glass frit) and the precipitate was rinsed with toluene. The filtrate was evaporated. The residue was diluted with petroleum ether (~20 mL) resulting a white precipitate which was separated by centrifugation. The clear solution was concentrated. The remaining oil was purified by vacuum distillation at 10 mbar (65-70°C) to give the product as a colourless oil, ((4-bromobutyl)oxy)dimethylsilane (7.65 g, 36.22 mmol, 69 % yield).

¹H NMR (500 MHz, Benzene-d₆) δ 4.78 (m, 1H), δ 3.36 (t, 2H), δ 2.96 (t, 2H), δ 1.68-1.56 (m, 2H), δ 1.46-1.34 (m, 2H), δ 0.10 (d, 6H)

### Example 36

### Synthesis of 1-(4-bromobutoxy)-1,1,3,3-tetramethyldisiloxane

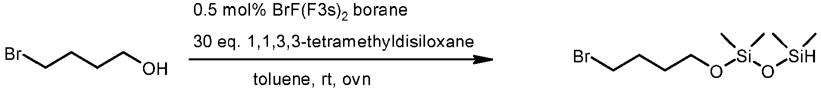

A flame dried 250 ml three neck round bottom flask was purged with the stream of argon. The flask was equipped with dropping funnel, thermometer, argon balloon and charged with 100 µL BrF(F3s)₂ borane catalyst solution (58.42 mg was dissolved in 100 µL) [borane catalysts are disclosed in WO2022/129966] and 1,1,3,3-tetramethyldisiloxane (105.34 g, 784.22 mmol, 30 eq.). To the reaction mixture the toluene solution of 4-bromo-1-butanol (4.00 g, 26.14 mmol in 16 ml of abs. toluene) was added dropwise and the reaction mixture was stirred overnight at room temperature. Then the reaction mixture was concentrated under reduced pressure. The crude product was purified by vacuum distillation using Vigreux column at 5 mbar (65°C-75°C) to give the product as a colourless oil, 1-(4-bromobutoxy)-1,1,3,3-tetramethyldisiloxane (4.56 g, 15.98 mmol, 61 % yield).

¹H NMR (500 MHz, Benzene-d₆) δ 4.99 (m, 1H), δ 3.49 (t, 2H), δ 3.00 (t, 2H), δ 1.74.1.61 (m, 2H), δ 1.54-1.40 (m, 2H), δ 0.18 (d, 6H), δ 0.10 (s, 6H)

### Mixed silyl acetals synthesis (general procedure E-F)

BrF(F3s)₂ and BrF(F4)₂ borane catalysts as disclosed in WO2022/129966 were applied in the synthesis, which are defined under each example.

### General Procedure E

A flame dried three necked round bottom flask was purged with the stream of argon. The flask was equipped with septum, thermometer and argon balloon and charged with the mixture of abs. toluene (1.0 ml toluene/1.0 mmol ester) and ester. To the reaction mixture the catalyst solution was added in one portion, followed by the dropwise addition of silane, maintaining the internal temperature between 20-30°C. The reaction mixture was stirred for hours at room temperature (specified in the examples). The reaction was monitored by TLC (hexane/ethyl acetate 9/1, PMA visualization and if different, it is specified in the example). After reaction completion 1 ml EtOAc, 0.6 ml MeCN and silica gel were added to the reaction mixture and stirred for further 10 mins and filtered off the silica gel and washed with 2×25 ml n-pentane. Volatiles were removed under reduced pressure to obtain the crude product that was purified by flash column chromatography or simple filtration via a short pad of silica to give the product.

### General Procedure F

A flame dried three necked round bottom flask was purged with the stream of argon. The flask was equipped with septum, thermometer and argon balloon and charged with the catalyst and abs. toluene (4.00 ml). The mixture of ester and silane was added dropwise to the solution of catalyst, maintaining the internal temperature between 20-30°C. The reaction mixture was stirred for 12 hours. The reaction was monitored by NMR or TLC (specified in the examples). After reaction completion 1 ml EtOAc, 0.6 ml MeCN and silica gel were added to the reaction mixture and stirred for further 10 mins and filtered off the silica gel and washed with 2×15 ml n-pentane. Volatiles were removed under reduced pressure to obtain the crude product that was purified by flash column chromatography or simple filtration via a short pad of silica to give the product.

### Examples of mixed silyl acetal synthesis

### Example 37

### Synthesis of ((6-bromo-1-(octyloxy)hexyl)oxy)dimethyl(octyl)silane

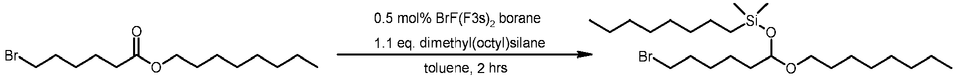

((6-bromo-1-(octyloxy)hexyl)oxy)dimethyl(octyl)silane was prepared from octyl 6-bromohexanoate (4.00 g, 13.02 mmol) and dimethyl(octyl)silane (1.1 eq., 2.47 g, 14.32 mmol) using 616 µl BrF(F3s)₂ borane catalyst solution (189 mg catalyst was dissolved in 4.00 ml abs toluene) according to **General Procedure E.** The reaction was completed after 2 hrs. The crude product was purified by flash column chromatography with gradient elution (0-10 % EtOAc in n-hexane) to give a transparent oil ((6-bromo-1-(octyloxy)hexyl)oxy)dimethyl(octyl)silane (4.72 g, 9.84 mmol, 76 % yield)

¹H NMR (500 MHz, Benzene-d₆) δ 4.79 (dt, 1H), δ 3.73-3.69 (dq, 1H), δ 3.35-3.31 (dq, 1H), δ 2.95 (t, 2H), δ 1.71-1.57 (m, 4H), δ 1.47-1.19 (m, 28H), δ 0.92-0.87 (t, 6H), δ 0.71 (dd, 2H), δ 0.2 (d, 6H)

### Example 38

### Synthesis of ((1-((6-bromohexyl)oxy)octyl)oxy)dimethyl(octyl)silane

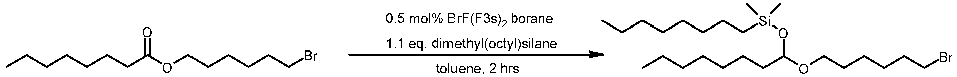

((1-((6-bromohexyl)oxy)octyl)oxy)dimethyl(octyl)silane was prepared from 6-bromohexyl octanoate (5.00 g, 16.27 mmol) and dimethyl(octyl)silane (1.1 eq., 3.09 g, 17.90 mmol) using 770 µl BrF(F3s)₂ borane catalyst solution (189 mg was dissolved in 4.00 ml abs. toluene) according to **General Procedure E.** The reaction was completed after 2 hrs. The crude product was purified by flash column chromatography with gradient elution (0-10 % EtOAc in n-hexane) to give a transparent oil ((1-((6-bromohexyl)oxy)octyl)oxy)dimethyl(octyl)silane (4.26 g, 8.88 mmol, 55 % yield)

¹H NMR (500 MHz, Benzene-d₆) δ 4.84 (t, 1H), δ 3.68-3.64 (dq, 1H), δ 3.30-3.27 (dq, 1H), δ 2.94 (t, 2H), δ 1.84-1.77 (m, 1H), δ 1.75-1.68 (m, 1H), δ 1.54-1.44 (m, 8H), δ 1.39 (m, 2H), δ 1.36-1.17 (m, 20H), δ 0.92-0.87 (t, 6H), δ 0.71 (dd, 2H), δ 0.22 (d, 6H)

### Example 39

### Synthesis of ((6-bromo-1-(decyloxy)hexyl)oxy)(hexyl)dimethylsilane

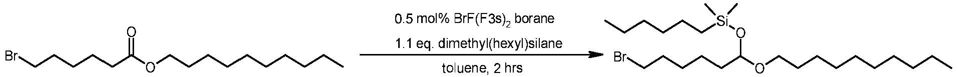

((6-bromo-1-(decyloxy)hexyl)oxy)(hexyl)dimethylsilane was prepared from decyl 6-bromohexanoate (4.00 g, 11.93 mmol) and dimethyl(hexyl)silane (1.1 eq., 1.89 g, 13.12 mmol) using 573 µl BrF(F3s)₂ borane catalyst solution (186 mg catalyst was dissolved in 4.00 ml abs toluene) according to **General Procedure E.** The reaction was completed after 2 hrs. The crude product was purified by flash column chromatography with gradient elution (0-10 % EtOAc in n-hexane) to give a transparent oil ((6-bromo-1-(decyloxy)hexyl)oxy)(hexyl)dimethylsilane (4.52 g, 9.42 mmol, 79 % yield)

¹H NMR (500 MHz, Benzene-d₆) δ 4.78 (t, 1H), δ 3.73-3.69 (dq, 1H), δ 336-331 (dq, 1H), δ 2.94 (t, 2H), δ 1.71-1.56 (m, 4H), δ 1.54-1.47 (m, 2H), δ 1.46-1.38 (m, 4H), δ 1.37-1.17 (m, 22H), δ 0.93-0.88 (t, 6H), δ 0.69 (dd, 2H), δ 0.21 (d, 6H)

### Example 40

### Synthesis of ((10-bromo-1-(octyloxy)decyl)oxy)(hexyl)dimethylsilane

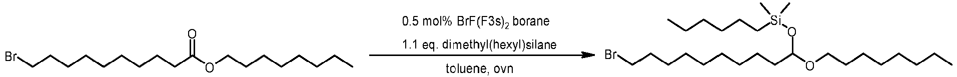

((10-bromo-1-(octyloxy)decyl)oxy)(hexyl)dimethylsilane was prepared from octyl 10-bromodecanoate (5.00 g, 11.69 mmol) and dimethyl(hexyl)silane (1.1 eq., 1.86 g, 12.86 mmol) using 517 µl BrF(F3s)₂ borane catalyst solution (202 mg was dissolved in 4.00 ml abs. toluene) according to **General Procedure E.** The reaction was completed after overnight. The crude product was purified by flash column chromatography with gradient elution (0-10 % EtOAc in n-hexane) to give a transparent oil ((10-bromo-1-(octyloxy)decyl)oxy)(hexyl)dimethylsilane (3.60 g, 7.10 mmol, 61 % yield)

¹H NMR (500 MHz, Benzene-d₆) δ 4.87 (t, 1H), δ 3.77-3.72 (dq, 1H), δ 339-335 (dq, 1H), δ 2.95 (t, 2H), δ 1.86-1.78 (m, 1H), δ 1.77-1.70 (m, 1H), δ 1.67-1.61 (p, 2H), δ 1.56-1.20 (m, 26H), δ 1.17-1.11 (p, 4H), δ 1.10-1.01 (m, 2H), δ 0.94-0.86 (m, 6H), δ 0.71 (dd, 2H), δ 0.23 (d, 6H)

### Example 41

### Synthesis of ((1-((6-bromohexyl)oxy)decyl)oxy)(hexyl)dimethylsilane

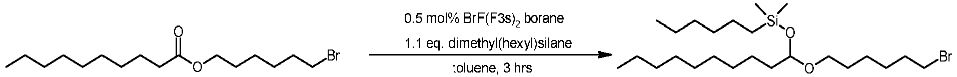

((1-((6-bromohexyl)oxy)decyl)oxy)(hexyl)dimethylsilane was prepared from 6-bromohexyl decanoate (5.00 g, 14.91 mmol) and dimethyl(hexyl)silane (1.1 eq., 2.37 g, 16.40 mmol) using 717 µl BrF(F3s)₂ borane catalyst solution (186 mg was dissolved in 4.00 ml abs. toluene) according to **General Procedure E.** The reaction was completed after 3 hrs. The crude product that was purified by flash column chromatography with gradient elution (0-10 % EtOAc in n-hexane) yielding a transparent oil ((1-((6-bromohexyl)oxy)decyl)oxy)(hexyl)dimethylsilane (5.04 g, 10.51 mmol, 71 % yield)

¹H NMR (500 MHz, Benzene-d₆) δ 4.84 (t, 1H), δ 3.68-3.64 (dq, 1H), δ 3.30-3.26 (dq, 1H), δ 2.94 (t, 2H), δ 1.85-1.77 (m, 1H), δ 1.76-1.68 (m, 1H), δ 1.61-1.41 (m, 8H), δ 1.40-1.15 (m, 22H), δ 0.92-0.88 (m, 6H), δ 0.70 (dd, 2H), δ 0.23 (d, 6H).

### Example 42

### Synthesis of ((1-((9-bromononyl)oxy)octyl)oxy)(hexyl)dimethylsilane

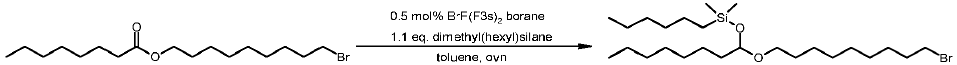

((1-((9-bromononyl)oxy)octyl)oxy)(hexyl)dimethylsilane was prepared from 9-bromononyl octanoate (5.00 g, 14.31 mmol) and dimethyl(hexyl)silane (1.1 eq., 2.27 g, 15.74 mmol) using 633 µl BrF(F3s)₂ borane catalyst solution (202 mg was dissolved in 4.00 ml abs. toluene) according to **General Procedure E.** The reaction was completed after overnight stirring. The crude product was purified by flash column chromatography with gradient elution (0-10 % EtOAc in n-hexane) yielding a transparent oil ((1-((9-bromononyl)oxy)octyl)oxy)(hexyl)dimethylsilane (6.00 g, 12.15 mmol, 85 % yield)

¹H NMR (500 MHz, Benzene-d₆) δ 4.87 (t, 1H), δ 3.77-3.72 (dq, 1H), δ 339-335 (dq, 1H), δ 2.95 (t, 2H), δ 1.86-1.78 (m, 1H), δ 1.77-1.68 (m, 1H), δ 1.66-1.58 (m, 2H), δ 1.57-1.18 (m, 24H), δ 1.17-1.10 (m, 4H) δ 1.09-1.01 (m, 2H), δ 0.92-0.86 (m, 6H), δ 0.71 (dd, 2H), δ 0.23 (d, 6H)

### Example 43

### Synthesis of 12-(5-bromopentyl)-10,10-dimethyl-9,11,13-trioxa-10-silahenicosane

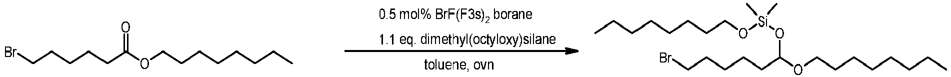

12-(5-bromopentyl)-10,10-dimethyl-9,11,13-trioxa-10-silahenicosane was prepared from octyl 6-bromohexanoate (5.00 g, 16.27 mmol) and dimethyl(octyloxy)silane (1.1 eq., 3.37 g, 17.90 mmol) using 503 µl BrF(F3s)₂ borane catalyst solution (253 mg was dissolved in 3.50 ml abs. toluene) according to **General Procedure** E. The reaction was completed after overnight stirring. The crude product was purified by flash column chromatography with gradient elution (0-10 % EtOAc in n-hexane) yielding a transparent oil 12-(5-bromopentyl)-10,10-dimethyl-9,11,13-trioxa-10-silahenicosane (3.78 g, 7.63 mmol, 47 % yield)

¹H NMR (500 MHz, Benzene-d₆) δ 4.93 (t, 1H), δ 3.85-3.83 (dq, 1H), δ 3.76-3.73 (t, 2H), δ 3.40-3.39 (dq, 1H), 2.97 (t, 2H), δ 1.80-1.60 (m, 6H), δ 1.60-1.50 (m, 2H), δ 1.50-1.30 (m, 6H), δ 1.57-1.18 (m, 18H), δ 0.92-0.89 (m, 6H), δ 0.23 (d, 6H)

### Example 44

### Synthesis of 1-bromo-8-heptyl-10,10-dimethyl-7,9,11-trioxa-10-silanonadecane

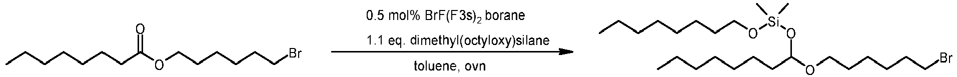

1-bromo-8-heptyl-10,10-dimethyl-7,9,11-trioxa-10-silanonadecane was prepared from 6-bromohexyl octanoate (5.00 g, 16.27 mmol) and dimethyl(octyloxy)silane (1.1 eq., 3.37 g, 17.90 mmol) using 706 µl BrF(F3s)₂ borane catalyst solution (206 mg was dissolved in 4.00 ml abs. toluene) according to **General Procedure** E. The reaction was completed after overnight stirring. The crude product that was purified by flash column chromatography with gradient elution (0-10 % EtOAc in n-hexane) yielding a transparent oil 1-bromo-8-heptyl-10,10-dimethyl-7,9,11-trioxa-10-silanonadecane (5.00 g, 10.08 mmol, 62 % yield)

¹H NMR (500 MHz, Benzene-d₆) δ 4.96 (t, 1H), δ 3.79-3.71 (dq, 3H), δ 3.34-3.30 (dq, 1H), δ 2.94 (t, 2H), δ 1.92-1.75 (m, 2H), δ 1.65-1.11 (m, 30H), δ 0.90-0.87 (m, 6H), δ 0.25 (d, 6H)

### Example 45

### Synthesis of 1-((6-bromo-1-(octyloxy)hexyl)oxy)-1,1,3,3-tetramethyl-3-octyldisiloxane

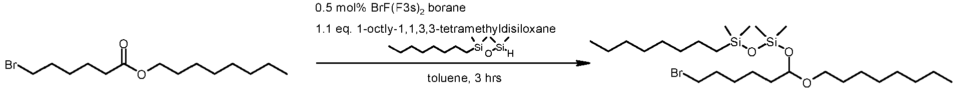

1-((6-bromo-1-(octyloxy)hexyl)oxy)-1,1,3,3-tetramethyl-3-octyldisiloxane was prepared from octyl 6-bromohexanoate (5.0 g, 16.27 mmol) and 1,1,3,3-tetramethyl-1-octyldisiloxane (1.1 eq., 4.41 g, 17.90 mmol) using 782 µl BrF(F3s)₂ borane catalyst solution (186 mg was dissolved in 4.00 ml abs. toluene) according to **General Procedure** E. The reaction was completed after 3 hrs. The crude product that was purified by flash column chromatography with gradient elution (0-10 % EtOAc in n-hexane) yielding a transparent oil 1-((6-bromo-1-(octyloxy)hexyl)oxy)-1,1,3,3-tetramethyl-3-octyldisiloxane (7.80 g, 14.08 mmol, 87 % yield)

¹H NMR (500 MHz, Benzene-d₆) δ 4.92 (dt, 1H), δ 3.85-3.80 (dq, 1H), δ 3.40-3.36 (dq, 1H), δ 2.96 (t, 2H), δ 1.79-1.61 (m, 4H), δ 1.56-1.50 (p, 2H), δ 1.48-1.20 (p, 26H), δ 0.92-0.87 (m, 6H), δ 0.66 (m, 2H), δ 0.23 (d, 6H), δ 0.19 (s, 6H).

### Example 46

### Synthesis of 1-((1-((6-bromohexyl)oxy)octyl)oxy)-1,1,3,3-tetramethyl-3-octyldisiloxane

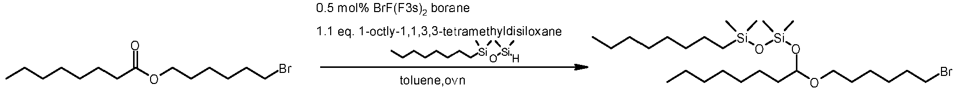

1-((1-((6-bromohexyl)oxy)octyl)oxy)-1,1,3,3-tetramethyl-3-octyldisiloxane was prepared from 6-bromohexyl octanoate (5.0 g, 16.27 mmol) and 1,1,3,3-tetramethyl-1-octyldisiloxane (1.1 eq., 4.41 g, 17.90 mmol) using 589 µl BrF(F3s)₂ borane catalyst solution (216 mg was dissolved in 3.50 ml abs. toluene) according to **General Procedure E.** The reaction was completed after overnight stirring. The crude product that was purified by flash column chromatography with gradient elution (0-10 % EtOAc in n-hexane) yielding a transparent oil 1-((1-((6-bromohexyl)oxy)octyl)oxy)-1,1,3,3-tetramethyl-3-octyldisiloxane (6.92 g, 12.49 mmol, 77 % yield)

¹H NMR (500 MHz, Benzene-d₆) δ 4.97 (t, 1H), δ 3.79-3.75 (dq, 1H), δ 335-330 (dq, 1H), δ 2.94 (t, 2H), δ 1.92-1.75 (m, 2H), δ 1.60-1.13 (m, 30H), δ 0.92-0.87 (m, 6H), δ 0.66 (m, 2H), δ 0.22 (d, 6H), δ 0.19 (s, 6H)

### Example 47

### Synthesis of 1-((6-bromo-1-(octyloxy)hexyl)oxy)-1,1,3,3-tetramethyl-3-(octyloxy)disiloxane

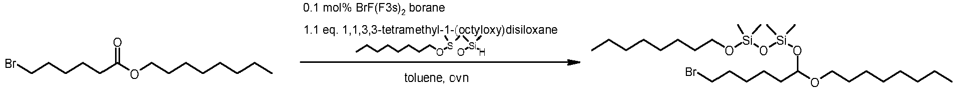

1-((6-bromo-1-(octyloxy)hexyl)oxy)-1,1,3,3-tetramethyl-3-(octyloxy)disiloxane was prepared from octyl 6-bromohexanoate (5.0 g, 16.27 mmol) and 1,1,3,3-tetramethyl-1-(octyloxy)disiloxane (1.1 eq., 4.70 g, 17.90 mmol) using 144 µl BrF(F3s)₂ borane catalyst solution (202 mg was dissolved in 4.00 ml abs. toluene) according to **General Procedure** E. The reaction was completed after overnight stirring. The crude product that was purified by flash column chromatography with gradient elution (0-10 % EtOAc in n-hexane) yielding a transparent oil 1-((6-bromo-1-(octyloxy)hexyl)oxy)-1,1,3,3-tetramethyl-3-(octyloxy)disiloxane (8.29 g, 14.55 mmol, 89 % yield).

¹H NMR (500 MHz, Benzene-d₆) δ 4.95 (dt, 1H), δ 3.86-3.82 (dq, 1H), δ 3.76-3.72 (t, 2H), δ 3.41-3.37 (dq, 1H), δ 2.96 (t, 2H), δ 1.82-1.59 (m, 6H), δ 1.56-1.51 (p, 2H), δ 1.46-1.35 (m, 6H), δ 1.34-1.18 (m, 18H), δ 0.90-0.87 (m, 6H), δ 0.27 (d, 6H), δ 0.21 (s, 6H).

### Example 48

### Synthesis of 1-((1-((6-bromohexyl)oxy)octyl)oxy)-1,1,3,3-tetramethyl-3-(octyloxy)disiloxan

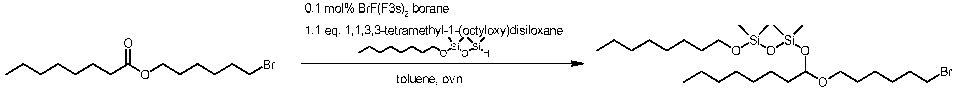

1-((1-((6-bromohexyl)oxy)octyl)oxy)-1,1,3,3-tetramethyl-3-(octyloxy)disiloxan was prepared from 6-bromohexyl octanoate (4.00 g, 13.01 mmol) and 1,1,3,3-tetramethyl-1-(octyloxy)disiloxane (1.1 eq., 3.76 g, 14.32 mmol) using 565 µl BrF(F3s)₂ borane catalyst solution (206 mg was dissolved in 4.00 ml abs. toluene) according to **General Procedure E.** The reaction was completed after overnight stirring. The crude product was purified by flash column chromatography with gradient elution (0-10 % EtOAc in n-hexane) to give a transparent oil 1-((1-((6-bromohexyl)oxy)octyl)oxy)-1,1,3,3-tetramethyl-3-(octyloxy)disiloxan (4.02 g, 7.05 mmol, 54 % yield)

¹H NMR (500 MHz, Benzene-d₆) δ 5.00 (dt, 1H), δ 3.82-3.71 (m, 3H), δ 3.36-3.31 (dq 1H), δ 2.94 (t, 2H), δ 1.95-1.76 (m, 2H), δ 1.68-1.45 (m, 8H), δ 1.45-1.11 (m, 22H), δ 0.90-0.87 (m, 6H), δ 0.29 (d, 6H), δ 0.22 (s, 6H)

### Example 49

### Synthesis of (6-bromohexyl)dimethyl((1-(octyloxy)octyl)oxy)silane

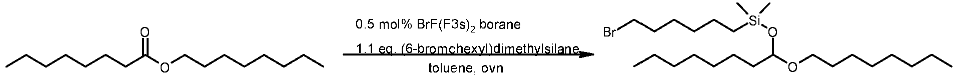

(6-bromohexyl)dimethyl((1-(octyloxy)octyl)oxy)silane was prepared from and octyl octanoate (5.00 g, 19.50 mmol) and (6-bromohexyl)dimethylsilane (1.1 eq., 4.79 g, 21.45 mmol) using 603 µl BrF(F3s)₂ borane catalyst solution (248 mg was dissolved in 3.43 ml abs. toluene) according to **General Procedure E.** The reaction was completed after overnight stirring. The crude product was purified by flash column chromatography with gradient elution (0-5 % EtOAc in n-hexane) to give a transparent oil (6-bromohexyl)dimethyl((1-(octyloxy)octyl)oxy)silane (9.11 g, 18.99 mmol, 97 % yield)

¹H NMR (500 MHz, Benzene-d₆) δ 4.87 (dt, 1H), δ 3.77-3.72 (dq, 1H), δ 3.40-3.35 (dq 1H), δ 2.99 (t, 2H), δ 1.87-1.80 (m, 1H), δ 1.77-1.70 (m, 1H), δ 1.68-1.63 (m, 2H), δ 1.57-1.49 (p, 4H), δ 1.48-1.40 (p, 2H), δ 1.39-1.14 (m, 22H), δ 0.92-0.88 (m, 6H), δ 0.65 (m, 2H), δ 0.23 (d, 6H).

### Example 50

### Synthesis of 1-bromo-10-heptyl-8,8-dimethyl-7,9,11-trioxa-8-silanonadecane

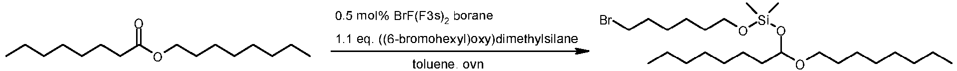

1-bromo-10-heptyl-8,8-dimethyl-7,9,11-trioxa-8-silanonadecane was prepared from octyl octanoate (5.00 g, 19.50 mmol) and ((6-bromohexyl)oxy)dimethylsilane (1.1 eq., 5.13 g, 21.45 mmol) using 603 µl BrF(F3s)₂ borane catalyst solution (253 mg was dissolved in 3.50 ml abs. toluene) according to **General Procedure E.** The reaction was completed after overnight stirring. The crude product that was purified by flash column chromatography with gradient elution (0-5 % EtOAc in n-hexane) yielding a transparent oil 1-bromo-10-heptyl-8,8-dimethyl-7,9,11-trioxa-8-silanonadecane (8.20 g, 16.54 mmol, 85 % yield).

¹H NMR (500 MHz, Benzene-d₆) δ 4.99 (dt, 1H), δ 3.86-3.84 (dq, 1H), δ 3.70-3.67 (t, 2H), δ 3.42-3.41 (dq 1H), δ 2.96 (t, 2H), δ 1.87-1.81 (m, 1H), δ 1.79-1.70 (m, 1H), δ 1.70-1.64 (m, 2H), δ 1.62-1.40 (m, 8H), δ 1.40-1.13 (m, 20H), δ 0.91-0.89 (m, 6H), δ 0.27 (d, 6H)

### Example 51

### Synthesis of 10-(5-bromopentyl)-15-hexyl-12,12-dimethyl-9,11,13-trioxa-12-silatricosane

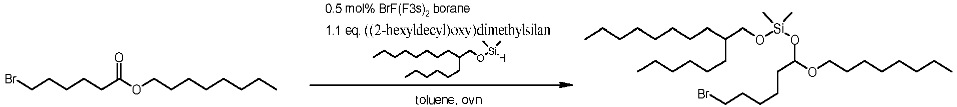

10-(5-bromopentyl)-15-hexyl-12,12-dimethyl-9,11,13-trioxa-12-silatricosane was prepared from octyl 6-bromohexanoate (5.00 g, 16.27 mmol) and ((2-hexyldecyl)oxy)dimethylsilane (1.1 eq., 5.38 g, 17.90 mmol) using 503 µl BrF(F3s)₂ borane catalyst solution (253 mg catalyst was dissolved in 3.50 ml abs toluene) according to **General Procedure E.** The reaction was completed after overnight stirring. The crude product was purified by flash column chromatography with gradient elution (0-2 % EtOAc in n-hexane) yielding a transparent oil 10-(5-bromopentyl)-15-hexyl-12,12-dimethyl-9,11,13-trioxa-12-silatricosane (6.66 g, 10.96 mmol, 67 % yield)

¹H NMR (500 MHz, Benzene-d₆) δ 4.93 (dt, 1H), δ 3.86-3.82 (dq, 1H), δ 3.72-3.71 (d, 2H), δ 3.42-3.37 (dq 1H), δ 2.96 (t, 2H), δ 1.81-1.47 (m, 9H), δ 1.47-1.18 (m, 36H), δ 0.91-0.88 (m, 9H), δ 0.26 (d, 6H)

### Example 52

### Synthesis of 1-bromo-8-heptyl-13-hexyl-10,10-dimethyl-7,9,11-trioxa-10-silahenicosane

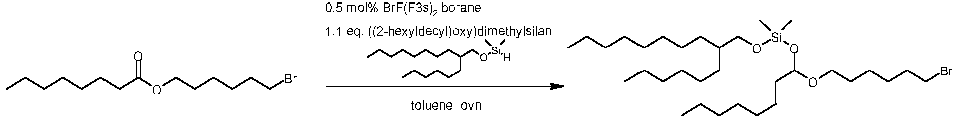

1-bromo-8-heptyl-13-hexyl-10,10-dimethyl-7,9,11-trioxa-10-silahenicosane was prepared from 6-bromohexyl octanoate (4.00 g, 13.01 mmol) and ((2-hexyldecyl)oxy)dimethylsilane (1.1 eq., 4.30 g, 14.32 mmol) using 402 µl BrF(F3s)₂ borane catalyst solution (253 mg was dissolved in 3.50 ml abs. toluene) according to **General Procedure E.** The reaction was completed after overnight stirring. The crude product was purified by flash column chromatography with gradient elution (0-2 % EtOAc in n-hexane) yielding a transparent oil 1-bromo-8-heptyl-13-hexyl-10,10-dimethyl-7,9,11-trioxa-10-silahenicosane (4.03 g, 6.63 mmol, 51 % yield).

¹H NMR (500 MHz, Benzene-d₆) δ 4.99 (dt, 1H), δ 3.82-3.78 (dq, 1H), 3.74-3.73 (d, 2H), 3.37-3.33 (dq 1H), 2.96 (t, 2H), δ 1.88-1.75 (m, 2H), δ 1.75-1.45 (m, 9H), δ 1.45-1.14 (m, 34H), δ 0.92-0.89 (m, 9H), δ 0.28 (d, 6H)

### Example 53

### Synthesis of triethyl(((9Z,12Z)-1-methoxyoctadeca-9,12-dien-1-yl)oxy)silane

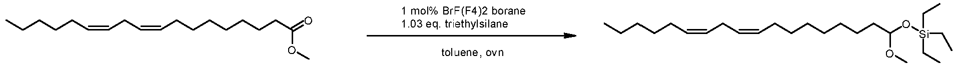

Triethyl(((9Z,12Z)-1-methoxyoctadeca-9,12-dien-1-yl)oxy)silane was prepared from methyl (9Z,12Z)-octadeca-9,12-dienoate (12.68 g, 43.07 mmol) and triethylsilane (5.16 g, 44.36 mmol) using 208 mg (0.43 mmol) BrF(F4)₂ borane catalyst according to **General Procedure F.** The reaction was monitored by ¹H NMR. After purification the product was obtained as a yellowish oil triethyl(((9Z,127)-1-methoxyoctadeca-9,12-dien-1-yl)oxy)silane (14.61 g, 35.56 mmol, 83 % yield) ¹H NMR (500 MHz, Benzene-d₆) δ 5.50-5.42 (m, 4H), δ 4.74 (t, 1H), δ 3.21 (s, 3H), δ 2.86 (t, 2H), δ 2.10-2.02 (m, 4H), δ 1.77-1.68 (m, 1H), δ 1.68-1.58 (m, 1H), δ 1.58-1.50 (m, 1H), δ 1.50-1.40 (m, 1H), δ 1.40-1.20 (m, 14H), δ 1.03-0.99 (t, 9H), δ 0.90-0.80 (m, 3H), δ 0.67-0.62 (q, 6H)

### Example 54

### Synthesis of triethyl(((9Z,12Z,15Z)-1-methoxyoctadeca-9,12,15-trien-1-yl)oxy)silane

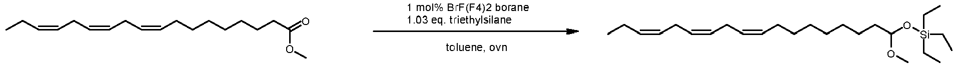

Triethyl(((9Z,12Z,15Z)-1-methoxyoctadeca-9,12,15-trien-1-yl)oxy)silane was prepared from methyl (9Z,12Z,15Z)-octadeca-9,12,15-trienoate (11.55 g, 39.50 mmol) and triethylsilane (4.73 g, 40.68 mmol) using 191 mg (0.39 mmol) BrF(F4)₂ borane catalyst according to **General Procedure F.** The reaction mixture was stirred overnight at 30°C. The reaction was monitored by TLC (hexane/ethyl acetate 9:1). After purification the product was obtained as a yellowish oil triethyl(((9Z,12Z,15Z)-1-methoxyoctadeca-9,12,15-trien-1-yl)oxy)silane (15.81 g, 38.68 mmol, 98 % yield)

¹H NMR (500 MHz, Benzene-d₆) δ 5.32-5.17 (m, 6H), δ 4.56 (t, 1H), δ 3.02 (s, 3H), δ 2.72-2.60 (m, 4H), δ 1.93-1.75 (m, 4H), δ 1.64-1.39 (m, 2H), δ 1.36-1.22 (m, 2H), δ 1.16-1.01 (m, 8H), δ 0.84 (t, 9H), δ 0.72 (m, 3H), δ 0.50-0.42 (q, 6H)

### Example 55

### Synthesis of (((5Z,8Z,11Z,14Z,17Z)-1-ethoxyicosa-5,8,11,14,17-pentaen-1-yl)oxy)triethylsilane

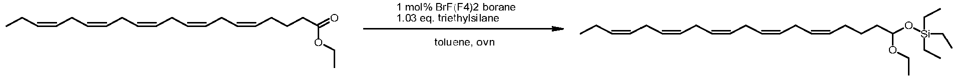

(((5Z,8Z,11Z,14Z,17Z)-1-ethoxyicosa-5,8,11,14,17-pentaen-1-yl)oxy)triethylsilane was prepared from ethyl (5Z,8Z,11Z,14Z,17Z)-icosa-5,8,11,14,17-pentaenoate (13.28 g, 40.17 mmol) and triethylsilane (4.81 g, 41.37 mmol) using 194 mg (0.40 mmol) BrF(F4)₂ borane catalyst according to **General Procedure** F. The reaction was monitored by ¹H NMR. The product was obtained as a yellowish oil (((5Z,8Z,11Z,14Z,17Z)-1-ethoxyicosa-5,8,11,14,17-pentaen-1-yl)oxy)triethylsilane (17.01 g, 38.08 mmol, 95 % yield)

¹H NMR (500 MHz, Benzene-d₆) δ 5.52-5.39 (m, 10H), δ 4.82 (t, 1H), δ 3.70-3.64 (dp, 1H), δ 3.37-3.31 (dp, 1H), δ 2.91-2.80 (m, 8H), δ 2.16-2.07 (m, 2H), δ 2.07-1.98 (m, 2H), δ 1.83-1.74 (m, 1H), δ 1.74-1.65 (m, 1H), δ 1.65-1.54 (m, 2H), δ 1.15 (t, 3H), δ 1.04 (t, 9H), δ 0.92 (t, 3H), δ 0.67 (q, 6H)

### Example 56

### Synthesis of triethyl(((4R)-1-methoxy-4-((3R,10S,13R)-3-methoxy-10,13-dimethylhexadecahydro-1H-cyclopenta[a]phenanthren-17-yl)pentyl)oxy)silane

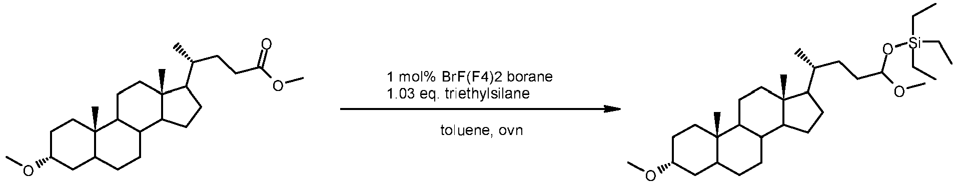

Triethyl(((4R)-1-methoxy-4-((3R,10S,13R)-3-methoxy-10,13-dimethylhexadecahydro-1H-cyclopenta[a]phenanthren-17-yl)pentyl)oxy)silane was prepared from methyl (4R)-4-((3R,10S,13R)-3-methoxy-10,13-dimethylhexadecahydro-1H-cyclopenta[a]phenanthren-17-yl)pentanoate (1.82 g, 4.50 mmol) and triethylsilane (0.57 g, 4.95 mmol) using 21.72 mg (0.045 mmol) BrF(F4)₂ borane catalyst according to **General Procedure F.** The reaction was monitored by TLC (hexane/ethyl acetate 9:1, PMA visualization). The crude product was purified by flash column chromatography with gradient elution (0-10 % EtOAc in n-hexane) to give a white solid triethyl(((4R)-1-methoxy-4-((3R,10S,13R)-3-methoxy-10,13-dimethylhexadecahydro-1H-cyclopenta[a]phenanthren-17-yl)pentyl)oxy)silane (0.84 g, 1.61 mmol, 36 % yield)

¹H NMR (500 MHz, Benzene-d₆) δ 4.81-476 (m, 1H), δ 4.76-4.68 (m, 1H), δ 3.41 (s, 3H), δ 3.25 (d, 3H), δ 1.95-1.10 (m, 25H), δ 1.10-1.02 (t, 12H), δ 1.00 (d, 3H), δ 0.75 (s, 3H), δ 0.71-0.66 (dq, 6H), δ 0.57 (s, 3H)

### Example 57

### Synthesis of 6-methoxy-6-((triethylsilyl)oxy)hexyl 2-(decylthio)hexanoate

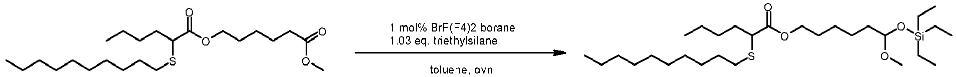

6-methoxy-6-((triethylsilyl)oxy)hexyl 2-(decylthio)hexanoate was prepared from 6-methoxy-6-oxohexyl 2-(decylthio)hexanoate (10.1 g, 24.24 mmol) and triethylsilane (3.10 g, 26.66 mmol) using 117 mg (0.24 mmol) BrF(F4)₂ borane catalyst according to **General Procedure F.** The reaction was monitored by TLC (hexane/ethyl acetate 9:1, PMA visualization). The crude product was purified by flash column chromatography with gradient elution (0-10 % EtOAc in n-hexane) yielding a transparent oil 6-methoxy-6-((triethylsilyl)oxy)hexyl 2-(decylthio)hexanoate (4.35 g, 8.16 mmol, 34 % yield).

¹H NMR (500 MHz, Benzene-d₆) δ 4.69 (t, 1H), δ 4.12-4.03 (m, 2H), δ 3.29 (dt, 1H), δ 3.19 (s, 3H) δ 3.75-3.65 (m, 1H), δ 3.65-3.57 (m, 1H), δ 2.06-1.95 (m, 1H), δ 1.79-1.77 (m, 1H), δ 1.77-1.63 (m, 1H), δ 1.62-1.54 (m, 3H), δ 1.53-1.46 (m, 2H), δ 1.43-1.14 (m, 22H), δ 1.02 (t, 9H), δ δ 0.90 (t, 3H), δ 0.80 (t, 3H), δ 0.64 (q, 6H)

### Example 58

### Synthesis of 6-methoxy-6-((triethylsilyl)oxy)hexyl 2-(pentylthio)decanoate

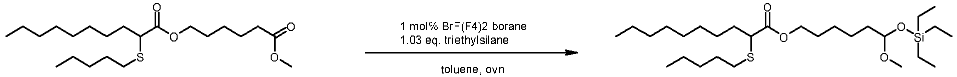

6-methoxy-6-((triethylsilyl)oxy)hexyl 2-(pentylthio)decanoate was prepared from 6-methoxy-6-oxohexyl 2-(pentylthio)decanoate (9.00 g, 22.35 mmol) and triethylsilane (2.86 g, 24.59 mmol) using 108 mg (0.22 mmol) BrF(F4)₂ borane catalyst according to **General Procedure F.** The reaction mixture was stirred overnight at room temperature. The reaction was monitored by TLC (hexane/ethyl acetate 9:1, PMA visualization). The crude product was purified by flash column chromatography with gradient elution (0-1 % EtOAc in n-hexane) to give a transparent oil 6-methoxy-6-((triethylsilyl)oxy)hexyl 2-(pentylthio)decanoate (5.79 g, 11.16 mmol, 50 % yield).

¹H NMR (500 MHz, Benzene-d₆) δ 4.69 (t, 1H), δ 4.14-4.03 (m, 2H), δ 3.31(dt, 1H), δ 3.19 (s, 3H) δ 3.73-3.64 (m, 1H), δ 3.62-3.53 (m, 1H), δ 2.09-1.98 (m, 1H), δ 1.84-1.72 (m, 1H), δ 1.70-1.62 (m, 1H), δ 1.62-1.46 (m, 5H), δ 1.46-1.33 (m, 4H), δ 1.31-1.12 (m, 16H), δ 1.02 (t, 9H), δ δ 0.89 (t, 3H), δ 0.81 (t, 3H), δ 0.64 (q, 6H)

### Example 59

### Synthesis of 6-ethoxy-6-((triethylsilyl)oxy)hexyl 2-hexyldecanoate

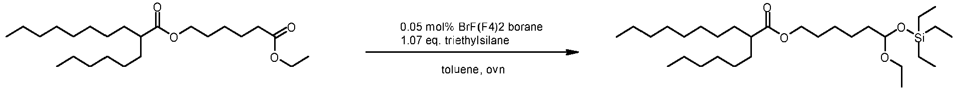

In a dried 100 ml flask equipped with a stirring bar, rubber septum, thermometer and nitrogen inlet, under inert conditions (nitrogen gas), 6-ethoxy-6-oxohexyl 2-hexyldecanoate (23.9 g, 600 mmol, 1.0 eq.) was dissolved in abs. toluene (30 mL). Next, a toluic solution of the catalyst, BrF(F4)₂ (14.49 mg in 600 µL abs. toluene) was added. Finally, triethylsilane (7.47 g, 10.3 mL, 64.2 mmol, 1.07 eq.) was added dropwise, keeping the temperature of the reaction between 24°C and 28°C. Sometimes, during the beginning of the reaction, evolution of hydrogen gas is observable, indicating the presence of small amounts of residual water in the system, but this does not affect the outcome of the reaction. The reaction was further stirred at room temperature overnight (approx. 20 hours). Monitoring of the reaction is done by TLC and NMR After reaction completion 1 ml EtOAc, 0.6 ml MeCN and silica gel were added to the reaction mixture and stirred for further 10 mins and filtered off the silica gel and washed with 2×50 ml n-pentane. Volatiles were removed under reduced pressure to obtain the product which was used without further purification (29.00 g, 57.89 mmol, 97 % yield).

¹H NMR (500 MHz, Benzene-d₆) δ 4.79 (t, 1H), δ 4.10 (t, 2H), δ 3.66 (m, 1H), δ 3.32 (m, 1H), δ 2.50-2.39 (m, 1H), δ 1.86-1.20 (br. m, 32H), δ 1.15 (t, 3H), δ 1.04 (t, 9H), δ .91 (q, 6H), δ .67 (q, 6H),

### Example 60

### Synthesis of 1-bromo-10-heptyl-8,8-dimethyl-7,9,11-trioxa-8-silaoctadecane

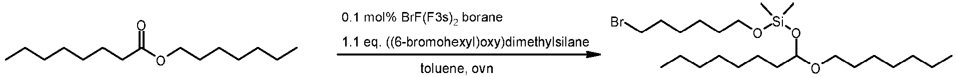

1-bromo-10-heptyl-8,8-dimethyl-7,9,11-trioxa-8-silaoctadecane was prepared from heptyl octanoate (727.19 mg, 3.00 mmol, 1.0 eq.) and ((6-bromohexyl)oxy)dimethylsilane (789.44 mg, 3.30 mmol, 1.1 eq.) using 100 µl BrF(F3s)₂ borane catalyst solution (1.34 mg was dissolved in 100 µl abs. toluene) according to **General Procedure E.** The reaction was completed after overnight stirring. The crude product that was purified by flash column chromatography with gradient elution (20 % - 66 % DCM in petroleum ether) yielding a transparent oil 1-bromo-10-heptyl-8,8-dimethyl-7,9,11-trioxa-8-silaoctadecane (1.34 g, 2.86 mmol, 95 % yield).

¹H NMR (500 MHz, Benzene-d₆) δ 4.96 (m, 1H), δ 3.84-3.79 (m, 1H), δ 3.66 (dt, 2H), δ 3.43-3.35 (m, 1H), δ 2.96 (t, 2H), δ 1.87-1.77 (m, 2H), δ 1.70-1.10 (br. m, 28H), δ 0.91-0.86 (m, 6H), δ 0.27 (m, 6H)

### Example 61

### Synthesis of 1-bromo-10-heptyl-8,8-dimethyl-7,9,11-trioxa-8-silaicosane

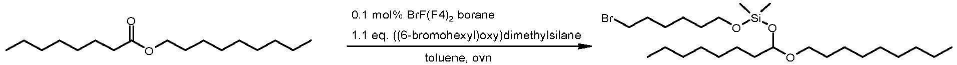

1-bromo-10-heptyl-8,8-dimethyl-7,9,11-trioxa-8-silaicosane was prepared from nonyl octanoate (811.35 mg, 3.00 mmol, 1.0 eq.) and ((6-bromohexyl)oxy)dimethylsilane (789. 44 mg, 3.30 mmol, 1.1 eq.) using 100 µl BrF(F4)₂ borane catalyst solution (1.45 mg was dissolved in 100 µl abs. toluene) according to **General Procedure E.** The reaction was completed after overnight stirring (TLC petroleum ether: chloroform 1:1, ~0.4 Rf). The crude product that was purified by flash column chromatography with gradient elution (20 % - 66% DCM in petroleum ether) yielding a transparent oil 1-bromo-10-heptyl-8,8-dimethyl-7,9,11-trioxa-8-silaicosane (1.32 g, 2.59 mmol, 86 % yield).

¹H NMR (500 MHz, Benzene-d₆) δ 4.99 (dt, 1H), δ 3.89-3.80 (m, 1H), δ 3.68 (t, 2H), δ 3.46-3.37 (m, 1H), δ 2.96 (t, 2H), δ 1.98-1.75 (m, 2H), δ 1.73-1.39 (br. m, 10H), δ 1.38-1.11 (br. m, 22H), δ 0.91 (m, 6H), δ 0.27 (d, 6H)

### Example 62

### Synthesis of 1-bromo-10-hexyl-8,8-dimethyl-7,9,11-trioxa-8-silanonadecane

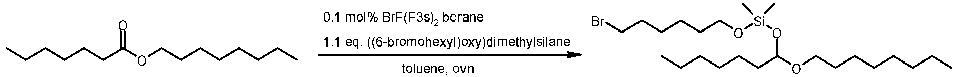

1-bromo-10-hexyl-8,8-dimethyl-7,9,11-trioxa-8-silanonadecane was prepared from octyl heptanoate (727.19 mg, 3.0 mmol) and ((6-bromohexyl)oxy)dimethylsilane (789.44 mg, 3.30 mmol, 1.1 eq.) using 100 µl BrF(F3s)₂ borane catalyst solution (1.34 mg was dissolved in 100 µl abs. toluene) according to **General Procedure E.** The reaction was completed after overnight stirring (TLC petroleum ether: chloroform 1:1, ~0.4 Rf). The crude product that was purified by flash column chromatography with gradient elution (20 % - 66 % DCM in petroleum ether) yielding a transparent oil 1-bromo-10-hexyl-8,8-dimethyl-7,9,11-trioxa-8-silanonadecane (1.26 g, 2.71 mmol, 90 % yield).

¹H NMR (500 MHz, Benzene-d₆) δ 4.98 (dd, 1H), δ 3.87-3.82 (m, 1H), δ 3.68 (t, 2H), δ 3.43-3.38 (m, 1H), δ 2.96 (t, 2H), δ 1.93-1.76 (m, 2H), δ 1.70-1.12 (br. m, 28H), δ 0.92-0.87 (m, 6H), δ 0.26 (d, 6H)

### Example 63

### Synthesis of 1-bromo-8,8-dimethyl-10-octyl-7,9,11-trioxa-8-silanonadecane

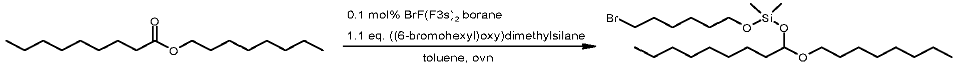

1-bromo-8,8-dimethyl-10-octyl-7,9,11-trioxa-8-silanonadecane was prepared from octyl nonanoate (811.35 mg, 3.0 mmol) and ((6-bromohexyl)oxy)dimethylsilane (789.44 mg, 3.30 mmol, 1.1 eq.) using 100 µl BrF(F3s)₂ borane catalyst solution (1.34 mg was dissolved in 100 µl abs. toluene) according to **General Procedure E.** The reaction was completed after overnight stirring (TLC petroleum ether: chloroform 1:1, ~0.4 Rf). The crude product that was purified by flash column chromatography with gradient elution (20 % - 66 % DCM in petroleum ether) yielding a transparent oil 1-bromo-8,8-dimethyl-10-octyl-7,9,11-trioxa-8-silanonadecane (1.29 g, 2.62 mmol, 87 % yield).

¹H NMR (500 MHz, Benzene-d₆) δ 4.99 (m, 1H), δ 3.88-3.83 (m, 1H), δ 3.68 (t, 2H), δ 3.44-3.39 (m, 1H), δ 2.96 (t, 2H), δ 1.90-1.80 (m, 2H), δ 1.71-1.14 (br. m, 32H), δ 0.92-0.88 (m, 6H), δ 0.27 (d, 6H)

### Example 64

### Synthesis of 1-bromo-8,8-dimethyl-10-nonyl-7,9,11-trioxa-8-silaheptadecane

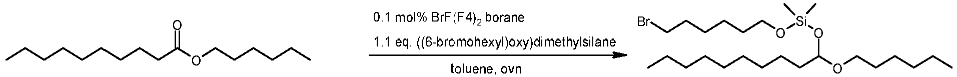

1-bromo-8,8-dimethyl-10-nonyl-7,9,11-trioxa-8-silaheptadecane was prepared from hexyl decanoate (769.3 mg, 3.00 mmol, 1.0 eq.) and ((6-bromohexyl)oxy)dimethylsilane (789.44 g, 3.30 mmol, 1.1 eq.) using 100 µl BrF(F4)₂ borane catalyst solution (1.45 mg was dissolved in 100 µl abs. toluene) according to **General Procedure E.** The reaction was completed after overnight stirring. The crude product that was purified by flash column chromatography with gradient elution (20 - 65 % DCM in petroleum ether) yielding a transparent oil 1-bromo-8,8-dimethyl-10-nonyl-7,9,11-trioxa-8-silaheptadecane (1.20 g, 2.42 mmol, 81 % yield).

¹H NMR (500 MHz, Benzene-d₆) δ 4.98 (m, 1H), δ 3.86-3.79 (m, 1H), δ 3.68 (t, 2H), δ 3.43-3.36 (m, 1H), δ 2.96 (t, 2H), δ 1.95-1.23 (br. m, 32H), δ 0.93-0.86 (m, 6H), δ 0.26 (d, 6H)

### Example 65

### Synthesis of 1-bromo-8,8-dimethyl-10-pentyl-7,9,11-trioxa-8-silahenicosane

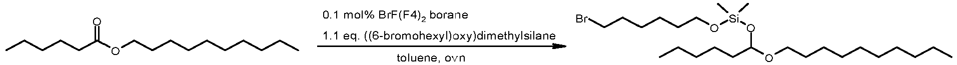

1-bromo-8,8-dimethyl-10-pentyl-7,9,11-trioxa-8-silahenicosane was prepared from decyl hexanoate (769.27 mg, 3.00 mmol) and ((6-bromohexyl)oxy)dimethylsilane ( 789.44 mg, 3.30 mmol, 1.1 eq.) using 100 µl BrF(F4)₂ borane catalyst solution (1.45 mg was dissolved 100 µl abs. toluene) according to **General Procedure** E. The reaction was completed after overnight stirring. The crude product that was purified by flash column chromatography with gradient elution (20 - 65 % DCM in petroleum ether) yielding a transparent oil 1-bromo-8,8-dimethyl-10-pentyl-7,9,11-trioxa-8-silahenicosane (1.04 g, 2.11 mmol, 70 % yield).

¹H NMR (500 MHz, Benzene-d₆) δ 4.98 (m, 1H), δ 3.88-3.81 (m, 1H), δ 3.68 (t, 2H), δ 3.45-3.37 (m, 1H), δ 2.96 (t, 2H), δ 1.95-1.27 (br. m, 32H), δ 0.95-0.84 (m, 6H), δ 0.26 (d, 6H)

### Example 66

### Synthesis of ((6-bromohexyl)oxy)dimethyl((oxacyclohexadecan-2-yl)oxy)silane

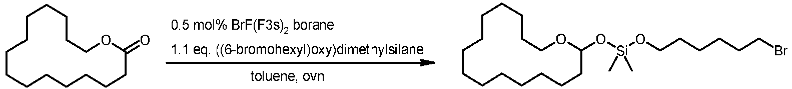

((6-bromohexyl)oxy)dimethyl((oxacyclohexadecan-2-yl)oxy)silane was prepared from oxacyclohexadecan-2-one (1.00 g, 3.827 mmol) and ((6-bromohexyl)oxy)dimethylsilane (1.00 g, 4.21 mmol, 1.1 eq.) using 100 µl BrF(F3s)₂ borane catalyst solution (8.55 mg was dissolved in 100 µl abs. toluene) according to **General Procedure E.** The reaction was completed after overnight stirring. The solvent was evaporated and yielding a transparent oil ((6-bromohexyl)oxy)dimethyl((oxacyclohexadecan-2-yl)oxy)silane (1.83 g, 3.83 mmol, 100 % yield).

¹H NMR (500 MHz, Benzene-d₆) δ 4.80 (dd, 1H), δ 3.85-3.78 (m, 1H), δ 3.68 (t, 2H), δ 3.40 (t, 2H), δ 3.31-3.22 (m, 1H), δ 1.90-1.83 (m, 2H), δ 1.75-1.18 (br. m, 32H), δ 0.15 (d, 6H)

### Example 67

### Synthesis of 1-(4-bromobutoxy)-1,1,3,3-tetramethyl-3-((1-(octyloxy)octyl)oxy)disiloxane

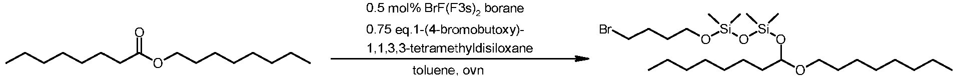

1-(4-bromobutoxy)-1,1,3,3-tetramethyl-3-((1-(octyloxy)octyl)oxy)disiloxane was prepared from octyl octanoate (750 mg, 2.925 mmol) and 1-(4-bromobutoxy)-1,1,3,3-tetramethyldisiloxane (0.75 eq., 625.9 mg, 2.190 mmol) using 100 µl BrF(F3s)₂ borane catalyst solution (6.54 mg was dissolved in 100 µl abs. toluene) according to **General Procedure E.** The reaction was completed after overnight stirring. The crude product that was purified by flash column chromatography with gradient elution (10-90 % EtOAc in petroleum ether) yielding a transparent oil 1-(4-bromobutoxy)-1,1,3,3-tetramethyl-3-((1-(octyloxy)octyl)oxy)disiloxane (1.27 g, 2.35 mmol, 80 % yield).

¹H NMR (500 MHz, Benzene-d₆) δ 4.79 (dd, 1H), δ 3.73-3.62 (m, 2H), δ 3.44 (t, 1H), δ 3.32-3.26 (m, 1H), δ 1.72-1.19 (br. m, 30H), δ 0.89-0.86 (m, 6H), δ 0.15-0.08 (d, 12H)

### Example 68

### Synthesis of 1-bromo-8,8,10-trimethyl-7,9,11-trioxa-8-silahenicosane

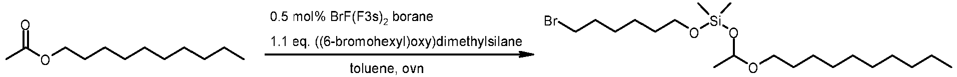

1-bromo-8,8,10-trimethyl-7,9,11-trioxa-8-silahenicosane was prepared from decyl acetate (400.03 mg, 1.997 mmol) and ((6-bromohexyl)oxy)dimethylsilane (477.73 mg, 1.997 mmol, 1.0 eq.) using 100 µl BrF(F3s)₂ borane catalyst solution (8.93 mg was dissolved in 100 µl abs. toluene) according to **General Procedure E.** The reaction was completed after overnight stirring. The crude product that was purified by flash column chromatography with gradient elution (0-100 % DCM and 1 % TEA in petroleum ether) yielding a transparent oil 1-bromo-8,8,10-trimethyl-7,9,11-trioxa-8-silahenicosane (743 mg, 1.69 mmol, 85 % yield).

¹H NMR (500 MHz, Benzene-d₆) δ 5.05 (m, 1H), δ 3.82-3.74 (m, 1H), δ 3.66-3.55 (m, 2H), δ 3.38-3.31 (m, 1H), δ 3.01-2.92 (m, 2H), δ 1.66-1.10 (br. m, 27H), δ 0.91 (t, 3H), δ 0.20 (t, 6H)

### Example 69

### Synthesis of 1-bromo-8,8-dimethyl-10-propyl-7,9,11-trioxa-8-silahenicosane

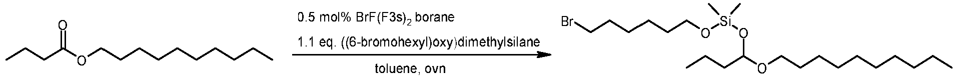

1-bromo-8,8-dimethyl-10-propyl-7,9,11-trioxa-8-silahenicosane was prepared from decyl butanoate (1.00 g, 4.028 mmol, 1.0 eq.) and ((6-bromohexyl)oxy)dimethylsilane (1.060 g, 4.431 mmol, 1.1 eq.) using 100 µl BrF(F3s)₂ borane catalyst solution (9.0 mg was dissolved in 100 µl ml abs. toluene) according to **General Procedure E.** The reaction was completed after overnight stirring. The crude product that was purified by flash column chromatography with gradient elution (20 - 65 % DCM in petroleum ether) yielding a transparent oil 1-bromo-8,8-dimethyl-10-propyl-7,9,11-trioxa-8-silahenicosane (1.88 g, 4.0 mmol, 100 % yield).

¹H NMR (500 MHz, Benzene-d₆) δ 4.81 (dd, 1H), δ 3.70-3.63 (m, 3H), δ 3.40 (t, 2H), δ 3.34-3.29 (m, 1H), δ 1.90-1.83 (m, 2H), δ 1.69-1.21 (br. m, 26H), δ 0.93-0.86 (m, 6H), δ 0.16 (d, 6H)

### Example 70

### Synthesis of 1-bromo-8,8-dimethyl-10-propyl-7,9,11-trioxa-8-silatricosane

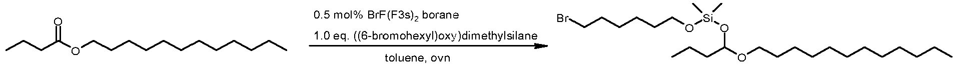

1-bromo-8,8-dimethyl-10-propyl-7,9,11-trioxa-8-silatricosane was prepared from dodecyl butanoate (750 mg, 2.925 mmol, 1.0 eq.) and ((6-bromohexyl)oxy)dimethylsilane (699.70 mg, 2.925 mmol, 1.0 eq.) using 100 µl BrF(F3s)₂ borane catalyst solution (6.54 mg was dissolved in 100 µl abs. toluene) according to **General Procedure E.** The reaction was completed after overnight stirring. The crude product that was purified by flash column chromatography with gradient elution (20 - 65 % DCM in petroleum ether) yielding a transparent oil 1-bromo-8,8-dimethyl-10-propyl-7,9,11-trioxa-8-silatricosane (1.38 g, 2.79 mmol, 96 % yield).

¹H NMR (500 MHz, Benzene-d₆) δ 4.81 (dd, 1H), δ 3.70-3.63 (m, 3H), δ 3.40 (t, 2H), δ 3.34-3.29 (m, 1H), δ 1.90-1.83 (m, 2H), δ 1.68-1.21 (br. m, 30H), δ 0.93-0.86 (m, 6H), δ 0.16 (d, 6H)

### Example 71

### Synthesis of 13-bromo-6,6-dimethyl-4-nonyl-3,5,7-trioxa-6-silatridecane

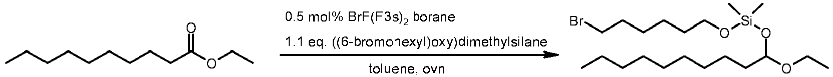

13-bromo-6,6-dimethyl-4-nonyl-3,5,7-trioxa-6-silatridecane was prepared from ethyl decanoate (400 mg, 1.997 mmol, 1.0 eq.) and ((6-bromohexyl)oxy)dimethylsilane (525.46 mg, 2.196 mmol, 1.1 eq.) using 100 µl BrF(F3s)₂ borane catalyst solution (4.46 mg was dissolved in 100 µl abs. toluene) according to **General Procedure E.** The reaction was completed after overnight stirring. The crude product that was filtered through a short silica gel (short column) yielding a transparent oil 13-bromo-6,6-dimethyl-4-nonyl-3,5,7-trioxa-6-silatridecane 883 mg, 1.51 mmol, 75 % yield).

¹H NMR (500 MHz, Benzene-d₆) δ 5.04 (dt, 1H), δ 3.89 (m, 1H), δ 3.76 (t, 2H), δ 3.48 (m, 1H), δ 3.06 (t, 2H), δ 2.01-1.93 (m, 2H), δ 1.70-1.52 (m, 6H), δ 1.51-1.33 (m, 12H), δ 1.28 (m, 7H), δ 1.01 (t, 3H), δ 0.33 (d, 6H)

### Example 72

### Synthesis of 13-bromo-6,6-dimethyl-4-undecyl-3,5,7-trioxa-6-silatridecane

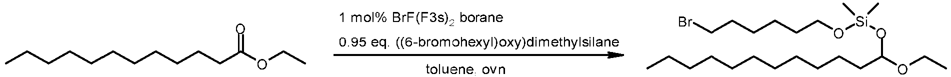

13-bromo-6,6-dimethyl-4-undecyl-3,5,7-trioxa-6-silatridecane was prepared from ethyl dodecanoate (1500 mg, 6.568 mmol, 1.0 eq.) and ((6-bromohexyl)oxy)dimethylsilane (1493 mg, 6.240 mmol, 0.95 eq.) using 100 µl BrF(F3s)₂ borane catalyst solution (29.36 mg was dissolved in 100 µl abs. toluene) according to **General Procedure E.** The reaction was completed after overnight stirring. The crude product that was filtered through a short silica gel (short column) yielding a transparent oil 13-bromo-6,6-dimethyl-4-undecyl-3,5,7-trioxa-6-silatridecane (3.04 g, 5.66 mmol, 86 % yield).

¹H NMR (500 MHz, Toluene-d₈) δ 4.92 (t, 1H), δ 3.81-3.73 (m, 1H), δ 3.63 (t, 2H), δ 3.39-3.32 (m, 1H), δ 2.95 (t, 2H), δ 1.88-1.74 (m, 2H), δ 1.61-1.10 (br. m, 29H), δ 0.90 (t, 3H), δ 0.21 (d, 6H)

### Example 73

### Synthesis of 15-bromo-8,8-dimethyl-6-nonyl-5,7,9-trioxa-8-silapentadecane

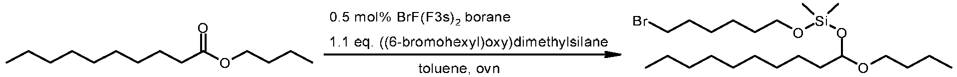

15-bromo-8,8-dimethyl-6-nonyl-5,7,9-trioxa-8-silapentadecane was prepared from butyl decanoate (400 mg, 1.75 mmol) and ((6-bromohexyl)oxy)dimethylsilane (460.91 g, 1.93 mmol, 1.1 eq.) using 100 µl BrF(F3s)₂ borane catalyst solution (7.83 mg was dissolved in 100 µl abs. toluene) according to **General Procedure** E. The reaction was completed after overnight stirring. The crude product that was filtered through a short silica gel (short column) yielding a transparent oil 15-bromo-8,8-dimethyl-6-nonyl-5,7,9-trioxa-8-silapentadecane (745 mg, 1.45 mmol, 83 % yield).

¹H NMR (500 MHz, Benzene-d₆) δ 4.97 (dt, 1H), δ 3.86-3.77 (m, 1H), δ 3.67 (t, 2H), δ 3.43-3.32 (m, 1H), δ 2.96 (t, 2H), δ 1.93-1.76 (m, 2H), δ 1.66-1.10 (br. m, 26H), δ 0.91 (t, 6H), δ 0.25 (d, 6H)

### Example 74

### Synthesis of 15-bromo-8,8-dimethyl-6-undecyl-5,7,9-trioxa-8-silapentadecane

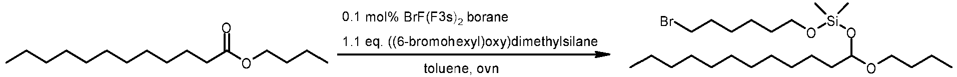

15-bromo-8,8-dimethyl-6-undecyl-5,7,9-trioxa-8-silapentadecane was prepared from butyl dodecanoate (200 mg, 0.78 mmol) and ((6-bromohexyl)oxy)dimethylsilane (205.24 mg, 0.858 mmol, 1.1 eq.) using 100 µl BrF(F3s)₂ borane catalyst solution (0.70 mg was dissolved in 100 µl abs. toluene) according to **General Procedure E.** The reaction was completed after overnight stirring. The crude product that was filtered through a short silica gel (short column) yielding a transparent oil 15-bromo-8,8-dimethyl-6-undecyl-5,7,9-trioxa-8-silapentadecane (415 mg, 0.78 mmol, 100 % yield).

¹H NMR (500 MHz, Benzene-d₆) δ 5.01 (dt, 1H), δ 3.90-3.82 (m, 1H), δ 3.72 (t, 2H), δ 3.46-3.39 (m, 1H), δ 3.02 (t, 2H), δ 2.00-1.79 (m, 2H), δ 1.70-1.15 (br. m, 30H), δ 0.99-0.93 (m, 6H), δ 0.29 (d, 6H)

### Example 75

### Synthesis of 13-bromo-4,6,6-trimethyl-3,5,7-trioxa-6-silatridecane

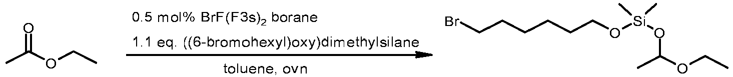

13-bromo-4,6,6-trimethyl-3,5,7-trioxa-6-silatridecane was prepared from ethyl acetate (200 mg, 2.27 mmol) and ((6-bromohexyl)oxy)dimethylsilane ( 597.35 mg, 2.50 mmol, 1.1 eq.) using 100 µl BrF(F3s)₂ borane catalyst solution (5.07 mg was dissolved in 100 µl abs. toluene) according to **General Procedure E.** The reaction was completed after overnight stirring. The crude product that was purified by flash column chromatography with gradient elution (0-30 % EtOAc and 1 % TEA in petroleum ether) yielding a transparent oil 13-bromo-4,6,6-trimethyl-3,5,7-trioxa-6-silatridecane (325 mg, 0.99 mmol, 44 % yield).

¹H NMR (500 MHz, Benzene-d₆) δ 5.01 (q, 1H), δ 3.80-3.66 (m, 1H), δ 3.61 (t, 2H), δ 3.36-3.26 (m, 1H), δ 2.95 (t, 2H), δ 1.56-1.36 (m, 7H), δ 1.20-1.10 (m, 7H), δ 0.18 (s, 6H)

### Example 76

### Synthesis of 1-bromo-9-heptyl-7,7-dimethyl-6,8,10-trioxa-7-silaoctadecane

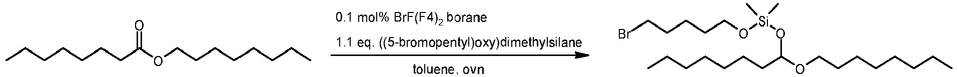

1-bromo-9-heptyl-7,7-dimethyl-6,8,10-trioxa-7- silaoctadecane was prepared from octly octanoate (1.54 mg, 6.0 mmol) and ((5-bromopentyl)oxy)dimethylsilane (1.486 mg, 6.6 mmol, 1.1 eq.) using 100 µl BrF(F3s)₂ borane catalyst solution (2.9 mg was dissolved in 100 µl abs. toluene) according to **General Procedure E.** The reaction was completed after overnight stirring. The crude product that was purified by flash column chromatography with gradient elution (0-5 % EtOAc in petroleum ether) yielding a transparent oil 1-bromo-9-heptyl-7,7-dimethyl-6,8,10-trioxa-7-silaoctadecane (2.63 g, 5.46 mmol, 91 % yield).

¹H NMR (500 MHz, Benzene-d₆) δ 5.08 (q, 1H), δ 3.97-3.90 (m, 1H), δ 3.73 (t, 2H), δ 3.54-3.47 (m, 1H), δ 3.07 (t, 2H), δ 2.05-1.84 (m, 2H), δ 1.81-1.27 (br. m, 28H), δ 1.05-0.95 (m, 6H), δ 0.35 (d, 6H)

### Example 77

### Synthesis of 1-bromo-10-heptyl-8,8-dimethyl-7,9-dioxa-11-thia-8-silaoctadecane

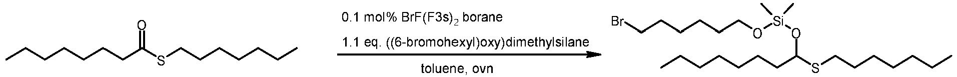

1-bromo-10-heptyl-8,8-dimethyl-7,9-dioxa-11-thia-8-silaoctadecane was prepared from octyl octanthioate (817.47 mg, 3.00 mmol) and ((6-bromohexyl)oxy)dimethylsilane (789.44 mg, 3.30 mmol, 1.1 eq.) using 100 µl BrF(F3s)₂ borane catalyst solution (1.34 mg was dissolved in 100 µl abs. toluene) according to **General Procedure E.** The reaction was completed after overnight stirring. The crude product that was purified by flash column chromatography with gradient elution (20 - 65 % DCM in petroleum ether) yielding a transparent oil 1-bromo-10-heptyl-8,8-dimethyl-7,9-dioxa-11-thia-8-silaoctadecane (1.40 g, 2.74 mmol, 91 % yield).

¹H NMR (500 MHz, Benzene-d₆) δ 5.09 (t, 1H), δ 3.74-3.67 (m, 2H), δ 3.00-2.92 (m, 2H), δ 2.81-3.63 (m, 2H), δ 2.09-1.85 (m, 2H), δ 1.75-1.06 (br. m, 24H), δ 0.92-0.81 (m, 10H), δ 0.29 (d, 6H)

### Example 78

### Synthesis of (Z)-1-bromo-10-heptyl-8,8-dimethyl-7,9,11-trioxa-8-silaicos-17-ene

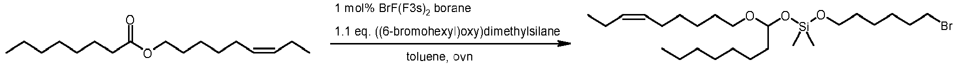

(Z)-1-bromo-10-heptyl-8,8-dimethyl-7,9,11-trioxa-8-silaicos-17-ene was prepared from (Z)-non-6-en-1-yl octanoate (1.0 g, 3.725 mmol) and ((6-bromohexyl)oxy)dimethylsilane (891.18 mg, 3.725 mmol, 1.0 eq.) using 100 µl BrF(F3s)₂ borane catalyst solution (16.65 mg was dissolved in 100 µl abs. toluene) according to **General Procedure E.** The reaction was completed after overnight stirring. The crude product that was filtered through a short silica gel (short column) yielding a transparent oil (Z)-1-bromo-10-heptyl-8,8-dimethyl-7,9,11-trioxa-8-silaicos-17-ene (1.66 g, 3.14 mmol, 84 % yield).

¹H NMR (500 MHz, Benzene-d₆) δ 5.49-5.36 (m, 2H), δ 4.97 (dd, 1H), δ 3.86-3.79 (m, 1H), δ 3.67 (t, 2H), δ 3.42-3.34 (m, 1H), δ 2.96 (t, 2H), δ 2.08-1.97 (m, 4H), δ 1.92-1.75 (m, 2H), δ 1.69-1.08 (br. m, 24H), δ 0.96-0.87 (m, 6H), δ 0.25 (d, 6H)

### Example 79

### Synthesis of (Z)-1-bromo-10-heptyl-8,8-dimethyl-7,9,11-trioxa-8-silaicos-14-ene

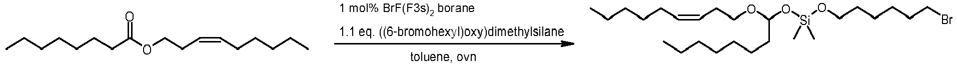

(Z)-1-bromo-10-heptyl-8,8-dimethyl-7,9,11-trioxa-8-silaicos-14-ene was prepared from (Z)-non-3-en-1-yl octanoate (xxx g, xxx mmol) and ((6-bromohexyl)oxy)dimethylsilane (891.18 mg, 3.725 mmol, 1.0 eq.) using 100 µl BrF(F3s)₂ borane catalyst solution (16.65 mg was dissolved in 100 µl abs. toluene) according to **General Procedure E.** The reaction was completed after overnight stirring. The crude product that was filtered through a short silica gel (short column) yielding a transparent oil (Z)-1-bromo-10-heptyl-8,8-dimethyl-7,9,11-trioxa-8-silaicos-14-ene (1.81 g, 3.30 mmol, 89 % yield).

¹H NMR (500 MHz, Benzene-d₆) δ 5.63-5.49 (m, 2H), δ 4.98 (dd, 1H), δ 3.90-3.82 (m, 1H), δ 3.67 (t, 2H), δ 3.48-3.40 (m, 1H), δ 2.96 (t, 2H), δ 2.49-2.43 (m, 2H), δ 2.11-2.03 (m, 2H), δ 1.61-1.40 (m, 6H), δ 1.38-1.10 (br. m, 20H), δ 0.96-0.84 (m, 6H), δ 0.25 (d, 6H)

### Example 80

### Synthesis of 10-bromo-3-((8Z,11Z)-heptadeca-8,11-dien-1-yl)-5,5-dimethyl-2,4,6-trioxa-5-siladecane

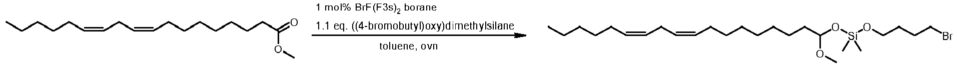

10-bromo-3-((8Z,11Z)-heptadeca-8,11-dien-1-yl)-5,5-dimethyl-2,4,6-trioxa-5-siladecane was prepared from methyl (9Z,12Z)-octadeca-9,12-dienoate (395.00 mg, 1.341 mmol) and ((4-bromobutyl)oxy)dimethylsilane (311.59 mg, 1.476 mmol, 1.1 eq.) using 100 µl BrF(F3s)₂ borane catalyst solution (6.00 mg was dissolved in 100 µl abs. toluene) according to **General Procedure E.** The reaction was completed after overnight stirring. The crude product that was purified by flash column chromatography with gradient elution (0-100 % EtOAc in n-hexane) yielding a transparent oil 10-bromo-3-((8Z,11Z)-heptadeca-8,11-dien-1-yl)-5,5-dimethyl-2,4,6-trioxa-5-siladecane (390 mg, 0.77 mmol, 58 % yield).

¹H NMR (500 MHz, Benzene-d₆) δ 5.53-5.40 (m, 4H), δ 4.77 (t, 1H), δ 3.52 (t, 2H), δ 3.24 (s, 3H), δ 3.01 (t, 2H), δ 2.87 (t, 2H), δ 2.12-2.01 (m, 4H), δ 1.85-1.61 (m, 2H), δ 1.53-1.07 (br. m, 20H), δ 0.94-0.79 (m, 3H), δ 0.15 (s, 6H)

### Chromatography methods for the purification of the lipids and intermediates

### Purification Method A

Eluent A: DCM:MeOH:aq. NH₃ 80:20:1, eluent B: DCM; full gradient A:B 0:100 -> 100:0. Sample loading ~200 mg; column size: 25 g of SiO₂. Column conditioned with ~400 mL of A and then with ~400 mL of B prior loading the sample.

### Purification Method B

Eluent A: EtOAc, eluent B: petroleum ether +1% Et₃N; full gradient A:B 0:100 -> 100:0. Sample loading ~200 mg; column size: 25 g of SiO₂. Column conditioned with ~400 mL of B prior loading the sample.

### Synthetic methods for the synthesis of the intermediates and lipids (general procedure G-P)

### General Procedure G

A MW reaction vial was charged with 1.0 eq. ω-bromo alkyl silyl mixed acetal and 1.0 eq. amine, abs. acetonitrile (1.0 mmol ω-bromo alkyl silyl mixed acetal/1.54 ml of abs. acetonitrile) and abs. cyclopentyl methyl ether (1.0 mmol ω-bromo alkyl silyl mixed acetal/0.51 ml of abs. cyclopentyl methyl ether). To the stirred reaction mixture K₂CO₃ (2.0 eq.) and KI (0.5 eq.) were added and purged with argon and sealed. The resulting mixture was stirred for 8 hours at 80°C. Then the reaction mixture was cooled down to room temperature and partitioned between ethyl acetate and brine and stirred vigorously for 15 minutes. The organic phase was separated and dried over Na₂SO₄, filtered and concentrated.

### General Procedure H

A MW reaction vial was charged with 2.5 eq. ω-bromo alkyl silyl mixed acetal and 1.0 eq. amine, 2.5 eq. diisopropyl ethyl amine, abs. ethanol (1.0 mmol ω-bromo alkyl silyl mixed acetal/2.4 ml of abs. ethanol) and purged with argon and sealed. The resulting mixture was stirred for 18 hours at 63°C. Then the reaction mixture was cooled down to room temperature and partitioned between ethyl acetate and brine and stirred vigorously for 15 minutes. The organic phase was separated and dried over Na₂SO₄, filtered and concentrated.

### General Procedure I

A MW reaction vial was charged with 2.5 eq. ω-bromo alkyl silyl mixed acetal and 1.0 eq. amine, abs. acetonitrile (1.0 mmol ω-bromo alkyl silyl mixed acetal/0.95 ml of abs. acetonitrile) and abs. cyclopentyl methyl ether (1.0 mmol ω-bromo alkyl silyl mixed acetal/0.31 ml of abs. cyclopentyl methyl ether). To the stirred reaction mixture K₂CO₃ (3.0 eq.) and KI (0.2 eq.) were added and purged with argon and sealed. The resulting mixture was stirred for 18 hours at 60°C. Then the reaction mixture was cooled down to room temperature and partitioned between ethyl acetate and brine and stirred vigorously for 15 minutes. The organic phase was separated and dried over Na₂SO₄, filtered and concentrated.

### General Procedure J

A MW reaction vial was charged with 1.0 eq. aldehyde, 1.0 eq. amine, 1.0 eq. acetic acid and abs. toluene (1.0 mmol aldehyde/6.5 ml of abs. toluene). After 15 minutes stirring 3.0 eq. sodium triacetoxyborohydride was added and the resulting mixture was stirred for overnight. Then the reaction mixture was diluted with ethyl acetate and NaHCO₃ solution and stirred vigorously for 5 minutes and the phases were separated. The organic phase was washed with brine, separated and dried over Na₂SO₄, filtered and concentrated.

### General Procedure K

A MW reaction vial was charged with ω-bromo alkyl silyl mixed acetal, amine, abs. acetonitrile (1.0 mmol ω-bromo alkyl silyl mixed acetal/4.0 ml of abs. acetonitrile) and abs. cyclopentyl methyl ether (ω-bromo alkyl silyl mixed acetal/1.33 ml of abs. cyclopentyl methyl ether). To the stirred reaction mixture K₂CO₃ (2.0 eq.) and KI (0.2 eq.) were added and purged with argon and sealed. The resulting mixture was stirred for 18 hours at 62°C. Then the reaction mixture was cooled down to room temperature and partitioned between ethyl acetate and brine and stirred vigorously for 15 minutes. The organic phase was separated and dried over Na₂SO₄, filtered and concentrated.

### General Procedure L

A MW reaction vial was charged with 1.0 eq. ω-bromo alkyl silyl mixed acetal, 20 eq. amine, 2.0 eq. K₂CO₃ and abs. ethanol (1.0 mmol ω-bromo alkyl silyl mixed acetal/0.32 ml of abs. ethanol) and purged with argon and sealed. The resulting mixture was stirred for overnight at room temperature. Then the reaction mixture was partitioned between ethyl acetate and brine and stirred vigorously for 15 minutes. The organic phase was separated and dried over Na₂SO₄, filtered and concentrated.

### General Procedure M

A MW reaction vial was charged with 1.0 eq. mixed silyl acetal, 3.0 eq. amine, tetrahydrofuran (1.0 mmol mixed silyl acetal /4.5 ml of tetrahydrofuran) and water (1.0 mmol mixed silyl acetal /0.9 ml of water). To the reaction mixture 3.0 eq. trifluoroacetic acid was added and stirred for 6 hours. Then 2.3 eq. sodium triacetoxyborohydride was added to the reaction mixture and stirred for overnight at 60°C. The reaction mixture was cooled down to room temperature and diluted with sat. NaHCO₃ solution and extracted with ethyl acetate. The organic phase was washed with brine, separated and dried over Na₂SO₄, filtered and concentrated.

### General Procedure N

A MW reaction vial was charged with 1.0 eq. ω-bromo alkyl ester, amine, 0.2 eq. KI, 2.2 eq. K₂CO₃ and abs. DMF (1.0 mmol ω-bromo alkyl ester /8.73 ml of abs. DMF) and purged with argon and sealed. The resulting mixture was stirred for 18 h at 63°C. Then the reaction mixture was cooled down to room temperature and diluted with sat. NaHCO₃ solution and extracted with ethyl acetate. The organic phase was washed with sat. NaHCO₃ solution, brine, separated and dried over Na₂SO₄, filtered and concentrated.

### General Procedure O

A MW reaction vial was charged with 2.5 eq. ω-bromo alkyl silyl mixed acetal, 1.0 eq. amine, 3.0 eq. K₂CO₃ 0.2 eq. KI and abs. DMF (1.0 mmol ω-bromo alkyl silyl mixed acetal/2.6 ml of abs. DMF) and purged with argon and sealed. The resulting mixture was stirred for 24 h at 64°C. Then the reaction mixture was concentrated to dryness under vacuum and the residue was purified by flash column chromatography.

### General Procedure P

A MW reaction vial was charged with 1.0 eq. propargyl amine derivative, 1.5 eq. azide derivative, 0.33 eq. CuBr, 0.33 eq. 1,1,4,7,7-Pentamethyldiethylenetriamine and abs. DMF (0.1 mmol propargyl amine derivative/3.0 ml of abs. DMF) and purged with argon and sealed. The resulting mixture was stirred for 20 h at 50°C. Then the reaction mixture was concentrated to dryness under vacuum and the residue was purified by flash column chromatography.

### Examples of the intermediate synthesis

### Example 81

### Synthesis of 4-((6-((dimethyl(octyl)silyl)oxy)-6-(octyloxy)hexyl)amino)butan-1-ol

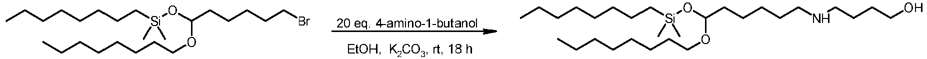

4-((6-((dimethyl(octyl)silyl)oxy)-6-(octyloxy)hexyl)amino)butan-1-ol was prepared from ((6-bromo-1-(octyloxy)hexyl)oxy)dimethyl(octyl)silane (300.0 mg, 0.625 mmol, 1.0 eq.) and 4-amino-1-butanol (1115.01 mg, 12.51 mmol, 20.0 eq.) according to **General Procedure** L. The crude product was purified by flash column chromatography according to **Purification Method** A to obtain the pure product as a colourless oil, 100.0 mg, 0.205 mmol, 33 % yield.

¹H NMR (500 MHz, Benzene-d₆) δ 4.89 (t, 1H), δ 3.78-3.73 (m, 1H), δ 3.65-3.62 (m, 2H), δ 3.39-3.34 (m, 1H), δ 2.31-2.27 (m, 4H), δ 1.83-1.20 (m, 36H), δ 0.94-0.88 (m, 6H), δ 0.76-0.72 (m, 2H), δ 0.26 (d, 6H)

### Example 82

### Synthesis of 13-heptyl-15,15-dimethyl-12,14-dioxa-5-aza-15-silatricosan-1-ol

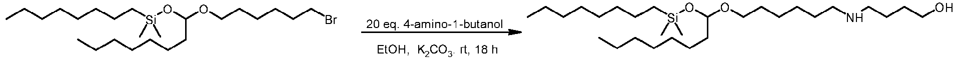

13-heptyl-15,15-dimethyl-12,14-dioxa-5-aza-15-silatricosan-1-ol was prepared from ((1-((6-bromohexyl)oxy)octyl)oxy)dimethyl(octyl)silane (300.0 mg, 0.625 mmol, 1.0 eq.) and 4-amino-1-butanol (1115.01 mg, 12.51 mmol, 20.0 eq.) according to **General Procedure L.** The crude product was purified by flash column chromatography according to **Purification Method A** to obtain the pure product as a colourless oil, 95.0 mg, 0.195 mmol, 31 % yield.

¹H NMR (500 MHz, Benzene-d₆) δ 4.89 (t, 1H), δ 3.77-3.71 (m, 1H), δ 3.64-3.61 (m, 2H), δ 3.39-3.32 (m, 1H), δ 2.30-2.29 (m, 4H), δ 1.87-1.18 (m, 36H), δ 0.92-0.88 (m, 6H), δ 0.75-0.71 (m, 2H), δ 0.25 (d, 6H)

### Example 83

### Synthesis of 4-((6-(octyloxy)-6-((1,1,3,3-tetramethyl-3-(octyloxy)disiloxaneyl)oxy)hexyl)amino)butan-1-ol

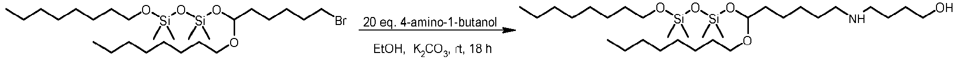

4-((6-(octyloxy)-6-((1,1,3,3-tetramethyl-3-(octyloxy)disiloxaneyl)oxy )hexyl)amino)butan-1-ol was prepared from 1-((6-bromo-1-(octyloxy)hexyl)oxy)-1,1,3,3-tetramethyl-3-(octyloxy)disiloxane (100.00 mg, 0.175 mmol, 1.0 eq.) and 4-amino-1-butanol (312.87 mg, 3.509 mmol, 20.0 eq.) according to **General Procedure L.** The crude product was purified by flash column chromatography according to **Purification Method A** to obtain the pure product as a colourless oil, 75.0 mg, 0.130 mmol, 74 % yield.

¹H NMR (500 MHz, Benzene-d₆) δ 5.03 (t, 1H), δ 3.93-3.84 (m, 1H), δ 3.78-3.75 (t, 2H), δ 3.64-3.61 (m, 2H), δ 3.49-3.35 (m, 1H), δ 2.36-2.25 (m, 4H), δ 1.93-1.17 (m, 36H), δ 0.92-0.88 (m, 6H), δ 0.31 (d, 6H), δ 0.25 (s, 6H)

### Example 84

### Synthesis of 15-heptyl-13,13-dimethyl-12,14,16-trioxa-5-aza-13-silatetracosan-1-ol

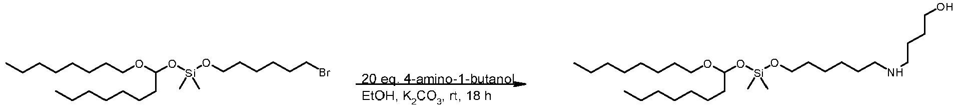

15-heptyl-13,13-dimethyl-12,14,16-trioxa-5-aza-13-silatetracosan-1-ol was prepared from 1-bromo-10-heptyl-8,8-dimethyl-7,9,11-trioxa-8-silanonadecane (500.0 mg, 1.010 mmol, 1.0 eq.) and 4-amino-1-butanol (1.798 g, 20.175 mmol, 20.0 eq.) according to **General Procedure L.** The crude product was purified by flash column chromatography according to **Purification Method A** to obtain the pure product as a colourless oil, 200.0 mg, 0.397 mmol, 40 % yield.

¹H NMR (500 MHz, Benzene-d₆) δ 5.01 (t, 1H), δ 3.93-3.79 (m, 1H), δ 3.75 (t, 2H), δ 3.61 (t, 1H), δ 3.44-3.40 (m, 2H), δ 2.32-2.27 (m, 2H), δ 1.96-1.14 (m, 38H), δ 0.93-0.87 (m, 6H), δ 0.28 (d, 6H)

### Example 85

### Synthesis of 4-(((5Z,8Z,11Z,14Z,17Z)-icosa-5,8,11,14,17-pentaen-1-yl)amino)butan-1-ol

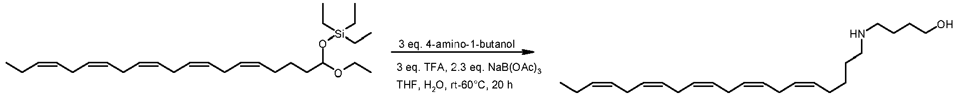

4-(((5Z,8Z,11Z,14Z,17Z)-icosa-5,8,11,14,17-pentaen-1-yl)amino)butan-1-ol was prepared from (((5Z,8Z,11Z,14Z,17Z)-1-ethoxyicosa-5,8,11,14,17-pentaen-1-yl)oxy)triethylsilane (1478.20 mg, 3.309 mmol, 1.0 eq.) and 4-amino-1-butanol (884.74 mg, 9.925 mmol, 3.0 eq.) according to **General Procedure M.** The crude product was purified by flash column chromatography according to **Purification Method A** to obtain the pure product as a colourless oil, 983.0 mg, 2.734 mmol, 83 % yield.

¹H NMR (500 MHz, CDCl₃) δ 5.43-5.27 (m, 10H), δ 398 (broad s, 2H), δ 3.57 (m, 2H), δ 2.88-2.76 (m, 8H), δ 2.68-2.61 (m, 4H), δ 2.12-2.03 (m, 4H), δ 1.71-1.60 (m, 4H), δ 1.57-1.48 (m, 2H), δ 1.43-1.35 (m, 2H), δ 0.97 (t, 3H)

### Example 86

### Synthesis of 6-((4-hydroxybutyl)amino)hexyl 2-hexyldecanoate

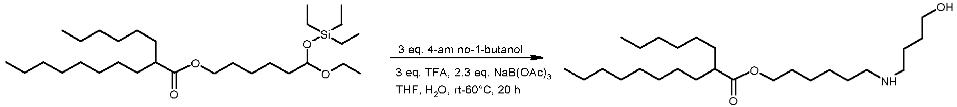

6-((4-hydroxybutyl)amino)hexyl 2-hexyldecanoate was prepared from 6-ethoxy-6-((triethylsilyl)oxy)hexyl 2-hexyldecanoate (1488.4 mg, 2.891 mmol, 1.0 eq.) and 4-amino-1-butanol (772.99 mg, 8.672 mmol, 3.0 eq.) according to **General Procedure M.** The crude product was purified by flash column chromatography according to **Purification Method A** to obtain the pure product as a colourless oil, 1056 mg, 2.469 mmol, 85 % yield.

¹H NMR (500 MHz, CDCl₃) δ 4.05 (t, 2H), δ 3.56 (m, 2H), δ 2.65-2.58 (m, 4H), δ 2.33-2.26 (m, 1H), δ 1.70-1.19 (m, 36H), δ 0.88-0.85 (dt, 6H)

### Example 87

### Synthesis of 6-((3-hydroxypropyl)amino)hexyl 2-hexyldecanoate

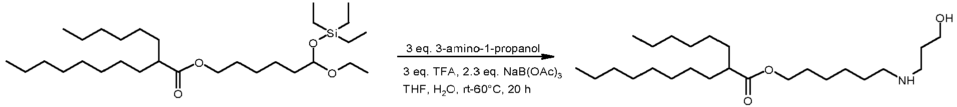

6-((3-hydroxypropyl)amino)hexyl 2-hexyldecanoate was prepared from 6-ethoxy-6-((triethylsilyl)oxy)hexyl 2-hexyldecanoate (1500 mg, 2.913 mmol, 1.0 eq.) and 3-aminopropanol (656.43 mg, 8.740 mmol, 3.0 eq.) according to **General Procedure M.** The crude product was purified by flash column chromatography according to **Purification Method A** to obtain the pure product as a colourless oil, 1265.0 mg, 1.651 mmol, 57 % yield.

¹H NMR (500 MHz, CDCl₃) δ 4.03 (t, 2H), δ 3.79 (t, 2H), δ 2.92 (t, 2H), δ 2.35-2.25 (m, 1H), δ 1.95-1.90 (t, 2H), δ 1.74-1.18 (m, 34H), δ 0.86 (t, 6H)

### Example 88

### Synthesis of 6-((2-hydroxyethyl)amino)hexyl 2-hexyldecanoate

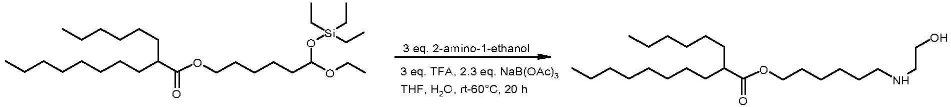

6-((2-hydroxyethyl)amino)hexyl 2-hexyldecanoate was prepared from 6-ethoxy-6-((triethylsilyl)oxy)hexyl 2-hexyldecanoate (1000 mg, 1.942 mmol, 1.0 eq.) and 2-amino-1-ethanol (355.9 mg, 5.827 mmol, 3.0 eq.) according to **General Procedure M.** The crude product was purified by flash column chromatography according to **Purification Method A** to obtain the pure product as a colourless oil, 590.0 mg, 1.476 mmol, 76 % yield.

¹H NMR (500 MHz, CDCl₃) δ 4.04 (t, 2H), δ 3.86 (t, 2H), δ 3.08-3.05 (m, 2H), δ 2.95 (t, 2H), δ 2.34-2.24 (m, 1H), δ 1.77-1.12 (m, 32H), δ 0.86 (t, 6H)

### Example 89

### Synthesis of 6-((2-(1-methylpyrrolidin-2-yl)ethyl)amino)hexyl 2-hexyldecanoate

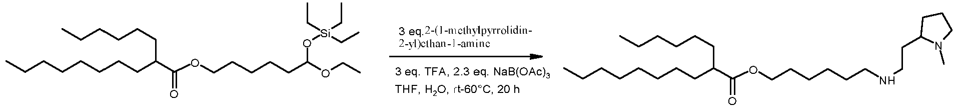

6-((2-(1-methylpyrrolidin-2-yl)ethyl)amino)hexyl 2-hexyldecanoate was prepared from 6-ethoxy-6-((triethylsilyl)oxy)hexyl 2-hexyldecanoate (850 mg, 1.651 mmol, 1.0 eq.) and 2-(1-methylpyrrolidin-2-yl)ethan-1-amine (634.98 mg, 4.953 mmol, 3.0 eq.) according to **General Procedure M.** The crude product was purified by flash column chromatography according to **Purification Method A** to obtain the pure product as a colorless oil, 120.0 mg, 0.257 mmol, 16 % yield.

¹H NMR (500 MHz, CDCl₃) δ 4.03 (t, 2H), δ 3.27-3.22 (m, 1H), δ 3.01-2.87 (m, 2H), δ 2.82-2.75 (m, 1H), δ 2.54 (s, 3H), δ 2.49-2.43 (m, 1H), δ 2.33-2.25 (m, 2H), δ 2.16-2.07 (m, 1H), δ 2.04-1.17 (br. m, 38H), δ 0.86 (t, 6H)

### Example 90

### Synthesis of 6-((4-hydroxybutyl)amino)hexyl 2-(pentvlthio)decanoate

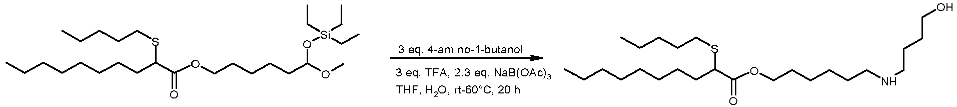

6-((4-hydroxybutyl)amino)hexyl 2-(pentylthio)decanoate was prepared from 6-methoxy-6-((triethylsilyl)oxy)hexyl 2-(pentylthio)decanoate (1500.00 mg, 2.891 mmol, 1.0 eq.) and 4-amino-1-butanol (772.99 mg, 8.672 mmol, 3.0 eq.) according to **General Procedure M.** The crude product was purified by flash column chromatography according to **Purification Method A** to obtain the pure product as a colourless oil, 1123.0 mg, 2.519 mmol, 87 % yield.

¹H NMR (500 MHz, CDCl₃) δ 4.11 (t, 2H), δ 3.56 (t, 2H), δ 3.22-3.19 (m, 1H), δ 2.65-2.42 (m, 6H), δ 1.89-1.80 (m, 1H), δ 1.70-1.16 (m, 31H), δ 0.90-0.85 (dt, 6H)

### Example 91

### Synthesis of 6-((4-hydroxybutyl)amino)hexyl 2-(decylthio)hexanoate

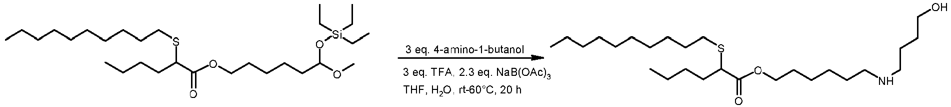

6-((4-hydroxybutyl)amino)hexyl 2-(decylthio)hexanoate was prepared from 6-methoxy-6-((triethylsilyl)oxy)hexyl2-(decylthio)hexanoate (400.00 mg, 0.751 mmol, 1.0 eq.) and 4-amino-1-butanol (200.71 mg, 2.252 mmol, 3.0 eq.) according to **General Procedure M.** The crude product was purified by flash column chromatography according to **Purification Method A** to obtain the pure product as a colorless oil, 211.0 mg, 0.459 mmol, 61 % yield.

¹H NMR (500 MHz, CDCl₃) δ 4.12 (t, 2H), δ 3.56 (m, 2H), δ 3.22-3.19 (m, 1H), δ 2.66-2.51 (m, 6H), δ 1.90-1.81 (m, 1H), δ 1.70-1.20 (m, 33H), δ 0.91-0.86 (dt, 6H)

### Example 92

### Synthesis of 4-(((4R)-4-((3R,10S,13R)-3-methoxy-10,13-dimethylhexadecahydro-1H-cyclopenta[a]phenanthren-17-yl)pentyl)amino)butan-1-ol

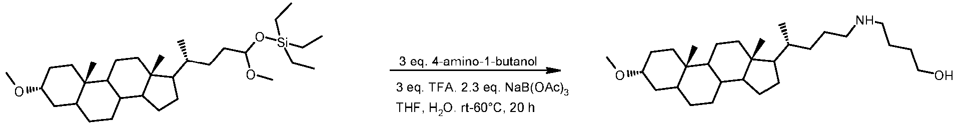

4-(((4R)-4-((3R,10S,13R)-3-methoxy-10,13-dimethylhexadecahydro-1H-cyclopenta[a]phenanthren-17-yl)pentyl)amino)butan-1-ol was prepared from triethyl(((4R)-1-methoxy-4-((3R,10S,13R)-3-methoxy-10,13-dimethylhexadecahydro-1H-cyclopenta[a]phenanthren-17-yl)pentyl)oxy)silane (237.00 mg, 0.455 mmol, 1.0 eq.) and 4-amino-1-butanol (121.67 mg, 1.365 mmol, 3.0 eq.) according to **General Procedure M.** The crude product was purified by flash column chromatography according to **Purification Method A** to obtain the pure product as a colorless oil, 178.0 mg, 0.398 mmol, 87 % yield.

¹H NMR (500 MHz, CDCl₃) δ 4.62 (m, 1H), δ 3.76 (s, 3H), δ 3.57 (t, 2H), δ 2.66-2.63 (m, 2H), δ 2.61-2.49 (m, 2H), δ 1.97-1.02 (m, 32H), δ 0.92-0.89 (m, 6H), δ 0.63 (s, 3H)

### Example 93

### Synthesis of 4-((4-hydroxybutyl)amino)butyl (4R)-4-((3R,10S,13R)-3-methoxy-10,13-dimethylhexadecahydro-1H-cyclopenta[a]phenanthren-17-yl)pentanoate

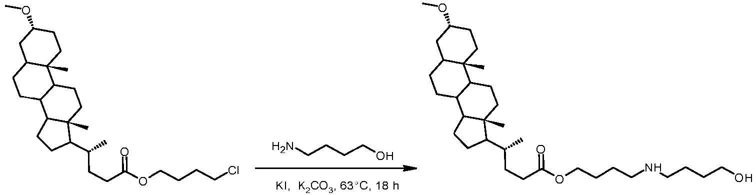

4-((4-hydroxybutyl)amino)butyl (4R)-4-((3R,10S,13R)-3-methoxy-10,13-dimethylhexadecahydro-1H-cyclopenta[a]phenanthren-17-yl)pentanoate was prepared from 4-chlorobutyl (4R)-4-((3R,10S,13R)-3-methoxy-10,13-dimethylhexadecahydro-1H-cyclopenta[a]phenanthren-17-yl)pentanoate (237.00 mg, 0.455 mmol, 1.0 eq.) and 4-aminobutanol (121.67 mg, 1.365 mmol, 3.0 eq.) according to **General Procedure K.** The crude product was purified by flash column chromatography according to **Purification Method A** to obtain the pure product as a colourless oil, 194.0 mg, 0.364 mmol, 80 % yield.

¹H NMR (500 MHz, CDCl₃) δ 4.05 (m, 2H), δ 3.60 (t, 2H), δ 3.35 (s, 3H), δ 3.18-3.11 (m, 1H), δ 2.90-2.85 (q, 2H), δ 2.80-2.71 (q, 3H), δ 2.36-2.29 (m, 1H), δ 2.22-2.14 (m, 1H), δ 1.93-1.50 (br. m, 16H), δ 1.44-0.88 (br. m, 18H), δ 0.90-0.88 (m, 6H), δ 0.62 (s, 3H)

### Example 94

### Synthesis of undecyl 6-((2-hydroxyethyl)amino)hexanoate

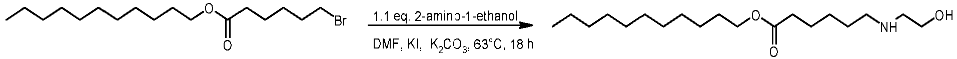

Undecyl 6-((2-hydroxyethyl)amino)hexanoate was prepared from undecyl 6-bromohexanoate (1000.00 mg, 2.862 mmol, 1.0 eq.) and 2-amino-1-ethanol (192.33 mg, 3.148 mmol, 1.1 eq.) according to **General Procedure N.** The crude product was purified by flash column chromatography according to **Purification Method A** to obtain the pure product as a colorless oil, 350.0 mg, 1.062 mmol, 37 % yield.

¹H NMR (500 MHz, CDCl₃) δ 4.04 (t, 2H), δ 3.63 (m, 2H), δ 2.76 (m, 2H), δ 2.61 (t, 2H), δ 2.29 (t, 2H), δ 2.09 (broad s, 2H), δ 1.67-1.57 (m, 4H), δ 1.54-1.47 (m, 2H), δ 1.39-1.22 (m, 18H), δ 0.87 (t, 3H)

### Example 95

### Synthesis of undecyl 6-((4-hydroxybutyl)amino)hexanoate

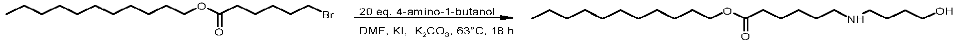

undecyl 6-((4-hydroxybutyl)amino)hexanoate was prepared from undecyl 6-bromohexanoate (1124.21 mg, 3.218 mmol, 1.0 eq.) and 4-amino-1-butanol (5737.05 mg, 64.36 mmol, 20 eq.) according to **General Procedure N.** The crude product was purified by flash column chromatography according to **Purification Method A** to obtain the pure product as a thick yellow oil, 969.0 mg, 2.607 mmol, 81 % yield.

¹H NMR (500 MHz, CDCl₃) δ 4.03 (t, 2H), δ 3.57 (t, 2H), δ 2.71-2.63 (m, 4H), δ 2.29 (t, 2H), δ 1.69-1.52 (m, 10H), δ 1.40-1.19 (m, 18H), δ 0.86 (t, 3H)

### Example 96

### Synthesis of heptadecan-9-yl 8-((2-hydroxyethyl)amino)octanoate

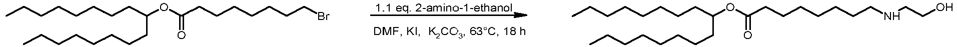

Heptadecan-9-yl 8-((2-hydroxyethyl)amino)octanoate was prepared from heptadecan-9-yl 8-bromooctanoate (200.00 mg, 0.412 mmol, 1.0 eq.) and 2-amino-1-ethanol (27.66 mg, 0.453 mmol, 1.1 eq.) according to **General Procedure N.** The crude product was purified by flash column chromatography according to **Purification Method A** to obtain the pure product as a colourless oil, 100.0 mg, 0.226 mmol, 55 % yield.

¹H NMR (500 MHz, CDCl₃) δ 4.89-4.83 (m, 1H), δ 3.62 (s, 2H), δ 2.78-2.75 (m, 2H), δ 2.60 (t, 2H), δ 2.27 (t, 2H), δ 1.65-1.56 (m, 2H), δ 1.53-1.41 (m, 6H), δ 1.36-1.16 (m, 30H), δ 0.87 (t, 6H)

### Example 97

### Synthesis of heptadecan-9-yl 8-((4-hydroxybutyl)amino)octanoate

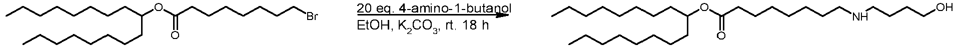

Heptadecan-9-yl 8-((4-hydroxybutyl)amino)octanoate was prepared from heptadecan-9-yl 8-bromooctanoate (950 mg, 1.841 mmol, 1.0 eq.) and 4-amino-1-butanol (3.28 g, 36.83 mmol, 20 eq.) according to **General Procedure L.** The crude product was purified by flash column chromatography according to **Purification Method A** to obtain the pure product as a colourless oil, 865.0 mg, 1.28 mmol, 69 % yield.

¹H NMR (500 MHz, CDCl₃) δ 5.19 (m, 1H), δ 3.80-3.76 (m, 2H), δ 3.48-3.40 (m, 2H), δ 2.61 (t, 2H), δ 2.51 (t, 2H), δ 2.28 (t, 2H), δ 1.83-1.10 (br. m, 40H), δ 0.98-0.91 (m, 6H)

### Example 98

### Synthesis of 6-((dimethyl(octyl)silyl)oxy)-6-(octyloxy)-N-(prop-2-yn-1-yl)hexan-1-amine

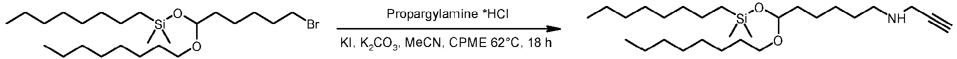

A MW reaction vial was charged with ((6-bromo-1-(octyloxy)hexyl)oxy)dimethyl(octyl)silane (400 mg, 0.834 mmol, 1.0 eq), propargylamine hydrochloride (381.69 mg, 4.170 mmol, 5.0 eq.), K₂CO₃ (806.79 mg, 5.838 mmol, 7.0 eq.), KI (13.84 mg, 0.083 mmol, 0.1 eq.), 1.5 ml of abs. acetonitrile and 1.0 ml of cyclopentyl methyl ether and purged with argon and sealed. The resulting mixture was stirred for 18 hours at 62°C. Then the reaction mixture was cooled down to room temperature and partitioned between ethyl acetate and brine and stirred vigorously for 15 minutes. The organic phase was separated and dried over Na₂SO₄, filtered and concentrated. The crude product was purified by flash column chromatography according to **Purification Method B** to obtain the pure product as a colorless oil, 260 mg, 0.573 mmol, 69 % yield.

¹H NMR (500 MHz, Benzene-d₆) δ 4.85 (t, 1H), δ 3.77-3.72 (q, 1H), δ 3.39-3.34 (q, 1H), δ 3.16 (d, 2H), δ 2.50 (t, 2H), δ 1.93 (t, 1H), δ 1.83-1.16 (m, 32H), δ 0.94-0.88 (m, 6H), δ 0.75-0.71 (m, 2H), δ 0.24 (d, 6H)

### Example 99

### Synthesis of 6-((dimethyl(octyl)silyl)oxy)-N-(6-((dimethyl(octyl)silyl)oxy)-6-(octyloxy)hexyl)-6-(octyloxy)-N-(prop-2-yn-1-yl)hexan-1-amine

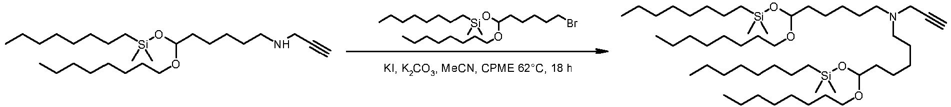

A MW reaction vial was charged with ((6-bromo-1-(octyloxy)hexyl)oxy)dimethyl(octyl)silane (431.22 mg, 0.899 mmol, 1.2 eq), 6-((dimethyl(octyl)silyl)oxy)-6-(octyloxy)-N-(prop-2-yn-1-yl)hexan-1-amine (340.00 mg, 0.749 mmol, 1.0 eq.), K₂CO₃ (207.09 mg, 1.499 mmol, 2.0 eq.), KI (12.44 mg, 0.075 mmol, 0.1 eq.), 1.5 ml of abs. acetonitrile and 0.5 ml of cyclopentyl methyl ether and purged with argon and sealed. The resulting mixture was stirred for 24 hours at 62°C. Then the reaction mixture was cooled down to room temperature and partitioned between ethyl acetate and brine and stirred vigorously for 15 minutes. The organic phase was separated and dried over Na₂SO₄, filtered and concentrated. The crude product was purified by flash column chromatography according to **Purification Method B** to obtain the pure product as a colourless oil, 530 mg, 0.622 mmol, 83 % yield.

¹H NMR (500 MHz, Benzene-d₆) δ 4.87 (t, 2H), δ 3.79-3.72 (q, 2H), δ 3.42-3.34 (q, 2H), δ 3.32 (d, 2H), δ 2.51 (t, 4H), δ 1.92 (t, 1H), δ 1.88-1.17 (m, 64H), δ 0.95-0.89 (m, 12H), δ 0.75-0.71 (m, 4H), δ 0.25 (d, 12H)

### Example 100

### Synthesis of 6-(octyloxy)-N-(6-(octyloxy)-6-((1,1,3,3-tetramethyl-3-octyldisiloxaneyl)oxy)hexyl)-N-(prop-2-yn-1-yl)-6-((1,1,3,3-tetramethyl-3-octyldisiloxaneyl)oxy)hexan-1-amine

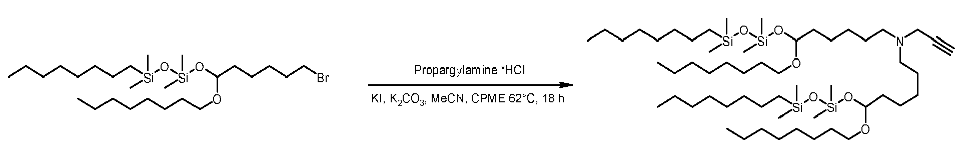

A MW reaction vial was charged with 1-((6-bromo-1-(octyloxy)hexyl)oxy)-1,1,3,3-tetramethyl-3-octyldisiloxane (453.74 mg, 0.819 mmol, 2.5 eq), propargylamine hydrochloride (30.00 mg, 0.328 mmol, 1.0 eq.), K₂CO₃ (181.18 mg, 1.311 mmol, 4.0 eq.), KI (10.88 mg, 0.065 mmol, 0.2 eq.), 1.2 ml of abs. acetonitrile and 0.4 ml of cyclopentyl methyl ether and purged with argon and sealed. The resulting mixture was stirred for 18 hours at 62°C. Then the reaction mixture was cooled down to room temperature and partitioned between ethyl acetate and brine and stirred vigorously for 15 minutes. The organic phase was separated and dried over Na₂SO₄, filtered and concentrated. The crude product was purified by flash column chromatography according to **Purification Method B** to obtain the pure product as a colourless oil, 83 mg, 0.083 mmol, 25 % yield.

¹H NMR (500 MHz, Benzene-d₆) δ 5.00 (t, 2H), δ 3.90-3.83 (q, 2H), δ 3.46-3.38 (q, 2H), δ 3.33 (d, 2H), δ 2.52 (t, 4H), δ 1.92-1.17 (m, 65H), δ 0.95-0.89 (m, 12H), δ 0.71-0.66 (m, 4H), δ 0.27 (d, 12H), δ 0.22 (s, 12H),

### Examples of the lipid synthesis

### Example 101

### Synthesis of 4-(bis(6-((dimethyl(octyl)silyl)oxy)-6-(octyloxy)hexyl)amino)butan-1-ol

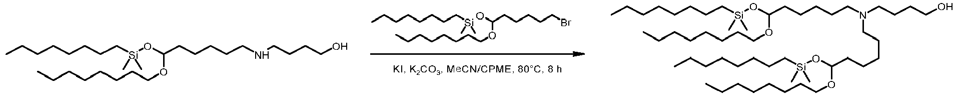

4-(bis(6-((dimethyl(octyl)silyl)oxy)-6-(octyloxy)hexyl)amino)butan-1-ol was prepared from ((6-bromo-1(octyloxy)hexyl)oxy)dimethyl(octyl)silane (93.4 mg, 0.195 mmol, 1.0 eq.) and 4-((6-((dimethyl(octyl)silyl)oxy)-6-(octyloxy)hexyl)amino)butan-1-ol (95.0 mg, 0.195 mmol, 1.0 eq.) according to **General Procedure G.** The crude product was purified by flash column chromatography twice according to **Purification Method A** and **B** to obtain the pure product as a colourless oil, 70 mg, 0.079 mmol, 40 % yield.

¹H NMR (500 MHz, Benzene-d₆) δ 4.88 (t, 2H), δ 3.79-3.72 (m, 2H), δ 3.62 (t, 2H), δ 3.41-3.36 (m, 2H), δ 2.33 (t, 4H), δ 2.24 (t, 2H), δ 1.77-1.25 (m, 68H), δ 0.94-0.89 (m, 12H), δ 0.76-0.72 (m, 4H), δ 0.26 (d, 12H); TOF MS ES⁺[M+H⁺]: 886.8069 m/z

### Example 102

### Synthesis of 5-(6-((1-((dimethyl(octyl)silyl)oxy)octyl)oxy)hexyl)-13-heptyl-15,15-dimethyl-12,14-dioxa-5-aza-15-silatricosan-1-ol

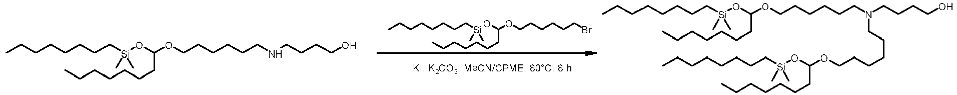

5-(6-((1-((dimethyl(octyl)silyl)oxy)octyl)oxy)hexyl)-13-heptyl-15,15-dimethyl-12,14-dioxa-5-aza-15-silatricosan-1-ol was prepared from ((1-((6-bromohexyl)oxy)octyl)oxy)dimethyl(octyl)silane (147.47 mg, 0.307 mmol, 1.0 eq.) and 13-heptyl-15,15-dimethyl-12,14-dioxa-5-aza-15-silatricosan-1-ol (150.00 mg, 0.307 mmol, 1.0 eq.) according to **General Procedure G.** The crude product was purified by flash column chromatography twice according to **Purification Method A** and **B** to obtain the pure product as a colourless oil, 100 mg, 0.113 mmol, 37 % yield.

¹H NMR (500 MHz, Benzene-d₆) δ 4.89 (t, 2H), δ 3.79-3.74 (m, 2H), δ 3.63 (t, 2H), δ 3.41-3.35 (m, 2H), δ 2.32 (m, 4H), δ 2.25 (t, 2H), δ 1.89-1.24 (m, 68H), δ 0.95-0.89 (m, 12H), δ 0.77-0.72 (m, 4H), δ 0.26 (d, 12H); TOF MS ES⁺ [M+H⁺]: 886.8039 m/z

### Example 103

### Synthesis of 4-(bis(6-(decyloxy)-6-((hexyldimethylsilyl)oxy)hexyl)amino)butan-1-ol

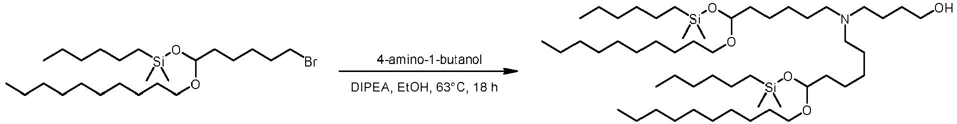

4-(bis(6-(decyloxy)-6-((hexyldimethylsilyl)oxy)hexyl)amino)butan-1-ol was prepared from ((6-bromo-1-(decyloxy)hexyl)oxy)(hexyl)dimethylsilane (403.27 mg, 0.841 mmol, 2.5 eq.) and 4-amino-1-butanol (29.98 mg, 0.336 mmol, 1.0 eq.) according to **General Procedure H.** The crude product was purified by flash column chromatography twice according to **Purification Method A** and **B** to obtain the pure product as a colourless oil, 45 mg, 0.051 mmol, 15 % yield.

¹H NMR (500 MHz, Benzene-d₆) δ 4.89 (t, 2H), δ 3.80-3.74 (m, 2H), δ 3.63 (t, 2H), δ 3.43-3.36 (m, 2H), δ 2.35-2.31 (m, 4H), δ 2.25 (t, 2H), δ 1.89-1.26 (m, 68H), δ 0.96-0.91 (m, 12H), δ 0.76-0.71 (m, 4H), δ 0.25 (d, 12H); TOF MS ES⁺ [M+H⁺]: 886.8082 m/z

### Example 104

### Synthesis of 5-(6-((1-((hexyldimethylsilyl)oxy)decyl)oxy)hexyl)-15,15-dimethyl-13-nonyl-12,14-dioxa-5-aza-15-silahenicosan-1-ol

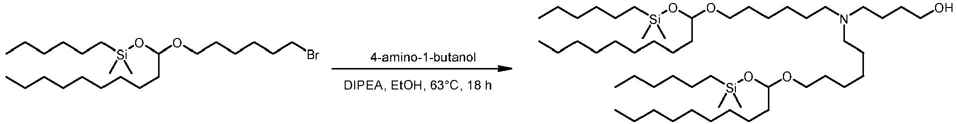

5-(6-((1-((hexyldimethylsilyl)oxy)decyl)oxy)hexyl)-15,15-dimethyl-13-nonyl-12,14-dioxa-5-aza-15-silahenicosan-1-ol was prepared from ((1-((6-bromohexyl)oxy)decyl)oxy)(hexyl)dimethylsilane (403.27 mg, 0.841 mmol, 2.5 eq.) and 4-amino-1-butanol (30.00 mg, 0.336 mmol, 1.0 eq.) according to **General Procedure H.** The crude product was purified by flash column chromatography according to **Purification Method A** to obtain the pure product as a colourless oil, 45 mg, 0.051 mmol, 15 % yield., 53 mg, 0.060 mmol, 18 % yield.

¹H NMR (500 MHz, Benzene-d₆) δ 4.89 (t, 2H), δ 3.78-3.73 (m, 2H), δ 3.63 (t, 2H), δ 3.41-3.35 (m, 2H), δ 2.34-2.30 (m, 4H), δ 2.25 (t, 2H), δ 1.89-1.23 (m, 68H), δ 0.96-0.90 (m, 12H), δ 0.75-0.70 (m, 4H), δ 0.25 (d, 12H); TOF MS ES⁺[M+H⁺]: 886.8081 m/z

### Example 105

### Synthesis of 5-(6-((dimethyl(octyloxy)silyl)oxy)-6-(octyloxy)hexyl)-13,13-dimethyl-11-(octyloxy)-12,14-dioxa-5-aza-13-siladocosan-1-ol

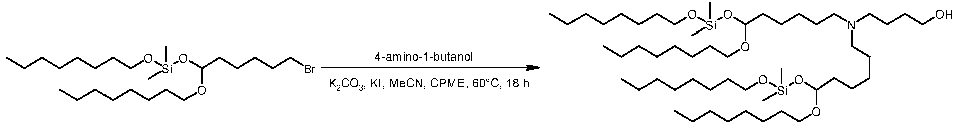

5-(6-((dimethyl(octyloxy)silyl)oxy)-6-(octyloxy)hexyl)-13,13-dimethyl-11-(octyloxy)-12,14-dioxa-5-aza-13-siladocosan-1-ol was prepared from 12-(5-bromopentyl)-10,10-dimethyl-9,11,13-trioxa-10-silahenicosane (416.72 mg, 0.841 mmol, 2.5 eq.) and 4-amino-1-butanol (29.98 mg, 0.336 mmol, 1.0 eq.) according to **General Procedure I.** The crude product was purified by flash column chromatography according to **Purification Method B** to obtain the pure product as a colourless oil, 187 mg, 0.204 mmol, 61 % yield.

¹H NMR (500 MHz, Benzene-d₆) δ 5.01(dt, 2H), δ 3.89-3.84 (m, 2H), δ 3.77 (t, 4H), δ 3.62 (t, 2H), δ 3.46-3.38 (m, 3H), δ 2.33 (m, 3H), δ 2.24 (t, 2H), δ 1.98-1.78 (m, 4H), δ 1.71-1.19 (m, 64H) δ 0.93-0.88 (m, 12H), δ 0.28 (d, 12H);TOF MS ES⁺ [M+H⁺]: 918.7984 m/z

### Example 106

### Synthesis of 13-heptyl-5-(8-heptyl-10,10-dimethyl-7,9,11-trioxa-10-silanonadecyl)-15,15-dimethyl-12,14,16-trioxa-5-aza-15-silatetracosan-1-ol

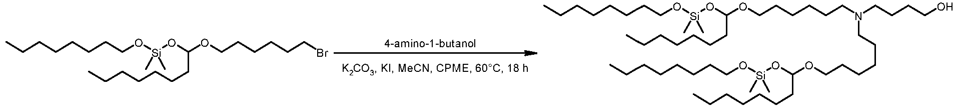

13-heptyl-5-(8-heptyl-10,10-dimethyl-7,9,11-trioxa-10-silanonadecyl)-15,15-dimethyl-12,14,16-trioxa-5-aza-15-silatetracosan-1-ol was prepared from 1-bromo-8-heptyl-10,10-dimethyl-7,9,11-trioxa-10-silanonadecane (416.72 mg, 0.840 mmol, 2.5 eq.) and 4-amino-1-butanol (29.98 mg, 0.336 mmol, 1.0 eq.) according to **General Procedure I.** The crude product was purified by flash column chromatography according to **Purification Method B** to obtain the pure product as a colourless oil, 146 mg, 0.159 mmol, 47 % yield.

¹H NMR (500 MHz, Benzene-d₆) δ 5.00 (dt, 2H), δ 3.89-3.84 (m, 2H), δ 3.79 (t, 4H), δ 3.63 (t, 2H), δ 3.45-3.38 (m, 2H), δ 2.34-2.30 (t, 4H), δ 2.25 (t, 2H), δ 1.96-1.79 (m, 4H), δ 1.73-1.22 (m, 64H) δ 0.94-0.89 (m, 12H), δ 0.30 (d, 12H); TOF MS ES⁺ [M+H⁺]: 918.7991 m/z

### Example 107

### Synthesis of 4-(bis(6-(octyloxy)-6-((1,1,3,3-tetramethyl-3-octyldisiloxaneyl)oxy)hexyl)amino)butan-1-ol

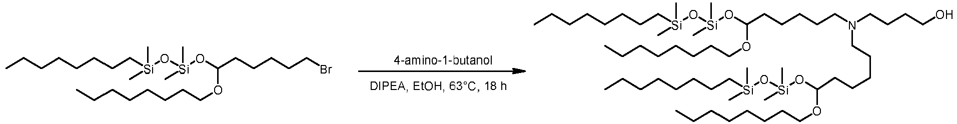

4-(bis(6-(octyloxy)-6-((1,1,3,3-tetramethyl-3-octyldisiloxaneyl)oxy)hexyl)amino)butan-1-ol was prepared from 1-((6-bromo-1-(octyloxy)hexyl)oxy)-1,1,3,3-tetramethyl-3-octyldisiloxane (465.62 mg, 0.841 mmol, 2.5 eq.) and 4-amino-1-butanol (29.98 mg, 0.336 mmol, 1.0 eq.) according to **General Procedure H.** The crude product was purified by flash column chromatography according to **Purification Method A** to obtain the pure product as a colourless oil, 87 mg, 0.084 mmol, 25 % yield.

¹H NMR (500 MHz, Benzene-d₆) δ 5.01 (dt, 2H), δ 3.91-3.84 (m, 2H), δ 3.61 (t, 2H), δ 3.45-3.41 (m, 2H), δ 2.35-2.32 (t, 4H), δ 2.25 (t, 2H), δ 1.96-1.77 (m, 4H), δ 1.75-1.15 (m, 64H), δ 0.94-0.89 (m, 12H);δ 0.70-0.66 (m, 4H), δ 0.27 (d, 12H), δ 0.22 (s, 12H);TOF MS ES⁺ [M+H⁺]: 1034.8458 m/z

### Example 108

### Synthesis of 13-heptyl-15,15,17,17-tetramethyl-5-(6-((1-((1,1,3,3-tetramethyl-3-octyldisiloxaneyl)oxy)octyl)oxy)hexyl)-12,14,16-trioxa-5-aza-15,17-disilapentacosan-1-ol

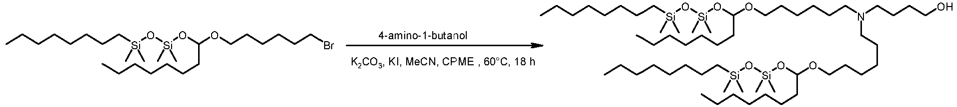

13-heptyl-15,15,17,17-tetramethyl-5-(6-((1-((1,1,3,3-tetramethyl-3-octyldisiloxaneyl)oxy)octyl)oxy)hexyl)-12,14,16-trioxa-5-aza-15,17-disilapentacosan-1-ol was prepared from 1-((1-((6-bromohexyl)oxy)octyl)oxy)-1,1,3,3-tetramethyl-3-octyldisiloxane (465.62 mg, 0.841 mmol, 2.5 eq.) and 4-amino-1-butanol (29.98 mg, 0.336 mmol, 1.0 eq.) according to **General Procedure I.** The crude product was purified by flash column chromatography according to **Purification Method A** to obtain the pure product as a colourless oil, 237 mg, 0.229 mmol, 66 % yield.

¹H NMR (500 MHz, Benzene-d₆) δ 5.01 (dt, 2H), δ 3.88-3.83 (m, 2H), δ 3.63 (t, 2H), δ 3.45-3.38 (m, 2H), δ 2.42-2.25 (m, 6H), δ 1.97-1.16 (m, 67H), δ 0.97-0.84 (m, 13H), δ 0.74-0.61 (m, 4H), δ 0.28 (d, 12H), δ 0.22 (s, 12H); TOF MS ES⁺[M+H⁺]: 1034.8467 m/z

### Example 109

### Synthesis of 4-(bis(6-(octyloxy)-6-((1,1,3,3-tetramethyl-3-(octyloxy)disiloxaneyl)oxy)hexyl)amino)butan-1-ol

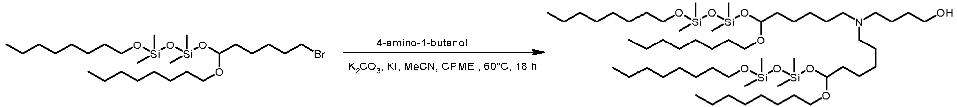

4-(bis(6-(octyloxy)-6-((1,1,3,3-tetramethyl-3-(octyloxy)disiloxaneyl)oxy )hexyl)amino)butan-1-ol was prepared from 1-((6-bromo-1-(octyloxy)hexyl)oxy)-1,1,3,3-tetramethyl-3-(octyloxy)disiloxane (319.90 mg, 0.561 mmol, 2.5 eq.) and 4-amino-1-butanol (20.02 mg, 0.225 mmol, 1.0 eq.) according to **General Procedure I.** The crude product was purified by flash column chromatography according to **Purification Method A** to obtain the pure product as a colourless oil, 60 mg, 0.056 mmol, 25 % yield.

¹H NMR (500 MHz, Benzene-d₆) δ 5.03 (dt, 2H), δ 3.91-3.85 (m, 2H), δ 3.76 (t, 4H), δ 3.61 (t, 2H), δ 3.47-3.38 (m, 2H), δ 2.35-2.31 (t, 4H), δ 2.25 (t, 2H) δ 1.96-1.79 (m, 4H), δ 1.72-1.21 (m, 64H), δ 0.94-0.87 (m, 12H), δ 0.31 (d, 12H), δ 0.25 (s, 12H); TOF MS ES⁺ [M+H⁺]: 1066.8368 m/z

### Example 110

### Synthesis of 13-heptyl-15,15,17,17-tetramethyl-5-(6-((1-((1,1,3,3-tetramethyl-3-(octyloxy)disiloxaneyl)oxy)octyl)oxy)hexyl)-12,14,16,18-tetraoxa-5-aza-15,17-disilahexacosan-1-ol

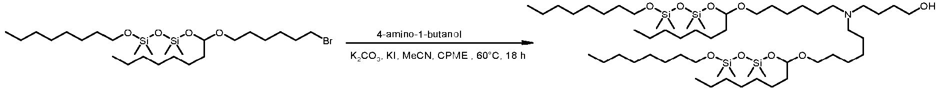

13-heptyl-15,15,17,17-tetramethyl-5-(6-((1-((1,1,3,3-tetramethyl-3-(octyloxy)disiloxaneyl)oxy)octyl)oxy)hexyl)-12,14,16,18-tetraoxa-5-aza-15,17-disilahexacosan-1-ol was prepared from 1-((1-((6-bromohexyl)oxy)octyl)oxy)-1,1,3,3-tetramethyl-3-(octyloxy)disiloxane (319.90 mg, 0.561 mmol, 2.5 eq.) and 4-amino-1-butanol (20.02 mg, 0.225 mmol, 1.0 eq.) according to **General Procedure I.** The crude product was purified by flash column chromatography according to **Purification Method A** to obtain the pure product as a colourless oil, 97 mg, 0.091 mmol, 41 % yield.

¹H NMR (500 MHz, Benzene-d₆) δ 5.04 (dt, 2H), δ 3.90-3.84 (m, 2H), δ 3.76 (t, 4H), δ 3.62 (t, 2H), δ 3.48-3.37 (m, 2H), δ 2.34-2.30 (m, 4H), δ 2.25 (t, 2H) δ 1.95-1.80 (m, 4H), δ 1.72-1.21 (m, 64H), δ 0.94-0.87 (m, 12H), δ 0.31 (d, 12H), δ 0.24 (s, 12H); TOF MS ES⁺ [M+H⁺]: 1066.8358 m/z

### Example 111

### Synthesis of 16-hexyl-5-(6-((((2-hexyldecyl)oxy)dimethylsilyl)oxy)-6-(octyloxy)hexyl)-13,13-dimethyl-11-(octyloxy)-12,14-dioxa-5-aza-13-silatetracosan-1-ol

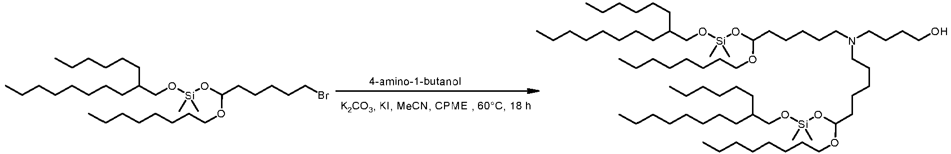

16-hexyl-5-(6-((((2-hexyldecyl)oxy)dimethylsilyl)oxy)-6-(octyloxy)hexyl)-13,13-dimethyl-11-(octyloxy)-12,14-dioxa-5-aza-13-silatetracosan-1-ol was prepared from 10-(5-bromopentyl)-15-hexyl-12,12-dimethyl-9,11,13-trioxa-12-silatricosane (511.07 mg, 0.841 mmol, 2.5 eq.) and 4-amino-1-butanol (29.98 mg, 0.336 mmol, 1.0 eq.) according to **General Procedure I.** The crude product was purified by flash column chromatography according to **Purification Method B** to obtain the pure product as a colourless oil, 269 mg, 0.232 mmol, 69 % yield.

¹H NMR (500 MHz, Benzene-d₆) δ 5.02 (dt, 2H), δ 3.91-3.84 (m, 2H), δ 3.75-3.73 (d, 4H), δ 3.61 (t, 2H), 3.48-3.40 (m, 2H), δ 2.34 (t, 4H), δ 2.26 (t, 2H), δ 1.97-1.76 (m, 4H), δ 1.73-1.18 (m, 92H), δ 0.95-0.89 (m, 18H), δ 0.29 (d, 12H);TOF MS ES⁺ [M_{fragment}+H⁺]: 918.7993 m/z

### Example 112

### Synthesis of 13-heptyl-5-(8-heptyl-13-hexyl-10,10-dimethyl-7,9,11-trioxa-10-silahenicosyl)-18-hexyl-15,15-dimethyl-12,14,16-trioxa-5-aza-15-silahexacosan-1-ol

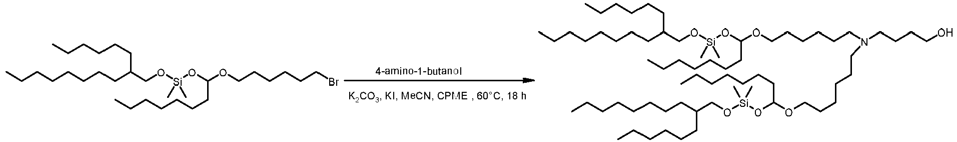

13-heptyl-5-(8-heptyl-13-hexyl-10,10-dimethyl-7,9,11-trioxa-10-silahenicosyl)-18-hexyl-15,15-dimethyl-12,14,16-trioxa-5-aza-15-silahexacosan-1-ol was prepared from 1-bromo-8-heptyl-13-hexyl-10,10-dimethyl-7,9,11-trioxa-10-silahenicosane (511.46 mg, 0.841 mmol, 2.5 eq.) and 4-amino-1-butanol (30.00 mg, 0.336 mmol, 1.0 eq.) according to **General Procedure I.** The crude product was purified by flash column chromatography according to **Purification Method B** to obtain the pure product as a colourless oil, 328 mg, 0.283 mmol, 84 % yield.

¹H NMR (500 MHz, Benzene-d₆) δ 5.01 (t, 2H), δ 3.90-3.83 (m, 2H), δ 3.74-3.73 (d, 4H), δ 3.62 (t, 2H), 3.46-3.38 (m, 2H), δ 2.33 (t, 4H), δ 2.25 (t, 2H), δ 1.94-1.78 (m, 4H), δ 1.72-1.19 (m, 92H), δ 0.95-0.87 (m, 18H), δ 0.29 (d, 12H);TOF MS ES⁺ [M_{fragment}+H⁺]: 918.7980 m/z

### Example 113

### Synthesis of 5-(6-(dimethyl((1-(octyloxy)octyl)oxy)silyl)hexyl)-14-heptyl-12,12-dimethyl-13,15-dioxa-5-aza-12-silatricosan-1-ol

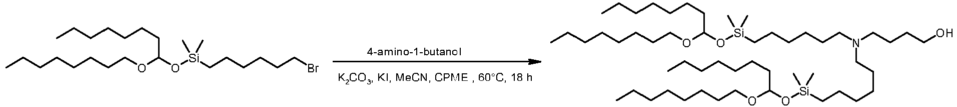

5-(6-(dimethyl((1-(octyloxy)octyl)oxy)silyl)hexyl)-14-heptyl-12,12-dimethyl-13,15-dioxa-5-aza-12-silatricosan-1-ol was prepared from (6-bromohexyl)dimethyl((1-(octyloxy)octyl)oxy)silane (403.58 mg, 0.841 mmol, 2.5 eq.) and 4-amino-1-butanol (30.00 mg, 0.336 mmol, 1.0 eq.) according to **General Procedure I.** The crude product was purified by flash column chromatography twice according to **Purification Method A** and **B** to obtain the pure product as a colourless oil, 97 mg, 0.109 mmol, 32 % yield.

¹H NMR (500 MHz, Benzene-d₆) δ 4.88 (m, 2H), δ 3.79-3.73 (m, 2H), δ 3.64 (t, 2H), δ 3.42-3.35 (m, 2H), δ 2.40-2.36 (m, 4H), δ 2.31-2.28 (m, 2H), δ 1.89-1.22 (m, 68H), δ 0.93-087 (m, 12H), δ 0.75-0.71 (m, 4H), δ 0.25 (d, 12H); TOF MS ES⁺ [M+H⁺]: 886.8121 m/z

### Example 114

### Synthesis of 15-heptyl-5-(10-heptyl-8,8-dimethyl-7,9,11-trioxa-8-silanonadecyl)-13,13-dimethyl-12,14,16-trioxa-5-aza-13-silatetracosan-1-ol

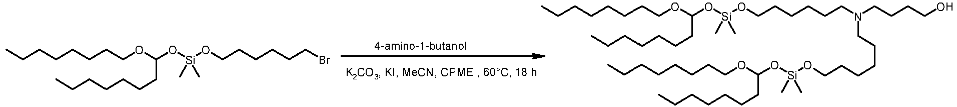

15-heptyl-5-(10-heptyl-8,8-dimethyl-7,9,11-trioxa-8-silanonadecyl)-13,13-dimethyl-12,14,16-trioxa-5-aza-13-silatetracosan-1-ol was prepared from 1-bromo-10-heptyl-8,8-dimethyl-7,9,11-trioxa-8-silanonadecane (416.72 mg, 0.841 mmol, 2.5 eq.) and 4-amino-1-butanol (29.98 mg, 0.336 mmol, 1.0 eq.) according to **General Procedure I.** The crude product was purified by flash column chromatography according to **Purification Method B** to obtain the pure product as a colourless oil, 169 mg, 0.184 mmol, 55 % yield.

¹H NMR (500 MHz, Benzene-d₆) δ 5.00 (m, 2H), δ 3.91-3.82 (m, 2H), δ 3.76 (t, 4H), δ 3.62 (t, 2H), δ 3.46-3.38 (m, 2H), δ 2.33 (t, 4H), δ 2.26 (t, 2H), δ 1.96-1.76 (m, 4H), δ 1.71-1.16 (m, 64H), δ 0.95-0.85 (m, 12H), δ 0.28 (d, 12H);TOF MS ES⁺ [M+H⁺]: 918.7974 m/z

### Example 115

### Synthesis of 15-heptyl-5-(10-heptyl-8,8-dimethyl-7,9-dioxa-11-thia-8-silanonadecyl)-13,13-dimethyl-12,14-dioxa-16-thia-5-aza-13-silatetracosan-1-ol

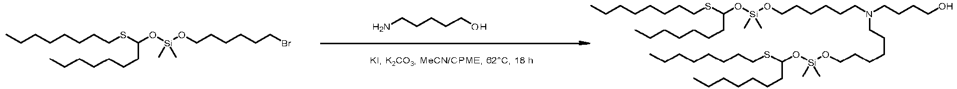

15-heptyl-5-(10-heptyl-8,8-dimethyl-7,9-dioxa-11-thia-8-silanonadecyl)-13,13-dimethyl-12,14-dioxa-16-thia-5-aza-13-silatetracosan-1-ol was prepared from 1-bromo-10-heptyl-8,8-dimethyl-7,9-dioxa-11-thia-8-silanonadecane (555.14 mg, 1.085 mmol, 2.5 eq.) and 4-amino-1-butanol (38.68 mg, 0.4339 mmol, 1.0 eq.) according to **General Procedure I.** The crude product was purified by flash column chromatography according to **Purification Method B** to obtain the pure product as a colourless oil, 170 mg, 0.179 mmol, 41 % yield.

¹H NMR (500 MHz, Benzene-d₆) δ 5.12 (m, 2H), δ 3.83-3.75 (m, 4H), δ 3.64-3.61 (t, 2H), δ 2.81-2.66 (m, 4H), δ 2.34 (t, 4H), δ 2.26 (t, 2H), δ 2.08-1.90 (m, 4H), δ 1.70-1.16 (m, 64H), δ 0.93-0.89 (m, 12H), δ 0.31 (d, 12H); TOF MS ES⁺[M+H⁺]: 950.7537 m/z

### Example 116

### Synthesis of 6-((dimethyl(octyl)silyl)oxy)-N-(6-((dimethyl(octyl)silyl)oxy)-6-(octyloxy)hexyl)-N-(2-(1-methylpyrrolidin-2-yl)ethyl)-6-(octyloxy)hexan-1-amine

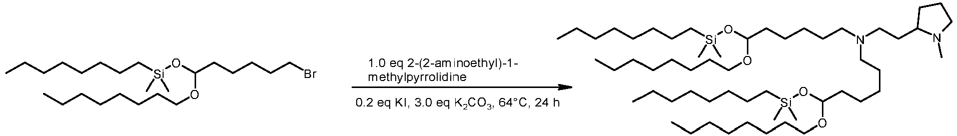

6-((dimethyl(octyl)silyl)oxy)-N-(6-((dimethyl(octyl)silyl)oxy)-6-(octyloxy)hexyl)-N-(2-(1-methylpyrrolidin-2-yl)ethyl)-6-(octyloxy)hexan-1-amine was prepared from ((6-bromo-1-(octyloxy)hexyl)oxy)dimethyl(octyl)silane (233.81 mg, 0.487 mmol, 2.5 eq.) and 2-(2-aminoethyl)-1-methylpyrrolidine (25.00 mg, 0.195 mmol, 1.0 eq.) according to **General Procedure O.** The crude product was purified by flash column chromatography according to **Purification Method A** to obtain the pure product as a colourless oil, 98 mg, 0.106 mmol, 54 % yield.

¹H NMR (500 MHz, Benzene-d₆) δ 4.89 (t, 2H), δ 3.78-3.73 (m, 2H), δ 3.41-3.35 (m, 2H), δ 3.01 (dt, 1H), δ 2.53-2.36 (m, 6H), δ 2.27 (s, 3H), δ 2.12-1.19 (m, 72H), δ 0.94-0.89 (m, 12H), δ 0.76-0.72 (4H), δ 0.25 (d, 12H); TOF MS ES⁺ [M+H⁺]: 925.8552 m/z

### Example 117

### Synthesis of 6-((dimethyl(octyloxy)silyl)oxy)-N-(6-((dimethyl(octyloxy)silyl)oxy)-6-(octyloxy)hexyl)-N-(2-(1-methylpyrrolidin-2-yl)ethyl)-6-(octyloxy)hexan-1-amine

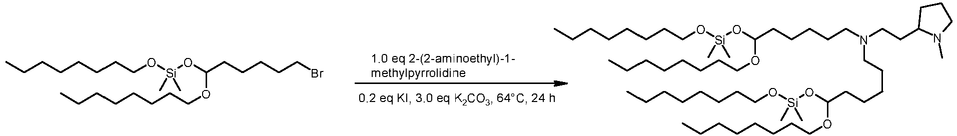

6-((dimethyl(octyloxy)silyl)oxy)-N-(6-((dimethyl(octyloxy)silyl)oxy)-6-(octyloxy)hexyl)-N-(2-(1-methylpyrrolidin-2-yl)ethyl)-6-(octyloxy)hexan-1-amine was prepared from 12-(5-bromopentyl)-10,10-dimethyl-9,11,13-trioxa-10-silahenicosane (386.58 mg, 0.780 mmol, 2.5 eq.) and 2-(2-aminoethyl)-1-methylpyrrolidine (40.00 mg, 0.312 mmol, 1.0 eq.) according to **General Procedure O.** The crude product was purified by flash column chromatography according to **Purification Method B** to obtain the pure product as a colourless oil, 33 mg, 0.034 mmol, 11 % yield.

¹H NMR (500 MHz, Benzene-d₆) δ 5.01 (t, 2H), δ 3.90-3.83 (m, 2H), δ 3.77 (t, 4H), δ 3.46-3.36 (m, 2H), δ 3.00 (dt, 1H), δ 2.53-2.36 (m, 6H), δ 2.29 (s, 3H), δ 2.10-1.17 (m, 72H), δ 0.93-0.88 (m, 12H);δ 0.28 (d, 12H); TOF MS ES⁺ [M_{fragment}+H⁺]: 845.7217 m/z

### Example 118

### Synthesis of N-(2-(1-methylpyrrolidin-2-yl)ethyl)-6-(octyloxy)-N-(6-(octyloxy)-6-((1,1,3,3-tetramethyl-3-octyldisiloxaneyl)oxy)hexyl)-6-((1,1,3,3-tetramethyl-3-octyldisiloxaneyl)oxy)hexan-1-amine

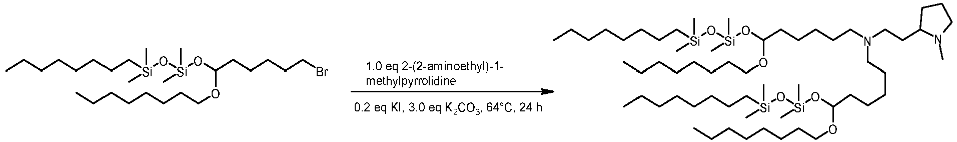

N-(2-(1-methylpyrrolidin-2-yl)ethyl)-6-(octyloxy)-N-(6-(octyloxy)-6-((1,1,3,3-tetramethyl-3-octyldisiloxaneyl)oxy)hexyl)-6-((1,1,3,3-tetramethyl-3-octyldisiloxaneyl)oxy)hexan-1-amine was prepared from 1-((6-bromo-1-(octyloxy)hexyl)oxy)-1,1,3,3-tetramethyl-3-octyldisiloxane (431.93 mg, 0.780 mmol, 2.5 eq.) and 2-(2-aminoethyl)-1-methylpyrrolidine (40.00 mg, 0.312 mmol, 1.0 eq.) according to **General Procedure O.** The crude product was purified twice by flash column chromatography according to **Purification Method A and B** to obtain the pure product as a colourless oil, 71 mg, 0.066 mmol, 21 % yield.

¹H NMR (500 MHz, Benzene-d₆) δ 5.02 (t, 2H), δ 3.90-3.84 (m, 2H), δ 3.46-3.30 (m, 2H), δ 3.00 (dt, 1H), δ 2.53-2.38 (m, 6H), δ 2.29 (s, 3H), δ 2.14-1.20 (m, 72H), δ 0.96-0.89 (m, 12H), δ 0.74-0.66 (m, 4H), δ 0.28 (d, 12H), δ 0.21 (s, 12H);TOF MS ES⁺ [M_{fragment}+H⁺]: 518.4431 m/z

### Example 119

### Synthesis of N-(2-(1-methylpyrrolidin-2-yl)ethyl)-6-(octyloxy)-N-(6-(octyloxy)-6-((1,1,3,3-tetramethyl-3-(octyloxy)disiloxaneyl)oxy)hexyl)-6-((1,1,3,3-tetramethyl-3-(octyloxy)disiloxaneyl)oxy)hexan-1-amine

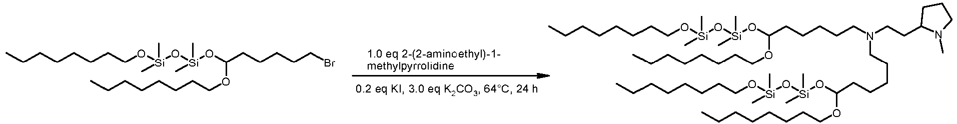

N-(2-(1-methylpyrrolidin-2-yl)ethyl)-6-(octyloxy)-N-(6-(octyloxy)-6-((1,1,3,3-tetramethyl-3-(octyloxy)disiloxaneyl)oxy)hexyl)-6-((1,1,3,3-tetramethyl-3-(octyloxy)disiloxaneyl)oxy )hexan-1-amine was prepared from 1-((6-bromo-1-(octyloxy)hexyl)oxy)-1,1,3,3-tetramethyl-3-(octyloxy)disiloxane (444.41 mg, 0.780 mmol, 2.5 eq.) and 2-(2-aminoethyl)-1-methylpyrrolidine (40.00 mg, 0.312 mmol, 1.0 eq.) according to **General Procedure O.** The crude product was purified twice by flash column chromatography according to **Purification Method A and B** to obtain the pure product as a colourless oil, 92 mg, 0.083 mmol, 27 % yield.

¹H NMR (500 MHz, Benzene-d₆) δ 5.04 (t, 2H), δ 3.91-3.85 (m, 2H), δ 3.76 (t, 4H), δ 3.47-3.36 (m, 2H), δ 3.00 (dt, 1H), δ 2.53-2.38 (m, 6H), δ 2.29 (s, 3H), δ 2.10-1.17 (m, 72H), δ 0.93-0.89 (m, 12H);δ 0.32 (d, 12H), δ 0.25 (s, 12H);TOF MS ES⁺ [M_{fragment}+H⁺]: 603.4640 m/z

### Example 120

### Synthesis of 6-((1-((dimethyl(octyl)silyl)oxy)octyl)oxy)-N-(6-((1-((dimethyl(octyl)silyl)oxy)octyl)oxy)hexyl)-N-(2-(1-methylpyrrolidin-2-yl)ethyl)hexan-1-amine

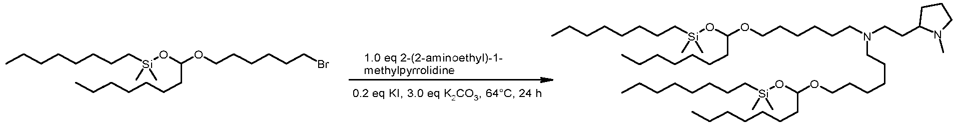

6-((1-((dimethyl(octyl)silyl)oxy)octyl)oxy)-N-(6-((1-((dimethyl(octyl)silyl)oxy)octyl)oxy)hexyl)-N-(2-(1-methylpyrrolidin-2-yl)ethyl)hexan-1-amine was prepared from ((1-((6-bromohexyl)oxy)octyl)oxy)dimethyl(octyl)silane (374.1 mg, 0.780 mmol, 2.5 eq.) and 2-(2-aminoethyl)-1-methylpyrrolidine (40.00 mg, 0.312 mmol, 1.0 eq.) according to **General Procedure O.** The crude product was purified twice by flash column chromatography according to **Purification Method A and B** to obtain the pure product as a colourless oil, 109 mg, 0.118 mmol, 38 % yield.

¹H NMR (500 MHz, Benzene-d₆) δ 4.88 (t, 2H), δ 3.79-3.73 (m, 2H), δ 3.41-3.35 (m, 2H), δ 3.00 (dt, 1H), δ 2.53-2.36 (m, 6H), δ 2.29 (s, 3H), δ 2.12-1.19 (m, 72H), δ 0.95-0.89 (m, 12H), δ 0.75-0.71 (4H), δ 0.25 (d, 12H)

### Example 121

### Synthesis of 8-heptyl-N-(8-heptyl-10,10-dimethyl-7,9,11-trioxa-10-silanonadecyl)-10,10-dimethyl-N-(2-(1-methylpyrrolidin-2-yl)ethyl)-7,9,11-trioxa-10-silanonadecan-1-amine

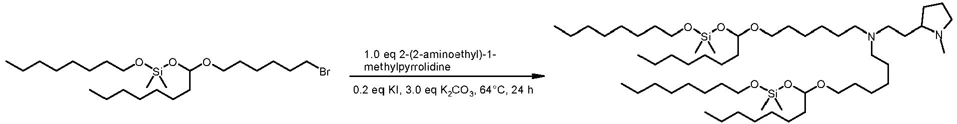

8-heptyl-N-(8-heptyl-10,10-dimethyl-7,9,11-trioxa-10-silanonadecyl)-10,10-dimethyl-N-(2-(1-methylpyrrolidin-2-yl)ethyl)-7,9,11-trioxa-10-silanonadecan-1-amine was prepared from 1-bromo-8-heptyl-10,10-dimethyl-7,9,11-trioxa-10-silanonadecane (386.58 mg, 0.780 mmol, 2.5 eq.) and 2-(2-aminoethyl)-1-methylpyrrolidine (40.00 mg, 0.312 mmol, 1.0 eq.) according to **General Procedure O.** The crude product was purified by flash column chromatography according to **Purification Method B** to obtain the pure product as a colourless oil, 60 mg, 0.063 mmol, 20 % yield.

¹H NMR (500 MHz, Benzene-d₆) δ 5.01 (t, 2H), δ 3.90-3.84 (m, 2H), δ 3.77 (t, 4H), δ 3.46-3.40 (m, 2H), δ 3.00 (dt, 1H), δ 2.53-2.37 (m, 6H), δ 2.29 (s, 3H), δ 2.10-1.17 (m, 72H), δ 0.93-0.89 (m, 12H), δ 0.29 (d, 12H)

### Example 122

### Synthesis of N-(2-(1-methylpyrrolidin-2-yl)ethyl)-6-((1-((1,1,3,3-tetramethyl-3-octyldisiloxaneyl)oxy)octyl)oxy)-N-(6-((1-((1,1,3,3-tetramethyl-3-octyldisiloxaneyl)oxy)octyl)oxy)hexyl)hexan-1-amine

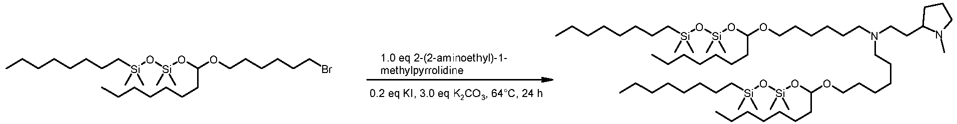

N-(2-(1-methylpyrrolidin-2-yl)ethyl)-6-((1-((1,1,3,3-tetramethyl-3-octyldisiloxaneyl)oxy)octyl)oxy)-N-(6-((1-((1,1,3,3-tetramethyl-3-octyldisiloxaneyl)oxy)octyl)oxy)hexyl)hexan-1-amine was prepared from 1-((1-((6-bromohexyl)oxy)octyl)oxy)-1,1,3,3-tetramethyl-3-octyldisiloxane (431.93 mg, 0.780 mmol, 2.5 eq.) and 2-(2-aminoethyl)-1-methylpyrrolidine (40.00 mg, 0.312 mmol, 1.0 eq.) according to **General Procedure O.** The crude product was purified by flash column chromatography according to **Purification Method B** to obtain the pure product as a colourless oil, 200 mg, 0.186 mmol, 60 % yield.

¹H NMR (500 MHz, Benzene-d₆) δ 5.01 (t, 2H), δ 3.91-3.83 (m, 2H), δ 3.45-3.37 (m, 2H), δ 2.99 (dt, 1H), δ 2.52-2.36 (m, 6H), δ 2.28 (s, 3H), δ 2.10-1.20 (m, 72H), δ 0.95-0.89 (m, 12H), δ 0.69-0.65 (m, 4H), δ 0.26 (d, 12H), δ 0.20 (s, 12H)

### Example 123

### Synthesis of N-(2-(1-methylpyrrolidin-2-yl)ethyl)-6-((1-((1,1,3,3-tetramethyl-3-(octyloxy)disiloxaneyl)oxy)octyl)oxy)-N-(6-((1-((1,1,3,3-tetramethyl-3-(octyloxy)disiloxaneyl)oxy)octyl)oxy hexyl)hexan-1-amine

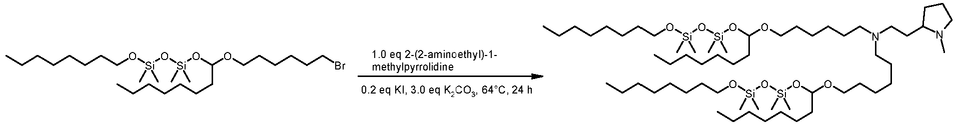

N-(2-(1-methylpyrrolidin-2-yl)ethyl)-6-((1-((1,1,3,3-tetramethyl-3-(octyloxy)disiloxaneyl)oxy)octyl)oxy)-N-(6-((1-((1,1,3,3-tetramethyl-3-(octyloxy)disiloxaneyl)oxy)octyl)oxy)hexyl)hexan-1-amine was prepared from 1-((1-((6-bromohexyl)oxy)octyl)oxy)-1,1,3,3-tetramethyl-3-(octyloxy)disiloxane (444.41 mg, 0.780 mmol, 2.5 eq.) and 2-(2-aminoethyl)-1-methylpyrrolidine (40.00 mg, 0.312 mmol, 1.0 eq.) according to **General Procedure O.** The crude product was purified by flash column chromatography according to **Purification Method B** to obtain the pure product as a colourless oil, 100 mg, 0.090 mmol, 29 % yield.

¹H NMR (500 MHz, Benzene-d₆) δ 5.03 (t, 2H), δ 3.93-3.83 (m, 2H), δ 3.76 (t, 4H), δ 3.46-3.35 (m, 2H), δ 3.00 (dt, 1H), δ 2.54-2.35 (m, 6H), δ 2.28 (s, 3H), δ 2.10-1.17 (m, 72H), δ 0.94-0.86 (m, 12H), δ 0.32 (d, 12H), δ 0.27 (s, 12H)

### Example 124

### Synthesis of 2-(4-((bis(6-((dimethyl(octyl)silyl)oxy)-6-(octyloxy)hexyl)amino)methyl)-1H-1,2,3-triazol-1-yl)ethan-1-ol

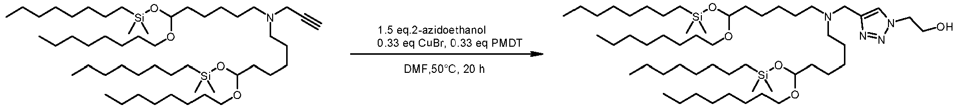

2-(4-((bis(6-((dimethyl(octyl)silyl)oxy)-6-(octyloxy)hexyl)amino)methyl)-1H-1,2,3-triazol-1-yl)ethan-1-ol was prepared from 6-((dimethyl(octyl)silyl)oxy)-N-(6-((dimethyl(octyl)silyl)oxy)-6-(octyloxy)hexyl)-6-(octyloxy)-N-(prop-2-yn-1-yl)hexan-1-amine (130.00 mg, 0.152 mmol, 1.0 eq.) and 2-azidoethanol (39.84 mg (50%), 0.229 mmol, 1.5 eq.) according to **General Procedure P.** The crude product was purified twice by flash column chromatography according to **Purification Method A and B** to obtain the pure product as a colourless oil, 63 mg, 0.067 mmol, 44 % yield.

¹H NMR (500 MHz, CDCl₃) δ 4.69 (t, 2H), δ 4.48-4.45 (m, 2H), δ 4.04-4.02 (m, 2H), δ 3.78 (s, 2H), δ 3.63-3.57 (m, 2H), δ 3.31-3.25 (m, 2H), δ 4.45 (t, 4H), δ 1.62-1.42 (m, 12H), δ 1.41-1.18 (m, 53H), δ 0.89-0.86 (m, 12H), δ 0.62-0.58 (m, 4H), δ 0.12 (d, 12H); TOF MS ES⁺ [M+H⁺]: 940.554 m/z

### Example 125

### Synthesis of (3R,4R,5S,6R)-3-(4-((bis(6-((dimethyl(octyl)silyl)oxy)-6-(octyloxy)hexyl)amino)methyl)-1H-1,2,3-triazol-1-yl)-6-(hydroxymethyl)tetrahydro-2H-pyran-2,4,5-triol

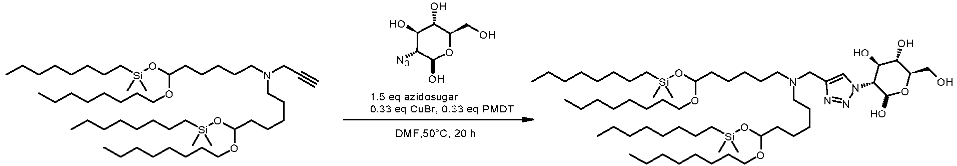

(3R,4R,5S,6R)-3-(4-((bis(6-((dimethyl(octyl)silyl)oxy)-6-(octyloxy)hexyl)amino)methyl)-1H-1,2,3-triazol-1-yl)-6-(hydroxymethyl)tetrahydro-2H-pyran-2,4,5-triol was prepared from 6-((dimethyl(octyl)silyl)oxy)-N-(6-((dimethyl(octyl)silyl)oxy)-6-(octyloxy)hexyl)-6-(octyloxy)-N-(prop-2-yn-1-yl)hexan-1-amine (130.00 mg, 0.152 mmol, 1.0 eq.) and 2-Azido-2-deoxy-D-glucose (46.93 mg, 0.229 mmol, 1.5 eq.) according to **General Procedure P.** The crude product was purified by flash column chromatography according to **Purification Method A** to obtain the pure product as a colourless oil, 89 mg, 0.084 mmol, 55 % yield.

¹H NMR (500 MHz, CDCl₃) δ 7.83 (s, 1H), δ 7.63 (s, 1H), δ 5.35 (s, 1H), δ 5.08 (d, 1H), δ 4.67 (t, 2H), δ 4.34-4.13 (m, 2H), δ 4.01 (d, 1H), δ 3.95-3.75 (m, 2H), δ 3.74-3.46 (br. m, 6H), δ 3.30-3.25 (q, 2H), δ 2.42-2.39 (br. m, 4H), δ 2.23 (m, 1H), δ 1.64-1.11 (m, 64H), δ 0.87 (m, 12H), δ 0.60 (m, 4H), δ 0.11 (d, 12H); LC ESI MS [M+H⁺]: 1057.843 m/z

### Example 126

### Synthesis of N-((2R,3R,4R,5S,6R)-2-(4-((bis(6-((dimethyl(octyl)silyl)oxy)-6-(octyloxy)hexyl)amino)methyl)-1H-1,2,3-triazol-1-yl)-4,5-dihydroxy-6-(hydroxymethyl)tetrahydro-2H-pyran-3-yl)acetamide

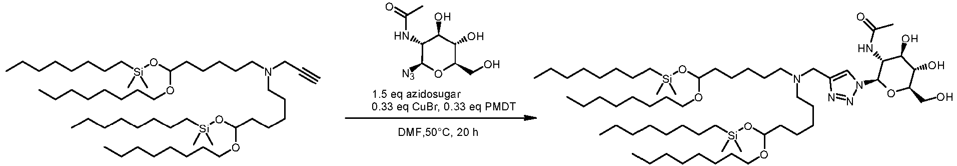

N-((2R,3R,4R,5S,6R)-2-(4-((bis(6-((dimethyl(octyl)silyl)oxy)-6-(octyloxy)hexyl)amino)methyl)-1H-1,2,3-triazol-1-yl)-4,5-dihydroxy-6-(hydroxymethyl)tetrahydro-2H-pyran-3-yl)acetamide was prepared from 6-((dimethyl(octyl)silyl)oxy)-N-(6-((dimethyl(octyl)silyl)oxy)-6-(octyloxy)hexyl)-6-(octyloxy)-N-(prop-2-yn-1-yl)hexan-1-amine (100.00 mg, 0.117 mmol, 1.0 eq.) and 2-Acetamido-2-deoxy-β-D-glucopyranosyl azide (43.32 mg, 0.176 mmol, 1.5 eq.) according to **General Procedure P.** The crude product was purified by flash column chromatography according to **Purification Method A** to obtain the pure product as a colourless oil, 88 mg, 0.080 mmol, 68 % yield.

¹H NMR (500 MHz, CDCl₃) δ 7.89 (s, 1H), δ 6.95 (s, 1H), δ 5.91 (d, 1H), δ 5.30-4.87 (s, 2H), δ 4.71-4.68 (m, 2H), δ 4.37-4.30 (m, 1H), δ 3.95-3.87 (m, 4H), δ 3.74-3.57 (m, 5H), δ 3.31-3.26 (m, 2H), δ 2.47-2.40 (m, 4H), δ 1.75 (s, 3H), δ 1.63-1.41 (m, 12H), δ 1.38-1.20 (m, 53H), δ 0.89-0.86 (m, 12H), δ 0.62-0.58 (m, 4H), δ 0.12 (d, 12H); LC ESI MS [M+H⁺]: 1098.624 m/z

### Example 127

### Synthesis of N-((2R,3R,4R,5S,6R)-2-(4-((bis(6-(octyloxy)-6-((1,1,3,3-tetramethyl-3-octyldisiloxaneyl)oxy)hexyl)amino)methyl)-1H-1,2,3-triazol-1-yl)-4,5-dihydroxy-6-(hydroxymethyl)tetrahydro-2H-pyran-3-yl)acetamide

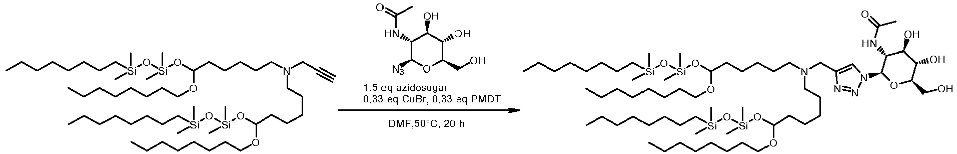

N-((2R,3R,4R,5S,6R)-2-(4-((bis(6-(octyloxy)-6-((1,1,3,3-tetramethyl-3-octyldisiloxaneyl)oxy)hexyl)amino)methyl)-1H-1,2,3-triazol-1-yl)-4,5-dihydroxy-6-(hydroxymethyl)tetrahydro-2H-pyran-3-yl)acetamide was prepared from 6-(octyloxy)-N-(6-(octyloxy)-6-((1,1,3,3-tetramethyl-3-octyldisiloxaneyl)oxy)hexyl)-N-(prop-2-yn-1-y1)-6-((1,1,3,3-tetramethyl-3-octyldisiloxaneyl)oxy)hexan-1-amine (83.00 mg, 0.083 mmol, 1.0 eq.) and 2-Acetamido-2-deoxy-β-D-glucopyranosyl azide (30.63 mg, 0.124 mmol, 1.5 eq.) according to **General Procedure P.** The crude product was purified by flash column chromatography according to **Purification Method A** to obtain the pure product as a colourless oil, 30 mg, 0.024 mmol, 29 % yield.

¹H NMR (500 MHz, CDCl₃) δ 8.04 (s, 1H), δ 7.21 (s, 1H), δ 5.89 (d, 1H), δ 4.77 (t, 2H), δ 4.39-4.36 (m, 1H), δ 4.14-3.83 (m, 6H), δ 3.69-3.65 (m, 3H), δ 3.31-3.25 (m, 2H), δ 2.90-2.26 (m, 6H), δ 1.75 (s, 3H), δ 1.65-1.45 (m, 12H), δ 1.43-1.13 (m, 53H), δ 0.89-0.85 (m, 12H), δ 0.55-0.51 (m, 4H), δ 0.11 (d, 12H), δ 0.09 (s, 12H); ); LC ESI MS [M+H⁺]: 1247.980 m/z

### Example 128

### Synthesis of 6-((6-((dimethyl(octyl)silyl)oxy)-6-(octyloxy)hexyl)(4-hydroxybutyl)amino)hexyl 2-hexyldecanoate

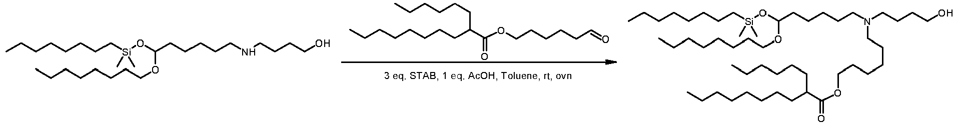

6-((6-((dimethyl(octyl)silyl)oxy)-6-(octyloxy)hexyl)(4-hydroxybutyl)amino)hexyl 2-hexyldecanoate was prepared from 4-((6-((dimethyl(octyl)silyl)oxy)-6-(octyloxy)hexyl)amino)butan-1-ol (150 mg, 0.307 mmol, 1.0 eq.) and 6-oxohexyl 2-hexyldecanoate (109.01 mg, 0.307 mmol, 1.0 eq.) according to **General Procedure J**. The crude product was purified by flash column chromatography according to **Purification Method A** to obtain the pure product as a colourless oil, 90 mg, 0.109 mmol, 35 % yield.

¹H NMR (500 MHz, Benzene-d₆) δ 4.90 (t, 1H), δ 4.12 (t, 2H), δ 3.80-3.74 (m, 1H), δ 3.62 (t, 2H), δ 3.42-3.34 (m, 2H), δ 2.48 (m, 1H), δ 2.35-2.23 (m, 5H), δ 1.90-1.22 (m, 68H), δ 0.95-0.88 (m, 12H), δ 0.77-0.73 (m, 2H), δ 0.26 (d, 6H); TOF MS ES⁺ [M+H⁺]: 826.7701 m/z

### Example 129

### Synthesis of 15-heptyl-7-(4-hydroxybutyl)-17,17-dimethyl-14,16-dioxa-7-aza-17-silapentacosyl 2-hexyldecanoate

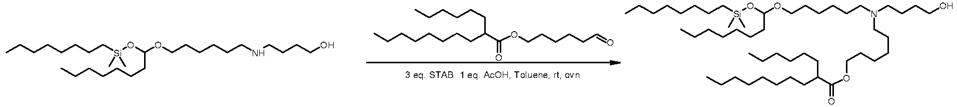

15-heptyl-7-(4-hydroxybutyl)-17,17-dimethyl-14,16-dioxa-7-aza-17-silapentacosyl 2-hexyldecanoate was prepared from 13-heptyl-15,15-dimethyl-12,14-dioxa-5-aza-15-silatricosan-1-ol (150 mg, 0.307 mmol, 1.0 eq.) and 6-oxohexyl 2-hexyldecanoate (109.01 mg, 0.307 mmol, 1.0 eq.) according to **General Procedure J**. The crude product was purified by flash column chromatography according to **Purification Method** A to obtain the pure product as a colourless oil, 38 mg, 0.046 mmol, 15 % yield.

¹H NMR (500 MHz, Benzene-d₆) δ 4.88 (t, 1H), δ 4.11 (t, 2H), δ 3.78-3.73 (m, 1H), δ 3.62 (t, 2H), δ 3.40-3.35 (m, 1H), δ 2.47(m, 2H), δ 2.35-2.22 (m, 5H), δ 1.87-1.12 (m, 68H), δ 0.93-0.88 (m, 12H), δ 0.75-0.71 (m, 2H), δ 0.25 (d, 6H); TOF MS ES⁺ [M+H⁺]: 826.7683 m/z

### Example 130

### Synthesis of 7-(4-hydroxybutyl)-15,15-dimethyl-13-(octyloxy)-14,16-dioxa-7-aza-15-silatetracosyl 2-hexyldecanoate

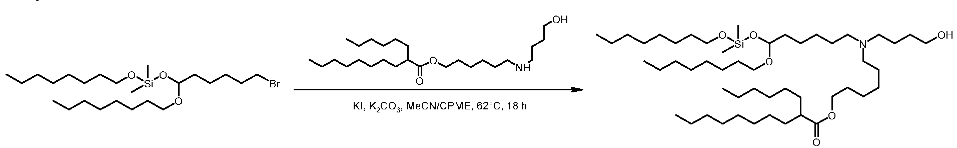

7-(4-hydroxybutyl)-15,15-dimethyl-13-(octyloxy)-14,16-dioxa-7-aza-15-silatetracosyl 2-hexyldecanoate was prepared from 12-(5-bromopentyl)-10,10-dimethyl-9,11,13-trioxa-10-silahenicosane (191.21 mg, 0.386 mmol, 1.1 eq.) and 6-((4-hydroxybutyl)amino)hexyl 2-hexyldecanoate (150 mg, 0.351 mmol, 1.0 eq.) according to **General Procedure K.** The crude product was purified by flash column chromatography according to **Purification Method A** to obtain the pure product as a colourless oil, 150 mg, 0.178 mmol, 51 % yield.

¹H NMR (500 MHz, Benzene-d6) δ 5.01 (t, 1H), δ 4.12 (t, 2H), δ 3.90-3.84 (m, 1H), δ 3.77 (t, 2H), δ 3.62 (t, 2H), δ 3.46-3.34 (m, 2H), δ 2.50-2.43 (m, 1H), δ 2.35-2.21 (m, 6H), δ 1.95-1.71 (m, 4H), δ 1.69-1.12 (m, 63H), δ 0.93-0.88 (m, 12H), δ 0.29 (d, 6H); TOF MS ES⁺ [M+H⁺]: 842.7618 m/z

### Example 131

### Synthesis of 15-heptyl-7-(4-hydroxybutyl)-17,17-dimethyl-14,16,18-trioxa-7-aza-17-silahexacosyl 2-hexyldecanoate

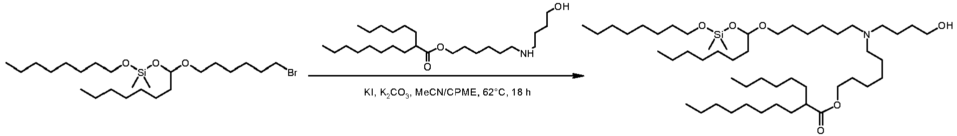

15-heptyl-7-(4-hydroxybutyl)-17,17-dimethyl-14,16,18-trioxa-7-aza-17-silahexacosyl 2-hexyldecanoate was prepared from 1-bromo-8-heptyl-10,10-dimethyl-7,9,11-trioxa-10-silanonadecane (173.83 mg, 0.351 mmol, 1.0 eq.) and 6-((4-hydroxybutyl)amino)hexyl 2-hexyldecanoate (150.00 mg, 0.351 mmol, 1.0 eq.) according to **General Procedure K.** The crude product was purified by flash column chromatography according to **Purification Method B** to obtain the pure product as a colourless oil, 153 mg, 0.182 mmol, 52 % yield.

¹H NMR (500 MHz, Benzene-d6) δ 5.00 (t, 1H), δ 4.11 (t, 2H), δ 3.92-3.83 (m, 1H), δ 3.76 (t, 2H), δ 3.62 (t, 2H), δ 3.44-3.40 (m, 2H), δ 2.47 (m, 1H), δ 2.34-2.23 (m, 6H), δ 1.95-1.73 (m, 4H), δ 1.69-1.12 (m, 63H), δ 0.93-0.88 (m, 12H), δ 0.29 (d, 6H); TOF MS ES⁺ [M+H⁺]: 842.7665 m/z

### Example 132

### Synthesis of 6-((4-hydroxybutyl)(6-(octyloxy)-6-((1,1,3,3-tetramethyl-3-octyldisiloxaneyl)oxy)hexyl)amino)hexyl 2-hexyldecanoate

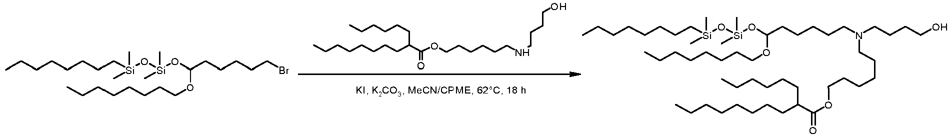

6-((4-hydroxybutyl)(6-(octyloxy)-6-((1,1,3,3-tetramethyl-3-octyldisiloxaneyl)oxy)hexyl)amino)hexyl 2-hexyldecanoate was prepared from 1-((6-bromo-1-(octyloxy)hexyl)oxy)-1,1,3,3-tetramethyl-3-octyldisiloxane (168.33 mg, 0.304 mmol, 1.0 eq.) and 6-((4-hydroxybutyl)amino)hexyl 2-hexyldecanoate (130 mg, 0.304 mmol, 1.0 eq.) according to **General Procedure K.** The crude product was purified by flash column chromatography according to **Purification Method B** to obtain the pure product as a colourless oil, 142 mg, 0.158 mmol, 52 % yield.

¹H NMR (500 MHz, Benzene-d₆) δ 5.01 (t, 1H), δ 4.12 (t, 2H), δ 3.92-3.83 (m, 1H), δ 3.62 (t, 2H), δ 3.47-3.39 (m, 1H), δ 2.47 (m, 1H), δ 2.36-2.23 (m, 6H), δ 1.99-1.08 (m, 68H), δ 0.95-0.88 (m, 12H), δ 0.71-0.66 (m, 2H), δ 0.27 (d, 6H), δ 0.22 (s, 6H); TOF MS ES⁺ [M+H⁺]: 900.7888 m/z

### Example 133

### Synthesis of 15-heptyl-7-(4-hydroxybutyl)-17,17,19,19-tetramethyl-14,16,18-trioxa-7-aza-17,19-disilaheptacosyl 2-hexyldecanoate

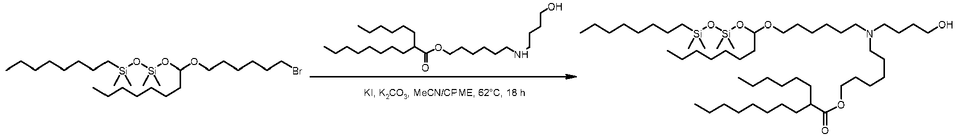

15-heptyl-7-(4-hydroxybutyl)-17,17,19,19-tetramethyl-14,16,18-trioxa-7-aza-17,19-disilaheptacosyl 2-hexyldecanoate was prepared from 1-((1-((6-bromohexyl)oxy)octyl)oxy)-1,1,3,3-tetramethyl-3-octyldisiloxane (194.22 mg, 0.351 mmol, 1.0 eq.) and 6-((4-hydroxybutyl)amino)hexyl 2-hexyldecanoate (150 mg, 0.351 mmol, 1.0 eq.) according to **General Procedure K.** The crude product was purified by flash column chromatography according to **Purification Method B** to obtain the pure product as a colourless oil, 169 mg, 0.188 mmol, 54 % yield.

¹H NMR (500 MHz, Benzene-d₆) δ 5.01 (t, 1H), δ 4.11 (t, 2H), δ 3.93-3.85 (m, 1H), δ 3.62 (t, 2H), δ 3.45-3.38 (m, 1H), δ 2.47 (m, 1H), δ 2.35-2.23 (m, 6H), δ 1.94-1.09 (m, 68H), δ 0.95-0.88 (m, 12H), δ 0.70-0.65 (m, 2H), δ 0.27 (d, 6H), δ 0.21 (s, 6H); TOF MS ES⁺ [M+H⁺]: 900.7840 m/z

### Example 134

### Synthesis of 6-((4-hydroxybutyl)(6-(octyloxy)-6-((1,1,3,3-tetramethyl-3-(octyloxy)disiloxaneyl)oxy)hexyl)amino)hexyl 2-hexyldecanoate

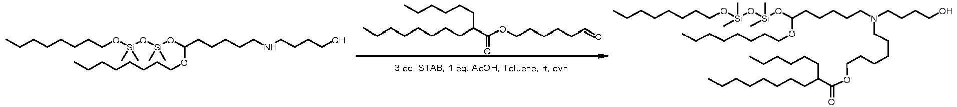

6-((4-hydroxybutyl)(6-(octyloxy)-6-((1,1,3,3-tetramethyl-3-(octyloxy)disiloxaneyl)oxy)hexyl)amino)hexyl 2-hexyldecanoate was prepared from 4-((6-(octyloxy)-6-((1,1,3,3-tetramethyl-3-(octyloxy)disiloxaneyl)oxy)hexyl)amino)butan-1-ol (126 mg, 0.218 mmol, 1.0 eq.) and 6-oxohexyl 2-hexyldecanoate (77.29 mg, 0.218 mmol, 1.0 eq.) according to **General Procedure J**. The crude product was purified by flash column chromatography according to **Purification Method A** to obtain the pure product as a colourless oil, 53 mg, 0.058 mmol, 27 % yield.

¹H NMR (500 MHz, Benzene-d₆) δ 5.03 (t, 1H), δ 4.11 (t, 2H), δ 3.91-3.85 (m, 1H), δ 3.76 (t, 2H), δ 3.61 (t, 2H), δ 3.46-3.41 (m, 1H), δ 2.46 (m, 1H), δ 2.35-2.23 (m, 6H), δ 1.96-1.11 (m, 68H), δ 0.93-0.87 (m, 12H), δ 0.30 (d, 6H), δ 0.24 (s, 6H); TOF MS ES⁺ [M+H⁺]: 916.7822 m/z

### Example 135

### Synthesis of 15-heptyl-7-(4-hydroxybutyl)-17,17,19,19-tetramethyl-14,16,18,20-tetraoxa-7-aza-17,19-disilaoctacosyl 2-hexyldecanoate

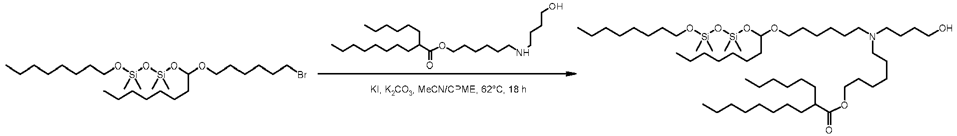

15-heptyl-7-(4-hydroxybutyl)-17,17,19,19-tetramethyl-14,16,18,20-tetraoxa-7-aza-17,19-disilaoctacosyl 2-hexyldecanoate was prepared from 1-((1-((6-bromohexyl)oxy)octyl)oxy)-1,1,3,3-tetramethyl-3-(octyloxy)disiloxane (190.51 mg, 0.334 mmol, 1.1 eq.) and 6-((4-hydroxybutyl)amino)hexyl 2-hexyldecanoate (130 mg, 0.304 mmol, 1.0 eq.) according to **General Procedure K.** The crude product was purified by flash column chromatography according to **Purification Method A** to obtain the pure product as a colourless oil, 51 mg, 0.056 mmol, 18 % yield.

¹H NMR (500 MHz, Benzene-d₆) δ 5.04 (t, 1H), δ 4.12 (t, 2H), δ 3.91-3.85 (m, 1H), δ 3.76 (t, 2H), δ 3.62 (t, 2H), δ 3.47-3.36 (m, 1H), δ 2.47 (m, 1H), δ 2.34-2.22 (m, 6H), δ 1.96-1.09 (m, 68H), δ 0.94-0.88 (m, 12H), δ 0.31 (d, 6H), δ 0.24 (s, 6H); TOF MS ES⁺ [M+H⁺]: 916.7834 m/z

### Example 136

### Synthesis of 18-hexyl-7-(4-hydroxybutyl)-15,15-dimethyl-13-(octyloxy)-14,16-dioxa-7-aza-15-silahexacosyl 2-hexyldecanoate

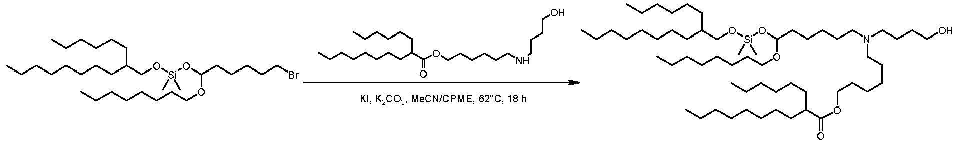

18-hexyl-7-(4-hydroxybutyl)-15,15-dimethyl-13-(octyloxy)-14,16-dioxa-7-aza-15-silahexacosyl 2-hexyldecanoate was prepared from 10-(5-bromopentyl)-15-hexyl-12,12-dimethyl-9,11,13-trioxa-12-silatricosane (234.50 mg, 0.386 mmol, 1.1 eq.) and 6-((4-hydroxybutyl)amino)hexyl 2-hexyldecanoate (150 mg, 0.351 mmol, 1.0 eq.) according to **General Procedure K.** The crude product was purified by flash column chromatography according to **Purification Method A** to obtain the pure product as a colourless oil, 179 mg, 0.188 mmol, 53 % yield.

¹H NMR (500 MHz, Benzene-d₆) δ 5.03 (t, 1H), δ 4.12 (t, 2H), δ 3.92-3.86 (m, 1H), δ 3.75 (d, 2H), δ 3.62 (t, 2H), δ 3.48-3.42 (m, 1H), δ 2.51-2.44 (m, 1H), δ 2.36-2.24 (m, 6H), δ 1.97-1.13 (m, 81H), δ 0.96-0.87 (m, 15H), δ 0.30 (d, 6H); TOF MS ES⁺ [M+H⁺]: 954.8895 m/z

### Example 137

### Synthesis of 15-heptyl-20-hexyl-7-(4-hydroxybutyl)-17,17-dimethyl-14,16,18-trioxa-7-aza-17-silaoctacosyl 2-hexyldecanoate

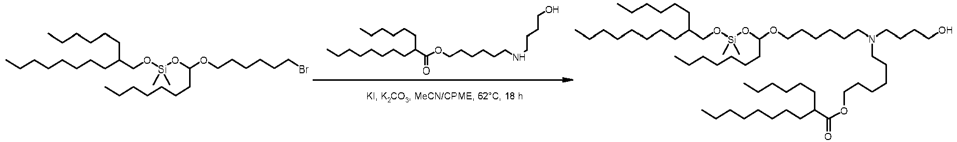

15-heptyl-20-hexyl-7-(4-hydroxybutyl)-17,17-dimethyl-14,16,18-trioxa-7-aza-17-silaoctacosyl 2-hexyldecanoate was prepared from 1-bromo-8-heptyl-13-hexyl-10,10-dimethyl-7,9,11-trioxa-10-silahenicosane (234.50 mg, 0.386 mmol, 1.1 eq.) and 6-((4-hydroxybutyl)amino)hexyl 2-hexyldecanoate (150 mg, 0.351 mmol, 1.0 eq.) according to **General Procedure K.** The crude product was purified by flash column chromatography according to **Purification Method A** to obtain the pure product as a colourless oil, 210 mg, 0.220 mmol, 63 % yield.

¹H NMR (500 MHz, Benzene-d₆) δ 5.03 (t, 1H), δ 4.12 (t, 2H), δ 3.91-3.85 (m, 1H), δ 3.75 (d, 2H), δ 3.62 (t, 2H), δ 3.46-3.40 (m, 1H), δ 2.50-2.43 (m, 1H), δ 2.34-2.24 (m, 6H), δ 1.97-1.12 (m, 81H), δ 0.96-0.87 (m, 12H), δ 0.30 (d, 6H); TOF MS ES⁺ [M+H⁺]: 954.8887 m/z

### Example 138

### Synthesis of 16-heptyl-7-(4-hydroxybutyl)-14,14-dimethyl-15,17-dioxa-7-aza-14-silapentacosyl 2-hexyldecanoate

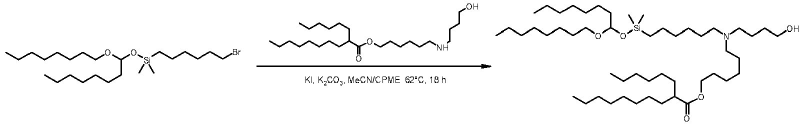

16-heptyl-7-(4-hydroxybutyl)-14,14-dimethyl-15,17-dioxa-7-aza-14-silapentacosyl 2-hexyldecanoate was prepared from (6-bromohexyl)dimethyl((1-(octyloxy)octyl)oxy)silane (185.04 mg, 0.386 mmol, 1.1 eq.) and 6-((4-hydroxybutyl)amino)hexyl 2-hexyldecanoate (150 mg, 0.351 mmol, 1.0 eq.) according to **General Procedure K.** The crude product was purified by flash column chromatography according to **Purification Method A** to obtain the pure product as a colourless oil, 197 mg, 0.238 mmol, 68 % yield.

¹H NMR (500 MHz, Benzene-d₆) δ 4.88 (dt, 1H), δ 4.12 (t, 2H), δ 3.79-3.73 (m, 1H), δ 3.63 (t, 2H), δ 3.41-3.35 (m, 1H), δ 2.54-2.42 (m, 1H), δ 2.39-2.26 (m, 6H), δ 1.88-1.13 (m, 68H), δ 0.94-0.87 (m, 12H), δ 0.76-0.72 (m, 2H), δ 0.26 (d, 6H); TOF MS ES⁺ [M+H⁺]: 826.7684 m/z

### Example 139

### Synthesis of 17-heptyl-7-(4-hydroxybutyl)-15,15-dimethyl-14,16,18-trioxa-7-aza-15-silahexacosyl 2-hexyldecanoate

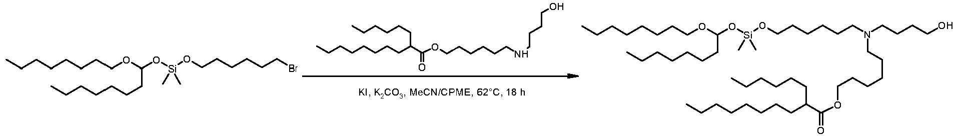

17-heptyl-7-(4-hydroxybutyl)-15,15-dimethyl-14,16,18-trioxa-7-aza-15-silahexacosyl 2-hexyldecanoate was prepared from 1-bromo-10-heptyl-8,8-dimethyl-7,9,11-trioxa-8-silanonadecane (191.21 mg, 0.386 mmol, 1.1 eq.) and 6-((4-hydroxybutyl)amino)hexyl 2-hexyldecanoate (150 mg, 0.351 mmol, 1.0 eq.) according to **General Procedure K.** The crude product was purified by flash column chromatography according to **Purification Method A** to obtain the pure product as a colourless oil, 94 mg, 0.112 mmol, 32 % yield.

¹H NMR (500 MHz, Benzene-d₆) δ 5.00 (t, 1H), δ 4.12 (t, 2H), δ 3.89-3.84 (m, 1H), δ 3.77 (t, 2H), δ 3.62 (t, 2H), δ 3.45-3.37 (m, 1H), δ 2.50-2.43 (m, 1H), δ 2.34-2.24 (m, 6H), δ 1.95-1.12 (m, 68H), δ 0.95-0.86 (m, 12H), δ 0.28 (d, 6H); TOF MS ES⁺ [M+H⁺]: 842.7627 m/z

### Example 140

### Synthesis of 17-heptyl-7-(4-hydroxybutyl)-15,15-dimethyl-14,16,18-trioxa-7-aza-15-silapentacosyl 2-hexyldecanoate

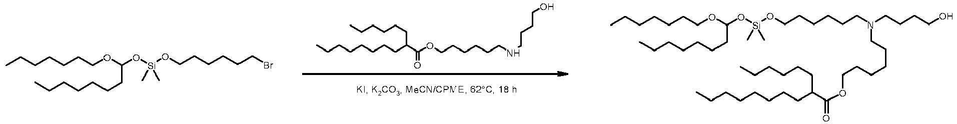

17-heptyl-7-(4-hydroxybutyl)-15,15-dimethyl-14,16,18-trioxa-7-aza-15-silapentacosyl 2-hexyldecanoate was prepared from 1-bromo-10-heptyl-8,8-dimethyl-7,9,11-trioxa-8-silaoctadecane (522.56 mg, 1.122 mmol, 1.2 eq.) and 6-((4-hydroxybutyl)amino)hexyl 2-hexyldecanoate (400 mg, 0.935 mmol, 1.0 eq.) according to **General Procedure K.** The crude product was purified by flash column chromatography according to **Purification Method A** to obtain the pure product as a colourless oil, 355 mg, 0.428 mmol, 46 % yield.

¹H NMR (500 MHz, Benzene-d₆) δ 4.96 (t, 1H), δ 4.09 (t, 2H), δ 3.85-3.80 (m, 1H), δ 3.74 (t, 2H), δ 3.61 (t, 2H), δ 3.41-3.36 (m, 1H), δ 2.47-2.40 (m, 1H), δ 2.32-2.25 (m, 6H), δ 1.91-1.15 (m, 66H), δ 0.92-0.87 (m, 12H), δ 0.25 (d, 6H); TOF MS ES⁺ [M+H⁺]: 828.7474 m/z

### Example 141

### Synthesis of 17-heptyl-7-(4-hydroxybutyl)-15,15-dimethyl-14,16,18-trioxa-7-aza-15-silaheptacosyl 2-hexyldecanoate

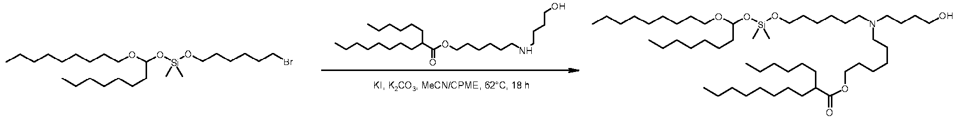

17-heptyl-7-(4-hydroxybutyl)-15,15-dimethyl-14,16,18-trioxa-7-aza-15-silaheptacosyl 2-hexyldecanoate was prepared from 1-bromo-10-heptyl-8,8-dimethyl-7,9,11-trioxa-8-silaicosane (326.04 mg, 0.640 mmol, 1.2 eq.) and 6-((4-hydroxybutyl)amino)hexyl 2-hexyldecanoate (228 mg, 0.533 mmol, 1.0 eq.) according to **General Procedure K.** The crude product was purified by flash column chromatography according to **Purification Method A** to obtain the pure product as a colourless oil, 144 mg, 0.168 mmol, 32 % yield.

¹H NMR (500 MHz, Benzene-d₆) δ 5.01 (t, 1H), δ 4.12 (t, 2H), δ 3.91-3.83 (m, 1H), δ 3.77 (t, 2H), δ 3.62 (t, 2H), δ 3.47-3.36 (m, 1H), δ 2.52-2.42 (m, 1H), δ 2.35-2.24 (m, 6H), δ 1.98-1.10 (br. m, 68H), δ 0.92-0.88 (m, 12H) δ 0.29 (d, 6H); TOF MS ES⁺ [M+H⁺]: 856.7791 m/z

### Example 142

### Synthesis of 17-hexyl-7-(4-hydroxybutyl)-15,15-dimethyl-14,16,18-trioxa-7-aza-15-silahexacosyl 2-hexyldecanoate

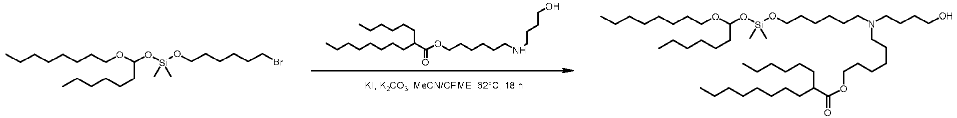

17-hexyl-7-(4-hydroxybutyl)-15,15-dimethyl-14,16,18-trioxa-7-aza-15-silahexacosyl 2-hexyldecanoate was prepared from 1-bromo-10-hexyl-8,8-dimethyl-7.9,11-trioxa-8-silanonadecane (522.56 mg, 1.122 mmol, 1.2 eq.) and 6-((4-hydroxybutyl)amino)hexyl 2-hexyldecanoate (400.0 mg, 0.935 mmol, 1.0 eq.) according to **General Procedure K.** The crude product was purified by flash column chromatography according to **Purification Method A** to obtain the pure product as a colourless oil, 385 mg, 0.465 mmol, 50 % yield.

¹H NMR (500 MHz, Benzene-d₆) δ 5.00 (t, 1H), δ 4.12 (t, 2H), δ 3.93-3.82 (m, 1H), δ 3.77 (t, 2H), δ 3.62 (t, 2H), δ 3.45-3.37 (m, 1H), δ 2.51-2.41 (m, 1H), δ 2.35-2.24 (m, 6H), δ 1.98-1.07 (br. m, 66H), δ 0.95-0.86 (m, 12H) δ 0.28 (d, 6H); TOF MS ES⁺ [M+H⁺]: 828.7489 m/z

### Example 143

### Synthesis of 7-(4-hydroxybutyl)-15,15-dimethyl-17-octyl-14,16,18-trioxa-7-aza-15-silahexacosyl 2-hexyldecanoate

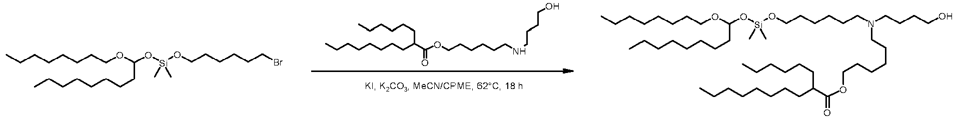

7-(4-hydroxybutyl)-15,15-dimethyl-17-octyl-14,16,18-trioxa-7-aza-15-silahexacosyl 2-hexyldecanoate was prepared from 1-bromo-8,8-dimethyl-10-octyl-7,9,11-trioxa-8-silanonadecane (554.04 mg, 1.122 mmol, 1.2 eq.) and 6-((4-hydroxybutyl)amino)hexyl 2-hexyldecanoate (400 mg, 0.935 mmol, 1.0 eq.) according to **General Procedure K.** The crude product was purified by flash column chromatography according to **Purification Method A** to obtain the pure product as a colourless oil, 398 mg, 0.465 mmol, 50 % yield.

¹H NMR (500 MHz, CDCl₃) δ 5.11 (t, 1H), δ 4.22 (t, 2H), δ 4.00-3.93 (m, 1H), δ 3.88 (t, 2H), δ 3.72 (t, 2H), δ 3.56-3.45 (m, 1H), δ 2.60-2.51 (m, 1H), δ 2.45-2.34 (m, 6H), δ 2.05-1.21 (br. m, 70H), δ 1.04-0.98 (m, 12H) δ 0.39 (d, 6H); TOF MS ES⁺ [M+H⁺]: 856.7808 m/z

### Example 144

### Synthesis of 18-(4-hydroxybutyl)-10,10-dimethyl-8-nonyl-7,9,11-trioxa-18-aza-10-silatetracosan-24-yl 2-hexyldecanoate

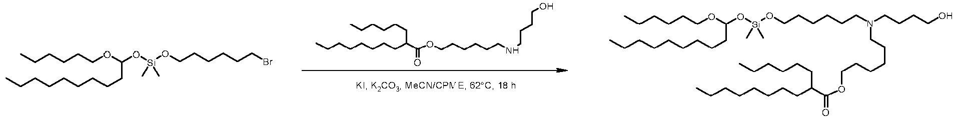

18-(4-hydroxybutyl)-10,10-dimethyl-8-nonyl-7,9,11-trioxa-18-aza-10-silatetracosan-24-yl 2-hexyldecanoate was prepared from 1-bromo-8,8-dimethyl-10-nonyl-7,9,11-trioxa-8-silaheptadecane (556.25 mg, 1.122 mmol, 1.2 eq.) and 6-((4-hydroxybutyl)amino)hexyl 2-hexyldecanoate (400 mg, 0.9352 mmol, 1.0 eq.) according to **General Procedure K.** The crude product was purified by flash column chromatography according to **Purification Method A** to obtain the pure product as a colourless oil, 344 mg, 0.408 mmol, 44 % yield.

H NMR (500 MHz, Benzene-d₆) δ 5.00 (t, 1H), δ 4.12 (t, 2H), δ 3.88-3.82 (m, 1H), δ 3.77 (t, 2H), δ 3.62 (t, 2H), δ 3.44-3.38 (m, 1H), δ 2.51-2.43 (m, 1H), δ 2.34-2.24 (m, 6H), δ 1.95-1.15 (br. m, 68H), δ 0.93-0.88 (m, 12H) δ 0.28 (d, 6H); TOF MS ES⁺ [M+H⁺]: 842.7653 m/z

### Example 145

### Synthesis of 7-(4-hydroxybutyl)-15,15-dimethyl-17-pentyl-14,16,18-trioxa-7-aza-15-silaoctacosyl 2-hexyldecanoate

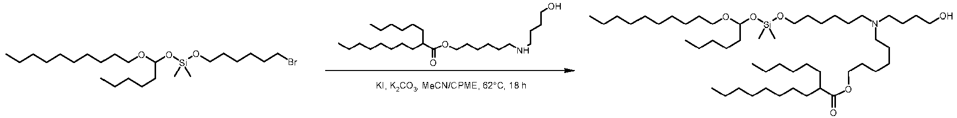

7-(4-hydroxybutyl)-15,15-dimethyl-17-pentyl-14,16,18-trioxa-7-aza-15-silaoctacosyl 2-hexyldecanoate was prepared from 1-bromo-8,8-dimethyl-10-pentyl-7,9,11-trioxa-8-silahenicosane (556.25 mg, 1.122 mmol, 1.2 eq.) and 6-((4-hydroxybutyl)amino)hexyl 2-hexyldecanoate (400 mg, 0.935 mmol, 1.0 eq.) according to **General Procedure K.** The crude product was purified by flash column chromatography according to **Purification Method B** to obtain the pure product as a colourless oil, 375 mg, 0.445 mmol, 48 % yield.

H NMR (500 MHz, Benzene-d₆) δ 4.97 (t, 1H), δ 4.10 (t, 2H), δ 3.87-3.82 (m, 1H), δ 3.75 (t, 2H), δ 3.61 (t, 2H), δ 3.42-3.38 (m, 1H), δ 2.47-2.41 (m, 1H), δ 2.33-2.23 (m, 6H), δ 1.91-1.10 (br. m, 68H), δ 0.93-0.88 (m, 12H) δ 0.26 (d, 6H); TOF MS ES⁺ [M+H⁺]: 842.7642 m/z

### Example 146

### Synthesis of 16-heptyl-7-(4-hydroxybutyl)-14,14-dimethyl-13,15,17-trioxa-7-aza-14-silapentacosyl 2-hexyldecanoate

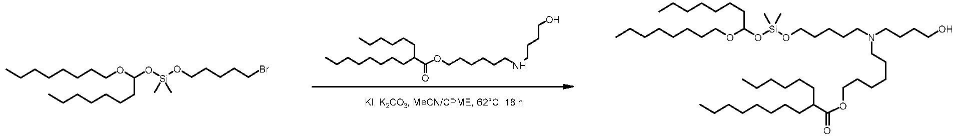

16-heptyl-7-(4-hydroxybutyl)-14,14-dimethyl-13,15,17-trioxa-7-aza-14-silapentacosyl 2-hexyldecanoate was prepared from 1-bromo-9-heptyl-7,7-dimethyl-6,8,10-trioxa-7-silaoctadecane (270.26 mg, 0.561 mmol, 1.2 eq.) and 6-((4-hydroxybutyl)amino)hexyl 2-hexyldecanoate (200 mg, 0.468 mmol, 1.0 eq.) according to **General Procedure K.** The crude product was purified by flash column chromatography according to **Purification Method A** to obtain the pure product as a colourless oil, 150 mg, 0.181 mmol, 39 % yield.

H NMR (500 MHz, Benzene-d₆) δ 5.01 (t, 1H), δ 4.12 (t, 2H), δ 3.90-3.83 (m, 1H), δ 3.78 (t, 2H), δ 3.62 (t, 2H), δ 3.47-3.39 (m, 1H), δ 3.36 (t, 2H), δ 2.34-2.24 (m, 6H), δ 1.96-1.08 (br. m, 66H), δ 0.94-0.88 (m, 12H) δ 0.29 (d, 6H); LC ESI MS [M+H⁺]: 828.860 m/z

### Example 147

### Synthesis of 17-heptyl-7-(4-hydroxybutyl)-15,15-dimethyl-14,16-dioxa-18-thia-7-aza-15-silahexacosyl 2-hexyldecanoate

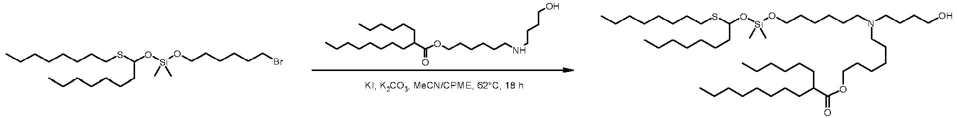

17-heptyl-7-(4-hydroxybutyl)-15,15-dimethyl-14,16-dioxa-18-thia-7-aza-15-silahexacosyl 2-hexyldecanoate was prepared from 1-bromo-10-heptyl-8,8-dimethyl-7,9-dioxa-11-thia-8-silanonadecane (574.28 mg, 1.122 mmol, 1.2 eq.) and 6-((4-hydroxybutyl)amino)hexyl 2-hexyldecanoate (400 mg, 0.935 mmol, 1.0 eq.) according to **General Procedure K.** The crude product was purified by flash column chromatography according to **Purification Method A** to obtain the pure product as a colourless oil, 288 mg, 0.330 mmol, 35 % yield.

H NMR (500 MHz, Benzene-d₆) δ 5.11 (t, 1H), δ 4.12 (t, 2H), δ 3.83-3.74 (m, 2H), δ 3.62 (t, 2H), δ 2.80-2.65 (m, 2H), δ 2.50-2.41 (m, 1H), δ 2.34-2.23 (m, 6H), δ 2.07-1.15 (br. m, 68H), δ 0.93-0.89 (m, 12H) δ 0.31 (d, 6H); TOF MS ES⁺[M+H⁺]: 858.7407 m/z

### Example 148

### Synthesis of 17-heptyl-7-(2-hydroxyethyl)-15,15-dimethyl-14,16,18-trioxa-7-aza-15-silahexacosyl 2-hexyldecanoate

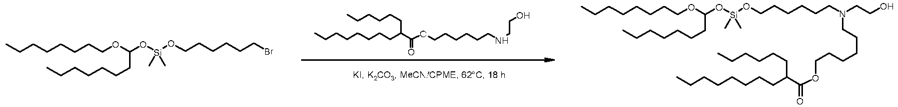

17-heptyl-7-(2-hydroxyethyl)-15,15-dimethyl-14,16,18-trioxa-7-aza-15-silahexacosyl 2-hexyldecanoate was prepared from 1-bromo-10-heptyl-8,8-dimethyl-7,9,11-trioxa-8-silanonadecane (654.83 mg, 1.321 mmol, 1.1 eq.) and 6-((2-hydroxyethyl)amino)hexyl 2-hexyldecanoate (480 mg, 1.201 mmol, 1.0 eq.) according to **General Procedure K.** The crude product was purified by flash column chromatography according to **Purification Method B** to obtain the pure product as a colourless oil, 190 mg, 0.233 mmol, 19 % yield.

¹H NMR (500 MHz, Benzene-d₆) δ 5.00 (dt, 1H), δ 4.12 (t, 2H), δ 3.91-3.82 (m, 1H), δ 3.77 (t, 2H), δ 3.52 (t, 2H), δ 3.46-3.38 (m, 1H), δ 2.52-2.43 (m, 1H), δ 2.38-2.24 (m, 6H), δ 1.97-1.07 (br. m, 64H), δ 0.95-0.88 (m, 12H), δ 0.28 (d, 6H); TOF MS ES⁺[M+H⁺]: 814.7322 m/z

### Example 149

### Synthesis of 9-heptyl-20-(4-hydroxybutyl)-7,7-dimethyl-8,10-dioxa-20-aza-7-silahexacosan-26-yl 2-hexyldecanoate

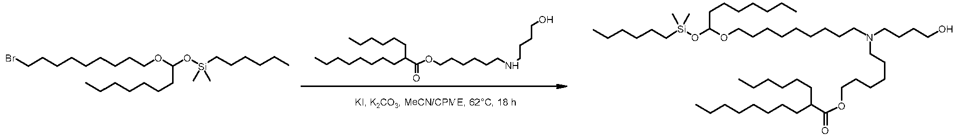

9-heptyl-20-(4-hydroxybutyl)-7,7-dimethyl-8,10-dioxa-20-aza-7-silahexacosan-26-yl 2-hexyldecanoate was prepared from ((1-((9-bromononyl)oxy)octyl)oxy)(hexyl)dimethylsilane (634.84 mg, 1.286 mmol, 1.1 eq.) and 6-((4-hydroxybutyl)amino)hexyl 2-hexyldecanoate (500 mg, 1.169 mmol, 1.0 eq.) according to **General Procedure K.** The crude product was purified by flash column chromatography according to **Purification Method B** to obtain the pure product as a colourless oil, 439 mg, 0.522 mmol, 45 % yield.

¹H NMR (500 MHz, Benzene-d₆) δ 4.89 (dt, 1H), δ 4.12 (t, 2H), δ 3.80-3.74 (m, 1H), δ 3.63 (t, 2H), δ 3.42-3.36 (m, 1H), δ 2.49-2.44 (m, 1H), δ 2.36-2.25 (m, 6H), δ 1.87-1.11 (br. m, 70H), δ 0.95-0.88 (m, 12H), δ 0.74-0.70 (m, 2H), δ 0.25 (d, 6H); TOF MS ES⁺ [M+H⁺]: 840.7838 m/z

### Example 150

### Synthesis of 17-heptyl-7-(4-hydroxybutyl)-13,13,15,15-tetramethyl-12,14,16,18-tetraoxa-7-aza-13,15-disilahexacosyl 2-hexyldecanoate

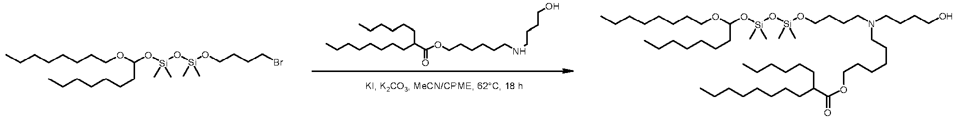

17-heptyl-7-(4-hydroxybutyl)-13,13,15,15-tetramethyl-12,14,16,18-tetraoxa-7-aza-13,15-disilahexacosyl 2-hexyldecanoate was prepared from 1-(4-bromobutoxy)-1,1,3,3-tetramethyl-3-((1-(octyloxy)octyl)oxy)disiloxane (501.6 mg, 0.926 mmol, 1.1 eq.) and 6-((4-hydroxybutyl)amino)hexyl 2-hexyldecanoate (360 mg, 0.842 mmol, 1.0 eq.) according to **General Procedure K.** The crude product was purified by flash column chromatography according to **Purification Method B** to obtain the pure product as a colourless oil, 194 mg, 0.218 mmol, 26 % yield.

¹H NMR (500 MHz, Benzene-d₆) δ 4.69 (t, 1H), δ 3.95 (t, 2H), δ 3.61-3.51 (m, 3H), δ 3.48-3.41 (m, 2H), δ 3.21-3.16 (m, 1H), δ 2.41-2.30 (m, 6H), δ 2.23-2.16 (m, 1H), δ 1.64-1.06 (br. m, 64H), δ 0.77 (t, 12H), δ 0.05 (d, 6H), δ 0.00 (s, 6H); LC ESI MS [M+H⁺]: 888.640 m/z

### Example 151

### Synthesis of 6-((6-((dimethyl((oxacyclohexadecan-2-yl)oxy)silyl)oxy)hexyl)(4-hydroxybutyl)amino)hexyl 2-hexyldecanoate

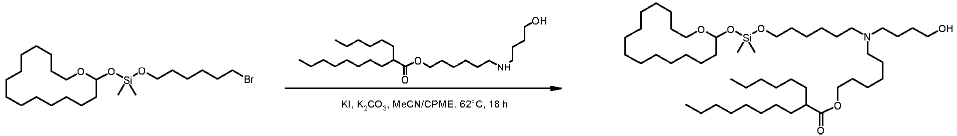

6-((6-((dimethyl((oxacyclohexadecan-2-yl)oxy)silyl)oxy)hexyl)(4-hydroxybutyl)amino)hexyl 2-hexyldecanoate was prepared from ((6-bromohexyl)oxy)dimethyl((oxacyclohexadecan-2-yl)oxy)silane (911.1 mg, 1.900 mmol, 1.25 eq.) and 6-((4-hydroxybutyl)amino)hexyl 2-hexyldecanoate (650 mg, 1.520 mmol, 1.0 eq.) according to **General Procedure K.** The crude product was purified by flash column chromatography according to **Purification Method B** to obtain the pure product as a colourless oil, 366 mg, 0.443 mmol, 29 % yield.

¹H NMR (500 MHz, Benzene-d₆) δ 4.96-4.93 (dd, 1H), δ 4.12 (t, 2H), δ 3.98-3.90 (m, 1H), δ 3.74 (t, 2H), δ 3.62 (t, 2H), δ 2.35-2.27 (m, 1H), δ 2.50-2.40 (m, 1H), δ 2.34-2.24 (m, 6H), δ 1.97-1.10 (br. m, 70H), δ 0.93-0.88 (m, 6H), δ 0.25 (d, 6H); LC ESI MS [M+H⁺]: 826.680 m/z

### Example 152

### Synthesis of (Z)-17-heptyl-7-(4-hydroxybutyl)-15,15-dimethyl-14,16,18-trioxa-7-aza-15-silaheptacos-24-en-1-yl 2-hexyldecanoate

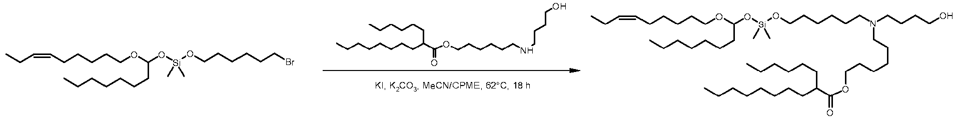

(Z)-17-heptyl-7-(4-hydroxybutyl)-15,15-dimethyl-14,16,18-trioxa-7-aza-15-silaheptacos-24-en-1-yl 2-hexyldecanoate was prepared from (Z)-1-bromo-10-heptyl-8,8-dimethyl-7,9,1 1-trioxa-8-silaicos-17-ene (1.073 g, 2.057 mmol, 1.1 eq.) and 6-((4-hydroxybutyl)amino)hexyl 2-hexyldecanoate (800 mg, 1.870 mmol, 1.0 eq.) according to **General Procedure K.** The crude product was purified by flash column chromatography according to **Purification Method B** to obtain the pure product as a colourless oil, 200 mg, 0.234 mmol, 12 % yield.

¹H NMR (500 MHz, Benzene-d₆) δ 5.48-5.38 (m, 2H), δ 4.98 (dt, 1H), δ 4.12 (t, 2H), δ 3.92-3.81 (m, 1H), δ 3.76 (t, 2H), δ 3.62 (t, 2H), δ 3.42-3.36 (m, 1H), δ 2.50-2.42 (m, 1H), δ 2.37-2.18 (m, 6H), δ 2.07-1.97 (m, 4H), δ 1.94-1.20 (br. m, 62H), δ 0.98-0.85 (m, 12H), δ 0.27 (d, 6H); LC ESI MS [M+H⁺]: 854.778 m/z

### Example 153

### Synthesis of (Z)-17-heptyl-7-(4-hydroxybutyl)-15,15-dimethyl-14,16,18-trioxa-7-aza-15-silaheptacos-21-en-1-yl 2-hexyldecanoate

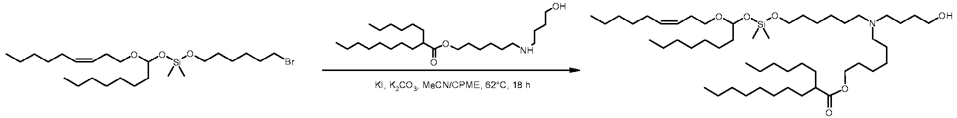

(Z)-17-heptyl-7-(4-hydroxybutyl)-15,15-dimethyl-14,16,18-trioxa-7-aza-15-silaheptacos-21-en-1-yl 2-hexyldecanoate was prepared from (Z)-1-bromo-10-heptyl-8,8-dimethyl-7,9,1 1-trioxa-8-silaicos-14-ene (1.073 g, 2.057 mmol, 1.1 eq.) and 6-((4-hydroxybutyl)amino)hexyl 2-hexyldecanoate (800 mg, 1.870 mmol, 1.0 eq.) according to **General Procedure K.** The crude product was purified by flash column chromatography according to **Purification Method B** to obtain the pure product as a colourless oil, 350 mg, 0.410 mmol, 22 % yield.

¹H NMR (500 MHz, Benzene-d₆) δ 5.63-5.49 (m, 2H), δ 5.00 (dt, 1H), δ 4.12 (t, 2H), δ 3.91-3.84 (m, 1H), δ 3.76 (t, 2H), δ 3.62 (t, 2H), δ 3.49-3.39 (m, 1H), δ 2.50-2.41 (m, 3H), δ 2.35-2.23 (m, 5H), δ 2.10-2.03 (m, 2H), δ 1.93-1.75 (m, 4H) δ 1.68-1.12 (br. m, 60H), δ 0.95-0.85 (m, 12H), δ 0.27 (d, 6H); LC ESI MS [M+H⁺]: 854.778 m/z

### Example 154

### Synthesis of 7-(4-hydroxybutyl)-15,15,17-trimethyl-14,16,18-trioxa-7-aza-15-silaoctacosyl 2-hexyldecanoate

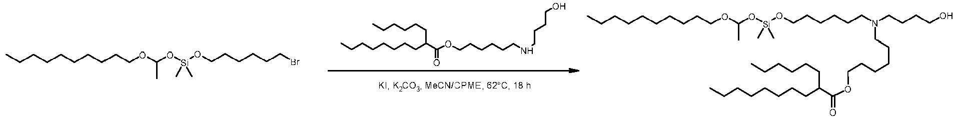

7-(4-hydroxybutyl)-15,15,17-trimethyl-14,16,18-trioxa-7-aza-15-silaoctacosyl 2-hexyldecanoate was prepared from 1-bromo-8,8,10-trimethyl-7,9,11-trioxa-8-silahenicosane (700.88 mg, 1.595 mmol, 1.1 eq.) and 6-((4-hydroxybutyl)amino)hexyl 2-hexyldecanoate (620 mg, 1.445 mmol, 1.0 eq.) according to **General Procedure K.** The crude product was purified by flash column chromatography according to **Purification Method B** to obtain the pure product as a colourless oil, 146 mg, 0.186 mmol, 13 % yield.

¹H NMR (500 MHz, Benzene-d₆) δ 5.09 (q, 1H), δ 4.12 (t, 2H), δ 3.85-3.79 (m, 1H), δ 3.73 (t, 2H), δ 3.62 (t, 2H), δ 3.40-3.35 (m, 1H), δ 2.51-2.45 (m, 1H), δ 2.34-2.23 (m, 6H), δ 1.85-1.76 (m, 2H), δ 1.70-1.14 (br. m, 62H), δ 0.93-0.87 (m, 9H), δ 0.24 (d, 6H); LC ESI MS [M+H⁺]: 786.693 m/z

### Example 155

### Synthesis of 7-(4-hydroxybutyl)-15,15-dimethyl-17-propyl-14,16,18-trioxa-7-aza-15-silaoctacosyl 2-hexyldecanoate

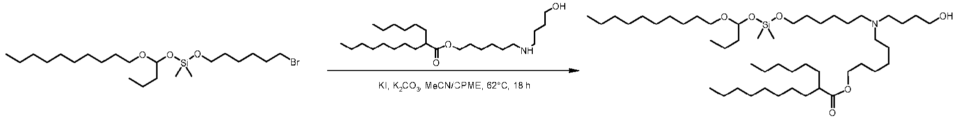

7-(4-hydroxybutyl)-15,15-dimethyl-17-propyl-14,16,18-trioxa-7-aza-15-silaoctacosyl 2-hexyldecanoate was prepared from 1-bromo-8,8-dimethyl-10-propyl-7,9,11-trioxa-8-silahenicosane (721.56 mg, 1.543 mmol, 1.1 eq.) and 6-((4-hydroxybutyl)amino)hexyl 2-hexyldecanoate (600 mg, 1.403 mmol, 1.0 eq.) according to **General Procedure K.** The crude product was purified by flash column chromatography according to **Purification Method B** to obtain the pure product as a colourless oil, 248 mg, 0.305 mmol, 22 % yield.

¹H NMR (500 MHz, Benzene-d₆) δ 4.97 (t, 1H), δ 4.11 (t, 2H), δ 3.87-3.81 (m, 1H), δ 3.75 (t, 2H), δ 3.61 (t, 2H), δ 3.42-3.37 (m, 1H), δ 2.50-2.43 (m, 1H), δ 2.33-2.23 (m, 6H), δ 1.89-1.15 (br. m, 64H), δ 0.96-0.87 (m, 12H) δ 0.26 (d, 6H); TOF MS ES⁺ [M+H⁺]: 814.7308 m/z

### Example 156

### Synthesis of 7-(4-hydroxybutyl)-15,15-dimethyl-17-propyl-14,16,18-trioxa-7-aza-15-silatriacontyl 2-hexyldecanoate

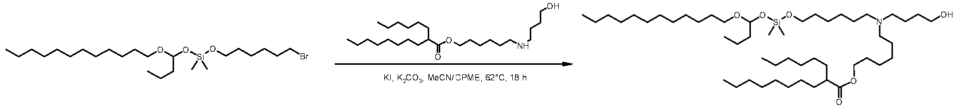

7-(4-hydroxybutyl)-15,15-dimethyl-17-propyl-14,16,18-trioxa-7-aza-15-silatriacontyl 2-hexyldecanoate was prepared from 1-bromo-8,8-dimethyl-10-propyl-7,9,11-trioxa-8-silatricosane (903.91 mg, 1.824 mmol, 1.2 eq.) and 6-((4-hydroxybutyl)amino)hexyl 2-hexyldecanoate (650 mg, 1.520 mmol, 1.0 eq.) according to **General Procedure K.** The crude product was purified by flash column chromatography according to **Purification Method B** to obtain the pure product as a colorless oil, 285 mg, 0.338 mmol, 22 % yield.

H NMR (500 MHz, Benzene-d₆) δ 4.97 (t, 1H), δ 4.11 (t, 2H), δ 3.89-3.81 (m, 1H), δ 3.75 (t, 2H), δ 3.62 (t, 2H), δ 3.44-3.37 (m, 1H), δ 2.50-2.42 (m, 1H), δ 2.36-2.23 (m, 6H), δ 1.89-1.15 (br. m, 68H), δ 0.96-0.90 (m, 12H) δ 0.26 (d, 6H); LC ESI MS [M+H⁺]: 842.890 m/z

### Example 157

### Synthesis of 14-(4-hydroxybutyl)-6,6-dimethyl-4-nonyl-3,5,7-trioxa-14-aza-6-silaicosan-20-yl 2-hexyldecanoate

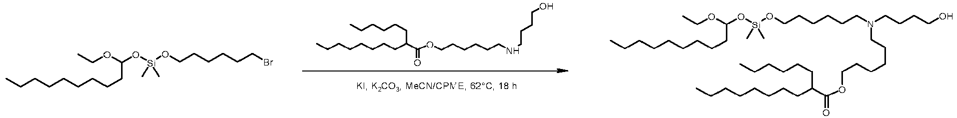

14-(4-hydroxybutyl)-6,6-dimethyl-4-nonyl-3,5,7-trioxa-14-aza-6-silaicosan-20-yl 2-hexyldecanoate was prepared from 13-bromo-6,6-dimethyl-4-nonyl-3,5,7-trioxa-6-silatridecane (644.36 mg, 1.466 mmol, 1.1 eq.) and 6-((4-hydroxybutyl)amino)hexyl 2-hexyldecanoate (570 mg, 1.33 mmol, 1.0 eq.) according to **General Procedure K.** The crude product was purified by flash column chromatography according to **Purification Method B** to obtain the pure product as a colourless oil, 100 mg, 0.127 mmol, 10 % yield.

¹H NMR (500 MHz, Benzene-d₆) δ 4.97 (dt, 1H), δ 4.12 (t, 2H), δ 3.87-3.80 (m, 1H), δ 3.75 (t, 2H), δ 3.62 (t, 2H), δ 3.45-3.35 (m, 1H), δ 2.50-2.43 (m, 1H), δ 2.35-2.23 (m, 6H), δ 1.94-1.72 (m, 4H), δ 1.65-1.14 (br. m, 60H), δ 0.95-0.88 (m, 9H), δ 0.26 (d, 6H); TOF MS ES⁺ [M+H⁺]: 786.7024 m/z

### Example 158

### Synthesis of 14-(4-hydroxybutyl)-6,6-dimethyl-4-undecyl-3,5,7-trioxa-14-aza-6-silaicosan-20-yl 2-hexyldecanoate

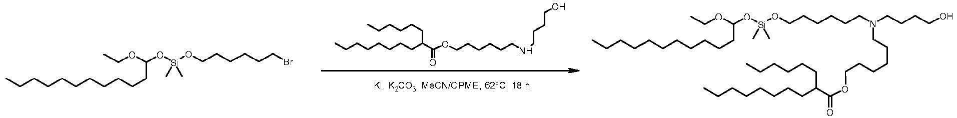

14-(4-hydroxybutyl)-6,6-dimethyl-4-undecyl-3,5,7-trioxa-14-aza-6-silaicosan-20-yl 2-hexyldecanoate was prepared from 13-bromo-6,6-dimethyl-4-undecyl-3,5,7-trioxa-6-silatridecane (829.38 mg, 1.543 mmol, 1.1 eq.) and 6-((4-hydroxybutyl)amino)hexyl 2-hexyldecanoate (600 mg, 1.403 mmol, 1.0 eq.) according to **General Procedure K.** The crude product was purified by flash column chromatography according to **Purification Method B** to obtain the pure product as a colourless oil, 190 mg, 0.233 mmol, 17 % yield.

¹H NMR (500 MHz, Benzene-d₆) δ 4.97 (t, 1H), δ 4.11 (t, 2H), δ 3.83-3.80 (m, 1H), δ 3.74 (t, 2H), δ 3.62 (t, 2H), δ 3.43-3.34 (m, 1H), δ 2.49-2.42 (m, 1H), δ 2.33-2.25 (m, 6H), δ 1.92-1.76 (m, 4H), δ 1.65-1.14 (br. m, 63H), δ 0.98-0.85 (m, 9H), δ 0.25 (d, 6H); LC ESI MS [M+H⁺]: 814.747 m/z

### Example 159

### Synthesis of 17-heptyl-15,15-dimethyl-7-(2-(1-methylpyrrolidin-2-yl)ethyl)-14,16,18-trioxa-7-aza-15-silahexacosyl 2-hexyldecanoate

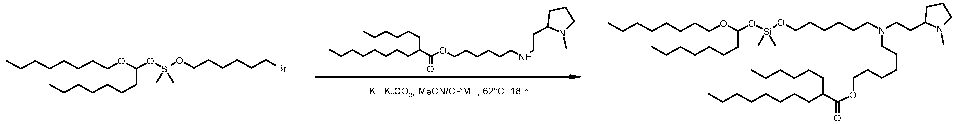

17-heptyl-15,15-dimethyl-7-(2-(1-methylpyrrolidin-2-yl)ethyl)-14,16,18-trioxa-7-aza-15-silahexacosyl 2-hexyldecanoate was prepared from 1-bromo-10-heptyl-8,8-dimethyl-7,9,11-trioxa-8-silanonadecane (233.6 mg, 0.471 mmol, 1.0 eq.) and 6-((2-(1-methylpyrrolidin-2-yl)ethyl)amino)hexyl 2-hexyldecanoate (220 mg, 0.471 mmol, 1.0 eq.) according to **General Procedure K.** The crude product was purified by flash column chromatography according to **Purification Method B** to obtain the pure product as a colourless oil, 72 mg, 0.082 mmol, 17 % yield.

¹H NMR (500 MHz, Benzene-d₆) δ 5.01 (dt, 1H), δ 4.13 (t, 2H), δ 3.91-3.84 (m, 1H), δ 3.78 (t, 2H), δ 3.45-3.40 (m, 1H), δ 3.00 (t, 1H) δ 2.55-2.35 (m, 7H), δ 2.29 (s, 3H), δ 2.12-2.00 (m, 2H) δ 1.96-1.20 (br. m, 72H), δ 0.94-0.89 (m, 12H), δ 0.29 (d, 6H); TOF MS ES⁺ [M+H⁺]: 881.8089 m/z

### Example 160

### Synthesis of 16-heptyl-14,14-dimethyl-7-(2-(1-methylpyrrolidin-2-yl)ethyl)-15,17-dioxa-7-aza-14-silapentacosyl 2-hexyldecanoate

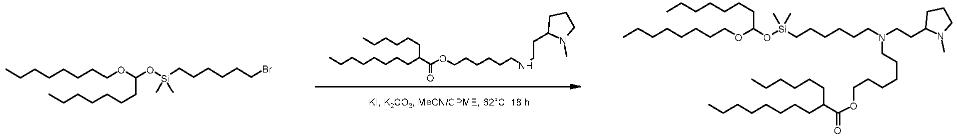

16-heptyl-14,14-dimethyl-7-(2-(1-methylpyrrolidin-2-yl)ethyl)-15,17-dioxa-7-aza-14-silapentacosyl 2-hexyldecanoate was prepared from (6-bromohexyl)dimethyl((1-(octyloxy)octyl)oxy)silane (81.76 mg, 0.170 mmol, 1.14 eq.) and 6-((2-(1-methylpyrrolidin-2-yl)ethyl)amino)hexyl 2-hexyldecanoate (70.00 mg, 0.15 mmol, 1.0 eq.) according to **General Procedure K.** The crude product was purified by flash column chromatography according to **Purification Method B** to obtain the pure product as a colourless oil, 40 mg, 0.046 mmol, 31 % yield.

¹H NMR (500 MHz, Benzene-d₆) δ 4.89 (t, 1H), δ 4.13 (t, 2H), δ 3.78-3.74 (m, 1H), δ 3.41-3.36 (m, 1H), δ 3.00 (td, 1H) δ 2.54-2.38 (m, 6H), δ 2.29 (s, 3H), δ 2.12-1.93 (m, 3H) δ 1.89-1.20 (br. m, 70H), δ 0.94-0.88 (m, 12H), δ 0.78-0.73 (m, 2H) δ 0.29 (d, 6H)

### Example 161

### Synthesis of 16-(4-hydroxybutyl)-8,8-dimethyl-6-nonyl-5,7,9-trioxa-16-aza-8-siladocosan-22-yl 2-hexyldecanoate

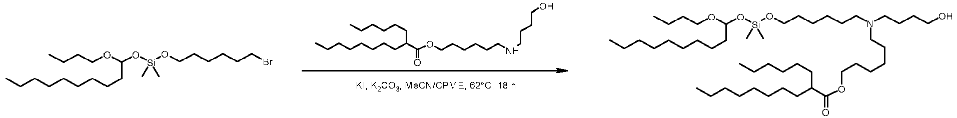

16-(4-hydroxybutyl)-8,8-dimethyl-6-nonyl-5,7,9-trioxa-16-aza-8-siladocosan-22-yl 2-hexyldecanoate was prepared from 15-bromo-8,8-dimethyl-6-nonyl-5,7,9-trioxa-8-silapentadecane (661.41 mg, 1.414 mmol, 1.1 eq.) and 6-((4-hydroxybutyl)amino)hexyl 2-hexyldecanoate (550 mg, 1.286 mmol, 1.0 eq.) according to **General Procedure K.** The crude product was purified by flash column chromatography according to **Purification Method A** to obtain the pure product as a colourless oil, 112 mg, 0.137 mmol, 11 % yield.

¹H NMR (500 MHz, Benzene-d₆) δ 4.99 (dt, 1H), δ 4.12 (t, 2H), δ 3.88-3.80 (m, 1H), δ 3.77 (t, 2H), δ 3.62 (t, 2H), δ 3.43-3.36 (m, 1H), δ 2.51-2.44 (m, 1H), δ 2.35-2.23 (m, 6H), δ 1.97-1.76 (m, 4H), δ 1.68-1.13 (br. m, 60H), δ 0.94-0.89 (m, 12H), δ 0.29 (d, 6H); LC ESI MS [M+H⁺]: 814.702 m/z

### Example 162

### Synthesis of 16-(4-hydroxybutyl)-8,8-dimethyl-6-undecyl-5,7,9-trioxa-16-aza-8-siladocosan-22-yl 2-hexyldecanoate

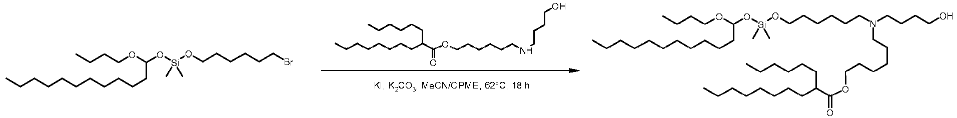

16-(4-hydroxybutyl)-8,8-dimethyl-6-undecyl-5,7,9-trioxa-16-aza-8-siladocosan-22-yl 2-hexyldecanoate was prepared from 15-bromo-8,8-dimethyl-6-undecyl-5,7,9-trioxa-8-silapentadecane (411.21 mg, 0.772 mmol, 1.1 eq.) and 6-((4-hydroxybutyl)amino)hexyl 2-hexyldecanoate (300 mg, 0.702 mmol, 1.0 eq.) according to **General Procedure K.** The crude product was purified by flash column chromatography according to **Purification Method A** to obtain the pure product as a colourless oil, 186 mg, 0.221 mmol, 32 % yield.

¹H NMR (500 MHz, Benzene-d₆) δ 4.99 (dt, 1H), δ 4.12 (t, 2H), δ 3.94-3.80 (m, 1H), δ 3.76 (t, 2H), δ 3.62 (t, 2H), δ 3.43-3.36 (m, 1H), δ 2.52-2.38 (m, 1H), δ 2.36-2.24 (m, 6H), δ 1.97-1.75 (m, 4H), δ 1.68-1.10 (br. m, 64H), δ 0.95-0.85 (m, 12H), δ 0.27 (d, 6H); TOF MS ES⁺ [M+H⁺]: 842.7631 m/z

### Example 163

### Synthesis of 14-(4-hydroxybutyl)-4,6,6-trimethyl-3,5,7-trioxa-14-aza-6-silaicosan-20-yl 2-hexyldecanoate

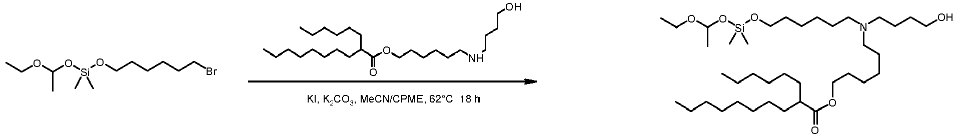

14-(4-hydroxybutyl)-4,6,6-trimethyl-3,5,7-trioxa-14-aza-6-silaicosan-20-yl 2-hexyldecanoate was prepared from 13-bromo-4,6,6-trimethyl-3,5,7-trioxa-6-silatridecane (311.48 mg, 0.952 mmol, 1.1 eq.) and 6-((4-hydroxybutyl)amino)hexyl 2-hexyldecanoate (370 mg, 0.865 mmol, 1.0 eq.) according to **General Procedure K.** The crude product was purified by flash column chromatography according to **Purification Method B** to obtain the pure product as a colourless oil, 170 mg, 0.252 mmol, 29 % yield.

¹H NMR (500 MHz, Benzene-d₆) δ 5.04 (q, 1H), δ 4.11 (t, 2H), δ 3.82-3.72 (m, 1H), δ 3.70 (t, 2H), δ 3.62 (t, 2H), δ 3.37-3.27 (m, 1H), δ 2.52-2.38 (m, 1H), δ 2.34-2.23 (m, 6H), δ 1.86-1.75 (m, 2H), δ 1.66-1.10 (br. m, 48H), δ 0.94-0.87 (m, 6H), δ 0.21 (s, 6H); TOF MS ES⁺ [M+H⁺]: 674.5767 m/z

### Example 164

### Synthesis of 17-heptyl-7-(3-hydroxypropyl)-15,15-dimethyl-14,16,18-trioxa-7-aza-15-silahexacosyl 2-hexyldecanoate

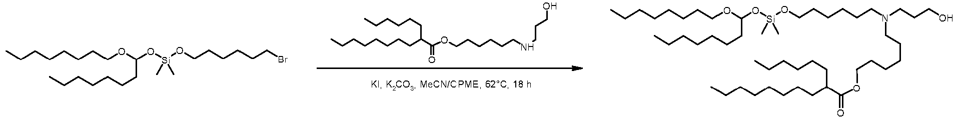

17-heptyl-7-(3-hydroxypropyl)-15,15-dimethyl-14,16,18-trioxa-7-aza-15-silahexacosyl 2-hexyldecanoate was prepared from 1-bromo-10-heptyl-8,8-dimethyl-7,9,11-trioxa-8-silanonadecane (498.19 mg, 1.00 mmol, 1.1 eq.) and 6-((3-hydroxypropyl)amino)hexyl 2-hexyldecanoate (378.02 mg, 0.914 mmol, 1.0 eq.) according to **General Procedure K.** The crude product was purified by flash column chromatography according to **Purification Method B** to obtain the pure product as a colourless oil, 50 mg, 0.060 mmol, 6 % yield.

¹H NMR (500 MHz, Benzene-d₆) δ 5.01 (dt, 1H), δ 4.12 (t, 2H), δ 3.91-3.82 (m, 1H), δ 3.75 (t, 2H), δ 3.46-3.38 (m, 1H), δ 2.52-2.43 (m, 1H), δ 2.41-2.21 (m, 6H), δ 1.95-1.76 (m, 4H), δ 1.69-1.10 (br. m, 64H), δ 0.97-0.81 (m, 12H), δ 0.29 (d, 6H); TOF MS ES⁺ [M+H⁺]: 828.7505 m/z

### Example 165

### Synthesis of 16-heptyl-7-(2-hydroxyethyl)-14,14-dimethyl-15,17-dioxa-7-aza-14-silapentacosyl 2-hexyldecanoate

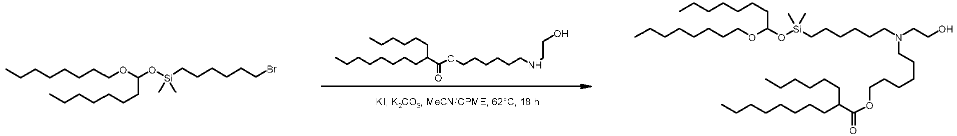

16-heptyl-7-(2-hydroxyethyl)-14,14-dimethyl-15,17-dioxa-7-aza-14-silapentacosyl 2-hexyldecanoate was prepared from (6-bromohexyl)dimethyl((1-(octyloxy)octyl)oxy)silane (263.80 mg, 0.550 mmol, 1.1 eq.) and 6-((2-hydroxyethyl)amino)hexyl 2-hexyldecanoate (200 mg, 0.500 mmol, 1.0 eq.) according to **General Procedure K.** The crude product was purified by flash column chromatography according to **Purification Method B** to obtain the pure product as a colourless oil, 180 mg, 0.225 mmol, 45 % yield.

¹H NMR (500 MHz, Benzene-d₆) δ 4.90-4.87 (m, 1H), δ 4.12 (t, 2H), δ 3.79-3.74 (m, 1H), δ 3.51 (t, 2H), δ 3.46-3.36 (m, 2H), δ 2.51-2.46 (m, 1H), δ 2.39-2.26 (m, 6H), δ 1.88-1.73 (m, 4H), δ 1.69-1.18 (br. m, 62H), δ 0.94-0.87 (m, 12H), δ 0.26 (d, 6H); TOF MS ES⁺ [M+H⁺]: 798.7377 m/z

### Example 166

### Synthesis 6-((6-((dimethyl(octyl)silyl)oxy)-6-(octyloxy)hexyl)(4-hydroxybutyl)amino)hexyl 2-(decylthio)hexanoate

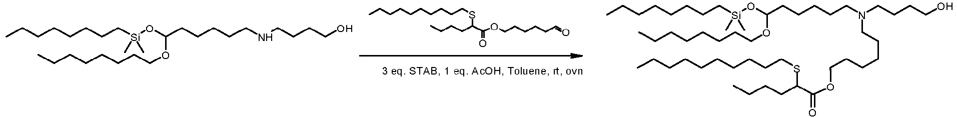

6-((6-((dimethyl(octyl)silyl)oxy)-6-(octyloxy)hexyl)(4-hydroxybutyl)amino)hexyl 2-(decylthio)hexanoate was prepared from 4-((6-((dimethyl(octyl)silyl)oxy)-6-(octyloxy)hexyl)amino)butan-1-ol (150.00 mg, 0.307 mmol, 1.0 eq.) and 6-oxohexyl 2-(decylthio)hexanoate (118.87 mg, 0.307 mmol, 1.0 eq.) according to **General Procedure J.** The crude product was purified twice by flash column chromatography according to **Purification Method A and B** to obtain the pure product as a colourless oil, 130 mg, 0.151 mmol, 49 % yield.

¹H NMR (500 MHz, Benzene-d₆) δ 4.89 (dt, 1H), δ 4.17-4.06 (m, 2H), δ 3.79-3.73 (m, 1H), δ 3.62 (t, 2H), δ 3.42-3.30 (m, 2H), δ 2.78-2.71 (m, 1H), δ 2.67-2.60 (m, 1H), δ 2.35-2.23 (m, 6H), δ 2.09-1.99 (m, 1H), δ 1.89-1.11 (m, 65H), δ 0.94-0.89 (m, 9H), δ 0.83 (t, 3H), δ 0.77-0.72 (m, 2H), δ 0.26 (d, 6H); TOF MS ES⁺ [M+H⁺]: 858.7409 m/z

### Example 167

### Synthesis 15-heptyl-7-(4-hydroxybutyl)-17,17-dimethyl-14,16-dioxa-7-aza-17-silapentacosyl 2-(decylthio)hexanoate

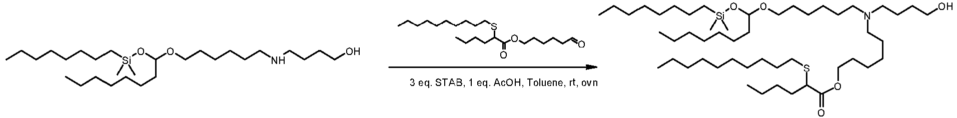

15-heptyl-7-(4-hydroxybutyl)-17,17-dimethyl-14,16-dioxa-7-aza-17-silapentacosyl 2-(decylthio)hexanoate was prepared from 13-heptyl-15,15-dimethyl-12,14-dioxa-5-aza-15-silatricosan-1-ol (150.00 mg, 0.307 mmol, 1.0 eq.) and 6-oxohexyl 2-(decylthio)hexanoate (118.87 mg, 0.307 mmol, 1.0 eq.) according to **General Procedure J.** The crude product was purified twice by flash column chromatography according to **Purification Method A and B** to obtain the pure product as a colourless oil, 142 mg, 0.165 mmol, 54 % yield.

¹H NMR (500 MHz, Benzene-d₆) δ 4.88 (dt, 1H), δ 4.16-4.07 (m, 2H), δ 3.79-3.73 (m, 1H), δ 3.62 (t, 2H), δ 3.41-3.30 (m, 2H), δ 2.77-2.70 (m, 1H), δ 2.67-2.60 (m, 1H), δ 2.34-2.23 (m, 6H), δ 2.08-1.99 (m, 1H), δ 1.88-1.12 (m, 65H), δ 0.94-0.88 (m, 9H), δ 0.83 (t, 3H), δ 0.76-0.72 (m, 2H), δ 0.25 (d, 6H); TOF MS ES⁺ [M+H⁺]: 858.7404 m/z

### Example 168

### Synthesis of 7-(4-hydroxybutyl)-15,15-dimethyl-13-(octyloxy)-14,16-dioxa-7-aza-15-silatetracosyl 2-(decylthio)hexanoate

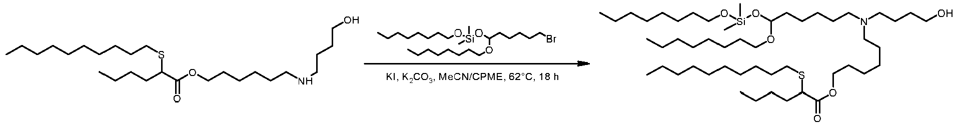

7-(4-hydroxybutyl)-15,15-dimethyl-13-(octyloxy)-14,16-dioxa-7-aza-15-silatetracosyl 2-(decylthio)hexanoate was prepared from 12-(5-bromopentyl)-10,10-dimethyl-9,11,13-trioxa-10-silahenicosane (177.88 mg, 0.359 mmol, 1.1 eq.) and 6-((4-hydroxybutyl)amino)hexyl 2-(decylthio)hexanoate (150.00 mg, 0.326 mmol, 1.0 eq.) according to **General Procedure K.** The crude product was purified twice by flash column chromatography according to **Purification Method A** to obtain the pure product as a colourless oil, 140 mg, 0.160 mmol, 49 % yield.

¹H NMR (500 MHz, Benzene-d₆) δ 5.01 (dt, 1H), δ 4.16-4.06 (m, 2H), δ 3.89-3.84 (m, 1H), δ 3.77 (t, 2H), δ 3.62 (t, 2H), δ 3.45-3.30 (m, 2H), δ 2.77-2.70 (m, 1H), δ 2.67-2.60 (m, 1H), δ 2.37-2.19 (m, 6H), δ 2.08-1.99 (m, 1H), δ 1.95-1.09 (m, 65H), δ 0.95-0.87 (m, 9H), δ 0.83 (t, 3H), δ 0.28 (d, 6H); TOF MS ES⁺ [M+H⁺]: 874.7339 m/z

### Example 169

### Synthesis of 15-heptyl-7-(4-hydroxybutyl)-17,17-dimethyl-14,16,18-trioxa-7-aza-17-silahexacosyl 2-(decylthio)hexanoate

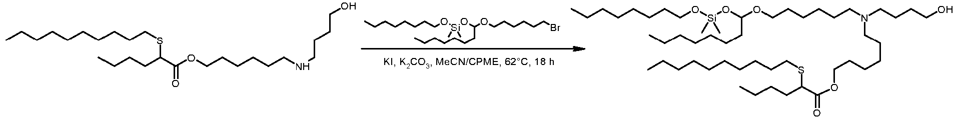

15-heptyl-7-(4-hydroxybutyl)-17,17-dimethyl-14,16,18-trioxa-7-aza-17-silahexacosyl 2-(decylthio)hexanoate was prepared from 1-bromo-8-heptyl-10,10-dimethyl-7,9,11-trioxa-10-silanonadecane (161.71 mg, 0.326 mmol, 1.0 eq.) and 6-((4-hydroxybutyl)amino)hexyl 2-(decylthio)hexanoate (150.00 mg, 0.326 mmol, 1.0 eq.) according to **General Procedure K.** The crude product was purified twice by flash column chromatography according to **Purification Method A** to obtain the pure product as a colourless oil, 123 mg, 0.141 mmol, 43 % yield.

¹H NMR (500 MHz, Benzene-d₆) δ 5.01 (dt, 1H), δ 4.16-4.07 (m, 2H), δ 3.93-3.84 (m, 1H), δ 3.77 (t, 2H), δ 3.62 (t, 2H), δ 3.45-3.30 (m, 2H), δ 3.78-3.71 (m, 1H), δ 2.67-2.60 (m, 1H), δ 2.34-2.23 (m, 6H), δ 2.09-2.00 (m, 1H), δ 1.95-1.11 (m, 65H), δ 0.94-0.89 (m, 9H), δ 0.83 (t, 3H), δ 0.28 (d, 6H); TOF MS ES⁺ [M+H⁺]: 874.7355 m/z

### Example 170

### Synthesis of 6-((4-hydroxybutyl)(6-(octyloxy)-6-((1,1,3,3-tetramethyl-3-octyldisiloxaneyl)oxy)hexyl)amino)hexyl 2-(decylthio)hexanoate

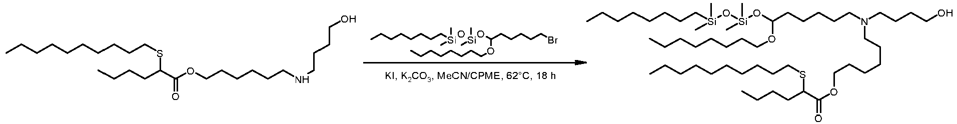

6-((4-hydroxybutyl)(6-(octyloxy)-6-((1,1,3,3-tetramethyl-3-octyldisiloxaneyl)oxy)hexyl)amino)hexyl 2-(decylthio)hexanoate was prepared from 1-((6-bromo-1-(octyloxy)hexyl)oxy)-1,1,3,3-tetramethyl-3-octyldisiloxane (156.59 mg, 0.2828 mmol, 1.0 eq.) and 6-((4-hydroxybutyl)amino)hexyl 2-(decylthio)hexanoate (130.00 mg, 0.2828 mmol, 1.0 eq.) according to **General Procedure K.** The crude product was purified twice by flash column chromatography according to **Purification Method B** to obtain the pure product as a colourless oil, 152 mg, 0.163 mmol, 58 % yield.

¹H NMR (500 MHz, Benzene-d₆) δ 5.00 (dt, 1H), δ 4.17-4.05 (m, 2H), δ 3.90-3.82 (m, 1H), δ 3.61 (t, 2H), δ 3.46-3.28 (m, 2H), δ 3.78-3.58 (m, 2H), δ 2.36-2.23 (m, 6H), δ 2.09-1.97 (m, 1H), δ 1.93-1.09 (m, 65H), δ 0.94-0.88 (m, 9H), δ 0.83 (t, 3H), δ 0.70-0.65 (m, 2H), δ 0.27 (d, 6H), δ 0.21 (d, 6H); TOF MS ES⁺ [M+H⁺]: 932.7584 m/z

### Example 171

### Synthesis of 15-heptyl-7-(4-hydroxybutyl)-17,17,19,19-tetramethyl-14,16,18-trioxa-7-aza-17,19-disilaheptacosyl 2-(decylthio)hexanoate

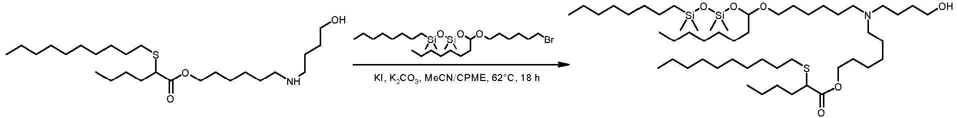

15-heptyl-7-(4-hydroxybutyl)-17,17,19,19-tetramethyl-14,16,18-trioxa-7-aza-17,19-disilaheptacosyl 2-(decylthio)hexanoate was prepared from 1-((1-((6-bromohexyl)oxy)octyl)oxy)-1,1,3,3-tetramethyl-3-octyldisiloxane (177.88 mg, 0.321 mmol, 0.98 eq.) and 6-((4-hydroxybutyl)amino)hexyl 2-(decylthio)hexanoate (150.00 mg, 0.326 mmol, 1.0 eq.) according to **General Procedure K.** The crude product was purified twice by flash column chromatography according to **Purification Method A** to obtain the pure product as a colourless oil, 184 mg, 0.197 mmol, 60 % yield.

¹H NMR (500 MHz, Benzene-d₆) δ 5.00 (t, 1H), δ 4.16-4.05 (m, 2H), δ 3.89-3.84 (m, 1H), δ 3.62 (t, 2H), δ 3.45-3.39 (m, 1H), δ 3.34-3.30 (m, 1H), δ 3.77-3.70 (m, 1H), δ 2.67-2.60 (m, 1H), δ 2.34-2.23 (m, 6H), δ 2.08-1.99 (m, 1H), δ 1.95-1.12 (m, 65H), δ 0.95-0.89 (m, 9H), δ 0.83 (t, 3H), δ 0.71-0.65 (m, 2H), δ 0.27 (d, 6H), δ 0.21 (d, 6H); TOF MS ES⁺ [M+H⁺]: 932.7599 m/z

### Example 172

### Synthesis 6-((4-hydroxybutyl)(6-(octyloxy)-6-((1,1,3,3-tetramethyl-3-(octyloxy)disiloxaneyl)oxy)hexyl)amino)hexyl 2-(decylthio)hexanoate

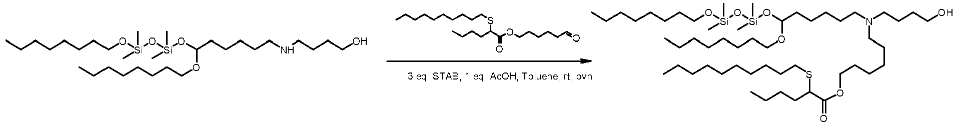

6-((4-hydroxybutyl)(6-(octyloxy)-6-((1,1,3,3-tetramethyl-3-(octyloxy)disiloxaneyl)oxy)hexyl)amino)hexyl 2-(decylthio)hexanoate was prepared from 4-((6-(octyloxy)-6-((1,1,3,3-tetramethyl-3-(octyloxy)disiloxaneyl)oxy)hexyl)amino)butan-1-ol (90.00 mg, 0.159 mmol, 1.0 eq.) and 6-oxohexyl 2-(decylthio)hexanoate (61.54 mg, 0.159 mmol, 1.0 eq.) according to **General Procedure J.** The crude product was purified twice by flash column chromatography according to **Purification Method A and B** to obtain the pure product as a colourless oil, 40 mg, 0.042 mmol, 26 % yield.

¹H NMR (500 MHz, Benzene-d₆) δ 5.04 (dt, 1H), δ 4.16-4.06 (m, 2H), δ 3.91-3.86 (m, 1H), δ 3.76 (t, 2H), δ 3.62 (t, 2H), δ 3.47-3.40 (m, 1H), δ 3.34-3.30 (m, 1H), δ 2.77-2.71 (m, 1H), δ 2.67-2.60 (m, 1H), δ 2.37-2.21 (m, 6H), δ 2.11-1.98 (m, 1H), δ 1.95-1.11 (m, 65H), δ 0.94-0.89 (m, 9H), δ 0.83 (t, 3H), δ 0.31 (d, 6H), δ 0.25 (s, 6H); TOF MS ES⁺ [M+H⁺]: 948.7556 m/z

### Example 173

### Synthesis of 15-heptyl-7-(4-hydroxybutyl)-17,17,19,19-tetramethyl-14,16,18,20-tetraoxa-7-aza-17,19-disilaoctacosyl 2-(decylthio)hexanoate

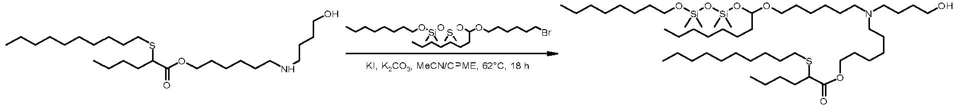

15-heptyl-7-(4-hydroxybutyl)-17,17,19,19-tetramethyl-14,16,18,20-tetraoxa-7-aza-17,19-disilaoctacosyl 2-(decylthio)hexanoate was prepared from 1-((1-((6-bromohexyl)oxy)octyl)oxy)-1,1,3,3-tetramethyl-3-(octyloxy)disiloxane (204.49 mg, 0.359 mmol, 1.1 eq.) and 6-((4-hydroxybutyl)amino)hexyl 2-(decylthio)hexanoate (150.00 mg, 0.326 mmol, 1.0 eq.) according to **General Procedure K.** The crude product was purified twice by flash column chromatography according to **Purification Method A** to obtain the pure product as a colourless oil, 192 mg, 0.202 mmol, 62 % yield.

¹H NMR (500 MHz, Benzene-d₆) δ 5.04 (dt, 1H), δ 4.16-4.06 (m, 2H), δ 3.90-3.85 (m, 1H), δ 3.76 (t, 2H), δ 3.62 (t, 2H), δ 3.46-3.40 (m, 1H), δ 3.33-3.30 (m, 1H), δ 2.77-2.70 (m, 1H), δ 2.67-2.60 (m, 1H), δ 2.34-2.23 (m, 6H), δ 2.08-1.99 (m, 1H), δ 1.96-1.12 (m, 65H), δ 0.95-0.88 (m, 9H), δ 0.83 (t, 3H), δ 0.31 (d, 6H), δ 0.24 (s, 6H); ; TOF MS ES⁺ [M+H⁺]: 948.7554 m/z

### Example 174

### Synthesis of 18-hexyl-7-(4-hydroxybutyl)-15,15-dimethyl-13-(octyloxy)-14,16-dioxa-7-aza-15-silahexacosyl 2-(decylthio)hexanoate

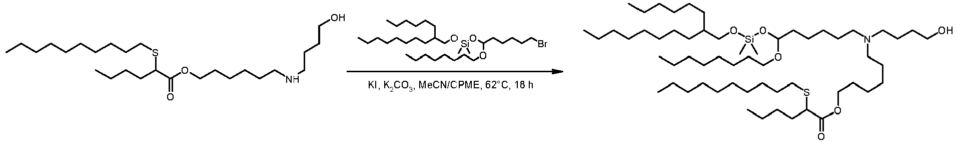

18-hexyl-7-(4-hydroxybutyl)-15,15-dimethyl-13-(octyloxy)-14,16-dioxa-7-aza-15-silahexacosyl 2-(decylthio)hexanoate was prepared from 10-(5-bromopentyl)-15-hexyl-12,12-dimethyl-9,11,13-trioxa-12-silatricosane (218.15 mg, 0.359 mmol, 1.1 eq.) and 6-((4-hydroxybutyl)amino)hexyl 2-(decylthio)hexanoate (150.00 mg, 0.326 mmol, 1.0 eq.) according to **General Procedure K.** The crude product was purified twice by flash column chromatography according to **Purification Method A** to obtain the pure product as a colourless oil, 158 mg, 0.160 mmol, 49 % yield.

¹H NMR (500 MHz, Benzene-d₆) δ 5.03 (dt, 1H), δ 4.16-4.07 (m, 2H), δ 3.92-3.86 (m, 1H), δ 3.76 (d, 2H), δ 3.62 (t, 2H), δ 3.48-3.42 (m, 1H), δ 3.34-3.30 (m, 1H), δ 2.77-2.70 (m, 1H), δ 2.67-2.60 (m, 1H), δ 2.36-2.24 (m, 6H), δ 2.08-1.99 (m, 1H), δ 1.97-1.12 (m, 78H), δ 0.95-0.89 (m, 12H), δ 0.83 (t, 3H), δ 0.30 (d, 6H); TOF MS ES⁺ [M+H⁺]: 986.8613 m/z

### Example 175

### Synthesis of 15-heptyl-20-hexyl-7-(4-hydroxybutyl)-17,17-dimethyl-14,16,18-trioxa-7-aza-17-silaoctacosyl 2-(decylthio)hexanoate

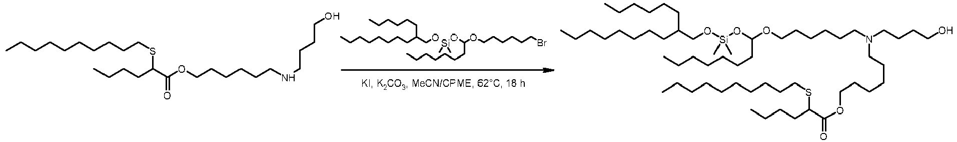

15-heptyl-20-hexyl-7-(4-hydroxybutyl)-17,17-dimethyl-14,16,18-trioxa-7-aza-17-silaoctacosyl 2-(decylthio)hexanoate was prepared from 1-bromo-8-heptyl-13-hexyl-10,10-dimethyl-7,9,11-trioxa-10-silahenicosane (218.15 mg, 0.359 mmol, 1.1 eq.) and 6-((4-hydroxybutyl)amino)hexyl 2-(decylthio)hexanoate (150.00 mg, 0.326 mmol, 1.0 eq.) according to **General Procedure K.** The crude product was purified twice by flash column chromatography according to **Purification Method A** to obtain the pure product as a colourless oil, 190 mg, 0.193 mmol, 59 % yield.

¹H NMR (500 MHz, Benzene-d₆) δ 5.02 (dt, 1H), δ 4.16-4.07 (m, 2H), δ 3.92-3.85 (m, 1H), δ 3.75 (d, 2H), δ 3.62 (t, 2H), δ 3.46-3.40 (m, 1H), δ 3.34-3.30 (m, 1H), δ 2.77-2.70 (m, 1H), δ 2.67-2.60 (m, 1H), δ 2.34-2.24 (m, 6H), δ 2.08-1.99 (m, 1H), δ 1.96-1.12 (m, 78H), δ 0.96-0.89 (m, 12H), δ 0.83 (t, 3H), δ 0.30 (d, 6H); LC ESI MS [M+H⁺]: 986.763 m/z

### Example 176

### Synthesis of 17-heptyl-7-(4-hydroxybutyl)-15,15-dimethyl-14,16,18-trioxa-7-aza-15-silahexacosyl 2-(decylthio)hexanoate

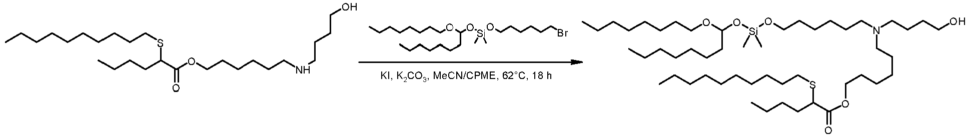

17-heptyl-7-(4-hydroxybutyl)-15,15-dimethyl-14,16,18-trioxa-7-aza-15-silahexacosyl 2-(decylthio)hexanoate was prepared from 1-bromo-10-heptyl-8,8-dimethyl-7,9,11-trioxa-8-silanonadecane (177.88 mg, 0.359 mmol, 1.1 eq.) and 6-((4-hydroxybutyl)amino)hexyl 2-(decylthio)hexanoate (150.00 mg, 0.326 mmol, 1.0 eq.) according to **General Procedure K.** The crude product was purified twice by flash column chromatography according to **Purification Method A** to obtain the pure product as a colourless oil, 94 mg, 0.107 mmol, 33 % yield.

¹H NMR (500 MHz, Benzene-d6) δ 5.00 (dt, 1H), δ 4.16-4.07 (m, 2H), δ 3.89-3.84 (m, 1H), δ 3.77 (t, 2H), δ 3.62 (t, 2H), δ 3.45-3.37 (m, 1H), δ 3.34-3.30 (m, 1H), δ 2.77-2.70 (m, 1H), δ 2.67-2.60 (m, 1H), δ 2.34-2.24 (m, 6H), δ 2.08-1.99 (m, 1H), δ 1.95-1.12 (m, 65H), δ 0.95-0.87 (m, 9H), δ 0.83 (t, 3H), δ 0.28 (d, 6H); TOF MS ES⁺ [M+H⁺]: 874.7365 m/z

### Example 177

### Synthesis of 6-((6-((dimethyl(octyl)silyl)oxy)-6-(octyloxy)hexyl)(4-hydroxybutyl)amino)hexyl 2-(pentylthio)decanoate

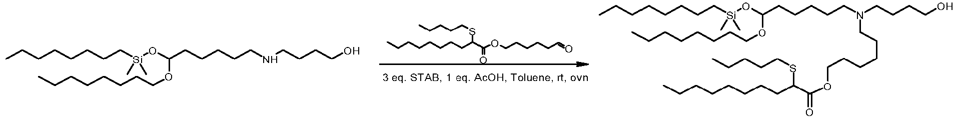

6-((6-((dimethyl(octyl)silyl)oxy)-6-(octyloxy)hexyl)(4-hydroxybutyl)amino)hexyl 2-(pentylthio)decanoate was prepared from 4-((6-((dimethyl(octyl)silyl)oxy)-6-(octyloxy)hexyl)amino)butan-1-ol (150.00 mg, 0.307 mmol, 1.0 eq.) and 6-oxohexyl 2-(pentylthio)decanoate (114.56 mg, 0.307 mmol, 1.0 eq.) according to **General Procedure J.** The crude product was purified twice by flash column chromatography according to **Purification Method A and B** to obtain the pure product as a colourless oil, 120 mg, 0.142 mmol, 46 % yield.

¹H NMR (500 MHz, Benzene-d₆) δ 4.89 (t, 1H), δ 4.17-4.07 (m, 2H), δ 3.82-3.74 (m, 1H), δ 3.62 (t, 2H), δ 3.42-3.33 (m, 2H), δ 2.75-2.69 (m, 1H), δ 2.65-2.58 (m, 1H), δ 2.35-2.23 (m, 6H), δ 2.12-2.03 (m, 1H), δ 1.89-1.11 (m, 63H), δ 0.95-0.89 (m, 9H), δ 0.84 (t, 3H), δ 0.77-0.73 (m, 2H), δ 0.26 (d, 6H); TOF MS ES⁺ [M+H⁺]: 844.7249 m/z

### Example 178

### Synthesis of 15-heptyl-7-(4-hydroxybutyl)-17,17-dimethyl-14,16-dioxa-7-aza-17-silapentacosyl 2-(pentylthio)decanoate

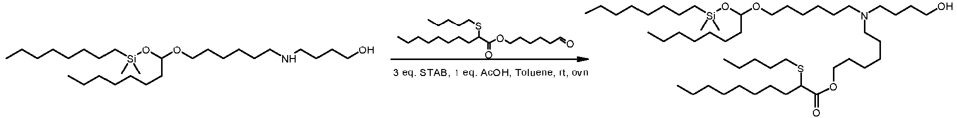

15-heptyl-7-(4-hydroxybutyl)-17,17-dimethyl-14,16-dioxa-7-aza-17-silapentacosyl 2-(pentylthio)decanoate was prepared from 13-heptyl-15,15-dimethyl-12,14-dioxa-5-aza-15-silatricosan-1-ol (150.00 mg, 0.307 mmol, 1.0 eq.) and 6-oxohexyl 2-(pentylthio)decanoate (114.56 mg, 0.307 mmol, 1.0 eq.) according to **General Procedure J.** The crude product was purified twice by flash column chromatography according to **Purification Method A and B** to obtain the pure product as a colourless oil, 142 mg, 0.168 mmol, 55 % yield.

¹H NMR (500 MHz, Benzene-d₆) δ 4.88 (t, 1H), δ 4.17-4.07 (m, 2H), δ 3.79-3.73 (m, 1H), δ 3.62 (t, 2H), δ 3.40-3.31 (m, 2H), δ 2.75-2.68 (m, 1H), δ 2.64-2.57 (m, 1H), δ 2.37-2.23 (m, 6H), δ 2.11-2.00 (m, 1H), δ 1.86-1.11 (m, 63H), δ 0.94-0.91 (m, 9H), δ 0.84 (t, 3H), δ 0.75-0.71 (m, 2H), δ 0.25 (d, 6H); TOF MS ES⁺ [M+H⁺]: 844.7228 m/z

### Example 179

### Synthesis of 7-(4-hydroxybutyl)-15,15-dimethyl-13-(octyloxy)-14,16-dioxa-7-aza-15-silatetracosyl 2-(pentylthio)decanoate

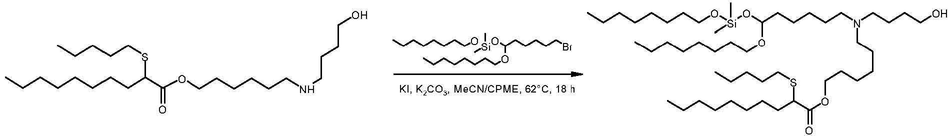

7-(4-hydroxybutyl)-15,15-dimethyl-13-(octyloxy)-14,16-dioxa-7-aza-15-silatetracosyl 2-(pentylthio)decanoate was prepared from 12-(5-bromopentyl)-10,10-dimethyl-9,11,13-trioxa-10-silahenicosane (183.47 mg, 0.370 mmol, 1.1 eq.) and 6-((4-hydroxybutyl)amino)hexyl 2-(pentylthio)decanoate (150 mg, 0.337 mmol, 1.0 eq.) according to **General Procedure K.** The crude product was purified by flash column chromatography according to **Purification Method A** to obtain the pure product as a colourless oil, 132 mg, 0.153 mmol, 46 % yield.

¹H NMR (500 MHz, Benzene-d₆) δ 5.01 (t, 1H), δ 4.17-4.07 (m, 2H), δ 3.89-3.82 (m, 1H), δ 3.77 (t, 2H), δ 3.62 (t, 2H), δ 3.46-3.28 (m, 2H), δ 2.75-2.68 (m, 1H), δ 2.64-2.58 (m, 1H), δ 2.35-2.22 (m, 6H), δ 2.11-2.02 (m, 1H), δ 1.95-1.76 (m, 3H), δ 1.71-1.12 (m, 60H), δ 0.94-0.87 (m, 9H), δ 0.84 (t, 3H), δ 0.28 (d, 6H); TOF MS ES⁺ [M+H⁺]: 860.7214 m/z

### Example 180

### Synthesis of 15-heptyl-7-(4-hydroxybutyl)-17,17-dimethyl-14,16,18-trioxa-7-aza-17-silahexacosyl 2-(pentylthio)decanoate

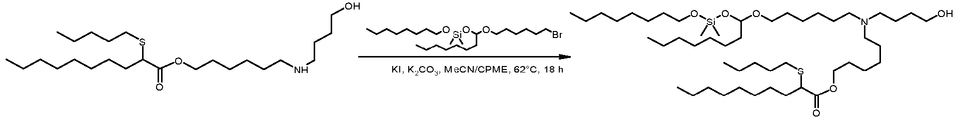

15-heptyl-7-(4-hydroxybutyl)-17,17-dimethyl-14,16,18-trioxa-7-aza-17-silahexacosyl 2-(pentylthio)decanoate was prepared from 1-bromo-8-heptyl-10,10-dimethyl-7,9,11-trioxa-10-silanonadecane (166.79 mg, 0.336 mmol, 1.0 eq.) and 6-((4-hydroxybutyl)amino)hexyl 2-(pentylthio)decanoate (150 mg, 0.336 mmol, 1.0 eq.) according to **General Procedure K.** The crude product was purified by flash column chromatography according to **Purification Method A** to obtain the pure product as a colourless oil, 100 mg, 0.116 mmol, 35 % yield.

¹H NMR (500 MHz, Benzene-d₆) δ 5.00 (t, 1H), δ 4.17-4.07 (m, 2H), δ 3.89-3.83 (m, 1H), δ 3.76 (t, 2H), δ 3.62 (t, 2H), δ 3.44-3.32 (m, 2H), δ 2.75-2.68 (m, 1H), δ 2.64-2.57 (m, 1H), δ 2.33-2.23 (m, 6H), δ 2.11-2.01 (m, 1H), δ 1.95-1.76 (m, 3H), δ 1.71-1.13 (m, 60H), δ 0.94-0.87 (m, 9H), δ 0.84 (t, 3H), δ 0.28 (d, 6H); TOF MS ES⁺ [M+H⁺]: 860.7202 m/z

### Example 181

### Synthesis of 6-((4-hydroxybutyl)(6-(octyloxy)-6-((1,1,3,3-tetramethyl-3-octyldisiloxaneyl)oxy)hexyl)amino)hexyl 2-(pentylthio)decanoate

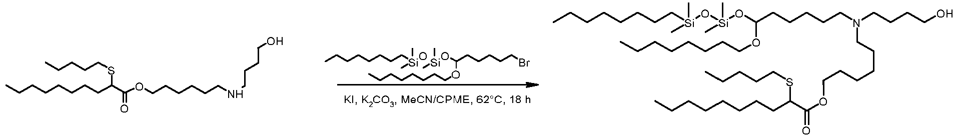

6-((4-hydroxybutyl)(6-(octyloxy)-6-((1,1,3,3-tetramethyl-3-octyldisiloxaneyl)oxy)hexyl)amino)hexyl 2-(pentylthio)decanoate was prepared from 1-((6-bromo-1-(octyloxy)hexyl)oxy)-1,1,3,3-tetramethyl-3-octyldisiloxane (186.36 mg, 0.337 mmol, 1.0 eq.) and 6-((4-hydroxybutyl)amino)hexyl 2-(pentylthio)decanoate (150 mg, 0.337 mmol, 1.0 eq.) according to **General Procedure K.** The crude product was purified by flash column chromatography according to **Purification Method B** to obtain the pure product as a colourless oil, 120 mg, 0.131 mmol, 39 % yield.

¹H NMR (500 MHz, Benzene-d₆) δ 5.00 (t, 1H), δ 4.18-4.05 (m, 2H), δ 3.90-3.82 (m, 1H), δ 3.61 (t, 2H), δ 3.46-3.30 (m, 2H), δ 2.75-2.55 (m, 2H), δ 2.36-2.23 (m, 6H), δ 2.12-1.99 (m, 1H), δ 1.94-1.73 (m, 3H) δ 1.70-1.12 (m, 60H), δ 0.95-0.88 (m, 9H), δ 0.84 (t, 3H), δ 0.70-0.65 (m, 2H), δ 0.27 (d, 6H), δ 0.21 (s, 6H); TOF MS ES⁺ [M+H⁺]: 918.7435 m/z

### Example 182

### Synthesis of 15-heptyl-7-(4-hydroxybutyl)-17,17,19,19-tetramethyl-14,16,18-trioxa-7-aza-17,19-disilaheptacosyl 2-(pentylthio)decanoate

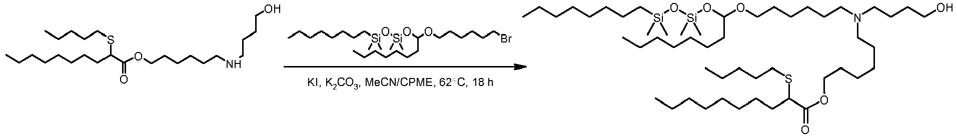

15-heptyl-7-(4-hydroxybutyl)-17,17,19,19-tetramethyl-14,16,18-trioxa-7-aza-17,19-disilaheptacosyl 2-(pentylthio)decanoate was prepared from 1-((1-((6-bromohexyl)oxy)octyl)oxy)-1,1,3,3-tetramethyl-3-octyldisiloxane (161.52 mg, 0.292 mmol, 1.0 eq.) and 6-((4-hydroxybutyl)amino)hexyl 2-(pentylthio)decanoate (130 mg, 0.292 mmol, 1.0 eq.) according to **General Procedure K.** The crude product was purified by flash column chromatography according to **Purification Method B** to obtain the pure product as a colourless oil, 122 mg, 0.133 mmol, 45 % yield.

¹H NMR (500 MHz, Benzene-d₆) δ 5.01 (t, 1H), δ 4.18-4.05 (m, 2H), δ 3.89-3.82 (m, 1H), δ 3.62 (t, 2H), δ 3.45-3.31 (m, 2H), δ 2.76-2.56 (m, 2H), δ 2.34-2.23 (m, 6H), δ 2.12-2.00 (m, 1H), δ 1.96-1.12 (m, 63H), δ 0.94-0.88 (m, 9H), δ 0.84 (t, 3H), δ 0.70-0.65 (m, 2H), δ 0.27 (d, 6H), δ 0.21 (s, 6H); TOF MS ES⁺ [M+H⁺]: 918.7426 m/z

### Example 183

### Synthesis of 6-((4-hydroxybutyl)(6-(octyloxy)-6-((1,1,3,3-tetramethyl-3-(octyloxy)disiloxaneyl)oxy)hexyl)amino)hexyl 2-(pentylthio)decanoate

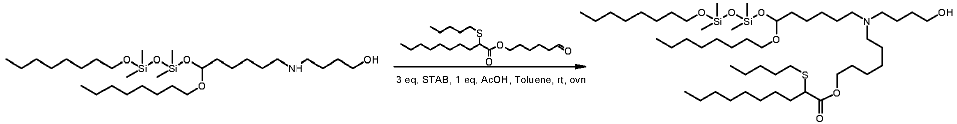

6-((4-hydroxybutyl)(6-(octyloxy)-6-((1,1,3,3-tetramethyl-3-(octyloxy)disiloxaneyl)oxy)hexyl)amino)hexyl 2-(pentylthio)decanoate was prepared from 4-((6-(octyloxy)-6-((1,1,3,3-tetramethyl-3-(octyloxy)disiloxaneyl)oxy)hexyl)amino)butan-1-ol (121.00 mg, 0.209 mmol, 1.0 eq.) and 6-oxohexyl 2-(pentylthio)decanoate (78.00 mg, 0.209 mmol, 1.0 eq.) according to **General Procedure J.** The crude product was purified twice by flash column chromatography according to **Purification Method A and B** to obtain the pure product as a colourless oil, 53 mg, 0.057 mmol, 27 % yield.

¹H NMR (500 MHz, Benzene-d₆) δ 5.04 (t, 1H), δ 4.17-4.07 (m, 2H), δ 3.91-3.86 (m, 1H), δ 3.76 (t, 2H), δ 3.62 (t, 2H), δ 3.47-3.33 (m, 2H), δ 2.75-2.68 (m, 1H), δ 2.65-2.58 (m, 1H), δ 2.36-2.23 (m, 6H), δ 2.12-2.02 (m, 1H), δ 1.97-1.11 (m, 63H), δ 0.93-0.89 (m, 9H), δ 0.84 (t, 3H), δ 0.32 (d, 6H), δ 0.25 (s, 6H); TOF MS ES⁺ [M+H⁺]: 934.7384 m/z

### Example 184

### Synthesis of 15-heptyl-7-(4-hydroxybutyl)-17,17,19,19-tetramethyl-14,16,18,20-tetraoxa-7-aza-17,19-disilaoctacosyl 2-(pentylthio)decanoate

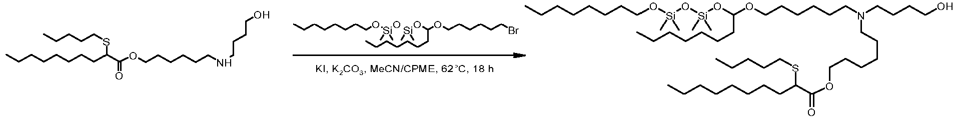

15-heptyl-7-(4-hydroxybutyl)-17,17,19,19-tetramethyl-14,16,18,20-tetraoxa-7-aza-17,19-disilaoctacosyl 2-(pentylthio)decanoate was prepared from 1-((1-((6-bromohexyl)oxy)octyl)oxy)-1,1,3,3-tetramethyl-3-(octyloxy)disiloxane (182.80 mg, 0.321 mmol, 1.1 eq.) and 6-((4-hydroxybutyl)amino)hexyl 2-(pentylthio)decanoate (130.00 mg, 0.292 mmol, 1.0 eq.) according to **General Procedure K.** The crude product was purified twice by flash column chromatography according to **Purification Method A and B** to obtain the pure product as a colourless oil, 101 mg, 0.109 mmol, 37 % yield.

¹H NMR (500 MHz, Benzene-d₆) δ 5.04 (t, 1H), δ 4.17-4.07 (m, 2H), δ 3.90-3.85 (m, 1H), δ 3.76 (t, 2H), δ 3.62 (t, 2H), δ 3.46-3.32 (m, 2H), δ 2.75-2.68 (m, 1H), δ 2.64-2.57 (m, 1H), δ 2.34-2.23 (m, 6H), δ 2.11-2.02 (m, 1H), δ 1.96-1.10 (m, 63H), δ 0.92-0.89 (m, 9H), δ 0.84 (t, 3H), δ 0.31 (d, 6H), δ 0.24 (s, 6H); TOF MS ES⁺[M+H⁺]: 934.7424 m/z

### Example 185

### Synthesis of 18-hexyl-7-(4-hydroxybutyl)-15,15-dimethyl-13-(octyloxy)-14,16-dioxa-7-aza-15-silahexacosyl 2-(pentylthio)decanoate

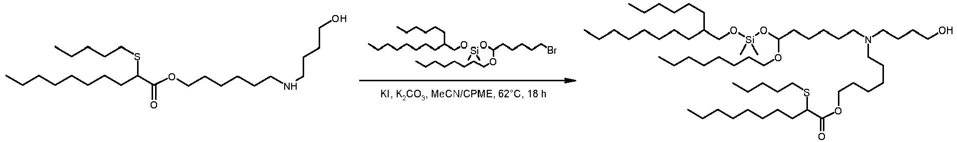

18-hexyl-7-(4-hydroxybutyl)-15,15-dimethyl-13-(octyloxy)-14,16-dioxa-7-aza-15-silahexacosyl 2-(pentylthio)decanoate was prepared from 10-(5-bromopentyl)-15-hexyl-12,12-dimethyl-9,11,13-trioxa-12-silatricosane (225.01 mg, 0.370 mmol, 1.1 eq.) and 6-((4-hydroxybutyl)amino)hexyl 2-(pentylthio)decanoate (150.00 mg, 0.336 mmol, 1.0 eq.) according to **General Procedure K.** The crude product was purified twice by flash column chromatography according to **Purification Method A and B** to obtain the pure product as a colourless oil, 130 mg, 0.134 mmol, 40 % yield.

¹H NMR (500 MHz, Benzene-d₆) δ 5.03 (t, 1H), δ 4.17-4.07 (m, 2H), δ 3.92-3.86 (m, 1H), δ 3.76 (d, 2H), δ 3.62 (t, 2H), δ 3.48-3.32 (m, 2H), δ 2.75-2.68 (m, 1H), δ 2.65-2.58 (m, 1H), δ 2.36-2.24 (m, 6H), δ 2.11-2.02 (m, 1H), δ 1.97-1.12 (m, 76H), δ 0.96-0.86 (m, 12H), δ 0.84 (t, 3H), δ 0.30 (d, 6H); TOF MS ES⁺ [M+H⁺]: 972.8441 m/z

### Example 186

### Synthesis of 15-heptyl-20-hexyl-7-(4-hydroxybutyl)-17,17-dimethyl-14,16,18-trioxa-7-aza-17-silaoctacosyl 2-(pentylthio)decanoate

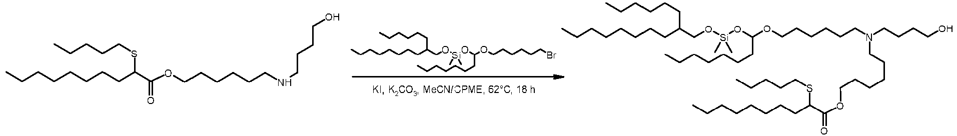

15-heptyl-20-hexyl-7-(4-hydroxybutyl)-17,17-dimethyl-14,16,18-trioxa-7-aza-17-silaoctacosyl 2-(pentylthio)decanoate was prepared from 1-bromo-8-heptyl-13-hexyl-10,10-dimethyl-7,9,11-trioxa-10-silahenicosane (204.56 mg, 0.336 mmol, 1.0 eq.) and 6-((4-hydroxybutyl)amino)hexyl 2-(pentylthio)decanoate (150.00 mg, 0.336 mmol, 1.0 eq.) according to **General Procedure K.** The crude product was purified by flash column chromatography according to **Purification Method B** to obtain the pure product as a colourless oil, 161 mg, 0.165 mmol, 49 % yield.

¹H NMR (500 MHz, Benzene-d₆) δ 5.03 (t, 1H), δ 4.19-4.07 (m, 2H), δ 3.94-3.85 (m, 1H), δ 3.76 (d, 2H), δ 3.62 (t, 2H), δ 3.48-3.32 (m, 2H), δ 2.77-2.57 (m, 2H), δ 2.35-2.23 (m, 6H), δ 2.14-2.01 (m, 1H), δ 1.97-1.12 (m, 76H), δ 0.96-0.89 (m, 12H), δ 0.84 (t, 3H), δ 0.30 (s, 6H); TOF MS ES⁺ [M+H⁺]: 972.8463 m/z

### Example 187

### Synthesis of 16-heptyl-7-(4-hydroxybutyl)-14,14-dimethyl-15,17-dioxa-7-aza-14-silapentacosyl 2-(pentylthio)decanoate

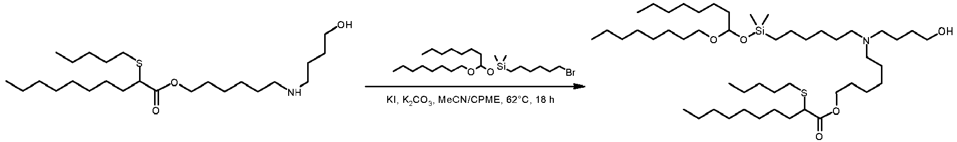

16-heptyl-7-(4-hydroxybutyl)-14,14-dimethyl-15,17-dioxa-7-aza-14-silapentacosyl 2-(pentylthio)decanoate was prepared from (6-bromohexyl)dimethyl((1-(octyloxy)octyl)oxy)silane (177.55 mg, 0.370 mmol, 1.1 eq.) and 6-((4-hydroxybutyl)amino)hexyl 2-(pentylthio)decanoate (150.00 mg, 0.336 mmol, 1.0 eq.) according to **General Procedure K.** The crude product was purified by flash column chromatography according to **Purification Method A** to obtain the pure product as a colourless oil, 178 mg, 0.211 mmol, 63 % yield.

¹H NMR (500 MHz, Benzene-d₆) δ 4.88 (t, 1H), δ 4.17-4.07 (m, 2H), δ 3.79-3.73 (m, 1H), δ 3.63 (t, 2H), δ 3.41-3.31 (m, 2H), δ 2.75-2.68 (m, 1H), δ 2.64-2.57 (m, 1H), δ 2.37-2.24 (m, 6H), δ 2.11-2.01 (m, 1H), δ 1.88-1.13 (m, 63H), δ 0.92-0.89 (m, 9H), δ 0.84 (t, 3H), δ 0.76-0.72 (m, 2H), δ 0.24 (d, 6H); TOF MS ES⁺ [M+H⁺]: 844.7252 m/z

### Example 188

### Synthesis of 17-heptyl-7-(4-hydroxybutyl)-15,15-dimethyl-14,16,18-trioxa-7-aza-15-silahexacosyl 2-(pentylthio)decanoate

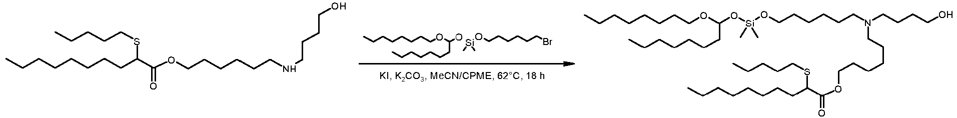

17-heptyl-7-(4-hydroxybutyl)-15,15-dimethyl-14,16,18-trioxa-7-aza-15-silahexacosyl 2-(pentylthio)decanoate was prepared from 1-bromo-10-heptyl-8,8-dimethyl-7,9,11-trioxa-8-silanonadecane (183.47 mg, 0.370 mmol, 1.1 eq.) and 6-((4-hydroxybutyl)amino)hexyl 2-(pentylthio)decanoate (150.00 mg, 0.336 mmol, 1.0 eq.) according to **General Procedure K.** The crude product was purified by flash column chromatography according to **Purification Method A** to obtain the pure product as a colourless oil, 79 mg, 0.092 mmol, 27 % yield.

¹H NMR (500 MHz, Benzene-d₆) δ 5.00 (t, 1H), δ 4.17-4.07 (m, 2H), δ 3.89-3.83 (m, 1H), δ 3.77 (t, 2H), δ 3.62 (t, 2H), δ 3.45-3.32 (m, 2H), δ 2.75-2.68 (m, 1H), δ 2.64-2.57 (m, 1H), δ 2.34-2.24 (m, 6H), δ 2.11-2.02 (m, 1H), δ 1.95-1.13 (m, 63H), δ 0.92-0.89 (m, 9H), δ 0.84 (t, 3H), δ 0.28 (d, 6H); TOF MS ES⁺ [M+H⁺]: 860.7207 m/z

### Example 189

### Synthesis of heptadecan-9-yl 8-((6-((dimethyl(octyl)silyl)oxy)-6-(octyloxy)hexyl)(2-hydroxyethyl)amino)octanoate

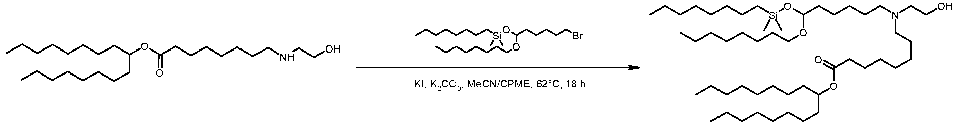

Heptadecan-9-yl 8-((6-((dimethyl(octyl)silyl)oxy)-6-(octyloxy)hexyl)(2-hydroxyethyl)amino)octanoate was prepared from ((6-bromo-1-(octyloxy)hexyl)oxy)dimethyl(octyl)silane (155.28 mg, 0.324 mmol, 1.1 eq.) and heptadecan-9-yl 8-((2-hydroxyethyl)amino)octanoate (130.00 mg, 0.294 mmol, 1.0 eq.) according to **General Procedure K.** The crude product was purified twice by flash column chromatography according to **Purification Method A** to obtain the pure product as a colourless oil, 180 mg, 0.214 mmol, 73 % yield.

¹H NMR (500 MHz, Benzene-d₆) δ 5.16 (m, 1H), δ 4.88 (t, 1H), δ 3.79-3.72 (m, 1H), δ 3.50 (t, 2H), δ 3.41-3.35 (m, 1H), δ 2.37-2.23 (m, 8H), δ 1.87-1.12 (m, 70H), δ 0.93-0.89 (m, 12H), δ 0.76-0.71 (m, 2H), δ 0.25 (d, 6H); TOF MS ES⁺[M+H⁺]: 840.7825 m/z

### Example 190

### Synthesis of heptadecan-9-yl 18-(2-hydroxyethyl)-10,10-dimethyl-12-(octyloxy)-9,11-dioxa-18-aza-10-silahexacosan-26-oate

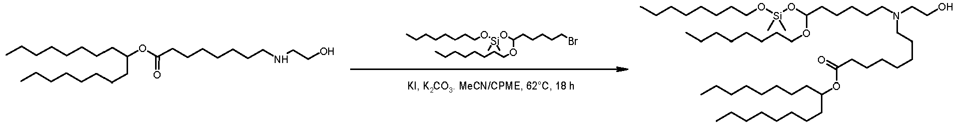

Heptadecan-9-yl 18-(2-hydroxyethyl)-10,10-dimethyl-12-(octyloxy)-9,11-dioxa-18-aza-10-silahexacosan-26-oate was prepared from 12-(5-bromopentyl)-10,10-dimethyl-9,11,13-trioxa-10-silahenicosane (160.45 mg, 0.324 mmol, 1.1 eq.) and heptadecan-9-yl 8-((2-hydroxyethyl)amino)octanoate (130.00 mg, 0.294 mmol, 1.0 eq.) according to **General Procedure K.** The crude product was purified twice by flash column chromatography according to **Purification Method A** to obtain the pure product as a colourless oil, 152 mg, 0.177 mmol, 60 % yield.

¹H NMR (500 MHz, Benzene-d₆) δ 5.20-5.14 (m, 1H), δ 5.01 (t, 1H), δ 3.90-3.84 (m, 1H), δ 3.77 (t, 2H), δ 3.50 (t, 2H), δ 3.44-3.36 (m, 1H), δ 2.37-2.23 (m, 8H), δ 1.94-1.13 (m, 70H), δ 0.96-0.87 (m, 12H), δ 0.28 (d, 6H); TOF MS ES⁺[M+H⁺]: 856.7795 m/z

### Example 191

### Synthesis of heptadecan-9-yl 8-((2-hydroxyethyl)(6-(octyloxy)-6-((1,1,3,3-tetramethyl-3-octyldisiloxaneyl)oxy)hexyl)amino)octanoate

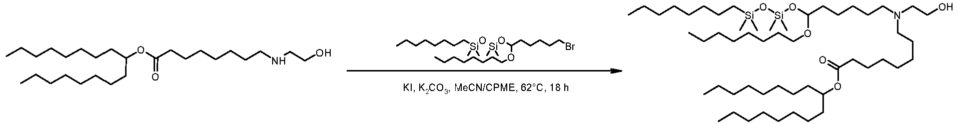

Heptadecan-9-yl 8-((2-hydroxyethyl)(6-(octyloxy)-6-((1,1,3,3-tetramethyl-3-octyldisiloxaneyl)oxy)hexyl)amino)octanoate was prepared from 1-((6-bromo-1-(octyloxy)hexyl)oxy)-1,1,3,3-tetramethyl-3-octyldisiloxane (179.28 mg, 0.324 mmol, 1.1 eq.) and heptadecan-9-yl 8-((2-hydroxyethyl)amino)octanoate (130.00 mg, 0.294 mmol, 1.0 eq.) according to **General Procedure K.** The crude product was purified twice by flash column chromatography according to **Purification Method A** to obtain the pure product as a colourless oil, 203 mg, 0.222 mmol, 75 % yield.

¹H NMR (500 MHz, Benzene-d₆) δ 5.19-5.12 (m, 1H), δ 5.01 (t, 1H), δ 3.89-3.83 (m, 1H), δ 3.50 (t, 2H), δ 3.45-3.38 (m, 1H), δ 2.37-2.24 (m, 8H), δ 1.93-1.12 (m, 70H), δ 0.93-0.89 (m, 12H), δ 0.69-0.65 (m, 2H), δ 0.27 (d, 6H), δ 0.21 (s, 6H); TOF MS ES⁺ [M+H⁺]: 914.7997 m/z

### Example 192

### Synthesis of heptadecan-9-yl 8-((2-hydroxyethyl)(6-(octyloxy)-6-((1,1,3,3-tetramethyl-3-(octyloxy)disiloxaneyl)oxy)hexyl)amino)octanoate

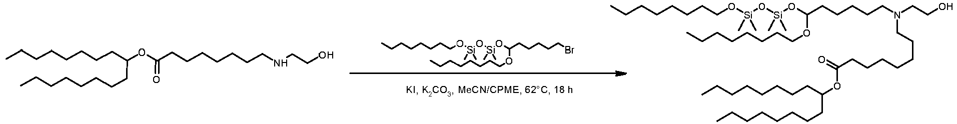

Heptadecan-9-yl 8-((2-hydroxyethyl)(6-(octyloxy)-6-((1,1,3,3-tetramethyl-3-(octyloxy)disiloxaneyl)oxy)hexyl)amino)octanoate was prepared from 1-((6-bromo-1-(octyloxy)hexyl)oxy)-1,1,3,3-tetramethyl-3-(octyloxy)disiloxane (184.46 mg, 0.324 mmol, 1.1 eq.) and heptadecan-9-yl 8-((2-hydroxyethyl)amino)octanoate (130.00 mg, 0.294 mmol, 1.0 eq.) according to **General Procedure K.** The crude product was purified twice by flash column chromatography according to **Purification Method A** to obtain the pure product as a colourless oil, 186 mg, 0.200 mmol, 68 % yield.

¹H NMR (500 MHz, Benzene-d₆) δ 5.19-5.13 (m, 1H), δ 5.04 (t, 1H), δ 3.91-3.85 (m, 1H), δ 3.75 (t, 2H), δ 3.50 (t, 2H), δ 3.46-3.37 (m, 1H), δ 2.37-2.23 (m, 8H), δ 1.95-1.12 (m, 70H), δ 0.95-0.87 (m, 12H), δ 0.31 (d, 6H), δ 0.24 (s, 6H)

### Example 193

### Synthesis of heptadecan-9-yl 10-heptyl-20-(4-hydroxybutyl)-12,12-dimethyl-9,11,13-trioxa-20-aza-12-silaoctacosan-28-oate

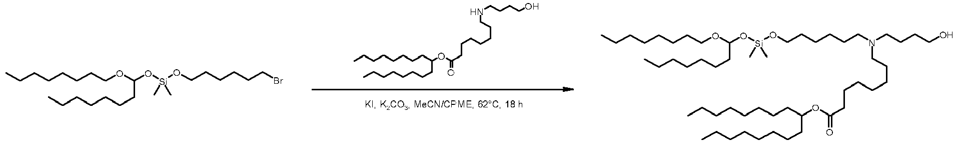

Heptadecan-9-yl 10-heptyl-20-(4-hydroxybutyl)-12,12-dimethyl-9,11,13-trioxa-20-aza-12-silaoctacosan-28-oate was prepared from 1-bromo-10-heptyl-8,8-dimethyl-7,9,11-trioxa-8-silanonadecane (649.92 mg, 1.311 mmol, 1.1 eq.) and heptadecan-9-yl 8-((4-hydroxybutyl)amino)octanoate (560 mg, 0.1.192 mmol, 1.0 eq.) according to **General Procedure K.** The crude product was purified twice by flash column chromatography according to **Purification Method B** to obtain the pure product as a colourless oil, 213 mg, 0.241 mmol, 20 % yield.

¹H NMR (500 MHz, Benzene-d₆) δ 5.16 (m, 1H), δ 5.00 (t, 1H), δ 3.91-3.82 (m, 1H), δ 3.76 (t, 2H), δ 3.62 (t, 2H), δ 3.46-3.38 (m, 2H), δ 2.36-2.24 (m, 7H), δ 1.96-1.77 (m, 2H) δ 1.71-1.12 (m, 73H), δ 0.94-0.87 (m, 12H), δ 0.28 (d, 6H); TOF MS ES⁺ [M+H⁺]: 884.8111 m/z

### Example 194

### Synthesis of 4-((6-((dimethyl(octyl)silyl)oxy)-6-(octyloxy)hexyl)((5Z,8Z,11Z,14Z,17Z)-icosa-5,8,11,14,17-pentaen-1-yl)amino)butan-1-ol

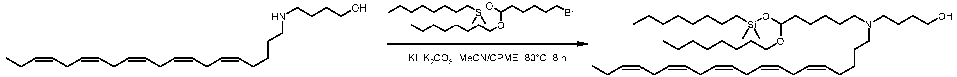

4-((6-((dimethyl(octyl)silyl)oxy)-6-(octyloxy)hexyl)((5Z,8Z,11Z,14Z,17Z)-icosa-5,8,11,14,17-pentaen-1-yl)amino)butan-1-ol was prepared from ((6-bromo-1-(octyloxy)hexyl)oxy)dimethyl(octyl)silane (148.06 mg, 0.309 mmol, 1 eq.) and 4-(((5Z,8Z,11Z,14Z,17Z)-icosa-5,8,11,14,17-pentaen-1-yl)amino)butan-1-ol (150 mg (purity 74 %), 0.309 mmol, 1.0 eq.) according to **General Procedure G.** The crude product was purified by flash column chromatography according to **Purification Method A** to obtain the pure product as a colourless oil, 100 mg, 0.132 mmol, 43 % yield.

¹H NMR (500 MHz, Benzene-d₆) δ 5.53-5.38 (m, 10H), δ 4.88 (dt, 1H), δ 3.78-3.73 (m, 1H), δ 3.61 (t, 2H), δ 3.41-3.35 (m, 1H), δ 2.92-2.83 (m, 8H), δ 2.35-2.34 (q, 4H), δ 2.25 (t, 2H), δ 2.10-1.99 (m, 4H), δ 1.88-1.19 (m, 40H), δ 0.95-0.89 (m, 9H), δ 0.76-0.72 (m, 2H), δ 0.25 (d, 6H); TOF MS ES⁺ [M+H⁺]: 758.6862 m/z

### Example 195

### Synthesis of 13-heptyl-5-((5Z,8Z,11Z,14Z,17Z)-icosa-5,8,11,14,17-pentaen-1-yl)-15,15-dimethyl-12,14-dioxa-5-aza-15-silatricosan-1-ol

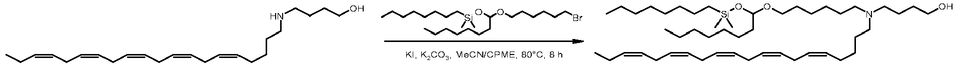

13-heptyl-5-((5Z,8Z,11Z,14Z,17Z)-icosa-5,8,11,14,17-pentaen-1-yl)-15,15-dimethyl-12,14-dioxa-5-aza-15-silatricosan-1-ol was prepared from ((1-((6-bromohexyl)oxy)octyl)oxy)dimethyl(octyl)silane (148.06 mg, 0.309 mmol, 1.0 eq.) and 4-(((5Z,8Z,11Z,14Z,17Z)-icosa-5,8,11,14,17-pentaen-1-yl)amino)butan-1-ol (150 mg (purity 74 %), 0.309 mmol, 1.0 eq.) according to **General Procedure G.** The crude product was purified by flash column chromatography according to **Purification Method A** to obtain the pure product as a colourless oil, 234 mg, 0.207 mmol, 67 % yield.

¹H NMR (500 MHz, Benzene-d₆) δ 5.60-5.39 (m, 10H), δ 4.89-4.85 (m, 1H), δ 3.78-3.73 (m, 1H), δ 3.61 (t, 2H), δ 3.40-3.31 (m, 1H), δ 2.91-2.81 (m, 8H), δ 2.32 (m, 4H), δ 2.26 (t, 2H), δ 2.10-2.01 (m, 4H), δ 1.88-1.19 (m, 40H), δ 0.96-0.89 (m, 9H), δ 0.78-0.71 (m, 2H), δ 0.24 (t, 6H); TOF MS ES⁺ [M+H⁺]: 758.6857 m/z

### Example 196

### Synthesis of 5-((5Z,8Z,11Z,14Z,17Z)-icosa-5,8,11,14,17-pentaen-1-yl)-13,13-dimethyl-11-(octyloxy)-12,14-dioxa-5-aza-13-siladocosan-1-ol

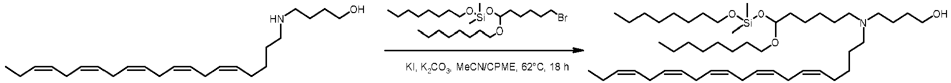

5-((5Z,8Z,11Z,14Z,17Z)-icosa-5,8,11,14,17-pentaen-1-yl)-13,13-dimethyl-11-(octyloxy)-12,14-dioxa-5-aza-13-siladocosan-1-ol was prepared from 12-(5-bromopentyl)-10,10-dimethyl-9,11,13-trioxa-10-silahenicosane (227.43 mg, 0.459 mmol, 1.1 eq.) and 4-(((5Z,8Z,11Z,14Z,17Z)-icosa-5,8,11,14,17-pentaen-1-yl)amino)butan-1-ol (150 mg, 0.417 mmol, 1 eq.) according to **General Procedure K.** The crude product was purified by flash column chromatography according to **Purification Method A** to obtain the pure product as a colourless oil, 145 mg, 0.187 mmol, 45 % yield.

¹H NMR (500 MHz, Benzene-d₆) δ 5.53-5.39 (m, 10H), δ 5.00 (m, 1H), δ 3.89-3.87 (m, 1H), δ 3.77 (t, 2H), δ 3.61 (t, 2H), δ 3.45-3.35 (m, 1H), δ 2.92-2.80 (m, 8H), δ 2.42-2.29 (m, 6H), δ 2.10-1.99 (m, 4H), δ 1.95-1.77 (m, 2H), δ 1.71-1.20 (m, 38H), δ 0.95-0.87 (m, 9H), δ 0.28 (d 6H); TOF MS ES⁺ [M+H⁺]: 774.6801 m/z

### Example 197

### Synthesis of 13-heptyl-5-((5Z,8Z,11Z,14Z,17Z)-icosa-5,8,11,14,17-pentaen-1-yl)-15,15-dimethyl-12,14,16-trioxa-5-aza-15-silatetracosan-1-ol

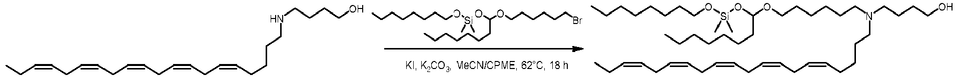

13-heptyl-5-((5Z,8Z,11Z,14Z,17Z)-icosa-5,8,11,14,17-pentaen-1-yl)-15,15-dimethyl-12,14,16-trioxa-5-aza-15-silatetracosan-1-ol was prepared from 1-bromo-8-heptyl-10,10-dimethyl-7,9,11-trioxa-10-silanonadecane (206.76 mg, 0.417 mmol, 1.0 eq.) and 4-(((5Z,8Z,11Z,14Z,17Z)-icosa-5,8,11,14,17-pentaen-1-yl)amino)butan-1-ol (150 mg, 0.417 mmol, 1.0 eq.) according to **General Procedure K.** The crude product was purified by flash column chromatography according to **Purification Method B** to obtain the pure product as a colourless oil, 155 mg, 0.200 mmol, 48 % yield.

¹H NMR (500 MHz, Benzene-d₆) δ 5.50-5.39 (m, 10H), δ 5.00 (t, 1H), δ 3.89-3.83 (m, 1H), δ 3.76 (t, 2H), δ 3.61 (t, 2H), δ 3.44-3.37 (m, 1H), δ 2.92-2.83 (m, 8H), δ 2.34-2.31 (m, 4H), δ 2.25 (t, 2H), δ 2.10-1.99 (m, 4H), δ 1.94-1.78 (m, 2H), δ 1.72-1.17 (m, 38H), δ 0.95-0.87 (m, 9H), δ 0.27 (d, 6H); TOF MS ES⁺ [M+H⁺]: 774.6811 m/z

### Example 198

### Synthesis of 4-(((5Z,8Z,11Z,14Z,17Z)-icosa-5,8,11,1-4,17-pentaen-1-yl)(6-(octyloay)-6-((1,1,3,3-tetramethyl-3-octyldisiloxaneyl)oxy)hexyl)amino)butan-1-ol

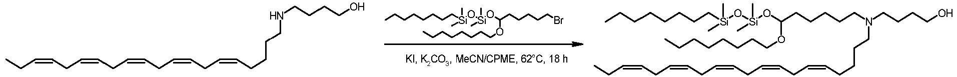

4-(((5Z,8Z,11Z,14Z,17Z)-icosa-5,8,11,14,17-pentaen-1-yl)(6-(octyloxy)-6-((1,1,3,3-tetramethyl-3-octyldisiloxaneyl)oxy)hexyl)amino)butan-1-ol was prepared from 1-((6-bromo-1-(octyloxy)hexyl)oxy)-1,1,3,3-tetramethyl-3-octyldisiloxane (231.02 mg, 0.417 mmol, 1.0 eq.) and 4-(((5Z,8Z,11Z,14Z,17Z)-icosa-5,8,11,14,17-pentaen-1-yl)amino)butan-1-ol (150 mg, 0.417 mmol, 1 eq.) according to **General Procedure K.** The crude product was purified by flash column chromatography according to **Purification Method B** to obtain the pure product as a colourless oil, 187 mg, 0.225 mmol, 54 % yield.

¹H NMR (500 MHz, Benzene-d₆) δ 5.52-5.38 (m, 10H), δ 5.00 (t, 1H), δ 3.89-3.83 (m, 1H), δ 3.61 (t, 2H), δ 3.44-3.37 (m, 1H), δ 2.92-2.80 (m, 8H), δ 2.36-2.29 (m, 4H), δ 2.25 (t, 2H), δ 2.09-1.99 (m, 4H), δ 1.94-1.77 (m, 2H), δ 1.72-1.19 (m, 38H), δ 0.95-0.88 (m, 9H), δ 0.69-0.65 (m, 2H), δ 0.26 (d, 6H), δ 0.21 (s, 6H); TOF MS ES⁺ [M+H⁺]: 832.7034 m/z

### Example 199

### Synthesis of 13-heptyl-5-((5Z,8Z,11Z,14Z,17Z)-icosa-5,8,11,14,17-pentaen-1-yl)-15,15,17,17-tetramethyl-12,14,16-trioxa-5-aza-15,17-disilapentacosan-1-ol

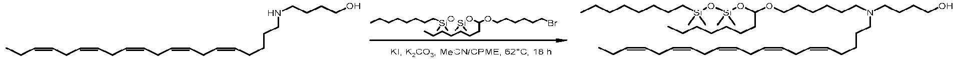

13-heptyl-5-((5Z,8Z,11Z,14Z,17Z)-icosa-5,8,11,14,17-pentaen-1-yl)-15,15,17,17-tetramethyl-12,14,16-trioxa-5-aza-15,17-disilapentacosan-1-ol was prepared from 1-((1-((6-bromohexyl)oxy)octyl)oxy)-1,1,3,3-tetramethyl-3-octyldisiloxane (231.02 mg, 0.417 mmol, 1.0 eq.) and 4-(((5Z,8Z,11Z,14Z,17Z)-icosa-5,8,11,14,17-pentaen-1-yl)amino)butan-1-ol (150 mg, 0.417 mmol, 1 eq.) according to **General Procedure K.** The crude product was purified by flash column chromatography according to **Purification Method B** to obtain the pure product as a colourless oil, 180 mg, 0.216 mmol, 52 % yield.

¹H NMR (500 MHz, Benzene-d₆) δ 5.55-5.40 (m, 10H), δ 5.01 (t, 1H), δ 3.93-3.81 (m, 1H), δ 3.61 (t, 2H), δ 3.45-3.37 (m, 1H), δ 2.92-2.83 (m, 8H), δ 2.37-2.17 (m, 6H), δ 2.11-1.98 (m, 4H), δ 1.95-1.75 (m, 2H), δ 1.73-1.18 (m, 38H), δ 0.96-0.88 (m, 9H), δ 0.70-0.65 (m, 2H), δ 0.29 (d, 6H), δ 0.22 (s, 6H); TOF MS ES⁺ [M+H⁺]: 832.7026 m/z

### Example 200

### Synthesis of 4-(((5Z,8Z,11Z,14Z,17Z)-icosa-5,8,11,1-4,17-pentaen-1-yl)(6-(octyloay)-6-((1,1,3,3-tetramethyl-3-(octyloxy)disiloxaneyl)oxy)hexyl)amino)butan-1-ol

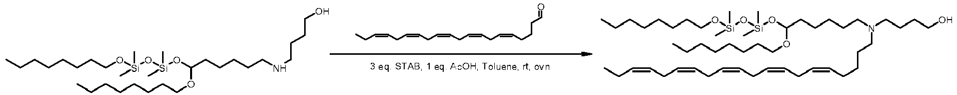

4-(((5Z,8Z,11Z,14Z,17Z)-icosa-5,8,11,14,17-pentaen-1-yl)(6-(octyloxy)-6-((1,1,3,3-tetramethyl-3-(octyloxy)disiloxaneyl)oxy)hexyl)amino)butan-1-ol was prepared from 4-((6-(octyloxy)-6-((1,1,3,3-tetramethyl-3-(octyloxy)disiloxaneyl)oxy)hexyl)amino)butan-1-ol (126 mg, 0.218 mmol, 1.0 eq.) and (5Z,8Z,11Z,14Z,17Z)-icosa-5,8,11,14,17-pentaenal (62.44 mg, 0.218 mmol, 1.0 eq.) according to **General Procedure J.** The crude product was purified by flash column chromatography according to **Purification Method A** to obtain the pure product as a colourless oil, 50 mg, 0.059 mmol, 27 % yield.

¹H NMR (500 MHz, Benzene-d₆) δ 5.53-5.41 (m, 10H), δ 5.01 (t, 1H), δ 3.93-3.85 (m, 1H), δ 3.76 (t, 2H) δ 3.62 (t, 2H), δ 3.47-3.37 (m, 1H), δ 2.92-2.82 (m, 8H), δ 2.36-2.32 (m, 4H), δ 2.26 (t, 2H) δ 2.10-1.99 (m, 4H), δ 1.95-1.79 (m, 2H), δ 1.72-1.20 (m, 38H), δ 0.95-0.89 (m, 9H), δ 0.31 (d, 6H), δ 0.25 (s, 6H); TOF MS ES⁺ [M+H⁺]: 848.6975 m/z

### Example 201

### Synthesis of 13-heptyl-5-((5Z,8Z,11Z,14Z,17Z)-icosa-5,8,11,14,17-pentaen-1-yl)-15,15,17,17-tetramethyl-12,14,16,18-tetraoxa-5-aza-15,17-disilahexacosan-1-ol

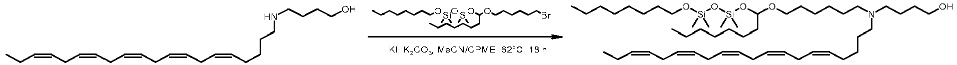

13-heptyl-5-((5Z,8Z,11Z,14Z,17Z)-icosa-5,8,11,14,17-pentaen-1-yl)-15,15,17,17-tetramethyl-12,14,16,18-tetraoxa-5-aza-15,17-disilahexacosan-1-ol was prepared from 1-((1-((6-bromohexyl)oxy)octyl)oxy)-1,1,3,3-tetramethyl-3-(octyloxy)disiloxane (209.17 mg, 0.367 mmol, 1.1 eq.) and 4-(((5Z,8Z,11Z,14Z,17Z)-icosa-5,8,11,14,17-pentaen-1-yl)amino)butan-1-ol (120 mg, 0.334 mmol, 1.0 eq.) according to **General Procedure K.** The crude product was purified by flash column chromatography according to **Purification Method A** to obtain the pure product as a colourless oil, 125 mg, 0.147 mmol, 44 % yield.

¹H NMR (500 MHz, Benzene-d₆) δ 5.53-5.39 (m, 10H), δ 5.04 (dt, 1H), δ 3.90-3.84 (m, 1H), δ 3.76 (t, 2H) δ 3.61 (t, 2H), δ 3.46-3.34 (m, 1H), δ 2.93-2.81 (m, 8H), δ 2.43-2.30 (m, 4H), δ 2.25 (t, 2H) δ 2.11-2.00 (m, 4H), δ 1.96-1.80 (m, 2H), δ 1.72-1.19 (m, 38H), δ 0.95-0.87 (m, 9H), δ 0.31 (d, 6H), δ 0.24 (s, 6H); TOF MS ES⁺ [M+H⁺]: 848.6992 m/z

### Example 202

### Synthesis of 16-hexyl-5-((5Z,8Z,11Z,14Z,17Z)-icosa-5,8,11,14,17-pentaen-1-yl)-13,13-dimethyl-11-(octyloxy)-12,14-dioxa-5-aza-13-silatetracosan-1-ol

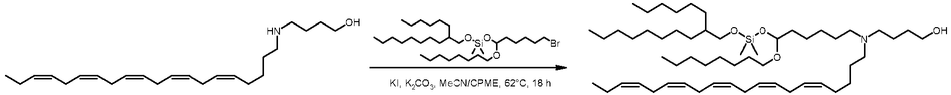

16-hexyl-5-((5Z,8Z,11Z,14Z,17Z)-icosa-5,8,11,14,17-pentaen-1-yl)-13,13-dimethyl-11-(octyloxy)-12,14-dioxa-5-aza-13-silatetracosan-1-ol was prepared from 10-(5-bromopentyl)-15-hexyl-12,12-dimethyl-9,11,13-trioxa-12-silatricosane (251.03 mg, 0.413 mmol, 1.1 eq.) and 4-(((5Z,8Z,11Z,14Z,17Z)-icosa-5,8,11,14,17-pentaen-1-yl)amino)butan-1-ol (135 mg, 0.375 mmol, 1.0 eq.) according to **General Procedure K.** The crude product was purified by flash column chromatography according to **Purification Method A** to obtain the pure product as a colourless oil, 185 mg, 0.209 mmol, 56 % yield.

¹H NMR (500 MHz, Benzene-d6) δ 5.53-5.39 (m, 10H), δ 5.02 (t, 1H), δ 3.92-3.86 (m, 1H), δ 3.76 (d, 2H), δ 3.61 (t, 2H), δ 3.48-3.38 (m, 1H), δ 2.93-2.82 (m, 8H), δ 2.37-2.30 (m, 4H), δ 2.26 (t, 2H), δ 2.10-1.99 (m, 4H), δ 1.96-1.79 (m, 2H), δ 1.73-1.23 (m, 51H), δ 0.96-0.89 (m, 12H), δ 0.30 (d, 6H); TOF MS ES⁺ [M+H⁺]: 886.8077 m/z

### Example 203

### Synthesis of 13-heptyl-18-hexyl-5-((5Z,8Z,11Z,14Z,17Z)-icosa-5,8,11,14,17-pentaen-1-yl)-15,15-dimethyl-12,14,16-trioxa-5-aza-15-silahexacosan-1-ol

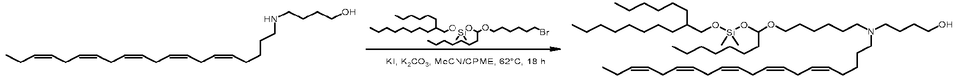

13-heptyl-18-hexyl-5-((5Z,8Z,11Z,14Z,17Z)-icosa-5,8,11,14,17-pentaen-1-yl)-15,15-dimethyl-12,14,16-trioxa-5-aza-15-silahexacosan-1-ol was prepared from 1-bromo-8-heptyl-13-hexyl-10,10-dimethyl-7,9,11-trioxa-10-silahenicosane (241.73 mg, 0.398 mmol, 1.1 eq.) and 4-(((5Z,8Z,11Z,14Z,17Z)-icosa-5,8,11,14,17-pentaen-1-yl)amino)butan-1-ol (130.00 mg, 0.361 mmol, 1.0 eq.) according to **General Procedure K.** The crude product was purified by flash column chromatography according to **Purification Method A** to obtain the pure product as a colourless oil, 197 mg, 0.222 mmol, 61 % yield.

¹H NMR (500 MHz, Benzene-d6) δ 5.53-5.39 (m, 10H), δ 5.02 (t, 1H), δ 3.90-3.85 (m, 1H), δ 3.75 (d, 2H), δ 3.61 (t, 2H), δ 3.46-3.37 (m, 1H), δ 2.93-2.82 (m, 8H), δ 2.35-2.30 (m, 4H), δ 2.25 (t, 2H), δ 2.10-1.99 (m, 4H), δ 1.97-1.80 (m, 2H), δ 1.71-1.24 (m, 51H), δ 0.95-0.89 (m, 12H), δ 0.30 (d, 6H); TOF MS ES⁺ [M+H⁺]: 886.8049 m/z

### Example 204

### Synthesis of 14-heptyl-5-((5Z,8Z,11Z,14Z,17Z)-icosa-5,8,11,14,17-pentaen-1-yl)-12,12-dimethyl-13,15-dioxa-5-aza-12-silatricosan-1-ol

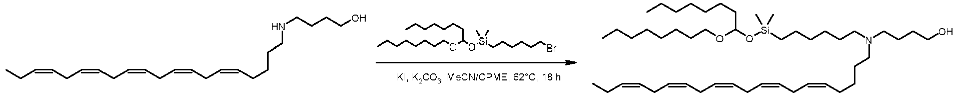

14-heptyl-5-((5Z,8Z,11Z,14Z,17Z)-icosa-5,8,11,14,17-pentaen-1-yl)-12,12-dimethyl-13,15-dioxa-5-aza-12-silatricosan-1-ol was prepared from (6-bromohexyl)dimethyl((1-(octyloxy)octyl)oxy)silane (198.08 mg, 0.413 mmol, 1.1 eq.) and 4-(((5Z,8Z,11Z,14Z,17Z)-icosa-5,8,11,14,17-pentaen-1-yl)amino)butan-1-ol (135.00 mg, 0.375 mmol, 1.0 eq.) according to **General Procedure K.** The crude product was purified by flash column chromatography according to **Purification Method A** to obtain the pure product as a colourless oil, 159 mg, 0.210 mmol, 56 % yield.

¹H NMR (500 MHz, Benzene-d6) δ 5.53-5.39 (m, 10H), δ 4.88 (t, 1H), δ 3.79-3.73 (m, 1H), δ 3.62 (t, 2H), δ 3.41-3.35 (m, 1H), δ 2.93-2.81 (m, 8H), δ 2.38-2.32 (m, 4H), δ 2.27 (t, 2H), δ 2.13-1.99 (m, 4H), δ 1.88-1.22 (m, 40H), δ 0.96-0.87 (m, 9H), δ 0.75-0.71 (m, 2H), δ 0.25 (d, 6H); TOF MS ES⁺ [M+H⁺]: 758.6862 m/z

### Example 205

### Synthesis of 15-heptyl-5-((5Z,8Z,11Z,14Z,17Z)-icosa-5,8,11,14,17-pentaen-1-yl)-13,13-dimethyl-12,14,16-trioxa-5-aza-13-silatetracosan-1-ol

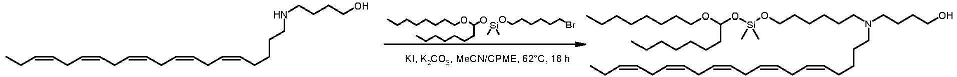

15-heptyl-5-((5Z,8Z,11Z,14Z,17Z)-icosa-5,8,11,14,17-pentaen-1-yl)-13,13-dimethyl-12,14,16-trioxa-5-aza-13-silatetracosan-1-ol was prepared from 1-bromo-10-heptyl-8,8-dimethyl-7,9,11-trioxa-8-silanonadecane (227.43 mg, 0.459 mmol, 1.1 eq.) and 4-(((5Z,8Z,11Z,14Z,17Z)-icosa-5,8,11,14,17-pentaen-1-yl)amino)butan-1-ol (150 mg, 0.417 mmol, 1.0 eq.) according to **General Procedure K.** The crude product was purified by flash column chromatography according to **Purification Method A** to obtain the pure product as a colourless oil, 155 mg, 0.200 mmol, 48 % yield.

¹H NMR (500 MHz, Benzene-d₆) δ 5.53-5.39 (m, 10H), δ 5.00 (t, 1H), δ 3.89-3.84 (m, 1H), δ 3.75 (t, 2H), δ 3.61 (t, 2H), δ 3.45-3.35 (m, 1H), δ 2.93-2.81 (m, 8H), δ 2.34-2.30 (m, 4H), δ 2.25 (t, 2H), δ 2.11-1.99 (m, 4H), δ 1.95-1.78 (m, 2H), δ 1.71-1.20 (m, 38H), δ 0.95-0.87 (m, 9H), δ 0.28 (d, 611); TOF MS ES⁺[M+H⁺]: 774.6809 m/z

### Example 206

### Synthesis of 15-heptyl-5-(4-hydroxybutyl)-13,13-dimethyl-12,14,16-trioxa-5-aza-13-silatetracosyl (9Z,12Z)-octadeca-9,12-dienoate

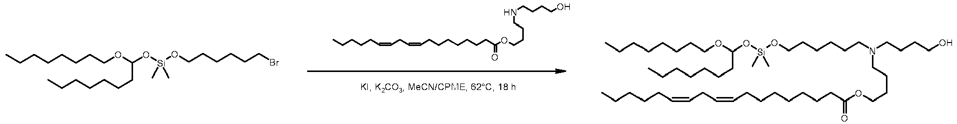

15-heptyl-5-(4-hydroxybutyl)-13,13-dimethyl-12,14,16-trioxa-5-aza-13-silatetracosyl (9Z,12Z)-octadeca-9,12-dienoate was prepared from 1-bromo-10-heptyl-8,8-dimethyl-7,9,11-trioxa-8-silanonadecane (501.9 mg, 1.013 mmol, 1.1 eq.) and 4-((4-hydroxybutyl)amino)butyl (9Z,12Z)-octadeca-9,12-dienoate (390.0 mg, 0.921 mmol, 1.0 eq.) according to **General Procedure K.** The crude product was purified by flash column chromatography according to **Purification Method B** to obtain the pure product as a colourless oil, 72 mg, 0.086 mmol, 9 % yield.

¹H NMR (500 MHz, Benzene-d₆) δ 5.54-5.43 (m, 4H), δ 4.99 (dd, 1H), δ 4.06 (t, 2H), δ 3.88-3.83 (m, 1H), δ 3.76 (t, 2H), δ 3.58 (t, 2H), δ 3.44-3.39 (m, 1H), δ 2.88 (t, 2H), δ 2.29-2.16 (m, 7H), δ 2.10-2.04 (m, 4H), δ 1.94-1.77 (m, 2H), δ 1.68-1.14 (m, 56H), δ 0.91-0.87 (m, 9H), δ 0.28 (d, 6H); TOF MS ES⁺[M+H⁺]: 838.7358 m/z

### Example 207

### Synthesis of 5-(3-((8Z,11Z)-heptadeca-8,11-dien-1-yl)-5,5-dimethyl-2,4,6-trioxa-5-siladecan-10-yl)-15-heptyl-13,13-dimethyl-12,14,16-trioxa-5-aza-13-silatetracosan-1-ol

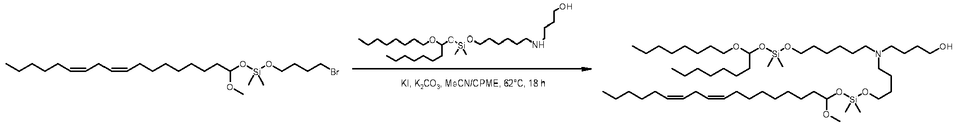

5-(3-((8Z,11Z)-heptadeca-8,11-dien-1-yl)-5,5-dimethyl-2,4,6-trioxa-5-siladecan-10-yl)-15-heptyl-13,13-dimethyl-12,14,16-trioxa-5-aza-13-silatetracosan-1-ol was prepared from 10-bromo-3-((8Z,11Z)-heptadeca-8,11-dien-1-yl)-5,5-imethyl-2,4,6-trioxa-5-siladecane (176.6 mg, 0.349 mmol, 1.1 eq.) and 15-heptyl-13,13-dimethyl-12,14,16-trioxa-5-aza-13-silatetracosan-1-ol (160 mg, 0.317 mmol, 1.0 eq.) according to **General Procedure K.** The crude product was purified by flash column chromatography according to **Purification Method B** to obtain the pure product as a colourless oil, 42 mg, 0.045 mmol, 14 % yield.

¹H NMR (500 MHz, Benzene-d₆) δ 5.55-5.44 (m, 4H), δ 5.01 (dd, 1H), δ 4.81 (dd, 1H), δ 3.93-3-84 (m, 1H), δ 3.79-3.72 (m, 4H), δ 3.61 (t, 2H), δ 3.45-3.38 (m, 1H), δ 3.32 (s, 3H), δ 2.90 (t, 2H), δ 2.45-2.24 (m, 6H), δ 2.12-2.06 (m, 4H), δ 1.96-1.75 (m, 4H), δ 1.71-1.20 (m, 58H), δ 0.93-0.88 (m, 9H), δ 0.30 (d, 6H), δ 0.25 (d, 6H); TOF MS ES⁺[M+H⁺]: 928.7825 m/z

### Example 208

### Synthesis of 5-(4-hydroxybutyl)-13,13,15-trimethyl-12,14,16-trioxa-5-aza-13-silahexacosyl (9Z,12Z)-octadeca-9,12-dienoate

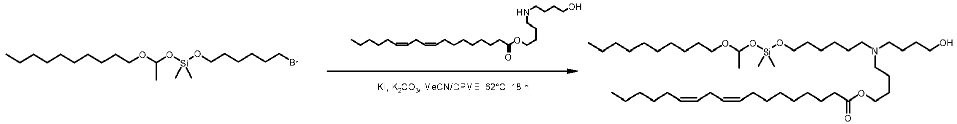

5-(4-hydroxybutyl)-13,13,15-trimethyl-12,14,16-trioxa-5-aza-13-silahexacosyl (9Z,12Z)-octadeca-9,12-dienoatewas prepared from 1-bromo-8,8,10-trimethyl-7,9,11-trioxa-8-silahenicosane (836.9 mg, 1.713 mmol, 1.1 eq.) and 4-((4-hydroxybutyl)amino)butyl (9Z,12Z)-octadeca-9,12-dienoate (660.0 mg, 1.558 mmol, 1.0 eq.) according to **General Procedure K.** The crude product was purified by flash column chromatography according to **Purification Method B** to obtain the pure product as a colourless oil, 50 mg, 0.064 mmol, 4 % yield.

¹H NMR (500 MHz, Benzene-d₆) δ 5.55-5.45 (m, 4H), δ 5.09 (q, 1H), δ 4.07 (t, 2H), δ 3.85-3.79 (m, 1H), δ 3.73 (t, 2H), δ 3.58 (t, 2H), δ 3.40-3.34 (m, 1H), δ 2.89 (t, 2H), δ 2.29-2.17 (m, 8H), δ 2.11-2.05 (m, 4H), δ 1.70-1.15 (m, 51H), δ 0.94-0.80 (m, 6H), δ 0.24 (d, 6H); LC ESI MS [M+H⁺]: 782.670 m/z

### Example 209

### Synthesis of undecyl 9-heptyl-20-(2-hydroxyethyl)-7,7-dimethyl-8,10-dioxa-20-aza-7-silahexacosan-26-oate

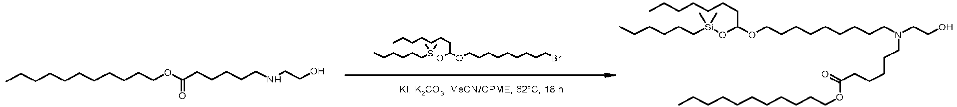

Undecyl 9-heptyl-20-(2-hydroxyethyl)-7,7-dimethyl-8,10-dioxa-20-aza-7-silahexacosan-26-oate was prepared from ((1-((9-bromononyl)oxy)octyl)oxy)(hexyl)dimethylsilane (206.00 mg, 0.417 mmol, 1.1 eq.) and undecyl 6-((2-hydroxyethyl)amino)hexanoate (125.00 mg, 0.379 mmol, 1.0 eq.) according to **General Procedure K.** The crude product was purified twice by flash column chromatography according to **Purification Method A** to obtain the pure product as a colourless oil, 201 mg, 0.2708 mmol, 71 % yield.

¹H NMR (500 MHz, Benzene-d₆) δ 4.89 (dt, 1H), δ 4.08 (t, 2H), δ 3.80-3.74 (m, 1H), δ 3.49 (t, 2H), δ 3.42-3.37 (m, 1H), δ 2.34 (t, 2H), δ 2.30-2.17 (m, 6H), δ 1.88-1.15 (m, 58H), δ 0.95-0.87 (m, 9H), δ 0.74-0.70 (m, 2H), δ 0.25 (d, 6H); TOF MS ES⁺ [M+H⁺]: 742.6735 m/z

### Example 210

### Synthesis of undecyl 6-((10-((hexyldimethylsilyl)oxy)-10-(octyloxy)decyl)(2-hydroxyethyl)amino)hexanoate

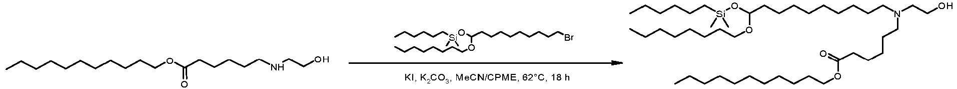

Undecyl 6-((10-((hexyldimethylsilyl)oxy)-10-(octyloxy)decyl)(2-hydroxyethyl)amino)hexanoate was prepared from ((10-bromo-1-(octyloxy)decyl)oxy)(hexyl)dimethylsilane (211.85 mg, 0.417 mmol, 1.1 eq.) and undecyl 6-((2-hydroxyethyl)amino)hexanoate (125.00 mg, 0.379 mmol, 1.0 eq.) according to **General Procedure K.** The crude product was purified twice by flash column chromatography according to **Purification Method A** to obtain the pure product as a colourless oil, 200 mg, 0.264 mmol, 70 % yield.

¹H NMR (500 MHz, Benzene-d₆) δ 4.89 (dt, 1H), δ 4.08 (t, 2H), δ 3.77-3.73 (m, 1H), δ 3.49 (t, 2H), δ 3.39-3.36 (m, 1H), δ 2.34 (t, 2H), δ 2.29-2.17 (m, 6H), δ 1.90-1.15 (m, 60H), δ 0.94-0.88 (m, 9H), δ 0.74-0.70 (m, 2H), δ 0.25 (d, 6H); TOF MS ES⁺ [M+H⁺]: 756.6902 m/z

### Example 211

### Synthesis of undecyl 17-heptyl-7-(4-hydroxybutyl)-15,15-dimethyl-14,16,18-trioxa-7-aza-15-silahexacosanoate

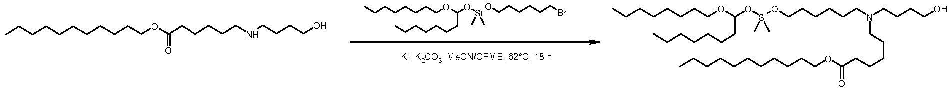

Undecyl 17-heptyl-7-(4-hydroxybutyl)-15,15-dimethyl-14,16,18-trioxa-7-aza-15-silahexacosanoate was prepared from 1-bromo-10-heptyl-8,8-dimethyl-7,9,11-trioxa-8-silanonadecane (485.15 mg, 0.979 mmol, 1.0 eq.) and undecyl 6-((4-hydroxybutyl)amino)hexanoate (350.0 mg, 0.979 mmol, 1.0 eq.) according to **General Procedure K.** The crude product was purified twice by flash column chromatography according to **Purification Method B** to obtain the pure product as a colourless oil, 179 mg, 0.231 mmol, 24 % yield.

¹H NMR (500 MHz, Benzene-d₆) δ 5.11 (dd, 1H), δ 4.18 (t, 2H), δ 3.87 (t, 2H), δ 3.71 (t, 2H), δ 3.55-3.46 (m, 1H), δ 2.41-2.29 (m, 7H), δ 2.06-1.88 (m, 2H), δ 1.80-1.28 (m, 59H), δ 1.04-0.99 (m, 9H), δ 0.39 (d, 6H); TOF MS ES⁺[M+H⁺]: 772.6857 m/z

### Example 212

### Synthesis of undecyl 6-((6-((dimethyl((oxacyclohexadecan-2-yl)oxy)silyl)oxy)hexyl)(4-hydroxybutyl)amino)hexanoate

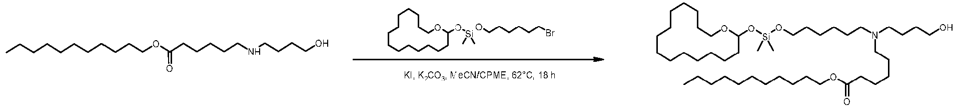

Undecyl 6-((6-((dimethyl((oxacyclohexadecan-2-yl)oxy)silyl)oxy)hexyl)(4-hydroxybutyl)amino)hexanoate was prepared from ((6-bromohexyl)oxy)dimethyl((oxacyclohexadecan-2-yl)oxy)silane (1.032 g, 2.153 mmol, 1.1 eq.) and undecyl 6-((4-hydroxybutyl)amino)hexanoate (700.0 mg, 1.958 mmol, 1.0 eq.) according to **General Procedure K.** The crude product was purified twice by flash column chromatography according to **Purification Method B** to obtain the pure product as a colourless oil, 250 mg, 0.331 mmol, 21 % yield.

¹H NMR (500 MHz, Benzene-d₆) δ 4.95-4.92 (dd, 1H), δ 4.06 (t, 2H), δ 3.95-3.90 (m, 1H), δ 3.73 (t, 2H), δ 3.60 (t, 2H), δ 3.40 (t, 1H), δ 3.29 (t, 1H), δ 2.30-2.17 (m, 7H), δ 1.94-1.87 (m, 1H), δ 1.84-1.80(m, 1H), δ 1.74-1.15 (m, 60H), δ 0.93-0.90 (m, 3H), δ 0.24 (d, 6H); TOF MS ES⁺ [M+H⁺]: 756.6555 m/z

### Example 213

### Synthesis of undecyl 7-(4-hydroxybutyl)-15,15,17-trimethyl-14,16,18-trioxa-7-aza-15-silaoctacosanoate

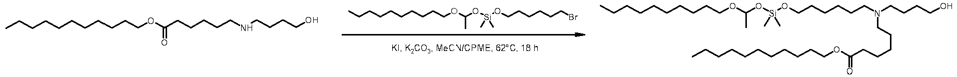

Undecyl 7-(4-hydroxybutyl)-15,15,17-trimethyl-14,16,18-trioxa-7-aza-15-silaoctacosanoate was prepared from 1-bromo-8,8,10-trimethyl-7,9,11-trioxa-8-silahenicosane (1250.0 mg, 2.842 mmol, 1.1 eq.) and undecyl 6-((4-hydroxybutyl)amino)hexanoate (960.0 mg, 2.583 mmol, 1.0 eq.) according to **General Procedure K.** The crude product was purified twice by flash column chromatography according to **Purification Method B** to obtain the pure product as a colourless oil, 435 mg, 0.607 mmol, 23 % yield.

¹H NMR (500 MHz, Benzene-d₆) δ 5.09 (q, 1H), δ 4.07 (t, 2H), δ 3.85-3.76 (m, 1H), δ 3.72 (t, 2H), δ 3.61 (t, 2H), δ 3.40-3.33 (m, 1H), δ 2.31-2.17 (m, 8H), δ 1.70-1.15 (m, 55H), δ 0.93-0.89 (m, 6H), δ 0.25 (d, 6H); TOF MS ES⁺ [M+H⁺]: 716.6218 m/z

### Example 214

### Synthesis 4-((6-((dimethyl(octyl)silyl)oxy)-6-(octyloxy)hexyl)((4R)-4-((3R,10S,13R)-3-methoxy-10,13-dimethylhexadecahydro-1H-cyclopenta[a]phenanthren-17-yl)pentyl)amino)butan-1-ol

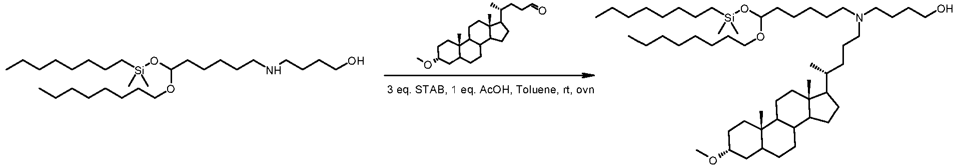

4-((6-((dimethyl(octyl)silyl)oxy)-6-(octyloxy)hexyl)((4R)-4-((3R,10S,13R)-3-methoxy-10,13-dimethylhexadecahydro-1H-cyclopenta[a]phenanthren-17-yl)pentyl)amino)butan-1-ol was prepared from 4-((6-((dimethyl(octyl)silyl)oxy)-6-(octyloxy)hexyl)amino)butan-1-ol (150.00 mg, 0.307 mmol, 1.0 eq.) and (4R)-4-((3R,10S,13R)-3-methoxy-10,13-dimethylhexadecahydro-1H-cyclopenta[a]phenanthren-17-yl)pentanal (115.17 mg, 0.307 mmol, 1.0 eq.) according to **General Procedure J.** The crude product was purified twice by flash column chromatography according to **Purification Method A and B** to obtain the pure product as a colourless oil, 120 mg, 0.142 mmol, 46 % yield.

¹H NMR (500 MHz, Benzene-d₆) δ 4.88 (dt, 1H), δ 4.78-4.70 (m, 1H), δ 3.79-3.73 (m, 1H), δ 3.64 (t, 2H), δ 3.43 (s, 3H), δ 3.43-3.36 (m, 1H), δ 2.42-2.38 (m, 4H), δ 2.31 (t, 2H), δ 1.97-0.70 (m, 78H), δ 0.61 (s, 3H), δ 0.26 (d, 6H); TOF MS ES⁺ [M+H⁺]: 846.7743 m/z

### Example 215

### Synthesis of 13-heptyl-5-((4R)-4-((3R,10S,13R)-3-methoxy-10,13-dimethylhexadecahydro-1H-cyclopenta[a]phenanthren-17-yl)pentyl)-15,15-dimethyl-12,14-dioxa-5-aza-15-silatricosan-1-ol

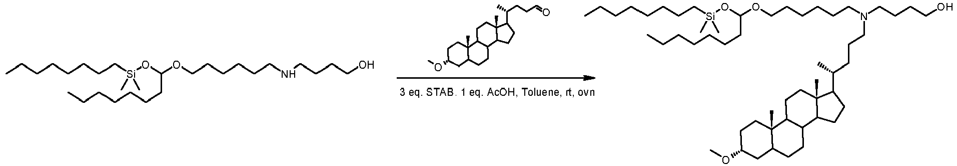

13-heptyl-5-((4R)-4-((3R,10S,13R)-3-methoxy-10,13-dimethylhexadecahydro-1H-cyclopenta[a]phenanthren-17-yl)pentyl)-15,15-dimethyl-12,14-dioxa-5-aza-15-silatricosan-1-ol was prepared from 13-heptyl-15,15-dimethyl-12,14-dioxa-5-aza-15-silatricosan-1-ol (150.00 mg, 0.307 mmol, 1.0 eq.) and (4R)-4-((3R,10S,13R)-3-methoxy-10,13-dimethylhexadecahydro-1H-cyclopenta[a]phenanthren-17-yl)pentanal (115.17 mg, 0.307 mmol, 1.0 eq.) according to **General Procedure J.** The crude product was purified twice by flash column chromatography according to **Purification Method A and B** to obtain the pure product as a colourless oil, 163 mg, 0.193 mmol, 63 % yield.

¹H NMR (500 MHz, Benzene-d₆) δ 4.87 (dt, 1H), δ 4.77-4.69 (m, 1H), δ 3.77-3.72 (m, 1H), δ 3.64 (t, 2H), δ 3.43 (s, 3H), δ 3.39-3.35 (m, 1H), δ 2.43-2.30 (m, 6H), δ 1.96-0.70 (m, 78H), δ 0.61 (s, 3H), δ 0.25 (d, 6H); TOF MS ES⁺[M+H⁺]: 846.7725 m/z

### Example 216

### Synthesis of 5-((4R)-4-((3R,10S,13R)-3-methoxy-10,13-dimethylhexadecahydro-1H-cyclopenta[a]phenanthren-17-yl)pentyl)-13,13-dimethyl-11-(octyloxy)-12,14-dioxa-5-aza-13-siladocosan-1-ol

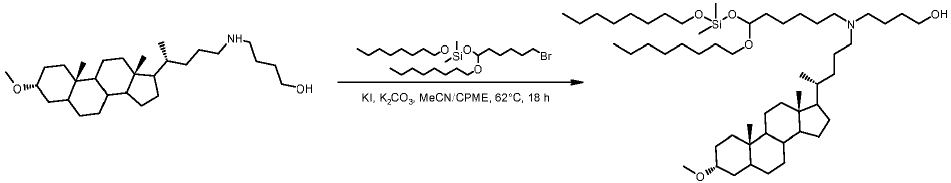

5-((4R)-4-((3R,10S,13R)-3-methoxy-10,13-dimethylhexadecahydro-1H-cyclopenta[a]phenanthren-17-yl)pentyl)-13,13-dimethyl-11-(octyloxy)-12,14-dioxa-5-aza-13-siladocosan-1-ol was prepared from 12-(5-bromopentyl)-10,10-dimethyl-9,11,13-trioxa-10-silahenicosane (113.25 mg, 0.228 mmol, 1.1 eq.) and 4-(((4R)-4-((3R,10S,13R)-3-methoxy-10,13-dimethylhexadecahydro-1H-cyclopenta[a]phenanthren-17-yl)pentyl)amino)butan-1-ol (93.00 mg, 0.208 mmol, 1.0 eq.) according to **General Procedure K.** The crude product was purified twice by flash column chromatography according to **Purification Method A** to obtain the pure product as a colourless oil, 58 mg, 0.067 mmol, 32 % yield.

¹H NMR (500 MHz, Benzene-d₆) δ 5.00 (dt, 1H), δ 4.78-4.70 (m, 1H), δ 3.89-3.84 (m, 1H), δ 3.77 (t, 2H), δ 3.64 (t, 2H), δ 3.45-3.43 (m, 4H), δ 2.32-2.29 (m, 6H), δ 1.97-0.75 (m, 76H), δ 0.61 (s, 3H), δ 0.28 (d, 6H); TOF MS ES⁺[M+H⁺]: 862.7687 m/z

### Example 217

### Synthesis of 13-heptyl-5-((4R)-4-((3R,10S,13R)-3-methoxy-10,13-dimethylhexadecahydro-1H-cyclopenta[a]phenanthren-17-yl)pentyl)-15,15-dimethyl-12,14,16-trioxa-5-aza-15-silatetracosan-1-ol

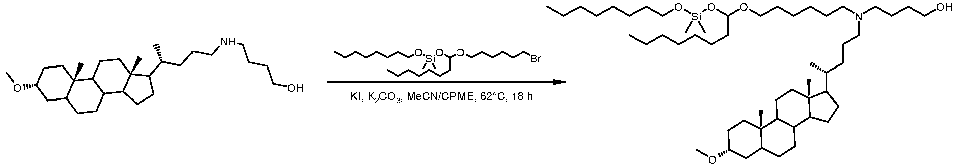

13-heptyl-5-((4R)-4-((3R,10S,13R)-3-methoxy-10,13-dimethylhexadecahydro-1H-cyclopenta[a]phenanthren-17-yl)pentyl)-15,15-dimethyl-12,14,16-trioxa-5-aza-15-silatetracosan-1-ol was prepared from 1-bromo-8-heptyl-10,10-dimethyl-7,9,11-trioxa-10-silanonadecane (113.25 mg, 0.228 mmol, 1.1 eq.) and 4-(((4R)-4-((3R,10S,13R)-3-methoxy-10,13-dimethylhexadecahydro-1H-cyclopenta[a]phenanthren-17-yl)pentyl)amino)butan-1-ol (93.00 mg, 0.208 mmol, 1.0 eq.) according to **General Procedure K.** The crude product was purified twice by flash column chromatography according to **Purification Method A** to obtain the pure product as a colourless oil, 39 mg, 0.045 mmol, 22 % yield.

¹H NMR (500 MHz, Benzene-d₆) δ 5.00 (dt, 1H), δ 4.78-4.68 (m, 1H), δ 3.88-3.83 (m, 1H), δ 3.77 (t, 2H), δ 3.66-3.61 (m, 2H), δ 3.46-3.39 (m, 4H), δ 2.45-2.25 (m, 6H), δ 1.97-0.75 (m, 76H), δ 0.61 (s, 3H), δ 0.28 (d, 6H); TOF MS ES⁺ [M+H⁺]: 862.7692 m/z

### Example 218

### Synthesis of 4-(((4R)-4-((3R,10S,13R)-3-methoxy-10,13-dimethylhexadecahydro-1H-cyclopenta[a]phenanthren-17-yl)pentyl)(6-(octyloxy)-6-((1,1,3,3-tetramethyl-3-octyldisiloxaneyl)oxy)hexyl)amino)butan-1-ol

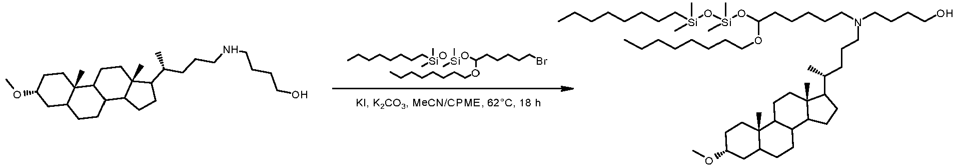

4-(((4R)-4-((3R,10S,13R)-3-methoxy-10,13-dimethylhexadecahydro-1H-cyclopenta[a]phenanthren-17-yl)pentyl)(6-(octyloxy)-6-((1,1,3,3-tetramethyl-3-octyldisiloxaneyl)oxy)hexyl)amino)butan-1-ol was prepared from 1-((6-bromo-1-(octyloxy)hexyl)oxy)-1,1,3,3-tetramethyl-3-octyldisiloxane (126.53 mg, 0.228 mmol, 1.1 eq.) and 4-(((4R)-4-((3R,10S,13R)-3-methoxy-10,13-dimethylhexadecahydro-1H-cyclopenta[a]phenanthren-17-yl)pentyl)amino)butan-1-ol (93.00 mg, 0.208 mmol, 1.0 eq.) according to **General Procedure K.** The crude product was purified twice by flash column chromatography according to **Purification Method A** to obtain the pure product as a colourless oil, 107 mg, 0.116 mmol, 56 % yield.

¹H NMR (500 MHz, Benzene-d₆) δ 5.01 (dt, 1H), δ 4.78-4.70 (m, 1H), δ 3.89-3.82 (m, 1H), δ 3.64 (t, 2H), δ 3.48-3.39 (m, 4H), δ 2.45-2.29 (m, 6H), δ 1.97-0.70 (m, 78H), δ 0.61 (s, 3H), δ 0.27 (d, 6H), δ 0.22 (s, 6H); TOF MS ES⁺[M+H⁺]: 920.7921 m/z

### Example 219

### Synthesis of 13-heptyl-5-((4R)-4-((3R,10S,13R)-3-methoxy-10,13-dimethylhexadecahydro-1H-cyclopenta[a]phenanthren-17-yl)pentyl)-15,15,17,17-tetramethyl-12,14,16-trioxa-5-aza-15,17-disilapentacosan-1-ol

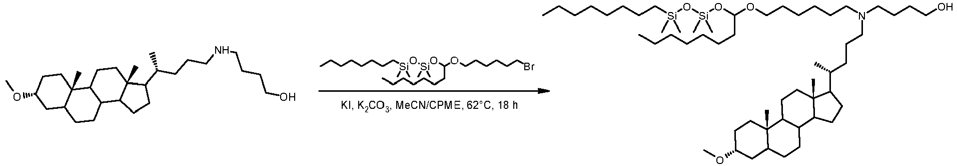

13-heptyl-5-((4R)-4-((3R,10S,13R)-3-methoxy-10,13-dimethylhexadecahydro-1H-cyclopenta[a]phenanthren-17-yl)pentyl)-15,15,17,17-tetramethyl-12,14,16-trioxa-5-aza-15,17-disilapentacosan-1-ol was prepared from 1-((1-((6-bromohexyl)oxy)octyl)oxy)-1,1,3,3-tetramethyl-3-octyldisiloxane (126.53 mg, 0.228 mmol, 1.1 eq.) and 4-(((4R)-4-((3R,10S,13R)-3-methoxy-10,13-dimethylhexadecahydro-1H-cyclopenta[a]phenanthren-17-yl)pentyl)amino)butan-1-ol (93.00 mg, 0.208 mmol, 1.0 eq.) according to **General Procedure K.** The crude product was purified by flash column chromatography according to **Purification Method A** to obtain the pure product as a colourless oil, 65 mg, 0.071 mmol, 34 % yield.

¹H NMR (500 MHz, Benzene-d₆) δ 5.01 (dt, 1H), δ 4.78-4.70 (m, 1H), δ 3.88-3.83 (m, 1H), δ 3.64 (t, 2H), δ 3.43-3.38 (m, 4H), δ 2.41-2.29 (m, 6H), δ 1.97-0.70 (m, 78H), δ 0.61 (s, 3H), δ 0.26 (d, 6H), δ 0.21 (s, 6H); TOF MS ES⁺[M+H⁺]: 920.7930 m/z

### Example 220

### Synthesis of 4-(((4R)-4-((3R,10S,13R)-3-methoxy-10,13-dimethylhexadecahydro-1H-cyclopenta[a]phenanthren-17-yl)pentyl)(6-(octyloxy)-6-((1,1,3,3-tetramethyl-3-(octyloxy)disiloxaneyl)oxy)hexyl)amino)butan-1-ol

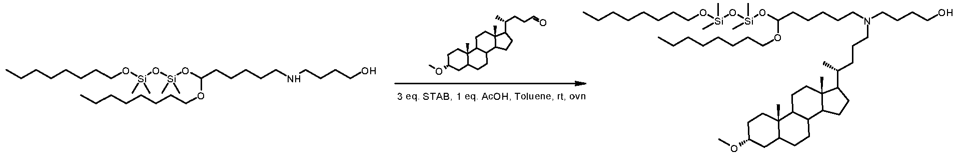

4-(((4R)-4-((3R,10S,13R)-3-methoxy-10,13-dimethylhexadecahydro-1H-cyclopenta[a]phenanthren-17-yl)pentyl)(6-(octyloxy)-6-((1,1,3,3-tetramethyl-3-(octyloxy)disiloxaneyl)oxy)hexyl)amino)butan-1-ol was prepared from 4-((6-(octyloxy)-6-((1,1,3,3-tetramethyl-3-(octyloxy)disiloxaneyl)oxy)hexyl)amino)butan-1-ol (121.00 mg, 0.209 mmol, 1.0 eq.) and (4R)-4-((3R,10S,13R)-3-methoxy-10,13-dimethylhexadecahydro-1H-cyclopenta[a]phenanthren-17-yl)pentanal (87.42 mg, 0.209 mmol, 1.0 eq.) according to **General Procedure J.** The crude product was purified twice by flash column chromatography according to **Purification Method A** to obtain the pure product as a colourless oil, 50 mg, 0.053 mmol, 25 % yield.

¹H NMR (500 MHz, Benzene-d₆) δ 5.03 (t, 1H), δ 4.77-4.70 (m, 1H), δ 3.92-3.85 (m, 1H), δ 3.76 (t, 2H), δ 3.63 (t, 2H), δ 3.46-3.37 (m, 4H), δ 2.48-2.29 (m, 6H), δ 1.97-0.75 (m, 76H), δ 0.61 (s, 3H), δ 0.31 (d, 6H), δ 0.25 (s, 6H); TOF MS ES⁺ [M+H⁺]: 936.7874 m/z

### Example 221

### Synthesis of 13-heptyl-5-((4R)-4-((3R,10S,13R)-3-methoxy-10,13-dimethylhexadecahydro-1H-cyclopenta[a]phenanthren-17-yl)pentyl)-15,15,17,17-tetramethyl-12,14,16,18-tetraoxa-5-aza-15,17-disilahexacosan-1-ol

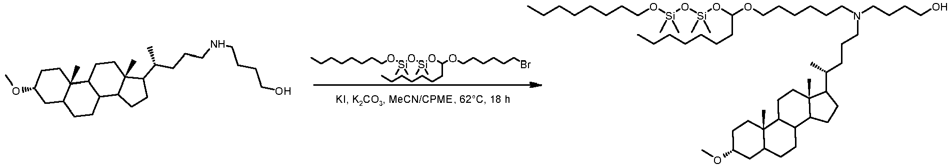

13-heptyl-5-((4R)-4-((3R,10S,13R)-3-methoxy-10,13-dimethylhexadecahydro-1H-cyclopenta[a]phenanthren-17-yl)pentyl)-15,15,17,17-tetramethyl-12,14,16,18-tetraoxa-5-aza-15,17-disilahexacosan-1-ol was prepared from 1-((1-((6-bromohexyl)oxy)octyl)oxy)-1,1,3,3-tetramethyl-3-(octyloxy)disiloxane (130.19 mg, 0.228 mmol, 1.1 eq.) and 4-(((4R)-4-((3R,10S,13R)-3-methoxy-10,13-dimethylhexadecahydro-1H-cyclopenta[a]phenanthren-17-yl)pentyl)amino)butan-1-ol (93.00 mg, 0.208 mmol, 1.0 eq.) according to **General Procedure K.** The crude product was purified by flash column chromatography according to **Purification Method A** to obtain the pure product as a colourless oil, 69 mg, 0.074 mmol, 35 % yield.

¹H NMR (500 MHz, Benzene-d₆) δ 5.03 (t, 1H), δ 4.78-4.70 (m, 1H), δ 3.90-3.84 (m, 1H), δ 3.76 (t, 2H), δ 3.64 (t, 2H), δ 3.47-3.37 (m, 4H), δ 2.43-2.29 (m, 6H), δ 1.97-0.75 (m, 76H), δ 0.61 (s, 3H), δ 0.31 (d, 6H), δ 0.24 (s, 6H); TOF MS ES⁺ [M+H⁺]: 936.7881 m/z

### Example 222

### Synthesis of 16-hexyl-5-((4R)-4-((3R,10S,13R)-3-methoxy-10,13-dimethylhexadecahydro-1H-cyclopenta[a]phenanthren-17-yl)pentyl)-13,13-dimethyl-11-(octyloxy)-12,14-dioxa-5-aza-13-silatetracosan-1-ol

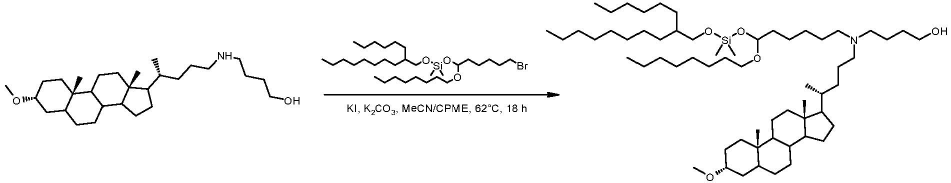

16-hexyl-5-((4R)-4-((3R,10S,13R)-3-methoxy-10,13-dimethylhexadecahydro-1H-cyclopenta[a]phenanthren-17-yl)pentyl)-13,13-dimethyl-11-(octyloxy)-12,14-dioxa-5-aza-13-silatetracosan-1-ol was prepared from 10-(5-bromopentyl)-15-hexyl-12,12-dimethyl-9,11,13-trioxa-12-silatricosane (194.14 mg, 0.319 mmol, 1.19 eq.) and 4-(((4R)-4-((3R,10S,13R)-3-methoxy-10,13-dimethylhexadecahydro-1H-cyclopenta[a]phenanthren-17-yl)pentyl)amino)butan-1-ol (120.00 mg, 0.268 mmol, 1.0 eq.) according to **General Procedure K.** The crude product was purified twice by flash column chromatography according to **Purification Method A and B** to obtain the pure product as a colourless oil, 100 mg, 0.103 mmol, 38 % yield.

¹H NMR (500 MHz, Benzene-d₆) δ 5.02 (t, 1H), δ 4.78-4.70 (m, 1H), δ 3.91-3.86 (m, 1H), δ 3.76 (d, 2H), δ 3.64 (t, 2H), δ 3.47-3.37 (m, 4H), δ 2.47-2.26 (m, 6H), δ 1.97-0.74 (m, 92H), δ 0.61 (s, 3H), δ 0.29 (d, 6H); TOF MS ES⁺[M+H⁺]: 974.8928 m/z

### Example 223

### Synthesis of 13-heptyl-18-hexyl-5-((4R)-4-((3R,10S,13R)-3-methoxy-10,13-dimethylhexadecahydro-1H-cyclopenta[a]phenanthren-17-yl)pentyl)-15,15-dimethyl-12,14,16-trioxa-5-aza-15-silahexacosan-1-ol

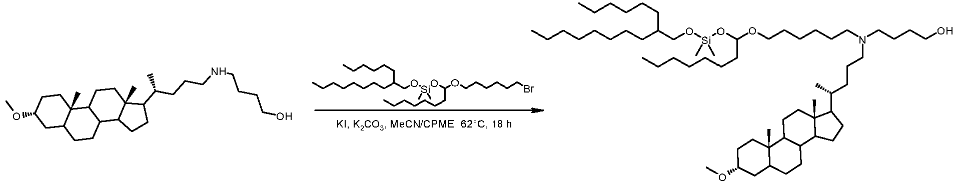

13-heptyl-18-hexyl-5-((4R)-4-((3R,10S,13R)-3-methoxy-10,13-dimethylhexadecahydro-1H-cyclopenta[a]phenanthren-17-yl)pentyl)-15,15-dimethyl-12,14,16-trioxa-5-aza-15-silahexacosan-1-ol was prepared from 1-bromo-8-heptyl-13-hexyl-10,10-dimethyl-7,9,11-trioxa-10-silahenicosane (179.21 mg, 0.295 mmol, 1.10 eq.) and 4-(((4R)-4-((3R,10S,13R)-3-methoxy-10,13-dimethylhexadecahydro-1H-cyclopenta[a]phenanthren-17-yl)pentyl)amino)butan-1-ol (120.00 mg, 0.268 mmol, 1.0 eq.) according to **General Procedure K.** The crude product was purified by flash column chromatography according to **Purification Method A** to obtain the pure product as a colourless oil, 109 mg, 0.112 mmol, 42 % yield.

¹H NMR (500 MHz, Benzene-d₆) δ 5.02 (t, 1H), δ 4.78-4.70 (m, 1H), δ 3.90-3.84 (m, 1H), δ 3.75 (d, 2H), δ 3.64 (t, 2H), δ 3.46-3.38 (m, 4H), δ 2.41-2.30 (m, 6H), δ 1.97-0.75 (m, 92H), δ 0.61 (s, 3H), δ 0.30 (d, 6H); TOF MS ES⁺[M+H⁺]: 974.8949 m/z

### Example 224

### Synthesis of 14-heptyl-5-((4R)-4-((3R,10S,13R)-3-methoxy-10,13-dimethylhexadecahydro-1H-cyclopenta[a]phenanthren-17-yl)pentyl)-12,12-dimethyl-13,15-dioxa-5-aza-12-silatricosan-1-ol

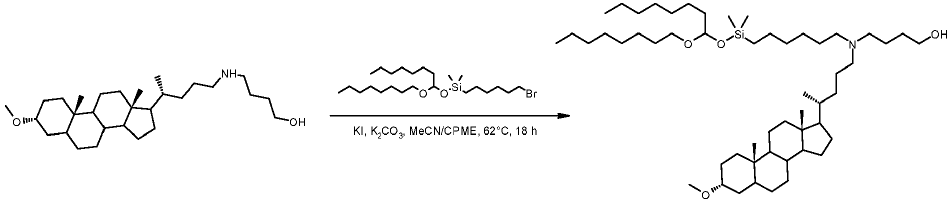

14-heptyl-5-((4R)-4-((3R,10S,13R)-3-methoxy-10,13-dimethylhexadecahydro-1H-cyclopenta[a]phenanthren-17-yl)pentyl)-12,12-dimethyl-13,15-dioxa-5-aza-12-silatricosan-1-ol was prepared from (6-bromohexyl)dimethyl((1-(octyloxy)octyl)oxy)silane (176.76 mg, 0.368 mmol, 1.10 eq.) and 4-(((4R)-4-((3R,10S,13R)-3-methoxy-10,13-dimethylhexadecahydro-1H-cyclopenta[a]phenanthren-17-yl)pentyl)amino)butan-1-ol (150.00 mg, 0.335 mmol, 1.0 eq.) according to **General Procedure K.** The crude product was purified by flash column chromatography according to **Purification Method A** to obtain the pure product as a colourless oil, 106 mg, 0.125 mmol, 37 % yield.

¹H NMR (500 MHz, Benzene-d₆) δ 4.88 (dt, 1H), δ 4.78-4.70 (m, 1H), δ 3.79-3.73 (m, 1H), δ 3.65 (t, 2H), δ 3.43 (s, 3H), δ 3.41-3.36 (m, 1H), δ 2.44-2.40 (m, 4H), δ 2.33 (t, 2H), δ 1.97-0.71 (m, 78H), δ 0.61 (s, 3H), δ 0.25 (d, 6H); TOF MS ES⁺ [M+H⁺]: 846.7731 m/z

### Example 225

### Synthesis of 15-heptyl-5-((4R)-4-((3R,10S,13R)-3-methoxy-10,13-dimethylhexadecahydro-1H-cyclopenta[a]phenanthren-17-yl)pentyl)-13,13-dimethyl-12,14,16-trioxa-5-aza-13-silatetracosan-1-ol

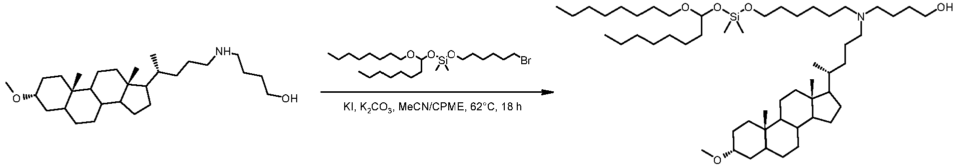

15-heptyl-5-((4R)-4-((3R,10S,13R)-3-methoxy-10,13-dimethylhexadecahydro-1H-cyclopenta[a]phenanthren-17-yl)pentyl)-13,13-dimethyl-12,14,16-trioxa-5-aza-13-silatetracosan-1-ol was prepared from 1-bromo-10-heptyl-8,8-dimethyl-7,9,11-trioxa-8-silanonadecane (152.21 mg, 0.307 mmol, 1.10 eq.) and 4-(((4R)-4-((3R,10S,13R)-3-methoxy-10,13-dimethylhexadecahydro-1H-cyclopenta[a]phenanthren-17-yl)pentyl)amino)butan-1-ol (125.00 mg, 0.279 mmol, 1.0 eq.) according to **General Procedure K.** The crude product was purified twice by flash column chromatography according to **Purification Method A and B** to obtain the pure product as a colourless oil, 43 mg, 0.050 mmol, 18 % yield.

¹H NMR (500 MHz, Benzene-d₆) δ 5.00 (td, 1H), δ 4.78-4.70 (m, 1H), δ 3.89-3.84 (m, 1H), δ 3.77 (t, 2H), δ 3.64 (t, 2H), δ 3.46-3.38 (m, 4H), δ 2.41-2.30 (m, 6H), δ 1.97-0.75 (m, 76H), δ 0.61 (s, 3H), δ 0.28 (d, 6H); TOF MS ES⁺[M+H⁺]: 862.7667 m/z

### Example 226

### Synthesis of 15-heptyl-5-(4-hydroxybutyl)-13,13-dimethyl-12,14,16-trioxa-5-aza-13-silatetracosyl (4R)-4-((3R,10S,13R)-3-methoxy-10,13-dimethylhexadecahydro-1H-cyclopenta[a]phenanthren-17-yl)pentanoate

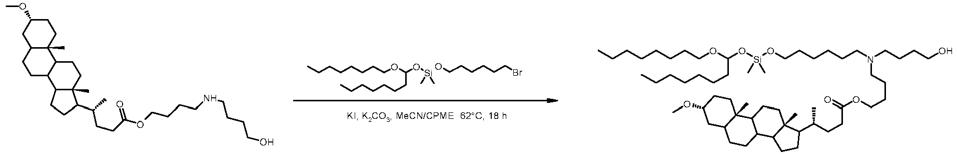

15-heptyl-5-(4-hydroxybutyl)-13,13-dimethyl-12,14,16-trioxa-5-aza-13-silatetracosyl (4R)-4-((3R,10S,13R)-3-methoxy-10,13-dimethylhexadecahydro-1H-cyclopenta[a]phenanthren-17-yl)pentanoate was prepared from 1-bromo-10-heptyl-8,8-dimethyl-7,9,11-trioxa-8-silanonadecane (582.14 mg, 1.174 mmol, 1.1 eq.) and 4-((4-hydroxybutyl)amino)butyl (4R)-4-((3R,10S,13R)-3-methoxy-10,13-dimethylhexadecahydro-1H-cyclopenta[a]phenanthren-17-yl)pentanoate (555.0 mg, 1.068 mmol, 1.0 eq.) according to **General Procedure K.** The crude product was purified by flash column chromatography according to **Purification Method B** to obtain the pure product as a colourless oil, 84 mg, 0.089 mmol, 83 % yield.

¹H NMR (500 MHz, Benzene-d₆) δ 5.01 (td, 1H), δ 4.10 (t, 2H), δ 3.90-3.84 (m, 1H), δ 3.77 (t, 2H), δ 3.59 (t, 2H), δ 3.45-3.40 (m, 1H), δ 3.25 (s, 3H), δ 3.09-3.02 (m, 1H), δ 2.43-2.21 (m, 8H), δ 2.00-1.20 (m, 62H), δ 1.03-0.79 (m, 16H), δ 0.60 (s, 3H), δ 0.29 (d, 6H); TOF MS ES⁻ [M+H⁺]: 948.8075 m/z

### Example 227

### Synthesis of 14-heptyl-5-(4-hydroxybutyl)-12,12-dimethyl-13,15-dioxa-5-aza-12-silatricosyl (4R)-4-((3R,10S,13R)-3-methoxy-10,13-dimethylhexadecahydro-1H-cyclopenta[a]phenanthren-17-yl)pentanoate

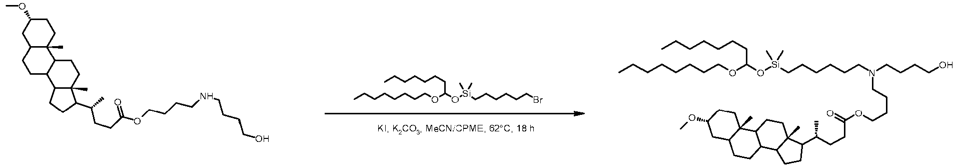

14-heptyl-5-(4-hydroxybutyl)-12,12-dimethyl-13,15-dioxa-5-aza-12-silatricosyl (4R)-4-((3R,10S,13R)-3-methoxy-10,13-dimethylhexadecahydro-1H-cyclopenta[a]phenanthren-17-yl)pentanoate was prepared from (6-bromohexyl)dimethyl((1-(octyloxy)octyl)oxy)silane (558.27 mg, 1.164 mmol, 1.1 eq.) and 4-((4-hydroxybutyl)amino)butyl (4R)-4-((3R,10S,13R)-3-methoxy-10,13-dimethylhexadecahydro-1H-cyclopenta[a]phenanthren-17-yl)pentanoate (550.0 mg, 1.058 mmol, 1.0 eq.) according to **General Procedure K.** The crude product was purified by flash column chromatography according to **Purification Method B** to obtain the pure product as a colourless oil, 250 mg, 0.268 mmol, 25 % yield.

¹H NMR (500 MHz, Benzene-d₆) δ 4.89 (td, 1H), δ 4.10 (t, 2H), δ 3.79-3.74 (m, 1H), δ 3.60 (t, 2H), δ 3.41-3.36 (m, 1H), δ 3.25 (s, 3H), δ 3.08-3.02 (m, 1H), δ 2.39-2.23 (m, 9H), δ 2.01-1.95 (m, 1H), δ 1.90-1.72 (m, 8H), δ 1.59-1.13 (m, 54H), δ 1.05-0.72 (m, 16H), δ 0.60 (s, 3H), δ 0.27 (d, 6H); TOF MS ES⁺ [M+H⁺]: 932.8140 m/z

### Preparation and characterisation of lipid nanoparticles, transfection and viability studies

The synthesized lipids were tested in term of *in vitro* transfection efficacy and toxicity on HeLa cells. In order to perform the mentioned biological tests, appropriate LNPs had to be formulated from the synthesized lipids. The formulation technique was different in **Method** A and **B.** The particle size, PDI and zeta potential of resulting LNPs were determined by means of Dynamic Light Scattering technique (DLS). Prior to biological testing the encapsulation efficiency (EE) of the loaded LNPs was determined by using RiboGreen RNA assay. In case of **Method** A Luciferase-mRNA while in case of **Method B** GFP-mRNA was transfected into the cells. In parallel with the transfection experiments, toxicity of the investigated LNPs was determined, in case of **Method A** resazurin assay while in case of **Method B** MTT assay was used. In the case of some lipids, the apparent pKa in LNPs was determined by using TNS titration based on the method described: Angew Chem Int Ed Engl. 2012, 51 (34), 8529-8533, doi: 10.1002/anie.201203263).

### METHOD A

### LNP formulation and dialysis (Method A)

### Preparation:

1. If needed, prepare stock solutions by solving known amounts of lipid powder into an appropriate solvent. The recommended solvent is Ethanol and the recommended concentration is typically in the range 10-50 mM. Higher concentrations are preferred to allow flexibility on mRNA-LNP concentration.
2. Remove the lipid stock solutions from the -20°C freezer. For long-term storage, -80°C is used.
3. Check the vials for precipitation, DOPE (CAS# 4004-05-1) (20 mM) and DSPE-PEG (CAS# 474922-26-4) (5mM) may need a 5-30 minute incubation step at 37°C with intermittent vortexing to resuspend all contents. Vials that are significantly precipitated (from repeated freeze-thawing) may not be recoverable. As a last resort, lipids may be heated to 50°C to resuspend, time should be kept as short as possible to prevent oxidation or other modifying or degradative processes.
4. Mix the lipids by vortexing for 2 minutes.
5. Cool the lipids stock solutions to room temperature by incubating on the bench, optionally protected from light. Cooling the solution prevent excessive evaporation of ethanol or similar solvents upon opening.
6. Meanwhile, thaw the RNA samples, including the latest SecNLuc control, by inserting the frozen tube in a room temperature aluminium block.
7. Prepare for each LNP-formulation with identical mRNA component, an RNA master mix according to Table 1 below in a fresh tube. Use of a master mix allows more precise comparison of lipid formulations relative to each other. Split the master mix over a number of fresh tubes equal to the number of samples + controls. Each tube contains 375 µl of mRNA in 10 mM Citrate pH 4.0.
8. Prepare for each LNP formulation with identical lipid composition a Lipid master mix according to Table 1 below in a fresh tube, exclude the lipid of interest. Use of a master mix allows more precise comparison of the novel ionizable lipid relative to each other. Split the master mix over a number of fresh tubes equal to the number of samples + controls. Each tube contains 65.5 µl of lipid master mix.
9. Add the ionizable lipid of interest to the equally divide lipid master mix. Mix well by pipetting up and down. Label the tube according to the ionizable lipid used.
10. Power on the 'L1 formulation machine' (L1 FM) and allow it to do a calibration of motor positions.

**Table 1: Lipid formulation solution**

| Name | Ionizable lipid | | Cationic lipid | | Helper lipid | | Core lipid | | Shield lipid | | EtOH | mRNA | Citrate 100mM | RFW |
|---|---|---|---|---|---|---|---|---|---|---|---|---|---|---|
| Sample | 59.2 µL | Tested lipid (10mM) | 4.8 µL | DOTAP (25mM) | 4.7 | DOPE (30mM) | 19.7 µL | RVS* (25mM) | 1.25 µL | DSPE-PEG (5mM) | 35 µL | 41 µL | 37.5 µL | 297 µL |
| Mastermix for 23 samples | | | 120 µL | | 117.5 µL | | 492.5 µL | | 31.25 µL | | 1500 µL | 1025 µL | 937.5 µL | 7425 µL |

| | | | | | | | | | | | | | | |
|---|---|---|---|---|---|---|---|---|---|---|---|---|---|---|
| * proprietary core lipid belongs to RiboPro B.V., described as compound 101 in patent application GB2302736.0 | | | | | | | | | | | | | | |

### Rinsing and pre-wetting:

11. Place a fresh microfluidic cartridge (COC-plastic) into the loading slot of the L1 FM.
12. Load the 'Receiving station' of the L1 FM with 2 ml Eppendorf tubes.
13. Fill one 'Rinse syringe' (A) with 1 ml RNAse-free H₂O and the other (B) with 1 ml 99% EtOH from the designated 'Wash' stock solutions
14. Attach the syringes in the correct orientation, the H₂O-filled syringe on the left and the EtOH-filled syringe on the right
15. Rinse the chip by pressing 'Button 2' (100 µg), hold the chip in place and check the movement of the syringes while the machine is working
16. Restart the machine for the next formulation.
17. Discard the flow-through

### Formulation:

18. Fill a fresh syringe (C) through a blunt 18G stainless steel needle with the previously prepared RNA mixture of step 6. The volume should be approximately 85% of total formulation volume + 25 µl (dead-volume). Make sure to remove all air bubbles by tapping.
19. Fill a fresh syringe (D) through a blunt 18G stainless steel needle with the Lipid master mix. The volume should be approximately 35% of total formulation volume + 25 µl (dead-volume). Again, Make sure to remove all bubbles.
20. Attach the syringes in the correct orientation, syringe A on the left and syringe B on the right
21. Load the 'Receiving station' with appropriate sized collection containers (2 ml Eppendorf tubes).
22. Press the microfluidic chip to the back of the machine with your thumb and hold, press the 'Button 1' (40 µg). The L1 FM pushes the total volume from the syringes in a perfect 3:1 (mRNA aqueous : lipid in EtOH) ratio through the microfluidic mixer chip with a total flow-rate of 12 ml/ml (Total flow rate (TFR)).
23. Remove the sample tube from the 'Receiving station' and label the tube accordingly.
24. Reset the machine to prepare with the next formulation.

### Dialysis:

25. Transfer the individual formulations to 100 kda MWCO, SpectraPor Biotech dialysis tubing, and close with designated clamps.
26. Submerge the dialysis bags in beakers filled with 1x PBS buffer pH 7.4, stir at 230 RPM at room temperature.
27. Refresh the 1x PBS buffer (pH 7.4) after 2 hours of incubation at room temperature, while stirring.
28. Refresh the buffer and incubate for 2 more hours at room temperature, while stirring.
29. Remove the dialysis bag from the dialysis setup and extract the LNP samples with a sterile pipette tip.
30. Dilute the samples to 1 ml with sterile 1x PBS and measure the Zeta potential, Size and PDI using the DLS machine.

### Size and size distribution measurements with dynamic light scattering (Method A)

Turn on the DLS (Dynamic Light Scattering) machine 30 minutes before use. This allows the laser to warm up and the sample station to equilibrate to the set working temperature (usually room temperature).
1. Optionally; if producing large volume in discrete batches (i.e., processed in multiple dialysis cassettes), pool the individual formulations for each of the unique constructs.
2. Unpack the folded capillary cells (DTS1070), use one sample per construct.
3. Flush the cuvette by holding it upside down (injection ports below) and inject 1 ml H₂O in one of the ports. This will dispense the fluid through the cell and out of the other port.
4. Repeat this step with a syringe filled with 1 ml air, this will dry the excess of water that might remain in the cell after flushing.
5. Turn the cuvette so that the injection ports are on the top.
6. Dilute 10 µl of dialyzed LNP formulation in 690 µl of 1x PBS buffer, pH 7.4.
7. Add the sample to the cell by using a clear syringe, do not exceed the 'Fill, Max' line on the cuvette.
8. Select the 'Size & Zeta' option in the 'Method builder' of the 'ZS Explorer' software, and measure the samples to determine the size, PDI and Zeta potential.

### Encapsulation efficiency (Method A)

### Solutions required:

1. Preparation of sample stock solutions: In the top row of the 96-well plate (Row A in the plate), add 297 µL of TE buffer pH 7.4 to a single well for each sample plus a single well for a PBS blank using a multichannel pipette.
2. Add 3 µL of sample (at 0.5mg/ml) to these wells for a final volume of 300 µL. Add 3 µL of PBS to the blank well. Pipette to mix. This is your stock solution for each sample. The final RNA concentration of these stock solutions should be approximately 4-7 µg/mL.
3. mRNA-LNP sample setup:
a. Add 50 µL of TE buffer pH 7.5 to the two wells directly below each sample (Rows B and C in the plate).
b. Add 50 µL of sample stock solution from Row A into the wells in Rows B and C (this assay is run in duplicate. All liquid handling should be done using a multichannel pipette).
c. Add 50 µL of Triton buffer to the wells in Rows D and E (in the plate) below each sample.
d. Add 50 µL of sample stock solution from Row A into the wells in Rows D and E.

4. RiboGreen RNA standard curve setup: Dilute the RNA standard to produce an RNA stock at a final concentration of 20 µg/mL in TE buffer pH 7.4. The final volume should be 150 µL. Set up a standard curve (in duplicate) as using the RNA stock (20 µg/mL siRNA), TE buffer pH 7.5, and Triton buffer according the Table 2 below.

**Table 2: RiboGreen RNA standard curve preparation**

| Final mRNA concentration | RNA stock (µl) | TE buffer (µl) | Triton buffer (µl) | Total volume (µl) |
|---|---|---|---|---|
| 2.5 | 25 | 25 | 50 | 100 |
| 1 | 10 | 40 | 50 | 100 |
| 0.5 | 5 | 45 | 50 | 100 |
| 0.25 | 2.5 | 47.5 | 50 | 100 |
| 0.1 | 1 | 49 | 50 | 100 |

5. Once samples and standard curve are plated, incubate the plate at 37°C for 10 min to lyse mRNA-LNP in the presence of Triton X-100.
6. Meanwhile, prepare RiboGreen Solution: Sum the total number of sample wells and standard curve wells. Add three to this number, and multiply the total by 100. This is the total volume, in µL, of RiboGreen Solution needed for this assay. In a 15 mL RNAse-free Falcon tube, dilute the RiboGreen Reagent 1:100 into TE buffer pH 7.5 to the total volume calculated.
7. Addition of RiboGreen Solution and sample readings: Remove a 96-well plate from 37°C incubator; let it cool to room temperature as RiboGreen binding is somewhat temperature dependent. Add 100 µL of RiboGreen Solution to each well. Pop any air bubbles with a needle. Read using fluorescent plate reader (excitation = 480, emission = 525).
8. Sample analysis: Use the data generated by the RiboGreen standard curve to calculate the concentration of mRNA.

### Transfection efficiency with Luciferase-mRNA (Method A)

### Control transfection preparation:

1. Prepare mRNA solution by adding 100 ng mRNA (SecNLuc, #P009024, RiboPro) to 5 µl Opti-MEM a. multiply by number of wells, include different concentrations and add 10% extra b. a standard set-up we use is 100 ng - 50 ng - 10 ng mRNA/well in triplicate, which would need 528 ng mRNA (includes 10% extra)

### Transfection:

2. (If applicable) Prepare different concentrations in Opti-MEM (for instance 100/50/10 ng).
3. Add 10 µl to each well and mix carefully by pipetting.
4. Mix by swirling the plate horizontally at modest speed. Reverse in direction 3 times.
5. Incubate for 24 hours (incubator).

### Nano-Glo Assay

### Background:

This protocol is applied to measure luciferase activity for secreted NanoLuc and is based on the protocol by Promega (Nano-Glo^{®} Luciferase Assay System #N1110-1150). The protocol is designed for a 384-wells plate, but volumes can be changed accordingly for other plates.

### Procedure:

1. Plate cells (HeLa) in 96-well plate, grow until 80% confluent and transfect with SecNanoLuc mRNA and incubate for the desired period (24h).
2. After incubation, the translated protein is accumulated in the medium. Transfer medium to a new plate or tubes
3. Transfer 12 µl of each sample to a black-walled, clear-bottom 384-well plate a. Black-walled plates are important to prevent shine-through of luminescence from other wells; alternatively, multiple wells between samples can be left empty
4. Set-up plate reader
5. Prepare the desired amount of Nano-Glo^{®} Luciferase Assay Reagent by combining one volume of Nano-Glo^{®} Luciferase Assay Substrate with 50 volumes of Nano-Glo^{®} Luciferase Assay Buffer
   a. composition: 100 mM MES pH 6; 1 mM EDTA; 0.5% NP-50; 150 mM KCl; 1 mM DTT; 35 mM Thiourea
   b. Briefly spin tubes containing substrate in a microcentrifuge before use
6. Add and mix (by pipetting) 12 µl Nano-Glo^{®} Luciferase Assay Reagent per well a. Wait approximately 3 minutes before measuring luminescence. The luminescence intensity will decay gradually, with a signal half-life of approximately 120 minutes at room temperature
7. Measure luminescence in plate reader using standard protocol luciferase (this includes mixing sample in plate reader)

### Notes

Thaw Nano-Glo^{®} Luciferase Assay buffer to room temperature, but do not exceed 25°C at any moment during the procedure.

### Cell viability assessment with Resazurin assay (Method A)

### Background:

This protocol is used to determine the metabolic activity of cells as a measure for cell viability after exposure to potentially toxic substances. Resazurin is converted in mitochondria by reduction to resorufin, which has a much higher fluorescence at ~600 nm compared to unconverted resazurin. A lower amount/percentage of conversion is correlated with reduced cellular metabolism and indirectly to cellular death and toxicity.. Care must be taken with interpretation of the read-out, as 50% reduction in resazurin conversion may mean 50% reduction of cellular metabolism of 100% alive cells, or 50% of cells dead, and any combination in between.

### Procedure:

1. Prepare 0.25 mg/ml stock solution resazurin in fresh cell culture medium. Resazurin is auto-reducing over time in solution, especially in medium, and fresh stocks should be prepared every 2-3 days. Stocks should be stored at 4°C and protected from light. For HeLa cells, DMEM/F12 with 10% FCS is used.
2. Dilute stock solution 10x in fresh cell culture medium to 0.025 mg/ml -> ready for application on cells. For HeLa cells, DMEM/F12 with 10% FCS is used.
3. Collect 96-well plate and replace cell-culture spent medium with 100 µl per well resazurin solution. Add some resazurin solution for background measurement in wells not containing cells.
4. Incubate for **50 minutes** in incubator at 37°C, 5% CO₂, 95% RH.
5. Transfer resazurin solution to a fresh 96- or 384-well plate.
6. Measure in plate reader using 540/25 nm (excitation) and 610/40 nm (emission) using standard settings (PTM low/OD-1.0).
7. Subtract the average background measurement from all samples. Set the average of the negative controls (well receiving OptiMEM only during transfection) to 100% and express all values as percentage of the control activity. Typically, at lower levels of treatment, elevation of metabolic activity is shown, whereas for toxic substances, at toxic concentrations sigmoidal curves can be obtained, with lower activity indicating higher toxicity.

### METHOD B

### LNP formulation (Method B)

### Solutions required:

0,33 ug/uL mRNA solution in DEPC-treated water (RiboPro, Off-The-Shelf mRNA; eGFP with Cap1)
0,04 ug/uL mRNA solution in DEPC-treated water (RiboPro, Off-The-Shelf mRNA; eGFP with Cap1)

**Table 3: Lipid formulation solution in absolute ethanol**

| Reagent | mol/L |
|---|---|
| Tested lipid | 7.36E-03 |
| ALC-0159 (CAS# 1849616-42-7) | 2.54E-04 |
| DSPC (CAS# 4539-70-2) | 1.49E-03 |
| Chol (CAS# 57-88-5) | 7.36E-03 |
| DOTAP (CAS# 132172-61-3) | 7.36E-03 |

MEM (Sigma, cat# M5650-500mL)
Dilutor: 10 mL MEM + 0,036 mL ethanol
Lipofectamine solution (Thermofisher/Invitrogen, cat#11668-019, 1 mg/mL solution)

### Procedure:

1. Negative control:
   a) Place 360 uL of Dilutor into an Eppendorf
2. Tested lipid formulation:
   In 1.5 mL eppendorf:
   a) Place 2.9 uL of 0.33 ug/uL mRNA solution into Eppendorf tube
   b) Add 1.94 µL of supplied ethanolic solution of tested lipid and immediately mix by working pipette up-and-down several times (work the solution up and down several times before transferring to saturate pipette tip with ethanol vapors)
   c) Incubate for 15 minutes.
   d) Add 536 µL of MEM. Mix the solution by vortexing.
   e) Incubate for 30 minutes.
3. Lipofectamine-50 control preparation (based on manufacturer's instructions)
   a) Place 13,5 µL of 0.04 ug/µL mRNA solution into 1.5 mL sterile Eppendorf tube.
   b) Add 527 µL of MEM
   c) Add 2,16 µL of Lipofectamine stock solution. Vortex.
   d) Incubate 15 min
4. Lipofectamine-6 control preparation:
   a) Place 45 µL Lipofectamine-50 solution into 1.5 mL sterile Eppendorf tube.
   b) Add 315 µL of MEM. Vortex.
   c) Incubate 15 min.

### Size and size distribution measurements with dynamic light scattering (Method B)

1. Transfer 100 uL of freshly prepared LNP formulation in DLS micro cuvette.
2. Place the formulation into Zetasizer pre-heated to 37 C.
3. Equilibrate the sample for 5 minutes.
4. Perform the size measurement in triplicate (settings: position and gain auto).
5. For QC examine correlation curve to have no disturbances. Report Z-average and PDI based on cumulants fit.

### Encapsulation efficiency (Method B)

### Solutions required:

Ribogreen (Invitrogen/Thermofisher, cat # 10207502)

**Table 4: Lipid formulation ethanolic solution**

| Reagent | mol/L |
|---|---|
| Tested lipid | 7.36E-03 |
| ALC-0159 (CAS# 1849616-42-7) | 2.54E-04 |
| DSPC (CAS# 4539-70-2) | 1.49E-03 |
| Chol (CAS# 57-88-5) | 7.36E-03 |
| DOTAP (CAS# 132172-61-3) | 7.36E-03 |

0,33 ug/uL mRNA solution in DEPC-treated water (RiboPro, Off-The-Shelf mRNA; eGFP with Cap1)
1X TE (Tris-EDTA) buffer solution (10 mM Tris-HCl, 1 mM EDTA, pH 7.5)
0.1% TritonX-100 in 1X TE (Tris-EDTA) buffer solution

### Procedure:

1. Prepare RiboGreen working solution:
   a) Dilute the RiboGreen reagent in 1X TE buffer according to the manufacturer's instructions (1:200 dilution).
2. Prepare RNA standards:
   a) Dilute a known concentration of RNA standard in 1X TE buffer to create a series of standards with different RNA concentrations, ranging from 0.1 to 1,000 ng/mL.
   b) Aliquot 100 µL of each standard into separate wells of a black 96-well plate.
3. Prepare liposome samples:
   a) Place 2.9 uL of 0.33 ug/uL mRNA solution into Eppendorf tube
   b) Add 1.94 uL Lipid formulation ethanolic solution and immediately mix by working pipette up-and-down several times (NB! work the solution up and down several times before transferring to saturate pipette tip with ethanol vapors)
   c) Incubate for 15 minutes.
   d) Add 268 uL of 1x TE buffer. Mix the solution by vortexing.
   e) Incubate for 30 minutes.
      Transfer 100 uL of the obtained sample into a separate eppendorf and dilute with additional 100 uL of 1x TE buffer. Vortex briefly = control sample
   g) Transfer 100 uL of the obtained sample into a separate eppendorf and dilute with 100 uL of 0.1% TritonX-100 solution. Vortex briefly, incubate at 37 C for 15 minutes = lysed sample.
   h) Aliquot 100 µL of control and lysed sample into separate wells of a black 96-well plate.
4. Perform the RiboGreen assay:
   a) Add 100 µL of the RiboGreen working solution to each well containing the RNA standards and liposome samples.
   b) Mix the contents of the wells gently by pipetting up and down or by shaking the plate on an orbital shaker.
   c) Incubate the plate in the dark at room temperature for 5-10 minutes.
   d) Using a fluorescence microplate reader, measure the fluorescence intensity of each well with an excitation wavelength of 480 nm and an emission wavelength of 525 nm.
   e) Record the fluorescence values for the RNA standards and liposome samples.
5. Calculate mRNA encapsulation efficiency:
   a) Plot the RNA standard curve by plotting the RNA concentration (ng/mL) on the x-axis and the corresponding fluorescence intensity on the y-axis. Fit the data to a linear regression model.
   b) Determine the concentration of mRNA in the liposome samples by interpolating their fluorescence values on the standard curve.
   c) Calculate the mRNA encapsulation efficiency as the percentage of the amount of mRNA in control samples of liposomes vs lysed sample of liposomes.

### Transfection efficiency with GFP-mRNA (Method B)

### Solutions required:

MEM (Sigma, cat# M5650-500mL)
Fetal Bovine Serum, FBS (Sigma, cat# F7524-500mL)
Solutions of the formulations (see above)
Dilutor: 10 mL MEM + 0,036 mL ethanol
Phosphate buffered saline, PBS

### Procedure:

1. Fill the edge wells of a 96-well plate with 100 µL media.
2. Plate 10,000 HeLa cells per well in 96 well plate. Do not use edge wells. Total 60 wells should be seeded. Incubate in MEM+10% FBS at 37°C in a humidified atmosphere with 5% CO₂ for 24h.
3. Examine under the microscope. Confluency must be at or nearly 80%.
4. Gently remove the media (aspirate). Place 50 µL of the following formulations:
   a) 6 wells with dilutor - negative control
   b) 6 wells with Lipofectamine-50 - positive control.
   c) 6 wells with Lipofectamine-6 - secondary positive control
   d) 6 wells per formulations - test samples.
5. Incubate plate for 1 hours at 37°C.
6. Add 50 µL of MEM+20% FBS (which will become 10% upon dilution).
7. Place plates into the incubator at 37°C in a humidified atmosphere with 5% CO₂ for 23 h (total 24 hours of incubation).
8. After 24 h, aspirate the media without disturbing the cells. Gently wash cells with 100 µL of warm PBS. Aspirate PBS.
9. Add 100 µL of fresh PBS into washed cells and read GFP fluorescence on the plate reader.
10. This can be followed with viability assessment with the MTT assay.

### Cell viability assessment with MTT assay (Method B)

### Solutions required:

MEM (Sigma, cat# M5650-500mL)
Fetal Bovine Serum, FBS (Sigma, cat# F7524-500mL)
Thiazolyl Blue Tetrazolium Bromide, MTT (TCI Chemicals, cat# D0801-5G)
Solutions of the formulations (see "Preparation of GFP mRNA loaded LNP" protocol)
Dilutor: 10 mL MEM + 0,036 mL ethanol
Phosphate buffered saline, PBS

### Procedure:

1. Plate 10,000 HeLa cells per well in 96 well plate. Do not use edge wells. Total 60 wells should be seeded. Incubate in MEM+10%FBS at 37°C in a humidified atmosphere with 5% CO₂ for 24 h.
2. Examine under the microscope. Confluency must be at or nearly 80%.
3. Gently remove the media (aspirate). Place 50 µL of the following formulations:
   a) 6 wells with dilutor - negative control
   b) 6 wells with Lipofectamine-50 - positive control.
   c) 6 wells with Lipofectamine-6 - secondary positive control
   d) 6 wells per formulations - test samples.
4. Incubate plate for 1 hours at 37 °C.
5. Add 50 uL of MEM+20% FBS (which will become 10% upon dilution).
6. Place plates into the incubator at 37 C for 23 hours (total 24 hours of incubation).
7. Prepare MTT solution at 1 mg/mL in sterile PBS. Filter-sterilize the solution using a 0.22 µm filter and protect it from light. This solution needs to be prepared fresh before the assay.
8. After the treatment duration, remove the treatment medium carefully from each well.
   a. Add 100 µL of the diluted MTT solution to each well.
   b. Incubate the plate for 2-4 hours at 37°C in a humidified atmosphere with 5% CO2.
9. After incubation, carefully remove the MTT solution from each well without disturbing the formazan crystals. Add 100-150 µL of a solubilization solution (isopropanol with 0.04 M HCl) to each well to dissolve the formazan crystals. Gently pipette up and down or shake the plate on an orbital shaker for 5-10 minutes to ensure complete solubilization.
10. Measure the absorbance of each well at 570 nm using a microplate reader. Measure the absorbance at a reference wavelength of 650 nm to subtract background absorbance.
11. Calculate the relative cell viability (%) by comparing with blank (untreated culture).

Results of the above experiments using lipids according to the invention as well as the reference compound are summarized in Table 5 below.

**Table 5.**

| **Example** | **Size (nm)** | **PDI** | **pKa** | **Zeta potential (mV)** | **EE (%)** | **Transfection* (Method A=100 ng, Method B=800 ng mRNA)** | **Viability % (Method A=100 ng, Method B=800 ng mRNA)** | **Method** |
|---|---|---|---|---|---|---|---|---|
| **102** | 212.9 | 0.06 | | -3.4 | 96 | 129513 | 99.7 | A |
| **104** | 184.4 | 0.043 | | -13.26 | 96 | 27631.5 | 107.6 | A |
| **107** | 152.1 | 0.186 | | -11.69 | 96 | 3677.5 | 104.1 | A |
| **108** | 198.5 | 0.211 | | -14.24 | 94 | 7926 | 103.8 | A |
| **113** | 174.6 | 0.11 | | -5.00 | 94 | 200295 | 100.7 | A |
| **115** | 130.2 | 0.13 | | 0.3 | 94 | 1135215 | 78.4 | A |
| **116** | 189.8 | 0.092 | | -14.17 | 96 | 118987 | 42.6 | A |
| **117** | 202.5 | 0.077 | | -14.14 | 98 | 7989 | 79.5 | A |
| **118** | 179.2 | 0.04 | | -15.92 | 98 | 57119.5 | 99.4 | A |
| **120** | 161.4 | 0.055 | | -13.18 | 98 | 469598.5 | 99.1 | A |
| **121** | 179.2 | 0.012 | | -11.47 | 98 | 80788.5 | 98.9 | A |
| **128** | 228.6 | 0.107 | | -14.77 | 97 | 647195.5 | 90.4 | A |
| **129** | 132.9 | 0.054 | | -12.48 | 96 | 962783 | 10.7 | A |
| **134** | 145.8 | 0.006 | | -11.99 | 98 | 398528 | 15.3 | A |
| **130** | 587.6 | 0.418 | | -11.85 | 90 | 428552 | 86.1 | A |
| **135** | 271.2 | 0.158 | | -16.11 | 91 | 538348 | 81.5 | A |
| **137** | 169.2 | 0.096 | | -16.61 | 97 | 1713925 | 82 | A |
| **136** | 305.2 | 0.259 | | -13.28 | 90 | 61666 | 85.8 | A |
| **139** | 137.4 | 0.14 | | -12.3 | 94 | 11829351 | 81.6 | A |
| **138** | 211.7 | 0.104 | | -16.19 | 95 | 1216014 | 72.1 | A |
| **131** | 244.2 | 0.219 | | -16.95 | 95 | 951145 | 97.6 | A |
| **133** | 135.7 | 0.039 | | -10.68 | 97 | 73879.5 | 82.8 | A |
| **140** | 130.8 | 0.05 | | -4.5 | 93 | 6292223 | 40.1 | A |
| **141** | 129.5 | 0.11 | | -10.6 | 95 | 4651434 | 46.0 | A |
| **142** | 125.1 | 0.06 | | -12.8 | 96 | 5491290 | 49.6 | A |
| **143** | 159.7 | 0.17 | | -5.5 | 95 | 4825007 | 72.1 | A |
| **144** | 133.9 | 0.15 | | -10.5 | 97 | 5688575 | 71.1 | A |
| **145** | 143.3 | 0.16 | | -7.7 | 97 | 5735227 | 100.5 | A |
| **146** | 116.7 | 0.07 | | -11.0 | 97 | 5015757 | 40.3 | A |
| **147** | 146.8 | 0.14 | | -7.5 | 96 | 4302243 | 80.9 | A |
| **149** | 283 | 0.190 | 6.34 | | | 34976 | 20.3 | B |
| **151** | 238.0 | 0.166 | 6.30 | | | 106106 | 46.6 | B |
| **152** | 229.0 | 0.132 | 6.35 | | | 46607 | 39.1 | B |
| **153** | 230.0 | 0.096 | 6.20 | | | 81818 | 2.7 | B |
| **154** | 248.0 | 0.185 | 6.37 | | | 67317 | 23.5 | B |
| **155** | 267.0 | 0.131 | 6.47 | | | 69688 | 28.0 | B |
| **156** | | | | | | 25588 | 52.3 | B |
| **157** | 254.0 | 0.184 | 6.52 | | | 57081 | 46.0 | B |
| **160** | | | | | | 9451 | 3.6 | B |
| **161** | | | | | | 26790 | 25.2 | B |
| **162** | | | | | | 10068 | 31.7 | B |
| **164** | | | | | | 38815 | 26.5 | B |
| **165** | | | | | | 8757 | 48.4 | B |
| **159** | 210 | 0.102 | 6.60 | | | 53830 | 29.9 | B |
| **167** | 202.9 | 0.04 | 6.42 | -13.69 | 98 | 3110369 | 90.8 | A |
| **172** | 151.7 | 0.07 | | -9.92 | 97 | 301328 | 94.4 | A |
| **178** | 209.9 | 0.058 | | -13.00 | 96 | 557798 | 34.6 | A |
| **183** | 143.9 | 0.044 | | -10.92 | 97 | 968235 | 91.1 | A |
| **168** | 224.2 | 0.085 | | -11.79 | 96 | 74333 | 86.3 | A |
| **179** | 189.1 | 0.038 | | -10.65 | 94 | 41726 | 85.5 | A |
| **171** | 178.9 | 0.017 | | -12.04 | 95 | 302489 | 87.7 | A |
| **173** | 180.5 | 0.033 | | -11.32 | 97 | 182533 | 115.6 | A |
| **184** | 237.5 | 0.146 | | -14.83 | 92 | 883795 | 87.2 | A |
| **175** | 161.2 | 0.068 | | -11.78 | 97 | 119238.5 | 80.9 | A |
| **174** | 222.6 | 0.159 | | -15.5 | 96 | 2292920 | 81.5 | A |
| **185** | 213.6 | 0.04 | | -16.4 | 97 | 1019830 | 81.7 | A |
| **176** | 175.00 | 0.18 | 6.48 | -15.0 | 95 | 12359273 | 102.0 | A |
| **188** | 160.0 | 0.08 | 6.34 | -18.1 | 98 | 11210992 | 77.5 | A |
| **187** | 334.9 | 0.255 | | -14.47 | 92 | 838984 | 101.9 | A |
| **170** | 207.5 | 0.210 | | -14.68 | 94 | 711747 | 97.3 | A |
| **181** | 188.6 | 0.049 | | -14.73 | 97 | 14238 | 107.0 | A |
| **177** | 166 | 0.100 | | -14.50 | 98 | 691264 | 56.7 | A |
| **166** | 223.6 | 0.185 | | -13.01 | 94 | 791474 | 94.5 | A |
| **169** | 159.7 | 0.09 | | -13.9 | 95 | 1379853 | 88.5 | A |
| **180** | 200.1 | 0.08 | | -15.01 | 98 | 821946 | 98.3 | A |
| **182** | 136.8 | 0.045 | | -12.56 | 96 | 227368 | 89.8 | A |
| **189** | 170 | 0.066 | | -10.68 | 94 | 45731 | 112.4 | A |
| **190** | 208.2 | 0.075 | | -11.40 | 93 | 15121 | 113.0 | A |
| **191** | 159.4 | 0.05 | | -10.35 | 96 | 34867 | 106.7 | A |
| **192** | 175.0 | 0.042 | | -12.28 | 97 | 11507 | 108.7 | A |
| **194** | 153.5 | 0.085 | | -16.45 | 96 | 152542 | 89.1 | A |
| **195** | 155.2 | 0.04 | | -5.8 | 96 | 180769 | 2.33 | A |
| **197** | 170 | 0.135 | | -12.3 | 97 | 497865 | 15.6 | A |
| **198** | 158 | 0.004 | | -13.07 | 98 | 53096 | 94.5 | A |
| **199** | 164.3 | 0.08 | | -12.42 | 97 | 144984 | 30.8 | A |
| **200** | 155.1 | 0.089 | | -13.03 | 97 | 225921 | 25.9 | A |
| **201** | 176.2 | 0.055 | | -13.1 | 95 | 25071 | 111.2 | A |
| **202** | 154.8 | 0.072 | | -10.0 | 96 | 37442 | 60.9 | A |
| **203** | 139.2 | 0.13 | | -14.95 | 94 | 161894 | 20.0 | A |
| **204** | 140 | 0.056 | | -10.63 | 97 | 282773 | 16.0 | A |
| **205** | 197.2 | 0.077 | | -13.13 | 97 | 95124 | 3.8 | A |
| **206** | | | 6.20 | | | 62404 | 34.2 | B |
| **207** | | | | | | 9906 | 64.2 | B |
| **209** | 161.6 | 0.018 | 6.61 | -12.11 | 97 | 2049133 | 82.6 | A |
| **210** | 181.2 | 0.097 | 6.40 | -18.75 | 99 | 1936082 | 81.1 | A |
| **211** | | | 6.38 | | | 69872 | | B |
| **214** | 191.2 | 0.14 | | -13.59 | 97 | 318542 | 106.3 | A |
| **215** | 204 | 0.211 | | -3.3 | 94 | 522805 | 95.8 | A |
| **216** | 269.1 | 0.228 | | -15.48 | 91 | 817325 | 82.3 | A |
| **217** | 178.1 | 0.897 | | -13.68 | 96 | 2231945 | 80.5 | A |
| **218** | 165.6 | 0.028 | | -12.88 | 97 | 94651 | 88.2 | A |
| **219** | 139.4 | 0.076 | | -16.69 | 98 | 531876 | 86.0 | A |
| **220** | 138.4 | 0.002 | | -14.55 | 98 | 367060 | 96.0 | A |
| **221** | 142.1 | 0.011 | | -13.99 | 96 | 73949 | 112.4 | A |
| **222** | 164.5 | 0.121 | | -16.36 | 94 | 1156093 | 77.9 | A |
| **223** | 206.5 | 0.15 | | -13.68 | 94 | 2167625 | 79.9 | A |
| **224** | 176.5 | 0.08 | | -16.58 | 97 | 4919513 | 72.9 | A |
| **225** | 117.9 | 0.04 | | -13.58 | 98 | 503272 | 69.6 | A |
| **ALC-0315 ref. (**CAS# 2036272-55-4**)** | 172.6 | 0.113 | | -11.12 | 94 | 836793 | 29.8 | A |
| | 249 | 0.149 | | | | 42602 | 31.5 | B |

### Conclusion

Thanks to the innovative modular approach leading to the novel ionizable cationic lipid library as described in the present invention, an extensive landscape of chemical space becomes readily accessible. By utilizing commercially available starting materials and implementing proprietary borane catalysts (as described in WO 2022/129966), compounds of formula (I) can be synthesized.

The findings from the LNP formulations and transfection studies provide evidence that the novel lipids incorporating silicon, as described in present invention, give rise to nanoparticles with suitable size, polydispersity index (PDI), and zeta potential. Furthermore, apparent pKa values were determined in certain instances, which align with the optimal range.

Moreover, the lipid nanoparticles formed from the formula (I) lipids revealed their potential for delivering GFP and Luciferase mRNA into cells while demonstrating low to moderate cell toxicity. Remarkably, a substantial number of formula (I) lipids from this study exhibit comparable or even superior transfection efficacy when compared to the reference compound ALC-0315.

## Claims

1. A compound having the structure of formula (I) 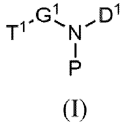
or its salt or stereoisomer,
wherein:
G¹ is unsubstituted C₂-C₉ alkylene,
- (CH₂)ₓ-CH=CH-(CH₂) _{y}-, wherein x is an integer selected from 1 to 6, y is an integer selected from 1 to 6, and the sum of x+y is an integer selected from 2 to 7, or
- (CH₂)_{w}-X-(CH₂) _{z}-, wherein w is an integer selected from 1 to 7, z is an integer selected from 2 to 7, the sum of w+z is an integer selected from 3 to 8, wherein-(CH₂)_{z}- is attached to N, and X is selected from O, S, SO and SO₂,
T¹ is 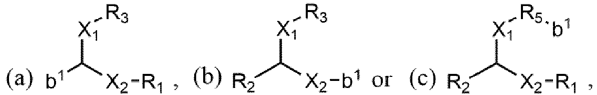 wherein
b¹ is a bond to G¹,
X₁ and X₂ are the same or different and each independently represents O or S,
R₁ is linear C₁-C₁₇ alkyl, non-linear C₃-C₁₇ alkyl, in both cases optionally containing one S, SO, SO₂, O, or Si(R^{a})₂, wherein R^{a} is C₁-C₆ alkyl, at any position in the carbon chain with the proviso that the heteroatom is not in the alpha or omega position of the carbon chain, C₃-C₁₇ alkenyl containing one double bond with the proviso that there is at least one -CH₂- group between the double bond and X₂, or polyunsaturated C₈-C₂₀ alkenyl with the proviso that there is at least one -CH₂- group between the first double bond and X₂, or
R₁ is 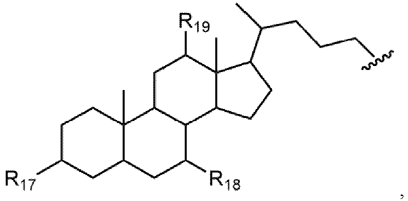 wherein R₁₇, R₁₈ and R₁₉ are the same or different and each independently can be H, OH, -C₁-C₆ alkoxy or fluorine;
R₂ is
linear C₁-C₁₇ alkyl, non-linear C₃-C₁₇ alkyl, in both cases optionally containing one S, SO, SO₂, O, or Si(R^{b})₂, wherein R^{b} is C₁-C₆ alkyl, at any position in the carbon chain with the proviso that the heteroatom is not in the alpha or omega position of the carbon chain , C₃-C₁₇ alkenyl containing one double bond with the proviso that there is at least one -CH₂- group between the double bond and the carbon linking X₁ and X₂, or polyunsaturated C₈-C₂₀ alkenyl with the proviso that there is at least one -CH₂- group between the first double bond and the carbon linking X₁ and X₂, or
R₂ is 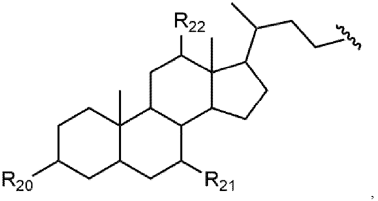 wherein R₂₀, R₂₁ and R₂₂ are the same or different and each independently can be H, OH, -C₁-C₆ alkoxy or fluorine,
with the proviso that R₁ and R₂ cannot be both at the same time, 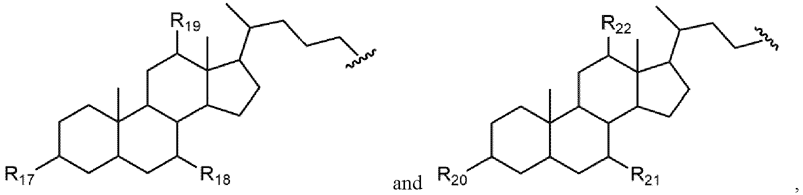 or
R₁ is connected to R₃ via alkylene or monounsaturated alkenyl forming a C₃-C₃₀-membered ring, or
R₂ is connected to R₃ via alkylene or monounsaturated alkenyl forming a C₃-C₃₀-membered ring, wherein the carbon atom of R₂ at the first or second position numbered from that carbon atom which is attached to the carbon linking X₁ and X₂, can be optionally replaced by O or S heteroatom, or
R₂ is connected to R₁ via alkylene or monounsaturated alkenyl forming a C₃-C₃₀-membered ring, wherein the carbon atom of R₂ at the first or second position numbered from that carbon atom which is attached to the carbon linking X₁ and X₂, can be optionally replaced by O or S heteroatom;
R₃ is 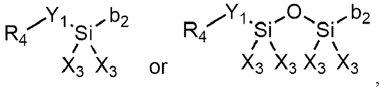
R₅-b¹ is 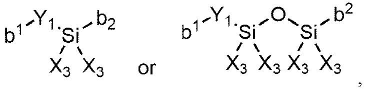
wherein
b² is a link to X₁,
Y₁ is -O- or -CH₂- or -O-CH₂-CH₂- wherein -CH₂- is attached to Si,
each X₃ is independently selected from the group consisting of C₁-C₄ alkyl or C₁-C₄ alkoxy,
R₄ is linear C₂-C₁₇ alkyl, or non-linear C₃-C₁₇ alkyl, in both cases optionally containing one S, SO, SO₂, O, or Si(R^{c})₂, wherein R^{c} is C₁-C₆ alkyl, at any position in the carbon chain with the proviso that the heteroatom is not in the alpha or omega position of the carbon chain, C₃-C₂₀ alkenyl containing one double bond with the proviso there is at least one -CH₂- group between the double bond and Y₁, or polyunsaturated C₈-C₂₀ alkenyl with the proviso there is at least one -CH₂- group between the first double bond and Y₁ or,
R⁴ is 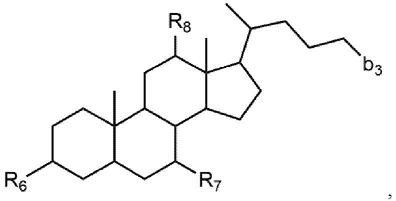
wherein b³ is a bond to Y₁,
R₆, R₇, R₈ are the same or different and each independently can be H, OH, -C₁-C₆ alkoxy or fluorine;
D¹ is selected from the group consisting of 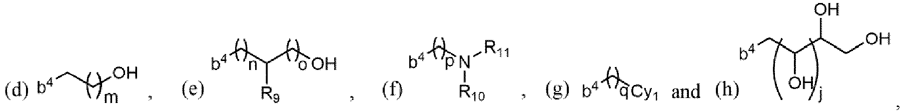 wherein
b⁴ is a bond to nitrogen,
R₉ can be C₁-C₆ alkyl, cyclopentyl, cyclohexyl, hydroxyl, hydroxymethyl, hydroxyethyl, phenyl, benzyl, 4-hydroxy benzyl, 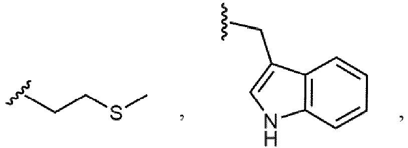
R₁₀ and R₁₁ are independently selected from H and C₁-C₆ alkyl,
m is an integer selected from 1 to 6,
n is an integer selected from 0 to 6,
o is an integer selected from 0 to 6,
p is an integer selected from 2 to 6,
q is an integer selected from 0 to 6,
j is an integer selected from 1 to 4,
Cy₁ is a C₃-C₆ cycloalkyl optionally substituted by one or more -OH, or
Cy₁ is a pyranose, furanose ring, which can be linked via any of its OH group, wherein the pyranose or furanose ring is optionally substituted by a -NH-CO-CH₃ group, adenine, guanine, uracil, cytosine, thymine, a mono- or oligosaccharide, or by a 4-, 5-, 6- or 7-membered heterocyclic ring containing 1, 2, 3 or 4 heteroatoms selected from N, O or S; or
Cy₁ is a 4-, 5-, 6- or 7-membered heterocyclic ring containing 1, 2, 3 or 4 heteroatoms selected from O, N or S, wherein the heterocyclic ring is optionally substituted by R₁₂,
wherein
R₁₂ represents C₁-C₆ alkyl, an arginine containing peptide, a pyranose or furanose ring attached by any of their ring-carbon atoms to the 4-, 5-, 6- or 7-membered heterocyclic ring, wherein the pyranose or furanose ring is optionally substituted by a -NH-CO-CH₃ group, adenine, guanine, uracil, cytosine, thymine or a mono- or oligosaccharide,
R₁₂ can be a group selected from (d), (e) or (f), wherein b⁴ is a bond to the heterocyclic ring, m, n, o, p, R₉, R₁₀ and R₁₁ are as defined above,
or
R₁₂ can be a 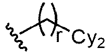 group wherein r is an integer selected from 1 to 4 and Cy₂ is a 4-, 5-, 6- or 7-membered heterocyclic ring containing 1, 2, 3 or 4 heteroatoms selected from O, N and S, optionally substituted by C₁-C₆ alkyl;
P is selected from
(i) G²-T²
wherein
G² is defined as G¹ above, where G¹ and G² may be identical or different;
T² is defined as T¹ above, b¹ is a bond to G², and wherein T¹ and T² may be identical or different; or
(ii) G²-T³
wherein
G² is as defined above;
T³ is 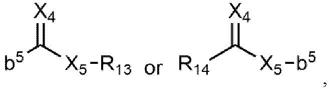
wherein
b⁵ is a bond to G²,
X₄ and X₅ can be the same or different and each independently is O, S, or NR^{d} wherein R^{d} is H, C₁-C₆ alkyl, -OH or C₁-C₆ alkoxy,
R₁₃ is linear C₂-C₁₇ alkyl, non-linear C₃-C₁₇ alkyl, both optionally containing one of S, SO, SO₂, O, Si(R^{e})₂, wherein R^{e} is C₁-C₆ alkyl at any position in the carbon chain with the proviso that the heteroatom is not in the alpha or omega position of the carbon chain, C₃-C₂₀ alkenyl containing one double bond with the proviso there is at least one -CH₂- group between the double bond and X₅ or polyunsaturated C₈-C₂₀ alkenyl with the proviso that there is at least one -CH₂-between the first double bond and X₅, or
R₁₃ is 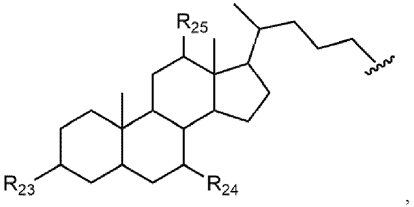 wherein R₂₃, R₂₄, R₂₅ are the same or different and each independently can be H, OH, - C₁-C₆ alkoxy or fluorine,
R₁₄ is linear C₂-C₁₇ alkyl, non-linear C₃-C₁₇ alkyl, both optionally containing one of S, SO, SO₂, O, Si(R^{f})₂, wherein R^{f} is C₁-C₆ alkyl at any position in the carbon chain with the proviso that the heteroatom is not in the alpha or omega position of the carbon chain, C₃-C₂₀ alkenyl containing one double bond with the proviso there is at least one -CH₂- group between the double bond and the carbon linking X₄ and X₅ or polyunsaturated C₈-C₂₀ alkenyl with the proviso that there is at least one -CH₂- between the first double bond and the carbon linking X₄ and X₅, or
R₁₄ is 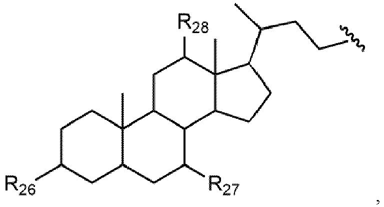 wherein R₂₆, R₂₇, R₂₈ are the same or different and each independently can be H, OH, - C₁-C₆ alkoxy or fluorine;
and (iii) T⁴, wherein
T⁴ is 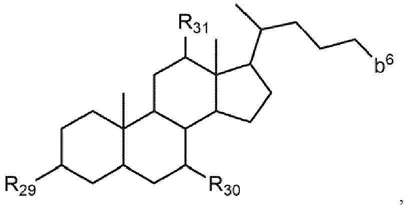 wherein, b⁶ is a bond to the nitrogen atom, R₂₉, R₃₀, R₃₁ can be the same or different and each independently can be H, OH, -C₁-C₆ alkoxy or fluorine;
C₄-C₂₀ alkenyl containing one double bond, with the proviso that there is at least one -CH₂-between the double bond and N, or
polyunsaturated C₈-C₂₀ alkenyl, with the proviso that there is at least one -CH₂- between the first double bond and N.

2. The compound of claim 1, wherein G¹ and, if present, G² are the same or different and each is independently linear C₄-C₉ alkylene, preferably C₅-C₉ alkylene, or, alternatively, each is independently linear C₅, C₆, C₇, or C₉ alkylene.

3. The compound of claim 1 or 2, wherein R₁ is linear C₁, C₂, C₄, C₆, C₇, C₈, C₉, C₁₀, or C₁₂ alkyl, or R₁ is linear C₉ alkenyl, and/or R₂ is linear C₁, C₃, C₅, C₆, C₇, C₈, C₉, or C₁₁ alkyl, preferably R₁ is linear C₂, C₈, C₁₀ alkyl, or R₁ is linear C₉ alkenyl and/or R₂ is linear C₁, C₃,C₇, or C₉ alkyl.

4. The compound of claim 3, wherein T¹ is (c) and R₁ is linear C₂, C₄, C₆, C₇, C₈, C₉, C₁₀, or C₁₂ alkyl, or R₁ is linear C₉ alkenyl and R₂ is linear C₁, C₃, C₅, C₆, C₇, C₈, C₉, or C₁₁ alkyl, preferably R₁ is linear C₂, C₈, C₁₀ alkyl, or R₁ is linear C₉ alkenyl, and R₂ is linear C₁, C₃, C₇, or C₉ alkyl.

5. The compound of any one of claims 1 to 3, wherein P is G²-T².

6. The compound of claim 5, wherein G¹-T¹ and G²-T² are identical.

7. The compound of any one of claims 1 to 3, wherein P is G²-T³.

8. The compound of claim 7, wherein T¹-G¹ has one of the following structures

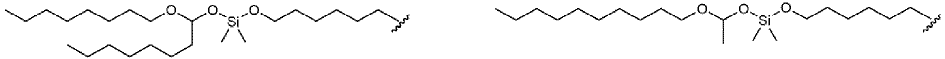

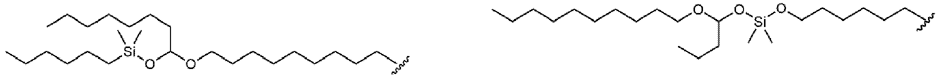

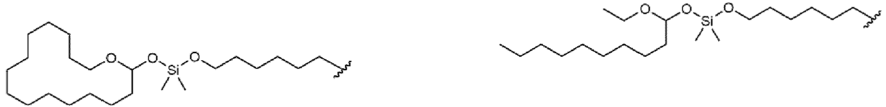

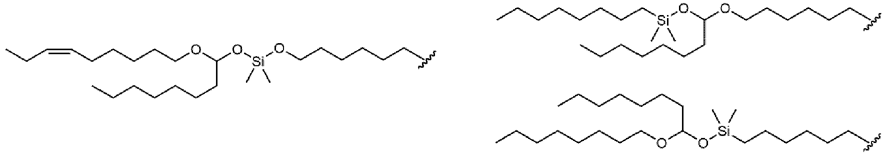

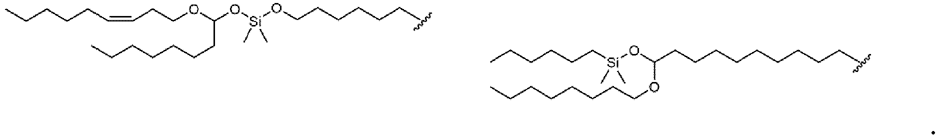

9. The compound of claim 7 or 8, wherein T³ has the following structure 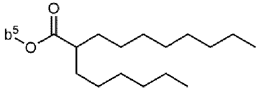 wherein b⁵ is the bond to G².

10. The compound of claim 7 or 8, wherein T³ has one of the following structures 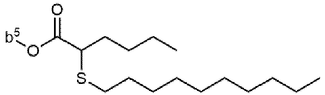 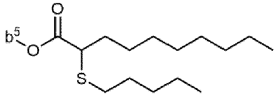 wherein b⁵ is the bond to G².

11. The compound of claim 7 or 8, wherein T³ has one of the following structures 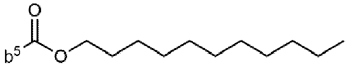 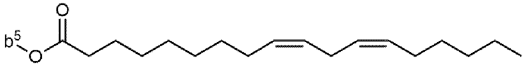 wherein b⁵ is the bond to G².

12. The compound of claim 7 or 8, wherein T³ has the following structure 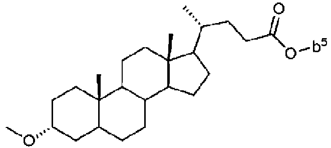 wherein b⁵ is the bond to G².

13. The compound of any one of claims 1 to 3, wherein P is T⁴.

14. The compound of claim 13, wherein T⁴ is 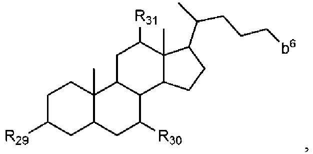 wherein, b⁶ is a bond to the nitrogen atom, R₂₉, R₃₀, R₃₁ can be the same or different and each independently can be H, OH, -C₁-C₆ alkoxy or fluorine; or
polyunsaturated C₈-C₂₀ alkenyl, with the proviso that there is at least one -CH₂- between the first double bond and N.

15. The compound of claim 14, wherein T¹-G¹ has one of the following structures

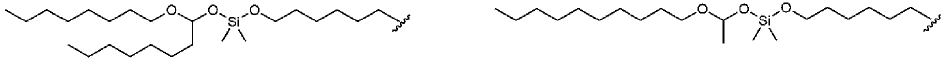

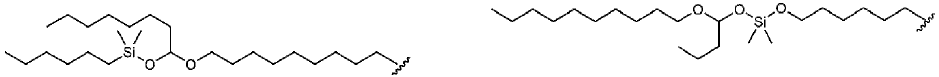

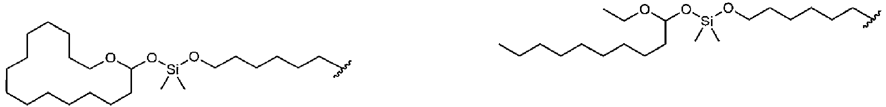

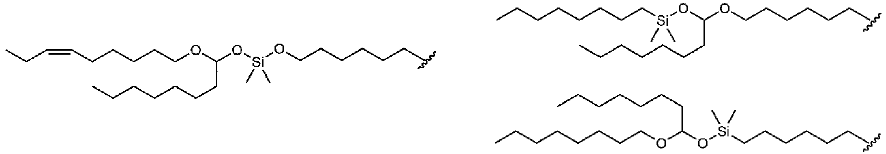

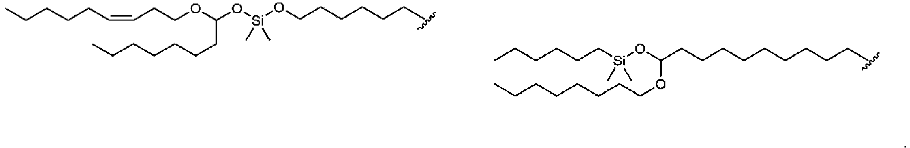

16. The compound of claim 14 or 15, wherein T⁴ has the following structure 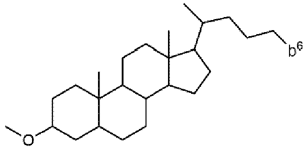 wherein b⁶ is a bond to the nitrogen atom.

17. The compound of any one of claims 1 to 16, wherein D¹ has one of the following structures:

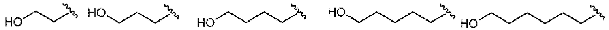

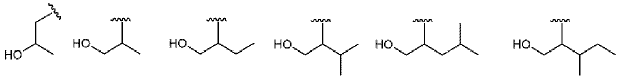

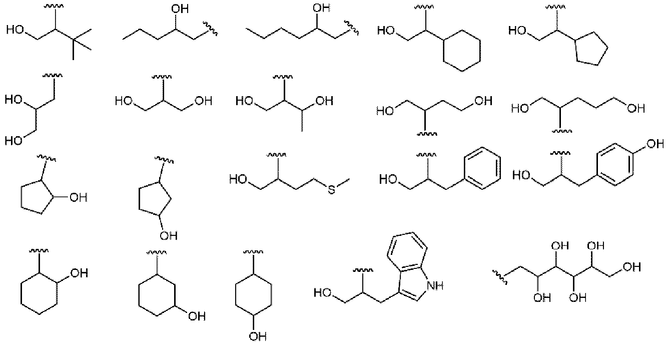

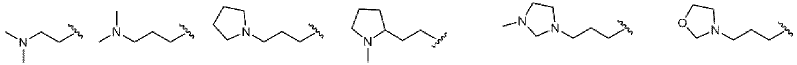

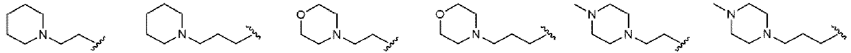

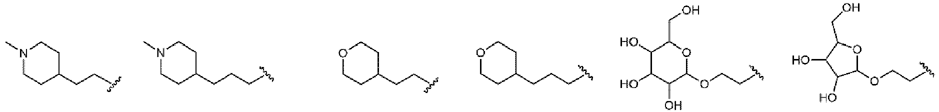

18. The compound of any one of claims 1 to 16, wherein D¹ has one of the following structures: 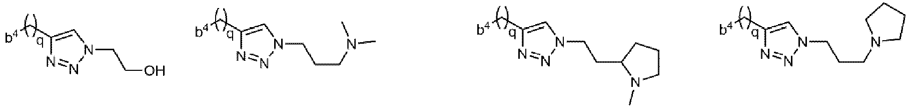 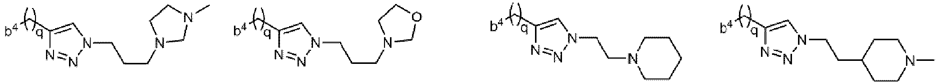 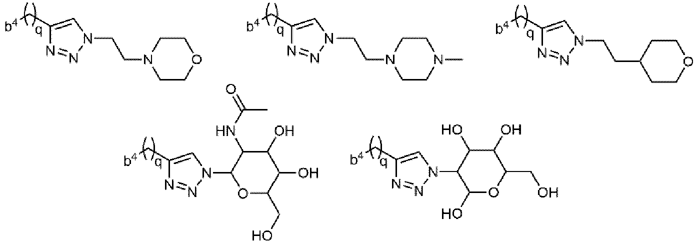 wherein q is an integer selected from 1 to 4 and b⁴ is a bond to nitrogen.

19. The compound of any one of claims 1-16, wherein D¹ has one of the following structures:

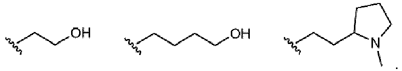

20. A compound of claim 1, which has any of the following structures:
| **Structure** | **IUPAC name** |
|---|---|
| 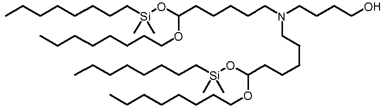 | 4-(bis(6-((dimethyl(octyl)silyl)oxy)-6-(octyloxy)hexyl)amino)butan-1-ol |
| 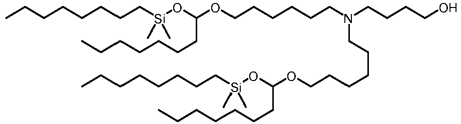 | 5-(6-((1-((dimethyl(octyl)silyl)oxy)octyl)oxy)hexyl)-13-heptyl-15,15-dimethyl-12,14-dioxa-5-aza-15-silatricosan-1-ol |
| 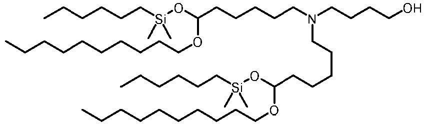 | 4-(bis(6-(decyloxy)-6-((hexyldimethylsilyl)oxy)hexyl)amino)butan-1-ol |
| 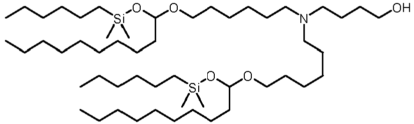 | 5-(6-((1-((hexyldimethylsilyl)oxy)decyl)oxy)hexyl)-15,15-dimethyl-13-nonyl-12,14-dioxa-5-aza-15-silahenicosan-1-ol |
| 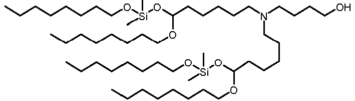 | 5-(6-((dimethyl(octyloxy)silyl)oxy)-6-(octyloxy)hexyl)-13,13-dimethyl-11-(octyloxy)-12,14-dioxa-5-aza-13-siladocosan-1-ol |
| 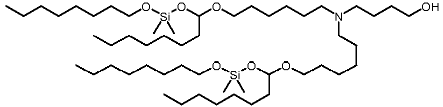 | 13-heptyl-5-(8-heptyl-10,10-dimethyl-7,9,11-trioxa-10-silanonadecyl)-15,15-dimethyl-12,14,16-trioxa-5-aza-15-silatetracosan-1-ol |
| 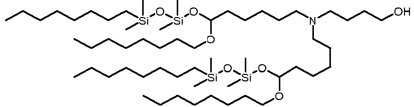 | 4-(bis(6-(octyloxy)-6-((1,1,3,3-tetramethyl-3-octyldisiloxaneyl)oxy)hexyl)amino)butan-1-ol |
| 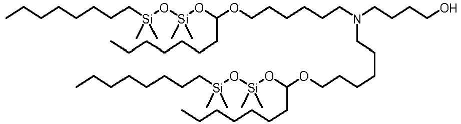 | 13-heptyl-15,15,17,17-tetramethyl-5-(6-((1-((1,1,3,3-tetramethyl-3-octyldisiloxaneyl)oxy)octyl)oxy)hexyl)-12,14,16-trioxa-5-aza-15,17-disilapentacosan-1-ol |
| 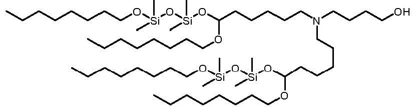 | 4-(bis(6-(octyloxy)-6-((1,1,3,3-tetramethyl-3-(octyloxy)disiloxaneyl)oxy)hexyl)amino)butan-1-ol |
| 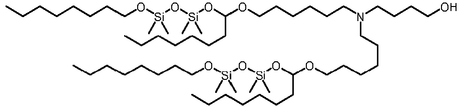 | 13-heptyl-15,15,17,17-tetramethyl-5-(6-((1-((1,1,3,3-tetramethyl-3-(octyloxy)disiloxaneyl)oxy)octyl)oxy)hexyl)-12,14,16,18-tetraoxa-5-aza-15,17-disilahexacosan-1-ol |
| 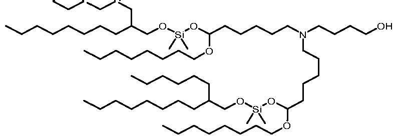 | 16-hexyl-5-(6-((((2-hexyldecyl)oxy)dimethylsilyl)oxy)-6-(octyloxy)hexyl)-13,13-dimethyl-11-(octyloxy)-12,14-dioxa-5-aza-13-silatetracosan-1-ol |
| 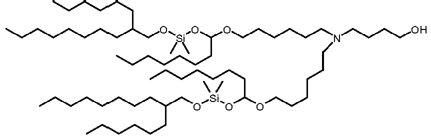 | 13-heptyl-5-(8-heptyl-13-hexyl-10,10-dimethyl-7,9,11-trioxa-10-silahenicosyl)-18-hexyl-15,15-dimethyl-12,14,16-trioxa-5-aza-15-silahexacosan-1-ol |
| 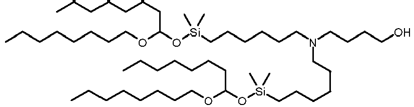 | 5-(6-(dimethyl((1-(octyloxy)octyl)oxy)silyl)hexyl)-14-heptyl-12,12-dimethyl-13,15-dioxa-5-aza-12-silatricosan-1-ol |
| 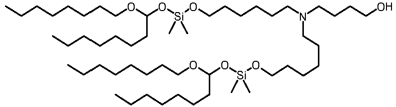 | 15-heptyl-5-(10-heptyl-8,8-dimethyl-7,9,11-trioxa-8-silanonadecyl)-13,13-dimethyl-12,14,16-trioxa-5-aza-13-silatetracosan-1-ol |
| 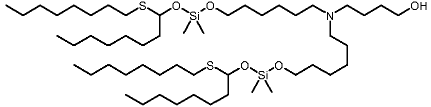 | 15-heptyl-5-(10-heptyl-8,8-dimethyl-7,9-dioxa-11-thia-8-silanonadecyl)-13,13-dimethyl-12,14-dioxa-16-thia-5-aza-13-silatetracosan-1-ol |
| 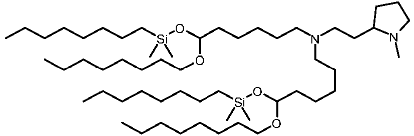 | 6-((dimethyl(octyl)silyl)oxy)-N-(6-((dimethyl(octyl)silyl)oxy)-6-(octyloxy)hexyl)-N-(2-(1-methylpyrrolidin-2-yl)ethyl)-6-(octyloxy)hexan-1-amine |
| 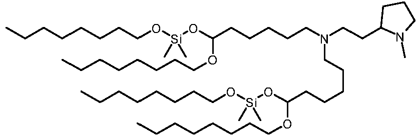 | 6-((dimethyl(octyloxy)silyl)oxy)-N-(6-((dimethyl(octyloxy)silyl)oxy)-6-(octyloxy)hexyl)-N-(2-(1-methylpyrrolidin-2-yl)ethyl)-6-(octyloxy)hexan-1-amine |
| 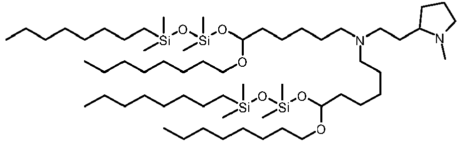 | N-(2-(1-methylpyrrolidin-2-yl)ethyl)-6-(octyloxy)-N-(6-(octyloxy)-6-((1,1,3,3-tetramethyl-3 -octyldisiloxaneyl)oxy)hexyl)-6-((1,1,3,3-tetramethyl-3-octyldisiloxaneyl)oxy)hexan-1-amine |
| 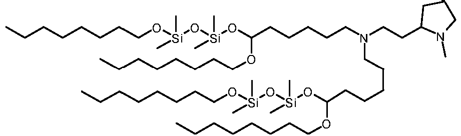 | N-(2-(1-methylpyrrolidin-2-yl)ethyl)-6-(octyloxy)-N-(6-(octyloxy)-6-((1,1,3,3-tetramethyl-3-(octyloxy)disiloxaneyl)oxy)hexyl)-6-((1,1,3,3-tetramethyl-3-(octyloxy)disiloxaneyl)oxy)hexan-1-amine |
| 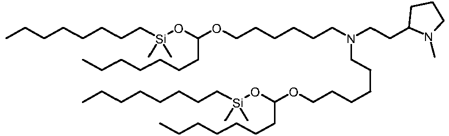 | 6-((1-((dimethyl(octyl)silyl)oxy)octyl)oxy)-N-(6-((1-((dimethyl(octyl)silyl)oxy)octyl)oxy)hexyl)-N-(2-(1-methylpyrrolidin-2-yl)ethyl)hexan-1-amine |
| 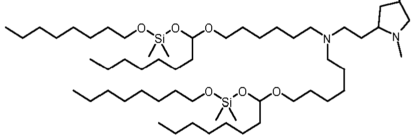 | 8-heptyl-N-(8-heptyl-10,10-dimethyl-7,9,11-trioxa-10-silanonadecyl)-10,10-dimethyl-N-(2-(1-methylpyrrolidin-2-yl)ethyl)-7,9,11-trioxa-10-silanonadecan-1-amine |
| 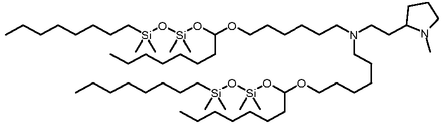 | N-(2-(1-methylpyrrolidin-2-yl)ethyl)-6-((1-((1,1,3,3-tetramethyl-3-octyldisiloxaneyl)oxy)octyl)oxy)-N-(6-((1-((1,1,3,3-tetramethyl-3-octyldisiloxaneyl)oxy)octyl)oxy)hexyl)hexan-1-amine |
| 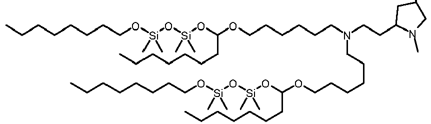 | N-(2-(1-methylpyrrolidin-2-yl)ethyl)-6-((1-((1,1,3,3-tetramethyl-3-(octyloxy)disiloxaneyl)oxy)octyl)oxy)-N-(6-((1-((1,1,3,3-tetramethyl-3-(octyloxy)disiloxaneyl)oxy)octyl)oxy)hexyl)hex an-1-amine |
| 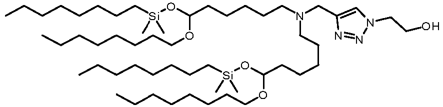 | 2-(4-((bis(6-((dimethyl(octyl)silyl)oxy)-6-(octyloxy)hexyl)amino)methyl)-1H-1,2,3-triazol-1-yl)ethan-1-ol |
| 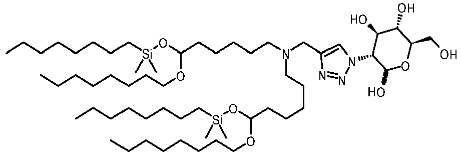 | (3R,4R,5S,6R)-3-(4-((bis(6-((dimethyl(octyl)silyl)oxy)-6-(octyloxy)hexyl)amino)methyl)-1H-1,2,3-triazol-1-yl)-6-(hydroxymethyl)tetrahydro-2H-pyran-2,4,5-triol |
| 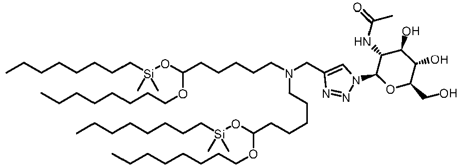 | N-((2R,3R,4R,5S,6R)-2-(4-((bis(6-((dimethyl(octyl)silyl)oxy)-6-(octyloxy)hexyl)amino)methyl)-1H-1,2,3-triazol-1-yl)-4,5-dihydroxy-6-(hydroxymethyl)tetrahydro-2H-pyran-3-yl)acetamide |
| 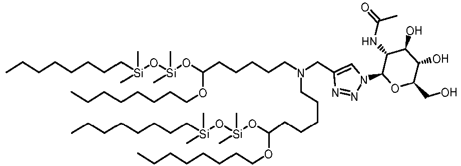 | N-((2R,3R,4R,5S,6R)-2-(4-((bis(6-(octyloxy)-6-((1,1,3,3-tetramethyl-3-octyldisiloxaneyl)oxy)hexyl)amino)methyl)-1H-1,2,3-triazol-1-yl)-4,5-dihydroxy-6-(hydroxymethyl)tetrahydro-2H-pyran-3-yl)acetamide |
| 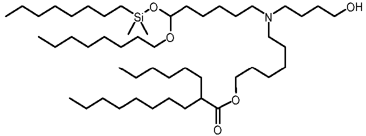 | 6-((6-((dimethyl(octyl)silyl)oxy)-6-(octyloxy)hexyl)(4-hydroxybutyl)amino)hexyl 2-hexyldecanoate |
| 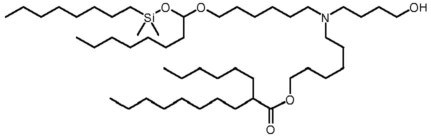 | 15-heptyl-7-(4-hydroxybutyl)-17,17-dimethyl-14,16-dioxa-7-aza-17-silapentacosyl 2-hexyldecanoate |
| 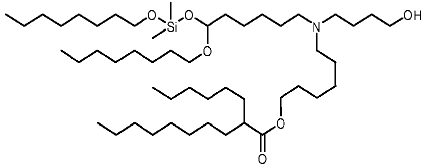 | 7-(4-hydroxybutyl)-15,15-dimethyl-13-(octyloxy)-14,16-dioxa-7-aza-15-silatetracosyl 2-hexyldecanoate |
| 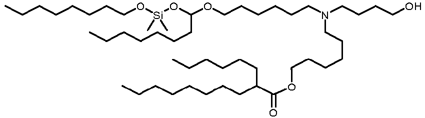 | 15-heptyl-7-(4-hydroxybutyl)-17,17-dimethyl-14,16,18-trioxa-7-aza-17-silahexacosyl 2-hexyldecanoate |
| 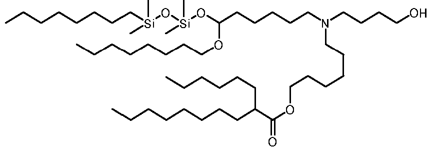 | 6-((4-hydroxybutyl)(6-(octyloxy)-6-((1,1,3,3-tetramethyl-3-octyldisiloxaneyl)oxy)hexyl)amino)hexyl 2-hexyldecanoate |
| 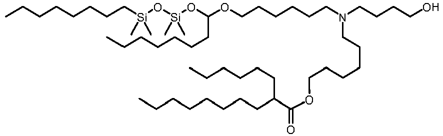 | 15-heptyl-7-(4-hydroxybutyl)-17,17,19,19-tetramethyl-14,16,18-trioxa-7-aza-17,19-disilaheptacosyl 2-hexyldecanoate |
| 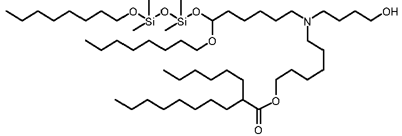 | 6-((4-hydroxybutyl)(6-(octyloxy)-6-((1,1,3,3-tetramethyl-3-(octyloxy)disiloxaneyl)oxy)hexyl)amino)hexyl 2-hexyldecanoate |
| 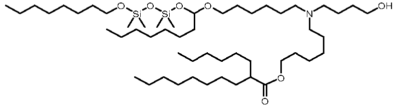 | 15-heptyl-7-(4-hydroxybutyl)-17,17,19,19-tetramethyl-14,16,18,20-tetraoxa-7-aza-17,19-disilaoctacosyl 2-hexyldecanoate |
| 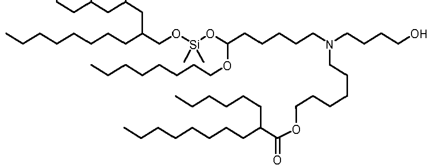 | 18-hexyl-7-(4-hydroxybutyl)-15,15-dimethyl-13-(octyloxy)-14,16-dioxa-7-aza-15-silahexacosyl 2-hexyldecanoate |
| 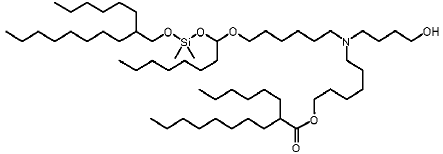 | 15-heptyl-20-hexyl-7-(4-hydroxybutyl)-17,17-dimethyl-14,16,18-trioxa-7-aza-17-silaoctacosyl 2-hexyldecanoate |
| 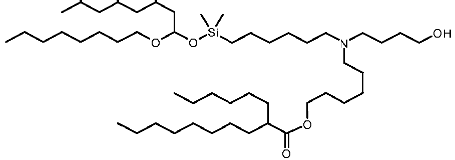 | 16-heptyl-7-(4-hydroxybutyl)-14,14-dimethyl-15,17-dioxa-7-aza-14-silapentacosyl 2-hexyldecanoate |
| 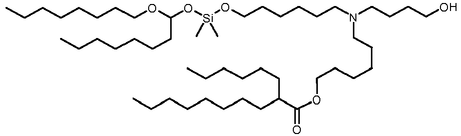 | 17-heptyl-7-(4-hydroxybutyl)-15,15-dimethyl-14,16,18-trioxa-7-aza-15-silahexacosyl 2-hexyldecanoate |
| 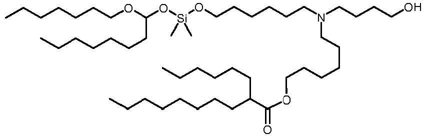 | 17-heptyl-7-(4-hydroxybutyl)-15,15-dimethyl-14,16,18-trioxa-7-aza-15-silapentacosyl 2-hexyldecanoate |
| 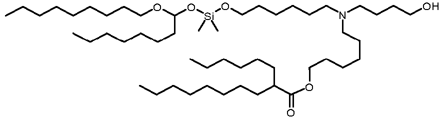 | 17-heptyl-7-(4-hydroxybutyl)-15,15-dimethyl-14,16,18-trioxa-7-aza-15-silaheptacosyl 2-hexyldecanoate |
| 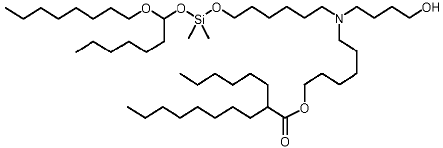 | 17-hexyl-7-(4-hydroxybutyl)-15,15-dimethyl-14,16,18-trioxa-7-aza-15-silahexacosyl 2-hexyldecanoate |
| 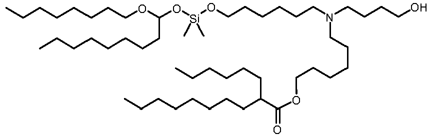 | 7-(4-hydroxybutyl)-15,15-dimethyl-17-octyl-14,16,18-trioxa-7-aza-15-silahexacosyl 2-hexyldecanoate |
| 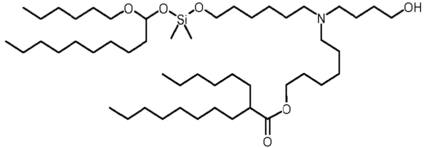 | 18-(4-hydroxybutyl)-10,10-dimethyl-8-nonyl-7,9,11-trioxa-18-aza-10-silatetracosan-24-yl 2-hexyldecanoate |
| 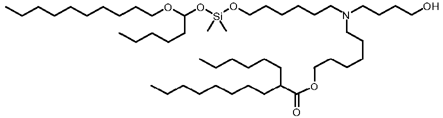 | 7-(4-hydroxybutyl)-15,15-dimethyl-17-pentyl-14,16,18-trioxa-7-aza-15-silaoctacosyl 2-hexyldecanoate |
| 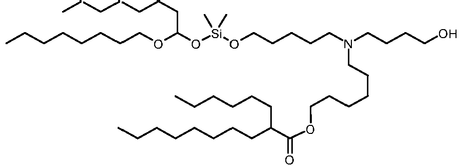 | 16-heptyl-7-(4-hydroxybutyl)-14,14-dimethyl-13,15,17-trioxa-7-aza-14-silapentacosyl 2-hexyldecanoate |
| 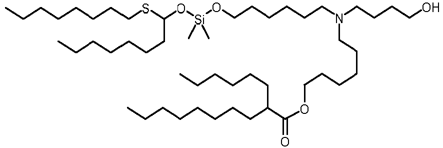 | 17-heptyl-7-(4-hydroxybutyl)-15,15-dimethyl-14,16-dioxa-18-thia-7-aza-15-silahexacosyl 2-hexyldecanoate |
| 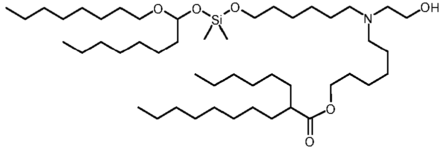 | 17-heptyl-7-(2-hydroxyethyl)-15,15-dimethyl-14,16,18-trioxa-7-aza-15-silahexacosyl 2-hexyldecanoate |
| 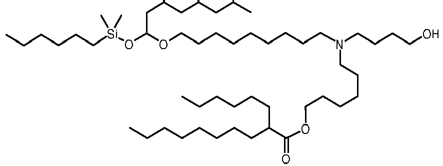 | 9-heptyl-20-(4-hydroxybutyl)-7,7-dimethyl-8,10-dioxa-20-aza-7-silahexacosan-26-yl 2-hexyldecanoate |
| 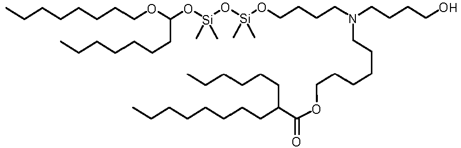 | 17-heptyl-7-(4-hydroxybutyl)-13,13,15,15-tetramethyl-12,14,16,18-tetraoxa-7-aza-13,15-disilahexacosyl 2-hexyldecanoate |
| 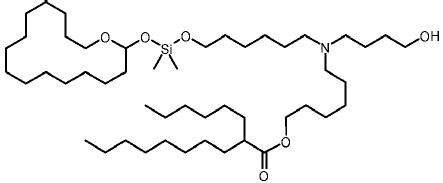 | 6-((6-((dimethyl((oxacyclohexadecan-2-yl)oxy)silyl)oxy)hexyl)(4-hydroxybutyl)amino)hexyl 2-hexyldecanoate |
| 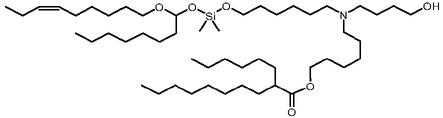 | (Z)-17-heptyl-7-(4-hydroxybutyl)-15,15-dimethyl-14,16,18-trioxa-7-aza-15-silaheptacos-24-en-1-yl 2-hexyldecanoate |
| 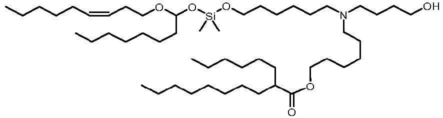 | (Z)-17-heptyl-7-(4-hydroxybutyl)-15,15-dimethyl-14,16,18-trioxa-7-aza-15-silaheptacos-21-en-1-yl 2-heayldecanoate |
| 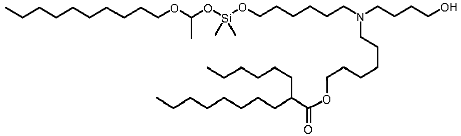 | 7-(4-hydroxybutyl)-15,15,17-trimethyl-14,16,18-trioxa-7-aza-15-silaoctacosyl 2-hexyldecanoate |
| 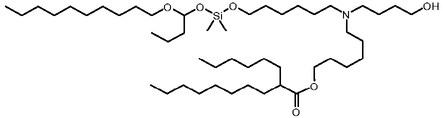 | 7-(4-hydroxybutyl)-15,15-dimethyl-17-propyl-14,16,18-trioxa-7-aza-15-silaoctacosyl 2-hexyldecanoate |
| 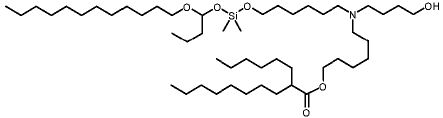 | 7-(4-hydroxybutyl)-15,15-dimethyl-17-propyl-14,16,18-trioxa-7-aza-15-silatriacontyl 2-hexyldecanoate |
| 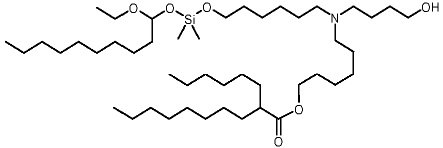 | 14-(4-hydroxybutyl)-6,6-dimethyl-4-nonyl-3,5,7-trioxa-14-aza-6-silaicosan-20-yl 2-hexyldecanoate |
| 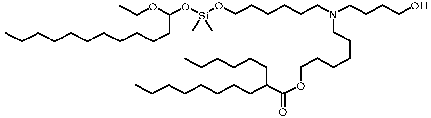 | 14-(4-hydroxybutyl)-6,6-dimethyl-4-undecyl-3,5,7-trioxa-14-aza-6-silaicosan-20-yl 2-hexyldecanoate |
| 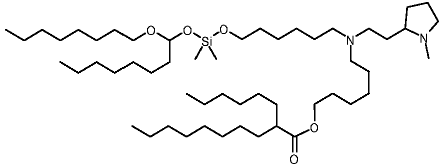 | 17-heptyl-15,15-dimethyl-7-(2-(1-methylpyrrolidin-2-yl)ethyl)-14,16,18-trioxa-7-aza-15-silahexacosyl 2-hexyldecanoate |
| 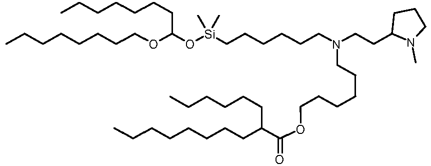 | 16-heptyl-14,14-dimethyl-7-(2-(1-methylpyrrolidin-2-yl)ethyl)-15,17-dioxa-7-aza-14-silapentacosyl 2-hexyldecanoate |
| 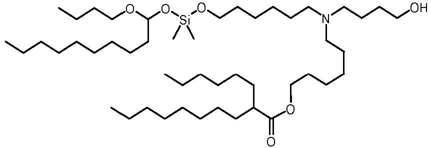 | 16-(4-hydroxybutyl)-8,8-dimethyl-6-nonyl-5,7,9-trioxa-16-aza-8-siladocosan-22-yl 2-hexyldecanoate |
| 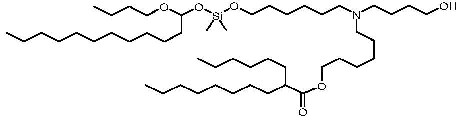 | 16-(4-hydroxybutyl)-8,8-dimethyl-6-undecyl-5,7,9-trioxa-16-aza-8-siladocosan-22-yl 2-hexyldecanoate |
| 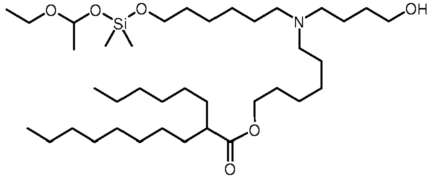 | 14-(4-hydroxybutyl)-4,6,6-trimethyl-3,5,7-trioxa-14-aza-6-silaicosan-20-yl 2-hexyldecanoate |
| 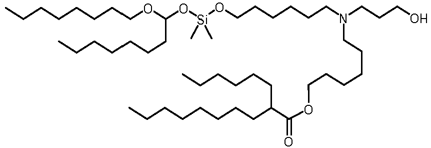 | 17-heptyl-7-(3-hydroxypropyl)-15,15-dimethyl-14,16,18-trioxa-7-aza-15-silahexacosyl 2-hexyldecanoate |
| 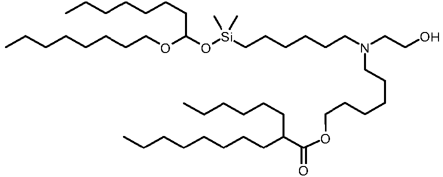 | 16-heptyl-7-(2-hydroxyethyl)-14,14-dimethyl-15,17-dioxa-7-aza-14-silapentacosyl 2-hexyldecanoate |
| 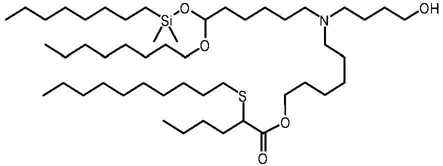 | 6-((6-((dimethyl(octyl)silyl)oxy)-6-(octyloxy)hexyl)(4-hydroxybutyl)amino)hexyl 2-(decylthio)hexanoate |
| 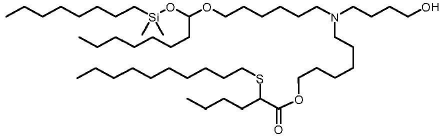 | 15-heptyl-7-(4-hydroxybutyl)-17,17-dimethyl-14,16-dioxa-7-aza-17-silapentacosyl 2-(decylthio)hexanoate |
| 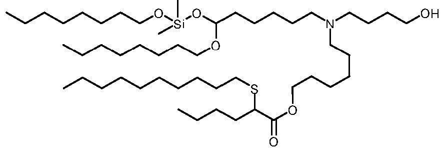 | 7-(4-hydroxybutyl)-15,15-dimethyl-13-(octyloxy)-14,16-dioxa-7-aza-15-silatetracosyl 2-(decylthio)hexanoate |
| 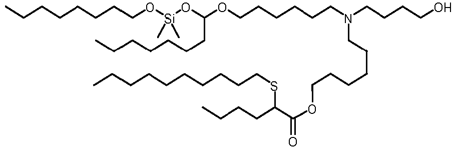 | 15-heptyl-7-(4-hydroxybutyl)-17,17-dimethyl-14,16,18-trioxa-7-aza-17-silahexacosyl 2-(decylthio)hexanoate |
| 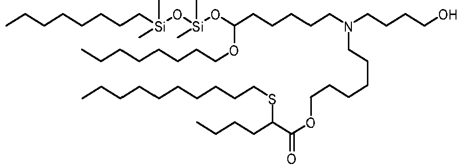 | 6-((4-hydroxybutyl)(6-(octyloxy)-6-((1,1,3,3-tetramethyl-3-octyldisiloxaneyl)oxy)hexyl)amino)hexyl 2-(decylthio)hexanoate |
| 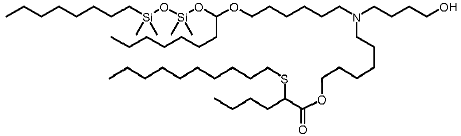 | 15-heptyl-7-(4-hydroxybutyl)-17,17,19,19-tetramethyl-14,16,18-trioxa-7-aza-17,19-disilaheptacosyl 2-(decylthio)hexanoate |
| 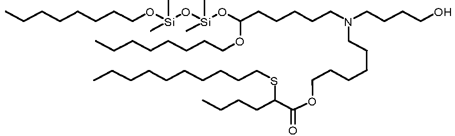 | 6-((4-hydroxybutyl)(6-(octyloxy)-6-((1,1,3,3-tetramethyl-3-(octyloxy)disiloxaneyl)oxy)hexyl)amino)hexyl 2-(decylthio)hexanoate |
| 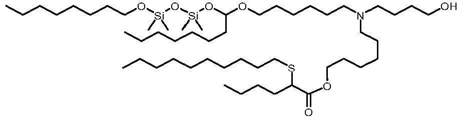 | 15-heptyl-7-(4-hydroxybutyl)-17,17,19,19-tetramethyl-14,16,18,20-tetraoxa-7-aza-17,19-disilaoctacosyl 2-(decylthio)hexanoate |
| 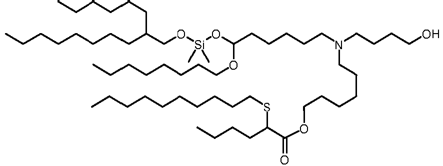 | 18-hexyl-7-(4-hydroxybutyl)-15,15-dimethyl-13-(octyloxy)-14,16-dioxa-7-aza-15-silahexacosyl 2-(decylthio)hexanoate |
| 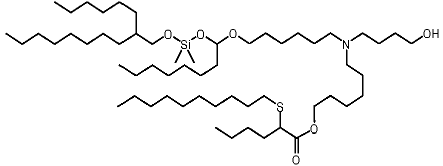 | 15-heptyl-20-hexyl-7-(4-hydroxybutyl)-17,17-dimethyl-14,16,18-trioxa-7-aza-17-silaoctacosyl 2-(decylthio)hexanoate |
| 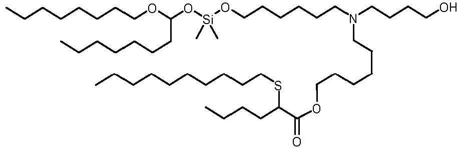 | 17-heptyl-7-(4-hydroxybutyl)-15,15-dimethyl-14,16,18-trioxa-7-aza-15-silahexacosyl 2-(decylthio)hexanoate |
| 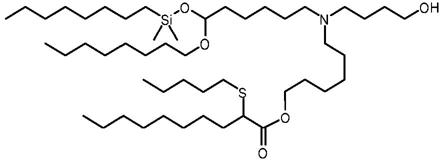 | 6-((6-((dimethyl(octyl)silyl)oxy)-6-(octyloxy)hexyl)(4-hydroxybutyl)amino)hexyl 2-(pentylthio)decanoate |
| 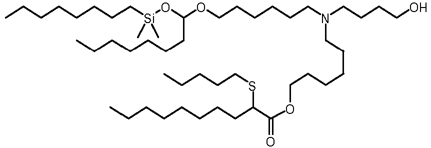 | 15-heptyl-7-(4-hydroxybutyl)-17,17-dimethyl-14,16-dioxa-7-aza-17-silapentacosyl 2-(pentylthio)decanoate |
| 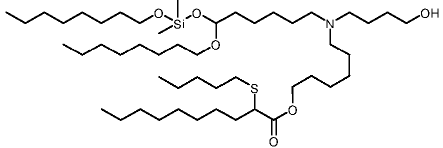 | 7-(4-hydroxybutyl)-15,15-dimethyl-13-(octyloxy)-14,16-dioxa-7-aza-15-silatetracosyl 2-(pentylthio)decanoate |
| 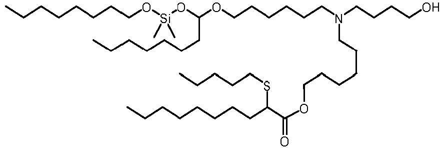 | 15-heptyl-7-(4-hydroxybutyl)-17,17-dimethyl-14,16,18-trioxa-7-aza-17-silahexacosyl 2-(pentylthio)decanoate |
| 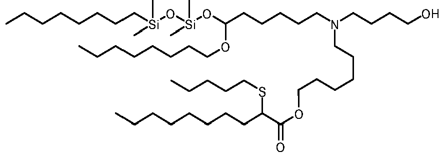 | 6-((4-hydroxybutyl)(6-(octyloxy)-6-((1,1,3,3-tetramethyl-3-octyldisiloxaneyl)oxy)hexyl)amino)hexyl 2-(pentylthio)decanoate |
| 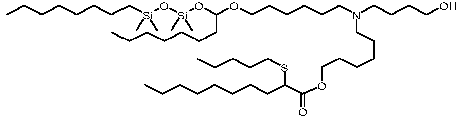 | 15-heptyl-7-(4-hydroxybutyl)-17,17,19,19-tetramethyl-14,16,18-trioxa-7-aza-17,19-disilaheptacosyl 2-(pentylthio)decanoate |
| 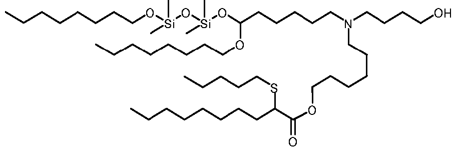 | 6-((4-hydroxybutyl)(6-(octyloxy)-6-((1,1,3,3-tetramethyl-3-(octyloxy)disiloxaneyl)oxy)hexyl)amino)hexyl 2-(pentylthio)decanoate |
| 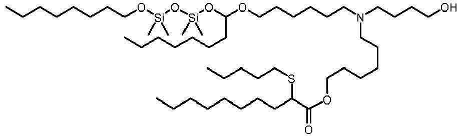 | 15-heptyl-7-(4-hydroxybutyl)-17,17,19,19-tetramethyl-14,16,18,20-tetraoxa-7-aza-17,19-disilaoctacosyl 2-(pentylthio)decanoate |
| 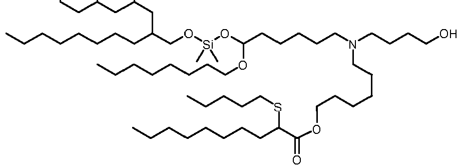 | 18-hexyl-7-(4-hydroxybutyl)-15,15-dimethyl-13-(octyloxy)-14,16-dioxa-7-aza-15-silahexacosyl 2-(pentylthio)decanoate |
| 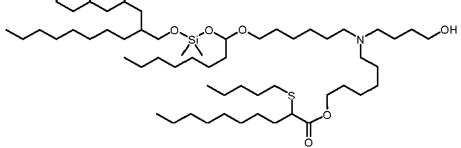 | 15-heptyl-20-hexyl-7-(4-hydroxybutyl)-17,17-dimethyl-14,16,18-trioxa-7-aza-17-silaoctacosyl 2-(pentylthio)decanoate |
| 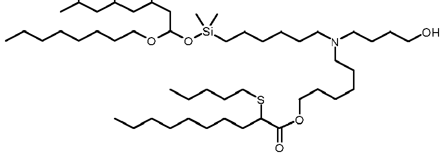 | 16-heptyl-7-(4-hydroxybutyl)-14,14-dimethyl-15,17-dioxa-7-aza-14-silapentacosyl 2-(pentylthio)decanoate |
| 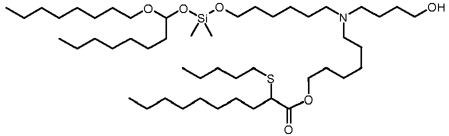 | 17-heptyl-7-(4-hydroxybutyl)-15,15-dimethyl-14,16,18-trioxa-7-aza-15-silahexacosyl 2-(pentylthio)decanoate |
| 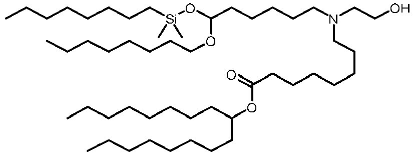 | heptadecan-9-yl 8-((6-((dimethyl(octyl)silyl)oxy)-6-(octyloxy)hexyl)(2-hydroxyethyl)amino)octanoate |
| 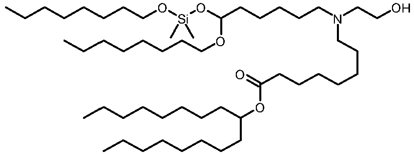 | heptadecan-9-yl 18-(2-hydroxyethyl)-10,10-dimethyl-12-(octyloxy)-9,11-dioxa-18-aza-10-silahexacosan-26-oate |
| 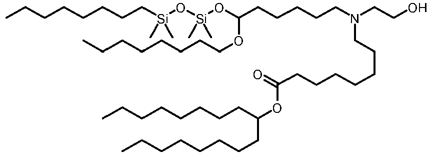 | heptadecan-9-yl 8-((2-hydroxyethyl)(6-(octyloxy)-6-((1,1,3,3-tetramethyl-3-octyldisiloxaneyl)oxy)hexyl)amino)octanoate |
| 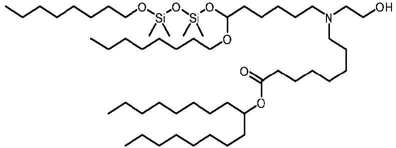 | heptadecan-9-yl 8-((2-hydroxyethyl)(6-(octyloxy)-6-((1,1,3,3-tetramethyl-3-(octyloxy)disiloxaneyl)oxy)hexyl)amino)octanoate |
| 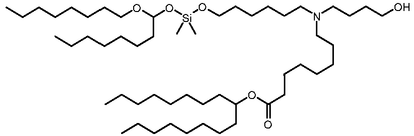 | heptadecan-9-yl 10-heptyl-20-(4-hydroxybutyl)-12,12-dimethyl-9,11,13-trioxa-20-aza-12-silaoctacosan-28-oate |
| 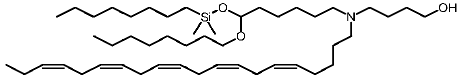 | 4-((6-((dimethyl(octyl)silyl)oxy)-6-(octyloxy)hexyl)((5Z,8Z,11Z,14Z,17Z)-icosa-5,8,11,14,17-pentaen-1-yl)amino)butan-1-ol |
| 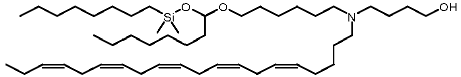 | 13-heptyl-5-((5Z,8Z,11Z,14Z,17Z)-icosa-5,8,11,14,17-pentaen-1-yl)-15,15-dimethyl-12,14-dioxa-5-aza-15-silatricosan-1-ol |
| 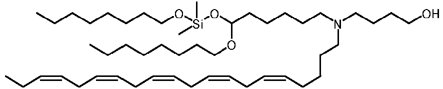 | 5-((5Z,8Z,11Z,14Z,17Z)-icosa-5,8,11,14,17-pentaen-1-yl)-13,13-dimethyl-11-(octyloxy)-12,14-dioxa-5-aza-13-siladocosan-1-ol |
| 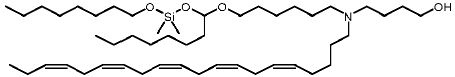 | 13-heptyl-5-((5Z,8Z,11Z,14Z,17Z)-icosa-5,8,11,14,17-pentaen-1-yl)-15,15-dimethyl-12,14,16-trioxa-5-aza-15-silatetracosan-1-ol |
| 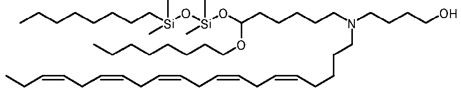 | 4-(((5Z,8Z,11Z,14Z,17Z)-icosa-5,8,11,14,17-pentaen-1-yl)(6-(octyloxy)-6-((1,1,3,3-tetramethyl-3-octyldisiloxaneyl)oxy)hexyl)amino)butan-1-ol |
| 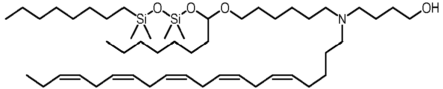 | 13-heptyl-5-((5Z,8Z,11Z,14Z,17Z)-icosa-5,8,11,14,17-pentaen-1-yl)-15,15,17,17-tetramethyl-12,14,16-trioxa-5-aza-15,17-disilapentacosan-1-ol |
| 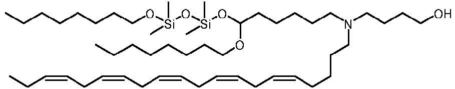 | 4-(((5Z,8Z,11Z,14Z,17Z)-icosa-5,8,11,14,17-pentaen-1-yl)(6-(octyloxy)-6-((1,1,3,3-tetramethyl-3-(octyloxy)disiloxaneyl)oxy)hexyl)amino)butan-1-ol |
| 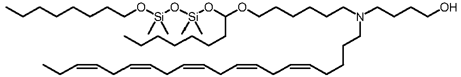 | 13-heptyl-5-((5Z,8Z,11Z,14Z,17Z)-icosa-5,8,11,14,17-pentaen-1-yl)-15,15,17,17-tetramethyl-12,14,16,18-tetraoxa-5-aza-15,17-disilahexacosan-1-ol |
| 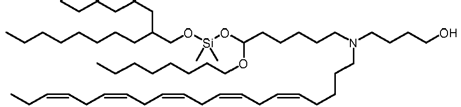 | 16-hexyl-5-((5Z,8Z,11Z,14Z,17Z)-icosa-5,8,11,14,17-pentaen-1-yl)-13,13-dimethyl-11-(octyloxy)-12,14-dioxa-5-aza-13-silatetracosan-1-ol |
| 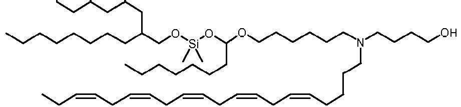 | 13-heptyl-18-hexyl-5-((5Z,8Z,11Z,14Z,17Z)-icosa-5,8,11,14,17-pentaen-1-yl)-15,15-dimethyl-12,14,16-trioxa-5-aza-15-silahexacosan-1-ol |
| 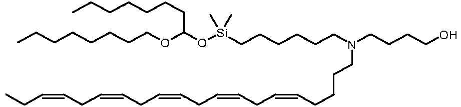 | 14-heptyl-5-((5Z,8Z,11Z,14Z,17Z)-icosa-5,8,11,14,17-pentaen-1-yl)-12,12-dimethyl-13,15-dioxa-5-aza-12-silatricosan-1-ol |
| 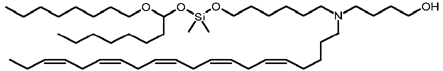 | 15-heptyl-5-((5Z,8Z,11Z,14Z,17Z)-icosa-5,8,11,14,17-pentaen-1-yl)-13,13-dimethyl-12,14,16-trioxa-5-aza-13-silatetracosan-1-ol |
| 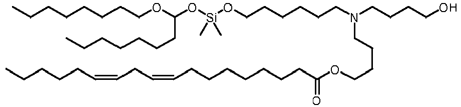 | 15-heptyl-5-(4-hydroxybutyl)-13,13-dimethyl-12,14,16-trioxa-5-aza-13-silatetracosyl (9Z,12Z)-octadeca-9,12-dienoate |
| 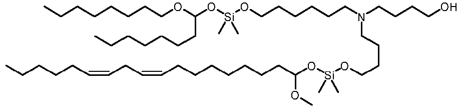 | 5-(3-((8Z,11Z)-heptadeca-8,11-dien-1-yl)-5,5-dimethyl-2,4,6-trioxa-5-siladecan-10-yl)-15-heptyl-13,13-dimethyl-12,14,16-trioxa-5-aza-13-silatetracosan-1-ol |
| 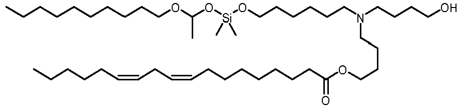 | 5-(4-hydroxybutyl)-13,13,15-trimethyl-12,14,16-trioxa-5-aza-13-silahexacosyl (9Z,12Z)-octadeca-9,12-dienoate |
| 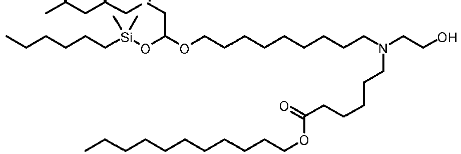 | undecyl 9-heptyl-20-(2-hydroxyethyl)-7,7-dimethyl-8,10-dioxa-20-aza-7-silahexacosan-26-oate |
| 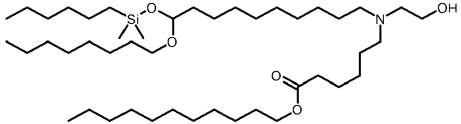 | undecyl 6-((10-((hexyldimethylsilyl)oxy)-10-(octyloxy)decyl)(2-hydroxyethyl)amino)hexanoate |
| 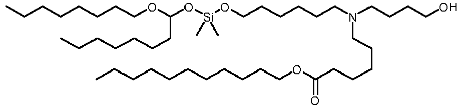 | undecyl 17-heptyl-7-(4-hydroxybutyl)-15,15-dimethyl-14,16,18-trioxa-7-aza-15-silahexacosanoate |
| 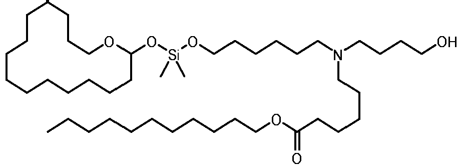 | undecyl 6-((6-((dimethyl((oxacyclohexadecan-2-yl)oxy)silyl)oxy)hexyl)(4-hydroxybutyl)amino)hexanoate |
| 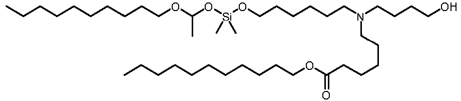 | undecyl 7-(4-hydroxybutyl)-15,15,17-trimethyl-14,16,18-trioxa-7-aza-15-silaoctacosanoate |
| 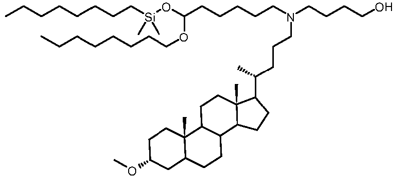 | 4-((6-((dimethyl(octyl)silyl)oxy)-6-(octyloay)heayl)((4R)-4-((3R,10S,13R)-3-methoxy-10,13-dimethylhexadecahydro-1H-cyclopenta[a]phenanthren-17-yl)pentyl)amino)butan-1-ol |
| 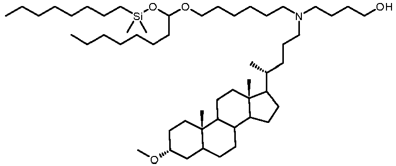 | 13-heptyl-5-((4R)-4-((3R,10S,13R)-3-methoxy-10,13-dimethylhexadecahydro-1H-cyclopenta[a]phenanthren-17-yl)pentyl)-15,15-dimethyl-12,14-dioxa-5-aza-15-silatricosan-1-ol |
| 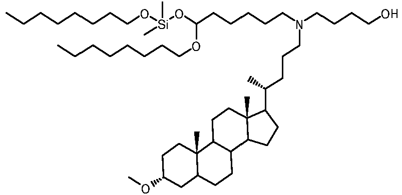 | 5-((4R)-4-((3R,10S,13R)-3-methoxy-10,13-dimethylhexadecahydro-1H-cyclopenta[a]phenanthren-17-yl)pentyl)-13,13-dimethyl-11-(octyloxy)-12,14-dioxa-5-aza-13-siladocosan-1-ol |
| 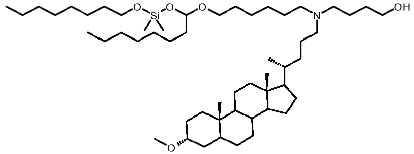 | 13-heptyl-5-((4R)-4-((3R,10S,13R)-3-methoxy-10,13-dimethylhexadecahydro-1H-cyclopenta[a]phenanthren-17-yl)pentyl)-15,15-dimethyl-12,14,16-trioxa-5-aza-15-silatetracosan-1-ol |
| 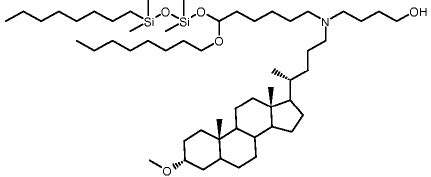 | 4-(((4R)-4-((3R,10S,13R)-3-methoxy-10,13-dimethylhexadecahydro-1H-cyclopenta[a]phenanthren-17-yl)pentyl)(6-(octyloxy)-6-((1,1,3,3-tetramethyl-3-octyldisiloxaneyl)oxy)hexyl)amino)butan-1-ol |
| 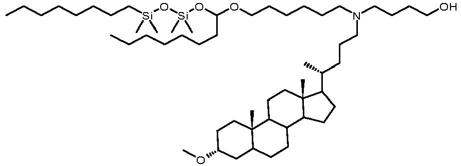 | 13-heptyl-5-((4R)-4-((3R,10S,13R)-3-methoxy-10,13-dimethylhexadecahydro-1H-cyclopenta[a]phenanthren-17-yl)pentyl)-15,15,17,17-tetramethyl-12,14,16-trioxa-5-aza-15,17-disilapentacosan-1-ol |
| 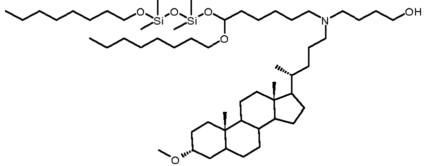 | 4-(((4R)-4-((3R,10S,13R)-3-methoxy-10,13-dimethylhexadecahydro-1H-cyclopenta[a]phenanthren-17-yl)pentyl)(6-(octyloxy)-6-((1,1,3,3-tetramethyl-3-(octyloxy)disiloxaneyl)oxy)hexyl)amino)butan-1-ol |
| 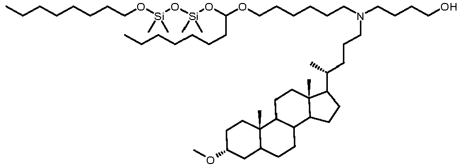 | 13-heptyl-5-((4R)-4-((3R,10S,13R)-3-methoxy-10,13-dimethylhexadecahydro-1H-cyclopenta[a]phenanthren-17-yl)pentyl)-15,15,17,17-tetramethyl-12,14,16,18-tetraoxa-5-aza-15,17-disilahexacosan-1-ol |
| 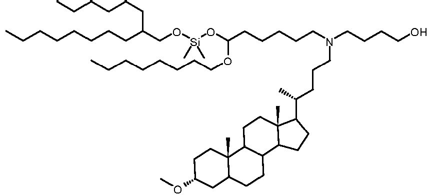 | 16-hexyl-5-((4R)-4-((3R,10S,13R)-3-methoxy-10,13-dimethylhexadecahydro-1H-cyclopenta[a]phenanthren-17-yl)pentyl)-13,13-dimethyl-11-(octyloxy)-12,14-dioxa-5-aza-13-silatetracosan-1-ol |
| 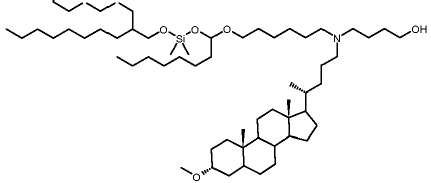 | 13-heptyl-18-hexyl-5-((4R)-4-((3R,10S,13R)-3-methoxy-10,13-dimethylhexadecahydro-1H-cyclopenta[a]phenanthren-17-yl)pentyl)-15,15-dimethyl-12,14,16-trioxa-5-aza-15-silahexacosan-1-ol |
| 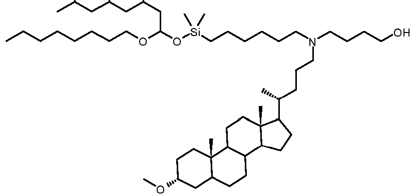 | 14-heptyl-5-((4R)-4-((3R,10S,13R)-3-methoxy-10,13-dimethylhexadecahydro-1H-cyclopenta[a]phenanthren-17-yl)pentyl)-12,12-dimethyl-13,15-dioxa-5-aza-12-silatricosan-1-ol |
| 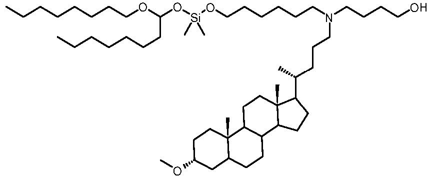 | 15-heptyl-5-((4R)-4-((3R,10S,13R)-3-methoxy-10,13-dimethylhexadecahydro-1H-cyclopenta[a]phenanthren-17-yl)pentyl)-13,13-dimethyl-12,14,16-trioxa-5-aza-13-silatetracosan-1-ol |
| 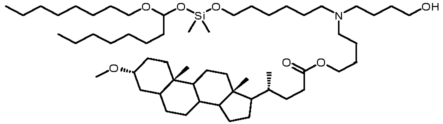 | 15-heptyl-5-(4-hydroxybutyl)-13,13-dimethyl-12,14,16-trioxa-5-aza-13-silatetracosyl (4R)-4-((3R,10S,13R)-3-methoxy-10,13-dimethylhexadecahydro-1H-cyclopenta[a]phenanthren-17-yl)pentanoate |
| 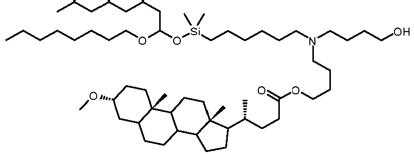 | 14-heptyl-5-(4-hydroxybutyl)-12,12-dimethyl-13,15-dioxa-5-aza-12-silatricosyl (4R)-4-((3R,10S,13R)-3-methoxy-10,13-dimethylhexadecahydro-1H-cyclopenta[a]phenanthren-17-yl)pentanoate |

21. Compound having any of the following structures (Ia), (Ib), (Ic), 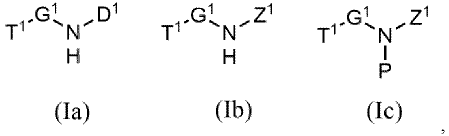 wherein D¹, G¹, T¹ and P are as defined in claim 1 and
Z¹ is 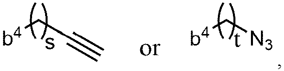
wherein b⁴ is a bond to N
s is an integer from 1 to 6
t is an integer from 2 to 6.

22. A compound having the following structure (II)) 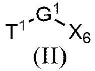 wherein G¹ is as defined in claim 1;
T¹ is 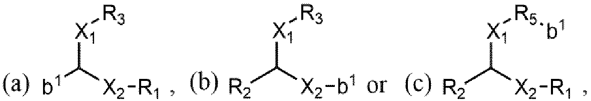 wherein b¹, X₁ and X₂, R₁, R₂, R₃, and R₅ are as defined in claim 1; and
X₆ is selected from Cl, Br, I, OMs, OTs, OTf.

23. A compound of claim 22, which has any of the following structures:
| **Structure** | **IUPAC name** |
|---|---|
| 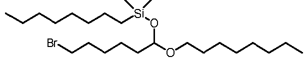 | ((6-bromo-1(octyloxy)hexyl)oxy)dimethyl(octyl)silane |
| 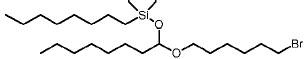 | ((1-((6-bromohexyl)oxy)octyl)oxy)dimethyl(octyl)silane |
| 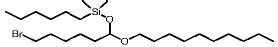 | ((6-bromo-1-(decyloxy)hexyl)oxy)(hexyl)dimethylsilane |
| 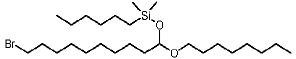 | ((10-bromo-1-(octyloxy)decyl)oxy)(hexyl)dimethylsilane |
| 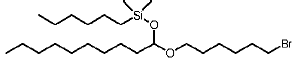 | ((1-((6-bromohexyl)oxy)decyl)oxy)(hexyl)dimethylsilane |
| 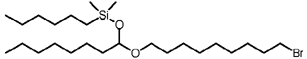 | ((1-((9-bromononyl)oxy)octyl)oxy)(hexyl)dimethylsilane |
| 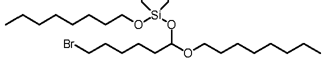 | 12-(5-bromopentyl)-10,10-dimethyl-9,11,13-trioxa-10-silahenicosane |
| 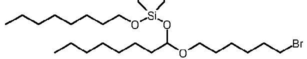 | 1-bromo-8-heptyl-10,10-dimethyl-7,9,11-trioxa-10-silanonadecane |
| 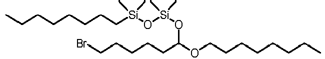 | 1-((6-bromo-1-(octyloxy)hexyl)oxy)-1,1,3,3-tetramethyl-3-octyldisiloxane |
| 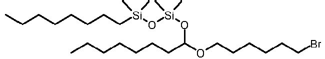 | 1-((1-((6-bromohexyl)oxy)octyl)oxy)-1,1,3,3-tetramethyl-3-octyldisiloxane |
| 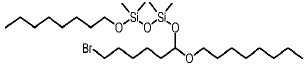 | 1-((6-bromo-1-(octyloxy)hexyl)oxy)-1,1,3,3-tetramethyl-3-(octyloxy)disiloxane |
| 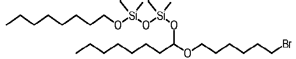 | 1-((1-((6-bromohexyl)oxy)octyl)oxy)-1,1,3,3-tetramethyl-3-(octyloxy)disiloxan |
| 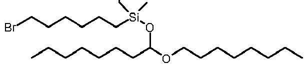 | (6-bromohexyl)dimethyl((1-(octyloxy)octyl)oxy)silane |
| 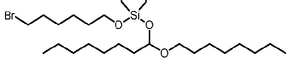 | 1-bromo-10-heptyl-8,8-dimethyl-7,9,11-trioxa-8-silanonadecane |
| 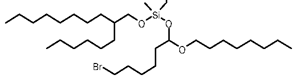 | 10-(5-bromopentyl)-15-hexyl-12,12-dimethyl-9,11,13-trioxa-12-silatricosane |
| 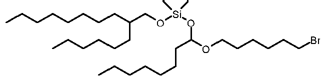 | 1-bromo-8-heptyl-13-hexyl-10,10-dimethyl-7,9,11-trioxa-10-silahenicosane |
| 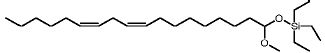 | triethyl(((9Z,12Z)-1-methoxyoctadeca-9,12-dien-1-yl)oxy)silane |
| 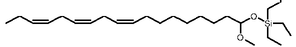 | triethyl(((9Z,12Z,15Z)-1-methoxyoctadeca-9,12,15-trien-1-yl)oxy)silane |
| 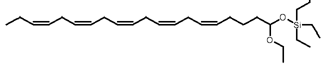 | (((5Z,8Z,11Z,14Z,17Z)-1-ethoxyicosa-5,8,11,14,17-pentaen-1-yl)oxy)triethylsilane |
| 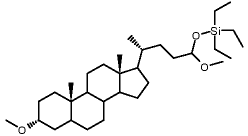 | triethyl(((4R)-1-methoxy-4-((3R,10S,13R)-3-methoxy-10,13-dimethylhexadecahydro-1H-cyclopenta[a]phenanthren-17-yl)pentyl)oxy)silane |
| 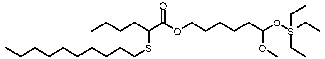 | 6-methoxy-6-((triethylsilyl)oxy)hexyl 2-(decylthio)hexanoate |
| 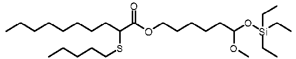 | 6-methoxy-6-((triethylsilyl)oxy)hexyl 2-(pentylthio)decanoate |
| 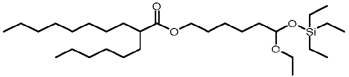 | 6-ethoxy-6-((triethylsilyl)oxy)hexyl 2-hexyldecanoate |
| 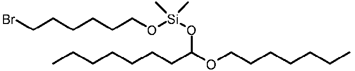 | 1-bromo-10-heptyl-8,8-dimethyl-7,9,11-trioxa-8-silaoctadecane |
| 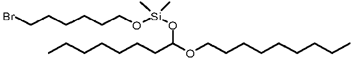 | 1-bromo-10-heptyl-8,8-dimethyl-7,9,11-trioxa-8-silaicosane |
| 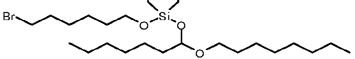 | 1-bromo-10-hexyl-8,8-dimethyl-7,9,11-trioxa-8-silanonadecane |
| 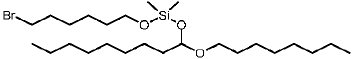 | 1-bromo-8,8-dimethyl-10-octyl-7,9,11-trioxa-8-silanonadecane |
| 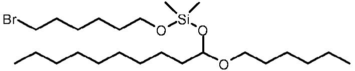 | 1-bromo-8,8-dimethyl-10-nonyl-7,9,11-trioxa-8-silaheptadecane |
| 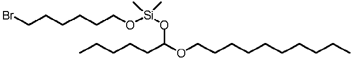 | 1-bromo-8,8-dimethyl-10-pentyl-7,9,11-trioxa-8-silahenicosane |
| 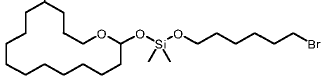 | ((6-bromohexyl)oxy)dimethyl((oxacyclohexadecan-2-yl)oxy)silane |
| 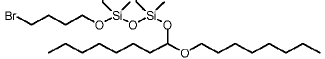 | 1-(4-bromobutoxy)-1,1,3,3-tetramethyl-3-((1-(octyloxy)octyl)oxy)disiloxane |
| 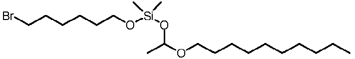 | 1-bromo-8,8,10-trimethyl-7,9,11-trioxa-8-silahenicosane |
| 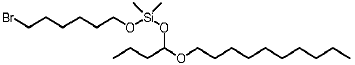 | 1-bromo-8,8-dimethyl-10-propyl-7,9,11-trioxa-8-silahenicosane |
| 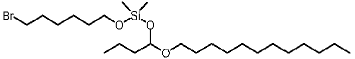 | 1-bromo-8,8-dimethyl-10-propyl-7,9,11-trioxa-8-silatricosane |
| 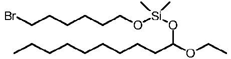 | 13-bromo-6,6-dimethyl-4-nonyl-3,5,7-trioxa-6-silatridecane |
| 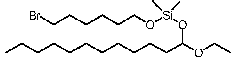 | 13-bromo-6,6-dimethyl-4-undecyl-3,5,7-trioxa-6-silatridecane |
| 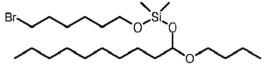 | 15-bromo-8,8-dimethyl-6-nonyl-5,7,9-trioxa-8-silapentadecane |
| 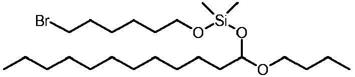 | 15-bromo-8,8-dimethyl-6-undecyl-5,7,9-trioxa-8-silapentadecane |
| 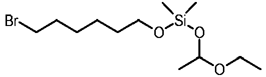 | 13-bromo-4,6,6-trimethyl-3,5,7-trioxa-6-silatridecane |
| 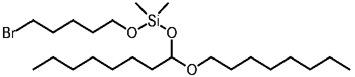 | 1-bromo-9-heptyl-7,7-dimethyl-6,8,10-trioxa-7-silaoctadecane |
| 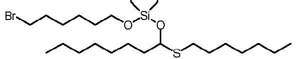 | 1-bromo-10-heptyl-8,8-dimethyl-7,9-dioxa-11-thia-8-silaoctadecane |
| 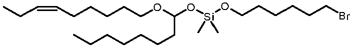 | (Z)-1-bromo-10-heptyl-8,8-dimethyl-7,9,11-trioxa-8-silaicos-17-ene |
| 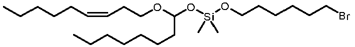 | (Z)-1-bromo-10-heptyl-8,8-dimethyl-7,9,11-trioxa-8-silaicos-14-ene |
| 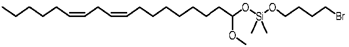 | 10-bromo-3-((8Z,11Z)-heptadeca-8,11-dien-1-yl)-5,5-dimethyl-2,4,6-trioxa-5-siladecane |

24. A lipid nanoparticle comprising a therapeutic agent and a compound according to any of claims 1 to 20, particularly a compound of claim 8, 9, 10, 11, 12 or 20.

## Patentansprüche

1. Verbindung mit der Struktur der Formel (I) 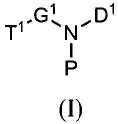
oder deren Salz oder Stereoisomer,
wobei:
G¹ ein unsubstituiertes C₂-C₉-Alkylen ist,
- (CH₂)ₓ-CH=CH-(CH₂)_{y}- ist, wobei x eine ganze Zahl von 1 bis 6 ist, y eine ganze Zahl von 1 bis 6 ist und die Summe von x+y eine ganze Zahl von 2 bis 7 ist, oder
- (CH₂)_{w}-X-(CH₂)_{z}- ist, wobei w eine ganze Zahl von 1 bis 7 ist, z eine ganze Zahl von 2 bis 7 ist, die Summe von w+z eine ganze Zahl von 3 bis 8 ist, wobei -(CH₂)_{z}- an N gebunden ist und X aus O, S, SO und SO₂ gewählt ist;
T¹ 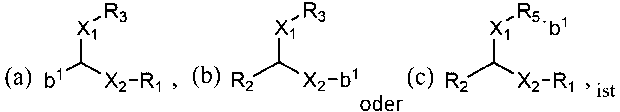 wobei
b¹ eine Bindung an G¹ ist,
X₁ und X₂ gleich oder verschieden sind und jeweils unabhängig voneinander O oder S darstellen, R₁ geradkettiges C₁-C₁₇-Alkyl, verzweigtes C₃-C₁₇-Alkyl, in beiden Fällen gegebenenfalls ein S, SO, SO₂, O oder Si(R^{a}₂) enthaltend, wobei R^{a} C₁-C₆-Alkyl ist, an einer beliebigen Position in der Kohlenstoffkette, mit der Maßgabe, dass sich das Heteroatom nicht in der Alpha- oder Omega-Position der Kohlenstoffkette befindet, C₃-C₁₇-Alkenyl, das eine Doppelbindung enthält, mit der Maßgabe, dass sich mindestens eine -CH₂- Gruppe zwischen der Doppelbindung und X₂ befindet, oder mehrfach ungesättigtes C₈-C₂₀-Alkenyl, mit der Maßgabe, dass sich mindestens eine -CH₂- Gruppe zwischen der ersten Doppelbindung und X₂ befindet, ist oder
R₁ 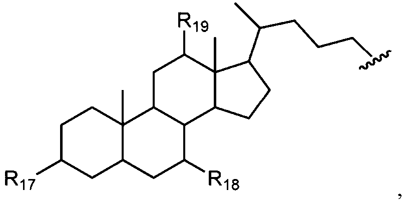 ist
wobei R₁₇, R₁₈ und R₁₉ gleich oder verschieden sind und jeweils unabhängig voneinander H, OH, C₁-C₆-Alkoxy oder Fluor sein können;
R₂ geradkettiges C₁-C₁₇-Alkyl, verzweigtes C₃-C₁₇-Alkyl, in beiden Fällen gegebenenfalls ein S, SO, SO₂, O oder Si(R^{b})₂ enthaltend, wobei R^{b} C₁-C₆-Alkyl ist, an einer beliebigen Position in der Kohlenstoffkette, mit der Maßgabe, dass sich das Heteroatom nicht in der Alpha- oder Omega-Position der Kohlenstoffkette befindet, C₃-C₁₇-Alkenyl, das eine Doppelbindung enthält, mit der Maßgabe, dass sich mindestens eine -CH₂- Gruppe zwischen der Doppelbindung und dem Kohlenstoffatom, das X₁ und X₂ verbindet, befindet oder mehrfach ungesättigtes C₈-C₂₀-Alkenyl, mit der Maßgabe, dass sich mindestens eine -CH₂- Gruppe zwischen der ersten Doppelbindung und dem Kohlenstoffatom, das X₁ und X₂ verbindet, befindet, ist oder
R₂ 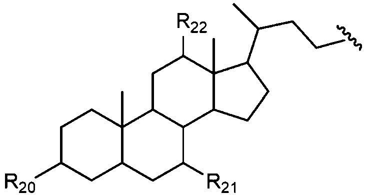 ist,
wobei R₂₀, R₂₁ und R₂₂ gleich oder verschieden sind und jeweils unabhängig voneinander H, OH, -C₁-C₆-Alkoxy oder Fluor sein können,
mit der Maßgabe, dass R₁ und R₂ nicht gleichzeitig 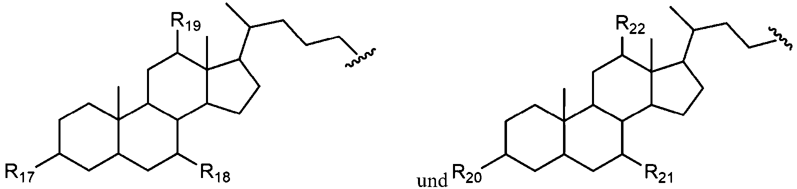 sein können, oder
R₁ über Alkylen oder einfach ungesättigtes Alkenyl an R₃ gebunden ist und einen C₅-C₃₀-gliedrigen Ring bildet, oder
R₂ über Alkylen oder einfach ungesättigtes Alkenyl mit R₃ gebunden ist und einen C₅-C₃₀-gliedrigen Ring bildet, wobei das Kohlenstoffatom von R₂ an der ersten oder zweiten Position, ,gezählt von jenem Kohlenstoffatom, das an das X₁ und X₂ verbindende Kohlenstoffatom gebunden ist, gegebenenfalls durch ein O- oder S-Heteroatom ersetzt sein kann, oder
R₂ über Alkylen oder einfach ungesättigtes Alkenyl an R₁ gebunden ist und einen C₃-C₃₀-Ring bildet, wobei das Kohlenstoffatom von R₂ an der ersten oder zweiten Position, gezählt von dem Kohlenstoffatom, das an das X₁ und X₂ verbindende Kohlenstoffatom gebunden ist, gegebenenfalls durch ein O- oder S-Heteroatom ersetzt sein kann;
R₃ 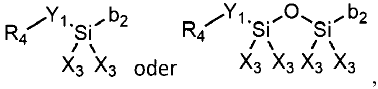 ist
R₅-b¹ 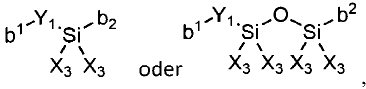 ist
wobei
b² eine Bindung zu X₁ ist,
Y₁ -O- oder -CH₂- oder -O-CH₂-CH₂- ist, wobei -CH₂- an Si gebunden ist, jedes X₃ unabhängig aus der Gruppe, bestehend aus C₁-C₄-Alkyl oder C₁-C₄-Alkoxy, ausgewählt ist,
R₄ geradkettiges C₂-C₁₇-Alkyl oder verzweigtes C₃-C₁₇-Alkyl, in beiden Fällen gegebenenfalls ein S, SO, SO₂, O oder Si(R^{c})₂ enthaltend, wobei R^{c} C₁-C₆-Alkyl an einer beliebigen Position in der Kohlenstoffkette ist, mit der Maßgabe, dass sich das Heteroatom nicht in der Alpha- oder Omega-Position der Kohlenstoffkette befindet, C₃-C₂₀-Alkenyl, das eine Doppelbindung enthält, mit der Maßgabe, dass sich mindestens eine -CH₂- Gruppe zwischen der Doppelbindung und Y₁ befindet, oder mehrfach ungesättigtes C₈-C₂₀-Alkenyl, mit der Maßgabe, dass sich mindestens eine -CH₂-Gruppe zwischen der ersten Doppelbindung und Y₁ befindet, ist oder
R⁴ 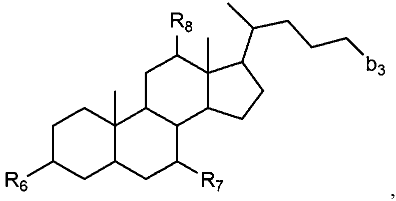
ist wobei b³ eine Bindung an Y₁ ist,
R₆, R₇ und R₈ gleich oder verschieden sind und jeweils unabhängig voneinander H, OH, -C₁-C₆-Alkoxy oder Fluor sein können;
D¹ ausgewählt aus der Gruppe bestehend aus 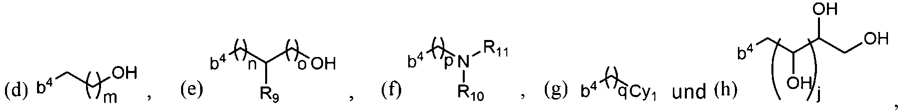 ist
wobei
b⁴ eine Bindung an Stickstoff ist,
R₉ C₁-C₆-Alkyl, Cyclopentyl, Cyclohexyl, Hydroxyl, Hydroxymethyl, Hydroxyethyl, Phenyl, Benzyl, 4-Hydroxybenzyl, 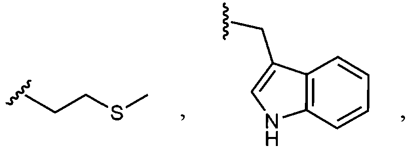 sein kann,
R₁₀ und R₁₁ unabhängig voneinander aus H und C₁-C₆-Alkyl ausgewählt sind,
m eine ganze Zahl von 1 bis 6 ist,
n eine ganze Zahl von 0 bis 6 ist,
o eine ganze Zahl von 0 bis 6 ist,
p eine ganze Zahl von 2 bis 6 ist,
q eine ganze Zahl von 0 bis 6 ist,
j eine ganze Zahl von 1 bis 4 ist,
Cy₁ ein C₃-C₆-Cycloalkyl, das gegebenenfalls durch ein oder mehrere -OH Gruppen substituiert ist, oder
Cy₁ ein Pyranose- oder Furanosering ist, der über eine seiner -OH Gruppen verknüpft sein kann, wobei der Pyranose- oder Furanosering gegebenenfalls durch eine -NH-CO-CH₃-Gruppe, Adenin, Guanin, Uracil, Cytosin, Thymin, ein Mono- oder Oligosaccharid oder durch einen 4-, 5-, 6- oder 7-gliedrigen heterocyclischen Ring, der 1, 2, 3 oder 4 Heteroatome enthält, ausgewählt aus N, O oder S, substituiert sein kann; oder
Cy₁ ein 4-, 5-, 6- oder 7-gliedriger heterocyclischer Ring ist, der 1, 2, 3 oder 4 Heteroatome enthält, ausgewählt aus O, N oder S, wobei der heterocyclische Ring gegebenenfalls durch R₁₂ substituiert ist,
wobei
R₁₂ C₁-C₆-Alkyl, ein Arginin enthaltendes Peptid, einen Pyranose- oder Furanosering, der über eines seiner Ring-Kohlenstoffatome an den 4-, 5-, 6- oder 7-gliedrigen heterocyclischen Ring gebunden ist, wobei der Pyranose- oder Furanosering gegebenenfalls durch eine -NH-CO-CH₃- Gruppe, Adenin, Guanin, Uracil, Cytosin, Thymin oder ein Mono- oder Oligosaccharid sustituiert ist,
R₁₂ eine Gruppe, ausgewählt aus (d), (e) oder (f), wobei b⁴ eine Bindung an den heterocyclischen Ring ist, m, n, o, p, R₉, R₁₀ und R₁₁ wie oben definiert sind,
oder
R₁₂ eine 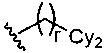 Gruppe sein kann, wobei r eine ganze Zahl von 1 bis 4 ist und Cy₂ ein 4-, 5-, 6- oder 7-gliedriger heterocyclischer Ring ist, der 1, 2, 3 oder 4 Heteroatome enthält, die aus O, N und S ausgewählt sind, und gegebenenfalls durch C₁-C₆-Alkyl substituiert ist;
P ausgewählt ist aus
(i) G²-T²
wobei
G² wie oben für G¹ definiert ist, wobei G¹ und G² identisch oder unterschiedlich sein können;
T² wie oben für T¹ definiert ist, b¹ eine Bindung an G² darstellt und wobei T¹ und T² identisch oder unterschiedlich sein können; oder
(ii) G²-T³
wobei
G² wie oben definiert ist;
T³ 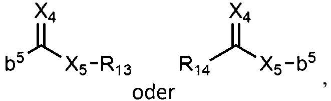
ist, wobei
b⁵ eine Bindung an G² ist,
X₄ und X₅ gleich oder verschieden sein können und jeweils unabhängig voneinander O, S oder NR^{d} sind, wobei R^{d} H, C₁-C₆-Alkyl, -OH oder C₁-C₆-Alkoxy ist,
R₁₃ geradkettiges C₂₋C₁₇-Alkyl, verzweigtes C₍₃₎₋C₁₇-Alkyl, wobei beide gegebenenfalls eine der folgenden Gruppen enthalten: S, SO, SO₂, O, Si(R^{e})₂, wobei R^{e} C₁-C₆-Alkyl an einer beliebigen Position in der Kohlenstoffkette ist, mit der Maßgabe, dass sich das Heteroatom nicht in der Alpha- oder Omega-Position der Kohlenstoffkette befindet, C₃-C₂₀-Alkenyl, das eine Doppelbindung enthält, mit der Maßgabe, dass sich mindestens eine -CH₂- Gruppe zwischen der Doppelbindung und X₅ befindet, oder mehrfach ungesättigtes C₈-C₂₀-Alkenyl, mit der Maßgabe, dass sich mindestens eine -CH₂- Gruppe zwischen der ersten Doppelbindung und X₅ befindet, oder
R₁₃ 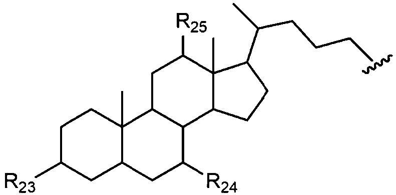 ist,
wobei R₂₃, R₂₄ und R₂₅ gleich oder verschieden sind und jeweils unabhängig voneinander H, OH, -C₁-C₆-Alkoxy oder Fluor sein können,
R₁₄ geradkettiges C₂-C₁₇-Alkyl oder verzweigtes C₃-C₁₇-Alkyl ist, wobei beide gegebenenfalls eines der folgenden Elemente enthalten: S, SO, SO₂, O, Si(R^{f} )₂, wobei R^{f} C₁-C₆-Alkyl an einer beliebigen Position in der Kohlenstoffkette ist, mit der Maßgabe, dass sich das Heteroatom nicht in der Alpha- oder Omega-Position der Kohlenstoffkette befindet, C₃-C₂₀-Alkenyl, das eine Doppelbindung enthält, mit der Maßgabe, dass sich mindestens eine -CH₂- Gruppe zwischen der Doppelbindung und dem Kohlenstoffatom befindet, das X₄ und X₅ verbindet, oder mehrfach ungesättigtes C₈-C₂₀-Alkenyl, mit der Maßgabe, dass sich mindestens eine -CH₂- Gruppe zwischen der ersten Doppelbindung und dem Kohlenstoffatom befindet, das X₄ und X₅ verbindet, oder
R₁₄ 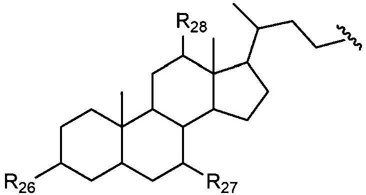 ist,
wobei R₂₆, R₂₇ und R₂₈ gleich oder verschieden sind und jeweils unabhängig voneinander H, OH, -C₁-C₆-Alkoxy oder Fluor sein können;
und (iii) T⁴, wobei
T⁴ 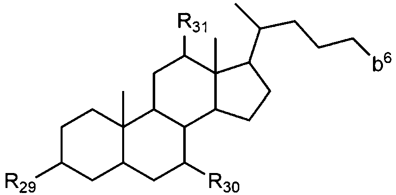 ist,
wobei b⁶ eine Bindung an das Stickstoffatom ist, R₂₉, R₃₀, R₃₁ gleich oder verschieden sein können und jeweils unabhängig voneinander H, OH, -C₁-C₆-Alkoxy oder Fluor sein können;
C₄-C₂₀-Alkenyl, das eine Doppelbindung enthält, mit der Maßgabe, dass sich mindestens eine - CH₂- Gruppe zwischen der Doppelbindung und N befindet, oder
mehrfach ungesättigtes C₈-C₂₀-Alkenyl, mit der Maßgabe, dass sich mindestens ein -CH₂-zwischen der ersten Doppelbindung und N befindet.

2. Die Verbindung nach Anspruch 1, wobei G¹ und, falls vorhanden, G² gleich oder verschieden sind und jeweils unabhängig voneinander geradkettiges C₄-C₉-Alkylen, vorzugsweise C₅-C₉-Alkylen sind, oder alternativ jeweils unabhängig voneinander geradkettiges C₅-, C₆-, C₇- oder C₉-Alkylen sind.

3. Verbindung nach Anspruch 1 oder 2, wobei R₁ geradkettiges C₁, C₂, C₄, C₆, C₇, C₈, C₉, C₁₀ oder C₁₂-Alkyl ist, oder R₁ geradkettiges C₉-Alkenyl ist, und/oder R₂ geradkettiges C₁, C₃, C₅, C₆, C₇, C₈, C₉ oder C₁₁-Alkyl ist, vorzugsweise R₁ geradkettiges C₂, C₈, C₁₀-Alkyl ist, oder R₁ geradkettiges C₉-Alkenyl ist und/oder R₂ geradkettiges C₁, C₃, C₇ oder C₉-Alkyl ist.

4. Verbindung nach Anspruch 3, wobei T¹ (c) ist und R₁ geradkettiges C₂, C₄, C₆, C₇, C₈, C₉, C₁₀ oder C₁₂-Alkyl ist, oder R₁ geradkettiges C₉-Alkenyl ist und R₂ geradkettiges C₁, C₃, C₅, C₆, C₇, C₈, C₉ oder C₁₁-Alkyl ist, vorzugsweise R₁ geradkettiges C₂, C₈, C₁₀-Alkyl ist, oder R₁ geradkettiges C₉-Alkenyl ist und R₂ geradkettiges C₁, C₃, C₇ oder C₉-Alkyl ist.

5. Verbindung nach einem der Ansprüche 1 bis 3, wobei P G²-T²ist.

6. Verbindung nach Anspruch 5, wobei G¹-T¹ und G²-T² identisch sind.

7. Verbindung nach einem der Ansprüche 1 bis 3, wobei P G²-T³ ist.

8. Verbindung nach Anspruch 7, wobei T¹-G¹ eine der folgenden Strukturen aufweist

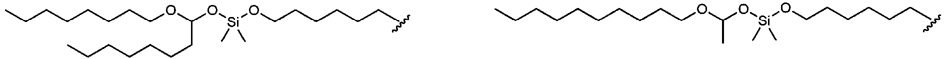

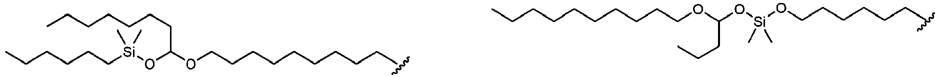

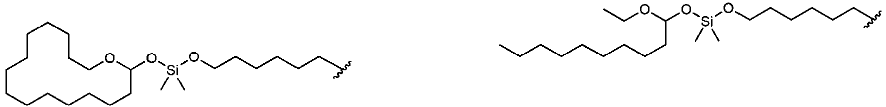

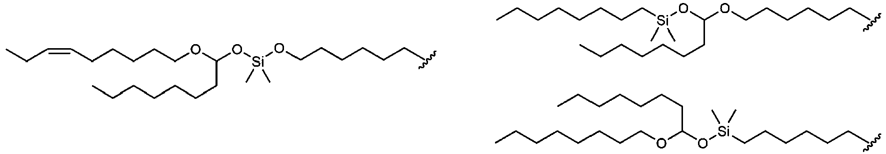

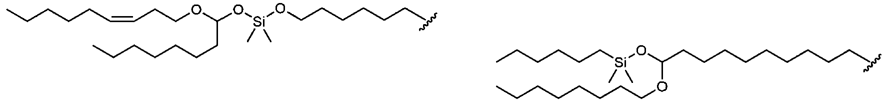

9. Verbindung nach Anspruch 7 oder 8, wobei T³ die folgende Struktur aufweist 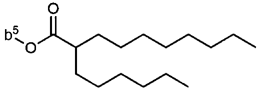 wobei b⁵ die Bindung an G² ist.

10. Verbindung nach Anspruch 7 oder 8, wobei T³ eine der folgenden Strukturen aufweist 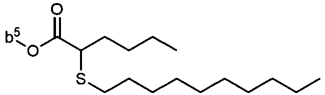 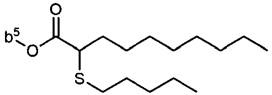 wobei b⁵ die Bindung an G² ist.

11. Verbindung nach Anspruch 7 oder 8, wobei T³eine der folgenden Strukturen aufweist 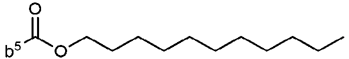 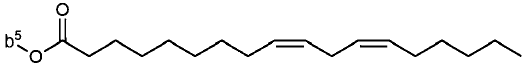 wobei b⁵ die Bindung an G² ist.

12. Verbindung nach Anspruch 7 oder 8, wobei T³ die folgende Struktur aufweist 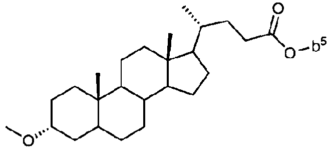 wobei b⁵ die Bindung an G² ist.

13. Verbindung nach einem der Ansprüche 1 bis 3, wobei P T⁴ist.

14. Verbindung nach Anspruch 13, wobei T⁴ 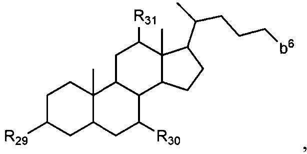 ist,
wobei b⁶ eine Bindung an das Stickstoffatom ist, R₂₉, R₃₀, R₃₁ gleich oder verschieden sein können und jeweils unabhängig voneinander H, OH, -C₁-C₆-Alkoxy oder Fluor sein können; oder
mehrfach ungesättigtes C₈ -C₂₀ -Alkenyl, mit der Maßgabe, dass sich mindestens ein -CH₂ - zwischen der ersten Doppelbindung und N befindet.

15. Verbindung nach Anspruch 14, wobei T¹ -G¹ eine der folgenden Strukturen aufweist

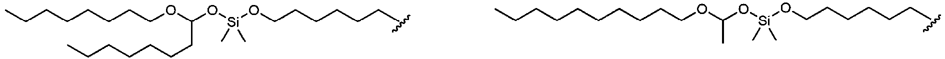

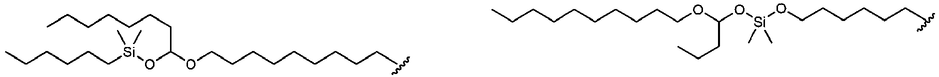

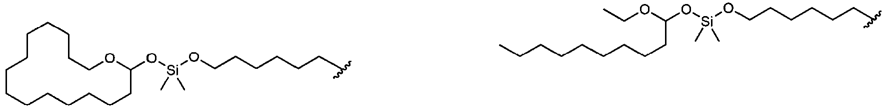

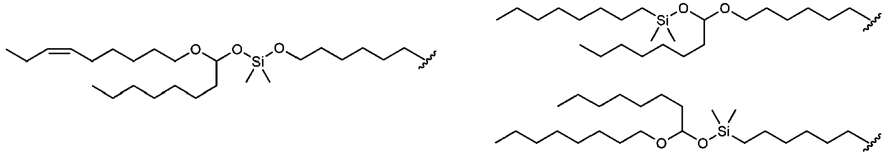

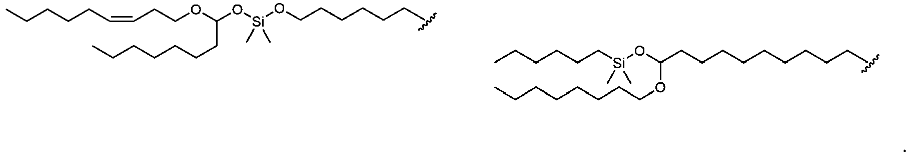

16. Verbindung nach Anspruch 14 oder 15, wobei T⁴die folgende Struktur aufweist 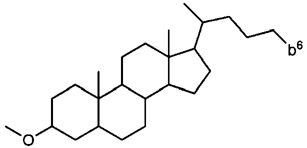 wobei b⁶ eine Bindung an das Stickstoffatom darstellt.

17. Verbindung nach einem der Ansprüche 1 bis 16, wobei D¹ eine der folgenden Strukturen aufweist:

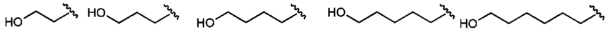

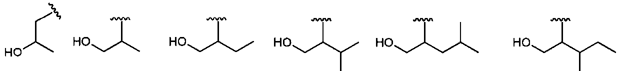

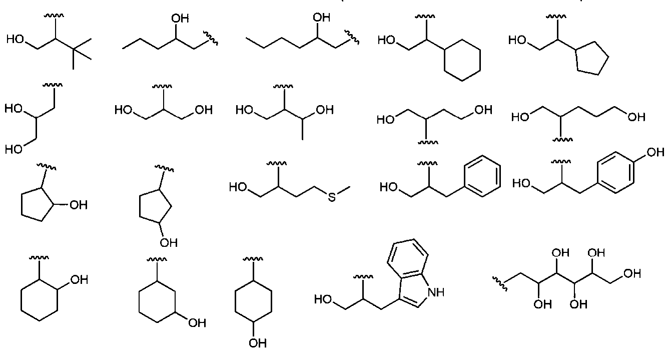

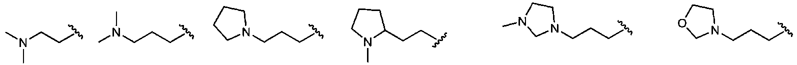

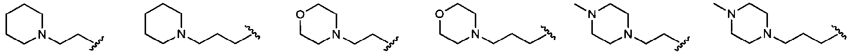

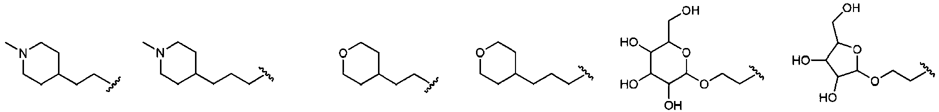

18. Verbindung nach einem der Ansprüche 1 bis 16, wobei D¹ eine der folgenden Strukturen aufweist: 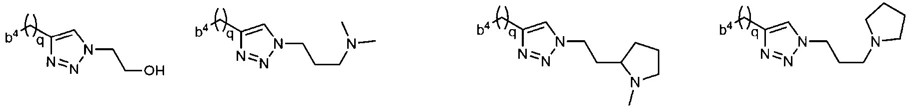 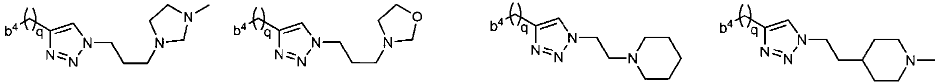 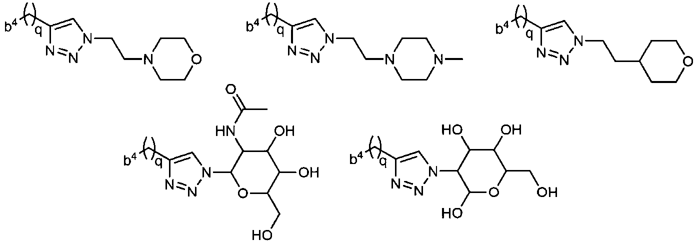 wobei q eine ganze Zahl von 1 bis 4 ist und b⁴ eine Bindung an Stickstoff darstellt.

19. Verbindung nach einem der Ansprüche 1 bis 16, wobei D¹ eine der folgenden Strukturen aufweist:

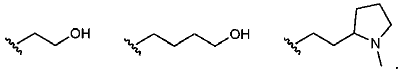

20. Verbindung nach Anspruch 1, die eine der folgenden Strukturen aufweist:
| **Struktur** | **IUPAC-Bezeichnung** |
|---|---|
| 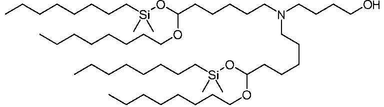 | 4-(bis(6-((Dimethyl(octyl)silyl)oxy)-6-(octyloxy)hexyl)amino)butan-1-ol |
| 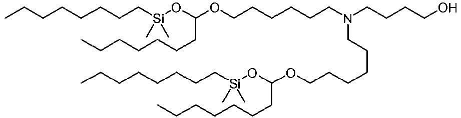 | 5-(6-((1-((Dimethyl(octyl)silyl)oxy)octyl)oxy)hexyl)-13-heptyl-15,15-dimethyl-12,14-dioxa-5-aza-15-silatricosan-1-ol |
| 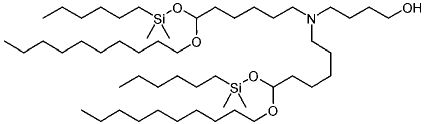 | 4-(bis(6-(Decyloxy)-6-((hexyldimethylsilyl)oxy)hexyl)amino)butan-1-ol |
| 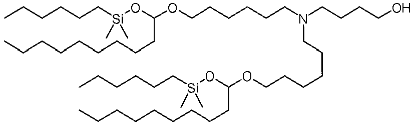 | 5-(6-((1-((Hexyldimethylsilyl)oxy)decyl)oxy)hexyl)-15,15-dimethyl-13-nonyl-12,14-dioxa-5-aza-15-silahenicosan-1-ol |
| 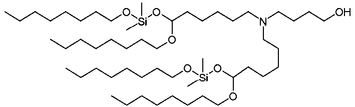 | 5-(6-((Dimethyl(octyloxy)silyl)oxy)-6-(octyloxy)hexyl)-13,13-dimethyl-11-(octyloxy)-12,14-dioxa-5-aza-13-siladocosan-1-ol |
| 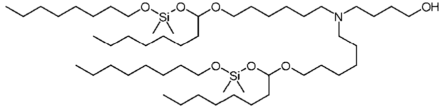 | 13-Heptyl-5-(8-heptyl-10,10-dimethyl-7,9,11-trioxa-10-silanonadecyl)-15,15-dimethyl-12,14,16-trioxa-5-aza-15-silatetracosan-1-ol |
| 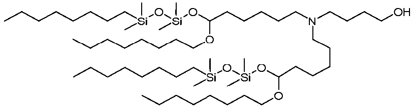 | 4-(bis(6-(Octyloxy)-6-((1,1,3,3-tetramethyl-3-octyldisiloxaneyl)oxy)hexyl)amino)butan-1-ol |
| 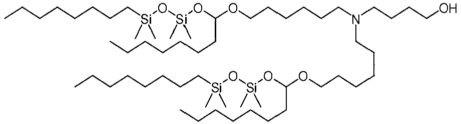 | 13-Heptyl-15,15,17,17-tetramethyl-5-(6-((1-((1,1,3,3-tetramethyl-3-octyldisiloxaneyl)oxy)octyl)oxy)hexyl)-12,14,16-trioxa-5-aza-15,17-disilapentacosan-1-ol |
| 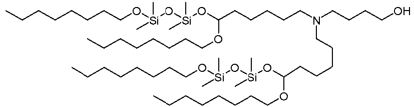 | 4-(bis(6-(Octyloxy)-6-((1,1,3,3-tetramethyl-3-(octyloxy)disiloxaneyl)oxy)hexyl)amino)butan-1-ol |
| 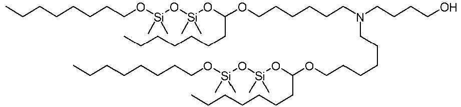 | 13-Heptyl-15,15,17,17-tetramethyl-5-(6-((1-((1,1,3,3-tetramethyl-3-(octyloxy)disiloxaneyl)oxy)octyl)oxy)hexyl)-12,14,16,18-tetraoxa-5-aza-15,17-disilahexacosan-1-ol |
| 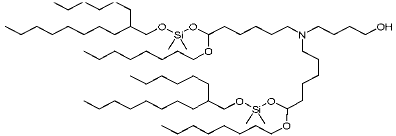 | 16-Hexyl-5-(6-((((2-hexyldecyl)oxy)dimethylsilyl)oxy)-6-(octyloxy)hexyl)-13,13-dimethyl-11-(octyloxy)-12,14-dioxa-5-aza-13-silatetracosan-1-ol |
| 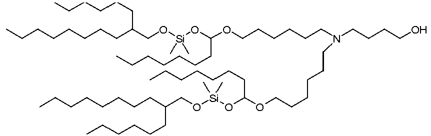 | 13-Heptyl-5-(8-heptyl-13-hexyl-10,10-dimethyl-7,9,11-trioxa-10-silahenicosyl)-18-hexyl-15,15-dimethyl-12,14,16-trioxa-5-aza-15-silahexacosan-1-ol |
| 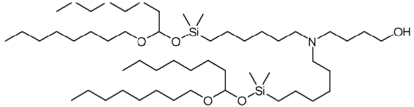 | 5-(6-(Dimethyl((1-(octyloxy)octyl)oxy)silyl)hexyl)-14-heptyl-12,12-dimethyl-13,15-dioxa-5-aza-12-silatricosan-1-ol |
| 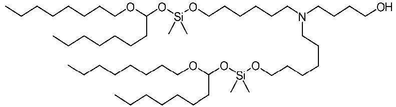 | 15-Heptyl-5-(10-heptyl-8,8-dimethyl-7,9,11-trioxa-8-silanonadecyl)-13,13-dimethyl-12,14,16-trioxa-5-aza-13-silatetracosan-1-ol |
| 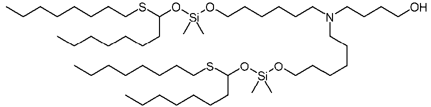 | 15-Heptyl-5-(10-heptyl-8,8-dimethyl-7,9-dioxa-11-thia-8-silanonadecyl)-13,13-dimethyl-12,14-dioxa-16-thia-5-aza-13-silatetracosan-1-ol |
| 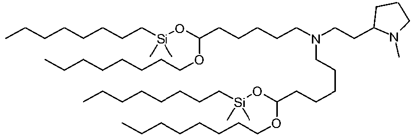 | 6-((Dimethyl(octyl)silyl)oxy)-N-(6-((dimethyl(octyl)silyl)oxy)-6-(octyloxy)hexyl)-N-(2-(1-methylpyrrolidin-2-yl)ethyl)-6-(octyloxy)hexan-1-amin |
| 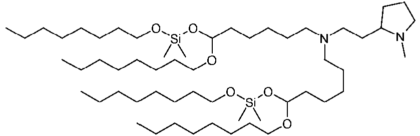 | 6-((Dimethyl(octyloxy)silyl)oxy)-N-(6-((dimethyl(octyloxy)silyl)oxy)-6-(octyloxy)hexyl)-N-(2-(1-methylpyrrolidin-2-yl)ethyl)-6-(octyloxy)hexan-1-amin |
| 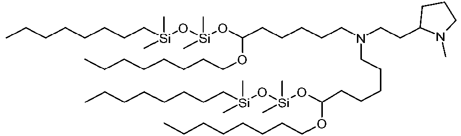 | N-(2-(1-Methylpyrrolidin-2-yl)ethyl)-6-(octyloxy)-N-(6-(octyloxy)-6-((1,1,3,3-tetramethyl-3-octyldisiloxaneyl)oxy)hexyl)-6-((1,1,3,3-tetramethyl-3-octyldisiloxaneyl)oxy)hexan-1-amin |
| 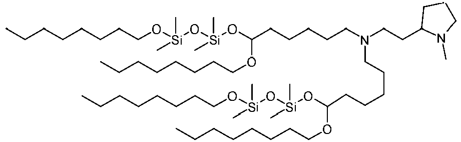 | N-(2-(1-Methylpyrrolidin-2-yl)ethyl)-6-(octyloxy)-N-(6-(octyloxy)-6-((1,1,3,3-tetramethyl-3-(octyloxy)disiloxaneyl)oxy)hexyl)-6-((1,1,3,3-tetramethyl-3-(octyloxy)disiloxaneyl)oxy)hexan-1-amin |
| 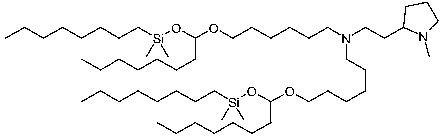 | 6-((1-((Dimethyl(octyl)silyl)oxy)octyl)oxy)-N-(6-((1-((dimethyl(octyl)silyl)oxy)octyl)oxy)hexyl)-N-(2-(1-methylpyrrolidin-2-yl)ethyl)hexan-1-amin |
| 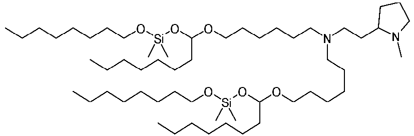 | 8-Heptyl-N-(8-heptyl-10,10-dimethyl-7,9,11-trioxa-10-silanonadecyl)-10,10-dimethyl-N-(2-(1-methylpyrrolidin-2-yl)ethyl)-7,9,11-trioxa-10-silanonadecan-1-amin |
| 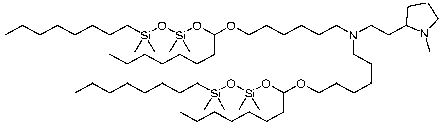 | N-(2-(1-Methylpyrrolidin-2-yl)ethyl)-6-((1-((1,1,3,3-tetramethyl-3-octyldisiloxaneyl)oxy)octyl)oxy)-N-(6-((1-((1,1,3,3-tetramethyl-3-octyldisiloxaneyl)oxy)octyl)oxy)hexyl)hexan-1-amin |
| 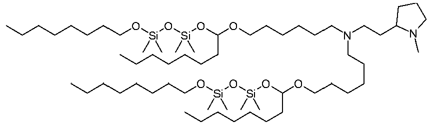 | N-(2-(1-Methylpyrrolidin-2-yl)ethyl)-6-((1-((1,1,3,3-tetramethyl-3-(octyloxy)disiloxaneyl)oxy)octyl)oxy)-N-(6-((1-((1,1,3,3-tetramethyl-3-(octyloxy)disiloxaneyl)oxy)octyl)oxy)hexyl) hexan-1-amin |
| 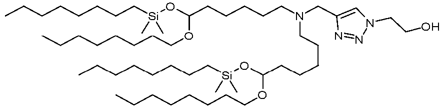 | 2-(4-((bis(6-((Dimethyl(octyl)silyl)oxy)-6-(octyloxy)hexyl)amino)methyl)-1H-1,2,3-triazol-1-yl)ethan-1-ol |
| 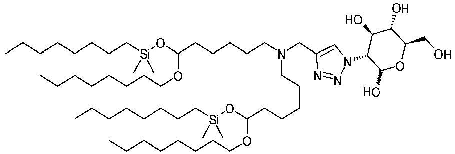 | (3R,4R,5S,6R)-3-(4-((bis(6-((Dimethyl(octyl)silyl)oxy)-6-(octyloxy)hexyl)amino)methyl)-1H-1,2,3-triazol-1-yl)-6-(hydroxymethyl)tetrahydro-2H-pyran-2,4,5-triol |
| 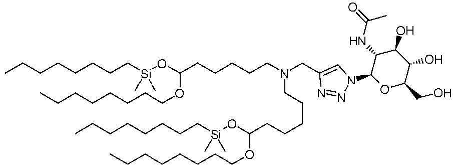 | N-((2R,3R,4R,5S,6R)-2-(4-((bis(6-((Dimethyl(octyl)silyl)oxy)-6-(octyloxy)hexyl)amino)methyl)-1H-1,2,3-triazol-1-yl)-4,5-dihydroxy-6-(hydroxymethyl)tetrahydro-2H-pyran-3-yl)acetamid |
| 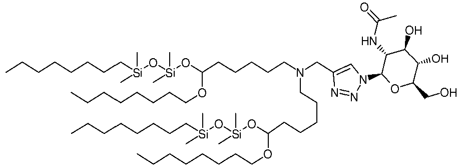 | N-((2R,3R,4R,5S,6R)-2-(4-((bis(6-(Octyloxy)-6-((1,1,3,3-tetramethyl-3-octyldisiloxaneyl)oxy)hexyl)amino)methyl)-1H-1,2,3-triazol-1-yl)-4,5-dihydroxy-6-(hydroxymethyl)tetrahydro-2H-pyran-3-yl)acetamid |
| 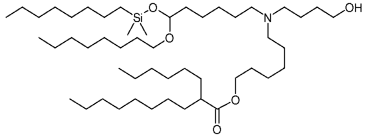 | 6-((6-((Dimethyl(octyl)silyl)oxy)-6-(octyloxy)hexyl)(4-hydroxybutyl)amino)hexyl-2-hexyldecanoat |
| 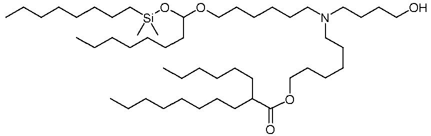 | 15-Heptyl-7-(4-hydroxybutyl)-17,17-dimethyl-14,16-dioxa-7-aza-17-silapentacosyl-2-hexyldecanoat |
| 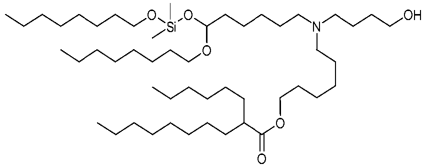 | 7-(4-Hydroxybutyl)-15,15-dimethyl-13-(octyloxy)-14,16-dioxa-7-aza-15-silatetracosyl-2-hexyldecanoat |
| 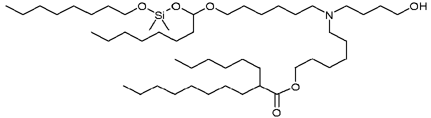 | 15-Heptyl-7-(4-hydroxybutyl)-17,17-dimethyl-14,16,18-trioxa-7-aza-17-silahexacosyl-2-hexyldecanoat |
| 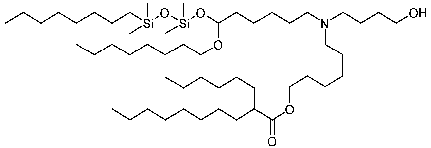 | 6-((4-Hydroxybutyl)(6-(octyloxy)-6-((1,1,3,3-tetramethyl-3-octyldisiloxaneyl)oxy)hexyl)amino)hexyl-2-hexyldecanoat |
| 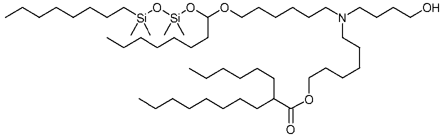 | 15-Heptyl-7-(4-hydroxybutyl)-17,17,19,19-tetramethyl-14,16,18-trioxa-7-aza-17,19-disilaheptacosyl-2-hexyldecanoat |
| 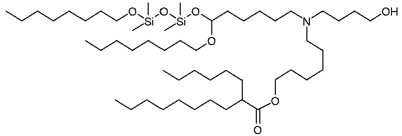 | 6-((4-Hydroxybutyl)(6-(octyloxy)-6-((1,1,3,3-tetramethyl-3-(octyloxy)disiloxaneyl)oxy)hexyl)amino)hexyl-2-hexyldecanoat |
| 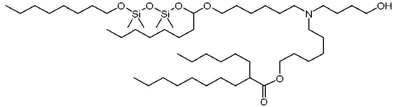 | 15-Heptyl-7-(4-hydroxybutyl)-17,17,19,19-tetramethyl-14,16,18,20-tetraoxa-7-aza-17,19-disilaoctacosyl-2-hexyldecanoat |
| 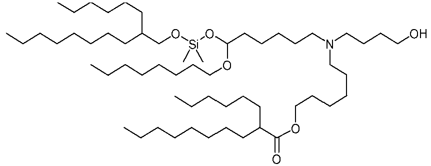 | 18-Hexyl-7-(4-hydroxybutyl)-15,15-dimethyl-13-(octyloxy)-14,16-dioxa-7-aza-15-silahexacosyl-2-hexyldecanoat |
| 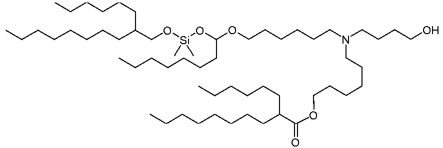 | 15-Heptyl-20-hexyl-7-(4-hydroxybutyl)-17,17-dimethyl-14,16,18-trioxa-7-aza-17-silaoctacosyl-2-hexyldecanoat |
| 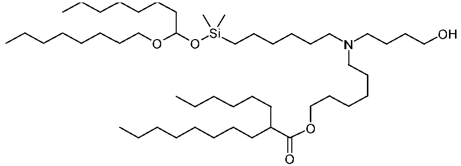 | 16-Heptyl-7-(4-hydroxybutyl)-14,14-dimethyl-15,17-dioxa-7-aza-14-silapentacosyl-2-hexyldecanoat |
| 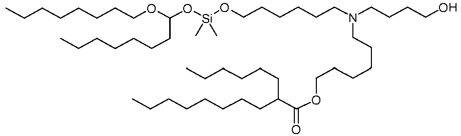 | 17-Heptyl-7-(4-hydroxybutyl)-15,15-dimethyl-14,16,18-trioxa-7-aza-15-silahexacosyl-2-hexyldecanoat |
| 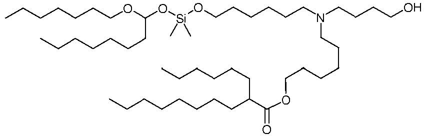 | 17-Heptyl-7-(4-hydroxybutyl)-15,15-dimethyl-14,16,18-trioxa-7-aza-15-silapentacosyl-2-hexyldecanoat |
| 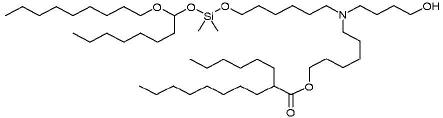 | 17-Heptyl-7-(4-hydroxybutyl)-15,15-dimethyl-14,16,18-trioxa-7-aza-15-silaheptacosyl-2-hexyldecanoat |
| 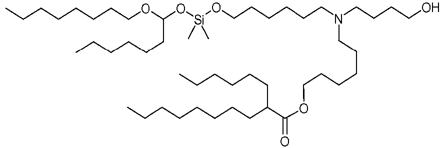 | 17-Hexyl-7-(4-hydroxybutyl)-15,15-dimethyl-14,16,18-trioxa-7-aza-15-silahexacosyl-2-hexyldecanoat |
| 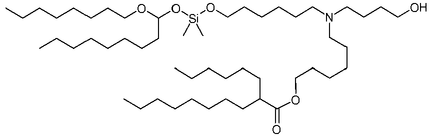 | 7-(4-Hydroxybutyl)-15,15-dimethyl-17-octyl-14,16,18-trioxa-7-aza-15-silahexacosyl-2-hexyldecanoat |
| 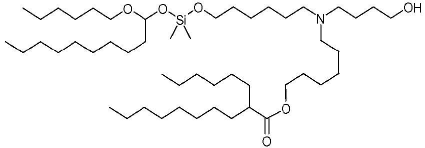 | 18-(4-Hydroxybutyl)-10,10-dimethyl-8-nonyl-7,9,11-trioxa-18-aza-10-silatetracosan-24-yl-2-hexyldecanoat |
| 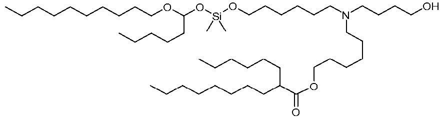 | 7-(4-Hydroxybutyl)-15,15-dimethyl-17-pentyl-14,16,18-trioxa-7-aza-15-silaoctacosyl-2-hexyldecanoat |
| 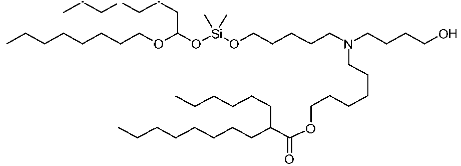 | 16-Heptyl-7-(4-hydroxybutyl)-14,14-dimethyl-13,15,17-trioxa-7-aza-14-silapentacosyl-2-hexyldecanoat |
| 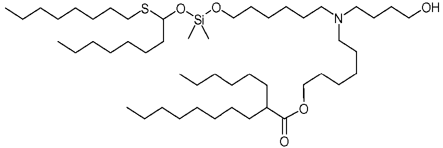 | 17-Heptyl-7-(4-hydroxybutyl)-15,15-dimethyl-14,16-dioxa-18-thia-7-aza-15-silahexacosyl-2-hexyldecanoat |
| 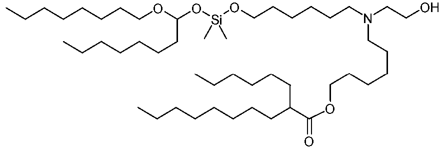 | 17-Heptyl-7-(2-hydroxyethyl)-15,15-dimethyl-14,16,18-trioxa-7-aza-15-silahexacosyl-2-hexyldecanoat |
| 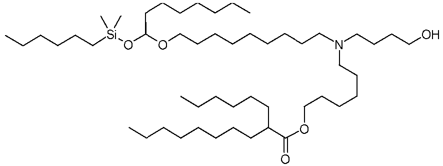 | 9-Heptyl-20-(4-hydroxybutyl)-7,7-dimethyl-8,10-dioxa-20-aza-7-silahexacosan-26-yl-2-hexyldecanoat |
| 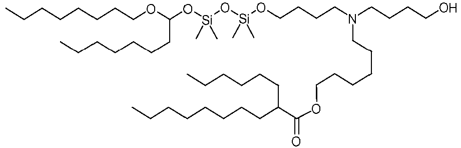 | 17-Heptyl-7-(4-hydroxybutyl)-13,13,15,15-tetramethyl-12,14,16,18-tetraoxa-7-aza-13,15-disilahexacosyl-2-hexyldecanoat |
| 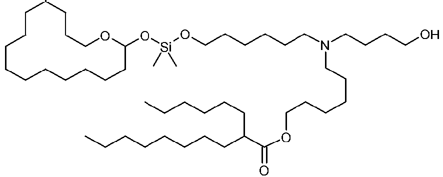 | 6-((6-((Dimethyl((oxacyclohexadecan-2-yl)oxy)silyl)oxy)hexyl)(4-hydroxybutyl)amino)hexyl-2-hexyldecanoat |
| 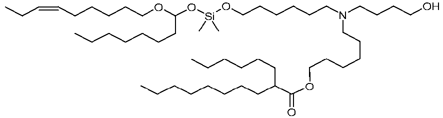 | (Z)-17-Heptyl-7-(4-hydroxybutyl)-15,15-dimethyl-14,16,18-trioxa-7-aza-15-silaheptacos-24-en-1-yl-2-hexyldecanoat |
| 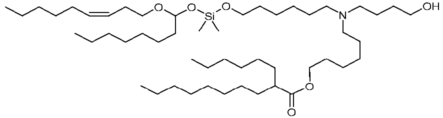 | (Z)-17-Heptyl-7-(4-hydroxybutyl)-15,15-dimethyl-14,16,18-trioxa-7-aza-15-silaheptacos-21-en-1-yl-2-hexyldecanoat |
| 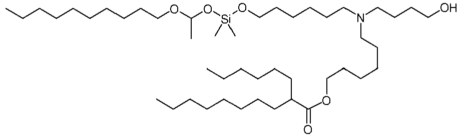 | 7-(4-Hydroxybutyl)-15,15,17-trimethyl-14,16,18-trioxa-7-aza-15-silaoctacosyl-2-hexyldecanoat |
| 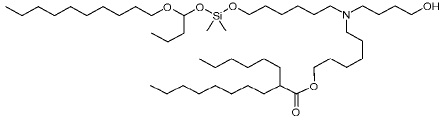 | 7-(4-Hydroxybutyl)-15,15-dimethyl-17-propyl-14,16,18-trioxa-7-aza-15-silaoctacosyl-2-hexyldecanoat |
| 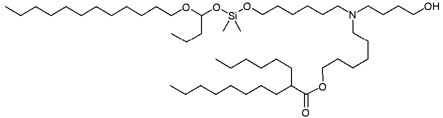 | 7-(4-Hydroxybutyl)-15,15-dimethyl-17-propyl-14,16,18-trioxa-7-aza-15-silatriacontyl-2-hexyldecanoat |
| 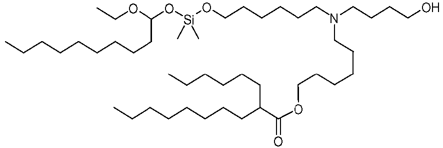 | 14-(4-Hydroxybutyl)-6,6-dimethyl-4-nonyl-3,5,7-trioxa-14-aza-6-silaicosan-20-yl-2-hexyldecanoat |
| 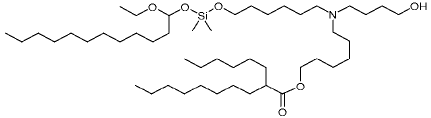 | 14-(4-Hydroxybutyl)-6,6-dimethyl-4-undecyl-3,5,7-trioxa-14-aza-6-silaicosan-20-yl-2-hexyldecanoat |
| 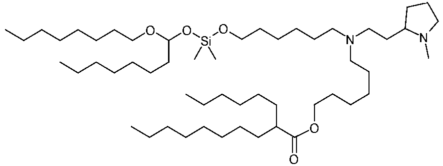 | 17-Heptyl-15,15-dimethyl-7-(2-(1-methylpyrrolidin-2-yl)ethyl)-14,16,18-trioxa-7-aza-15-silahexacosyl-2-hexyldecanoat |
| 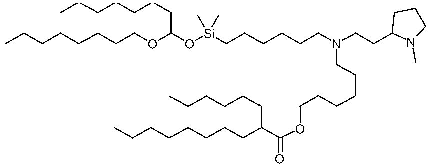 | 16-Heptyl-14,14-dimethyl-7-(2-(1-methylpyrrolidin-2-yl)ethyl)-15,17-dioxa-7-aza-14-silapentacosyl-2-hexyldecanoat |
| 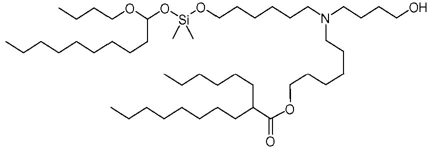 | 16-(4-Hydroxybutyl)-8,8-dimethyl-6-nonyl-5,7,9-trioxa-16-aza-8-siladocosan-22-yl-2-hexyldecanoat |
| 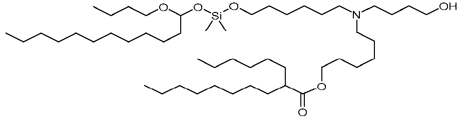 | 16-(4-Hydroxybutyl)-8,8-dimethyl-6-undecyl-5,7,9-trioxa-16-aza-8-siladocosan-22-yl-2-hexyldecanoat |
| 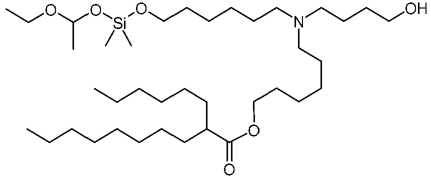 | 14-(4-Hydroxybutyl)-4,6,6-trimethyl-3,5,7-trioxa-14-aza-6-silaicosan-20-yl-2-hexyldecanoat |
| 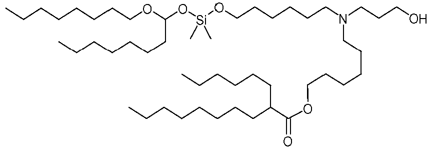 | 17-Heptyl-7-(3-hydroxypropyl)-15,15-dimethyl-14,16,18-trioxa-7-aza-15-silahexacosyl-2-hexyldecanoat |
| 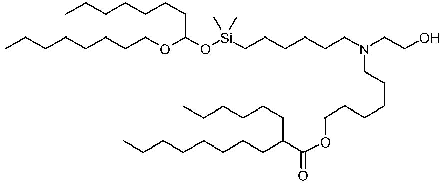 | 16-Heptyl-7-(2-hydroxyethyl)-14,14-dimethyl-15,17-dioxa-7-aza-14-silapentacosyl-2-hexyldecanoat |
| 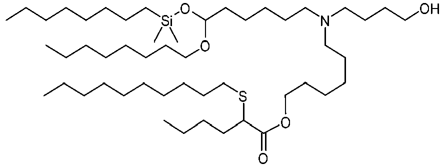 | 6-((6-((Dimethyl(octyl)silyl)oxy)-6-(octyloxy)hexyl)(4-hydroxybutyl)amino)hexyl-2-(decylthio)hexanoat |
| 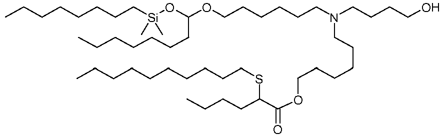 | 15-Heptyl-7-(4-hydroxybutyl)-17,17-dimethyl-14,16-dioxa-7-aza-17-silapentacosyl-2-(decylthio)hexanoat |
| 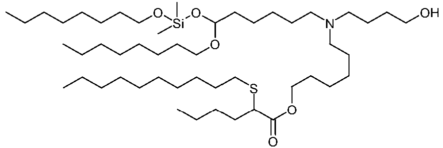 | 7-(4-Hydroxybutyl)-15,15-dimethyl-13-(octyloxy)-14,16-dioxa-7-aza-15-silatetracosyl-2-(decylthio)hexanoat |
| 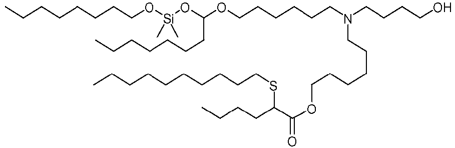 | 15-Heptyl-7-(4-hydroxybutyl)-17,17-dimethyl-14,16,18-trioxa-7-aza-17-silahexacosyl-2-(decylthio)hexanoat |
| 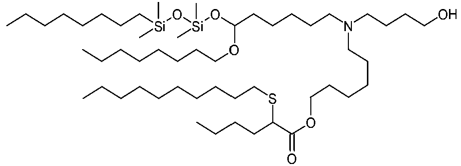 | 6-((4-Hydroxybutyl)(6-(octyloxy)-6-((1,1,3,3-tetramethyl-3-octyldisiloxaneyl)oxy)hexyl)amino)hexyl-2-(decylthio)hexanoat |
| 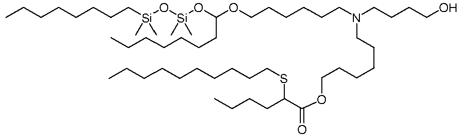 | 15-Heptyl-7-(4-hydroxybutyl)-17,17,19,19-tetramethyl-14,16,18-trioxa-7-aza-17,19-disilaheptacosyl-2-(decylthio)hexanoat |
| 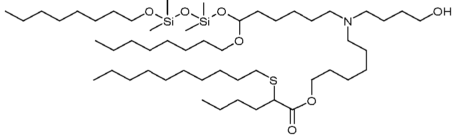 | 6-((4-Hydroxybutyl)(6-(octyloxy)-6-((1,1,3,3-tetramethyl-3-(octyloxy)disiloxaneyl)oxy)hexyl)amino)hexyl-2-(decylthio)hexanoat |
| 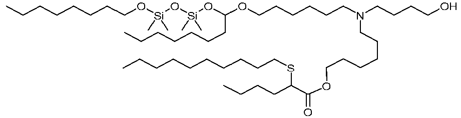 | 15-Heptyl-7-(4-hydroxybutyl)-17,17,19,19-tetramethyl-14,16,18,20-tetraoxa-7-aza-17,19-disilaoctacosyl-2-(decylthio)hexanoat |
| 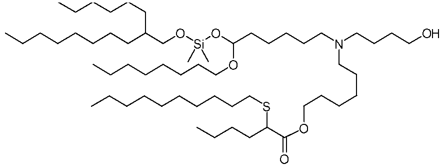 | 18-Hexyl-7-(4-hydroxybutyl)-15,15-dimethyl-13-(octyloxy)-14,16-dioxa-7-aza-15-silahexacosyl-2-(decylthio)hexanoat |
| 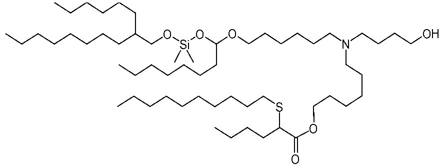 | 15-Heptyl-20-hexyl-7-(4-hydroxybutyl)-17,17-dimethyl-14,16,18-trioxa-7-aza-17-silaoctacosyl-2-(decylthio)hexanoat |
| 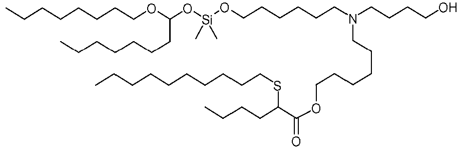 | 17-Heptyl-7-(4-hydroxybutyl)-15,15-dimethyl-14,16,18-trioxa-7-aza-15-silahexacosyl-2-(decylthio)hexanoat |
| 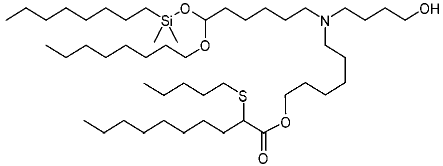 | 6-((6-((Dimethyl(octyl)silyl)oxy)-6-(octyloxy)hexyl)(4-hydroxybutyl)amino)hexyl-2-(pentylthio)decanoat |
| 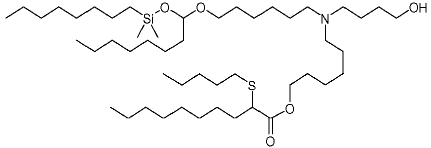 | 15-Heptyl-7-(4-hydroxybutyl)-17,17-dimethyl-14,16-dioxa-7-aza-17-silapentacosyl-2-(pentylthio)decanoat |
| 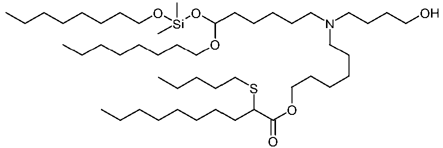 | 7-(4-Hydroxybutyl)-15,15-dimethyl-13-(octyloxy)-14,16-dioxa-7-aza-15-silatetracosyl-2-(pentylthio)decanoat |
| 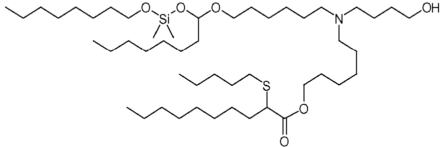 | 15-Heptyl-7-(4-hydroxybutyl)-17,17-dimethyl-14,16,18-trioxa-7-aza-17-silahexacosyl-2-(pentylthio)decanoat |
| 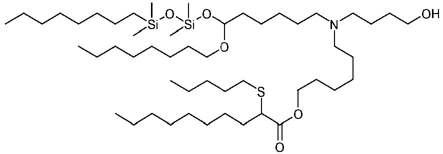 | 6-((4-Hydroxybutyl)(6-(octyloxy)-6-((1,1,3,3-tetramethyl-3-octyldisiloxaneyl)oxy)hexyl)amino)hexyl-2-(pentylthio)decanoat |
| 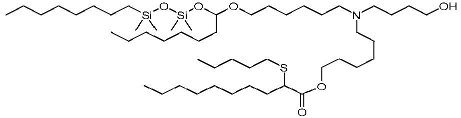 | 15-Heptyl-7-(4-hydroxybutyl)-17,17,19,19-tetramethyl-14,16,18-trioxa-7-aza-17,19-disilaheptacosyl-2-(pentylthio)decanoat |
| 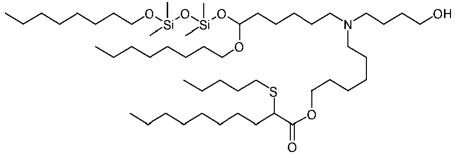 | 6-((4-Hydroxybutyl)(6-(octyloxy)-6-((1,1,3,3-tetramethyl-3-(octyloxy)disiloxaneyl)oxy)hexyl)amino)hexyl-2-(pentylthio)decanoat |
| 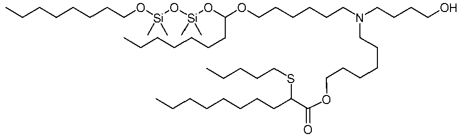 | 15-Heptyl-7-(4-hydroxybutyl)-17,17,19,19-tetramethyl-14,16,18,20-tetraoxa-7-aza-17,19-disilaoctacosyl-2-(pentylthio)decanoat |
| 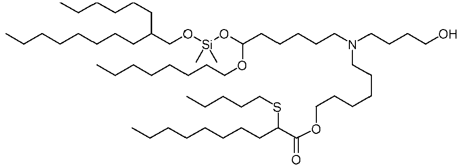 | 18-Hexyl-7-(4-hydroxybutyl)-15,15-dimethyl-13-(octyloxy)-14,16-dioxa-7-aza-15-silahexacosyl-2-(pentylthio)decanoat |
| 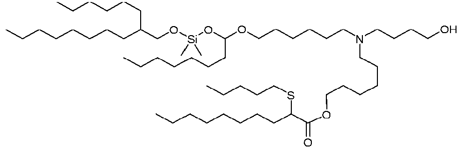 | 15-Heptyl-20-hexyl-7-(4-hydroxybutyl)-17,17-dimethyl-14,16,18-trioxa-7-aza-17-silaoctacosyl-2-(pentylthio)decanoat |
| 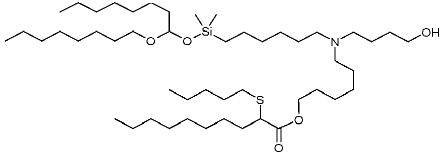 | 16-Heptyl-7-(4-hydroxybutyl)-14,14-dimethyl-15,17-dioxa-7-aza-14-silapentacosyl-2-(pentylthio)decanoat |
| 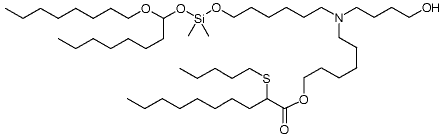 | 17-Heptyl-7-(4-hydroxybutyl)-15,15-dimethyl-14,16,18-trioxa-7-aza-15-silahexacosyl-2-(pentylthio)decanoat |
| 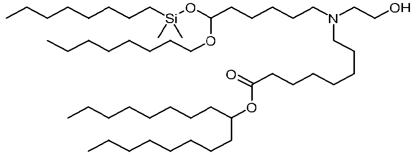 | Heptadecan-9-yl-8-((6-((dimethyl(octyl)silyl)oxy)-6-(octyloxy)hexyl)(2-hydroxyethyl)amino)octanoat |
| 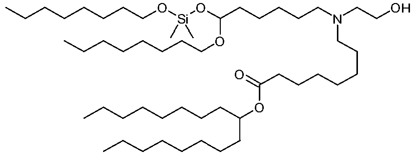 | Heptadecan-9-yl-18-(2-hydroxyethyl)-10,10-dimethyl-12-(octyloxy)-9,11-dioxa-18-aza-10-silahexacosan-26-oat |
| 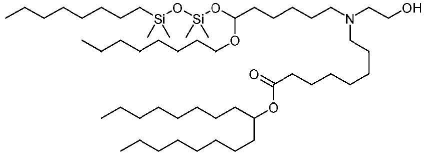 | Heptadecan-9-yl-8-((2-hydroxyethyl)(6-(octyloxy)-6-((1,1,3,3-tetramethyl-3-octyldisiloxaneyl)oxy)hexyl)amino)octanoat |
| 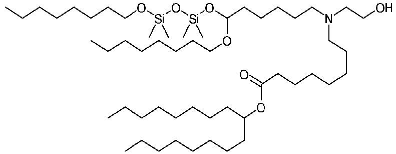 | Heptadecan-9-yl-8-((2-hydroxyethyl)(6-(octyloxy)-6-((1,1,3,3-tetramethyl-3-(octyloxy)disiloxaneyl)oxy)hexyl)amino)octanoat |
| 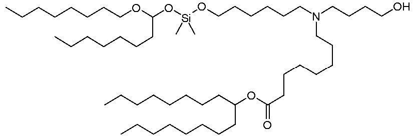 | Heptadecan-9-yl-10-heptyl-20-(4-hydroxybutyl)-12,12-dimethyl-9,11,13-trioxa-20-aza-12-silaoctacosan-28-oat |
| 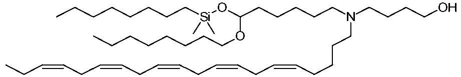 | 4-((6-((Dimethyl(octyl)silyl)oxy)-6-(octyloxy)hexyl)((5Z,8Z,11Z,14Z,17Z)-icosa-5,8,11,14,17-pentaen-1-yl)amino)butan-1-ol |
| 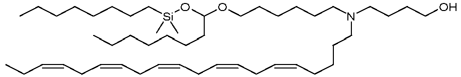 | 13-Heptyl-5-((5Z,8Z,11Z,14Z,17Z)-icosa-5,8,11,14,17-pentaen-1-yl)-15,15-dimethyl-12,14-dioxa-5-aza-15-silatricosan-1-ol |
| 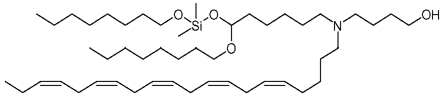 | 5-((5Z,8Z,11Z,14Z,17Z)-Icosa-5,8,11,14,17-pentaen-1-yl)-13,13-dimethyl-11-(octyloxy)-12,14-dioxa-5-aza-13-siladocosan-1-ol |
| 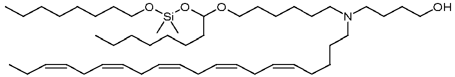 | 13-Heptyl-5-((5Z,8Z,11Z,14Z,17Z)-icosa-5,8,11,14,17-pentaen-1-yl)-15,15-dimethyl-12,14,16-trioxa-5-aza-15-silatetracosan-1-ol |
| 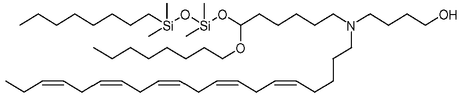 | 4-(((5Z,8Z,11Z,14Z,17Z)-Icosa-5,8,11,14,17-pentaen-1-yl)(6-(octyloxy)-6-((1,1,3,3-tetramethyl-3-octyldisiloxaneyl)oxy)hexyl)amino)butan-1-ol |
| 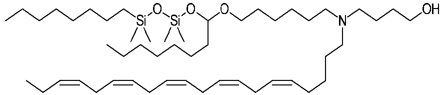 | 13-Heptyl-5-((5Z,8Z,11Z,14Z,17Z)-icosa-5,8,11,14,17-pentaen-1-yl)-15,15,17,17-tetramethyl-12,14,16-trioxa-5-aza-15,17-disilapentacosan-1-ol |
| 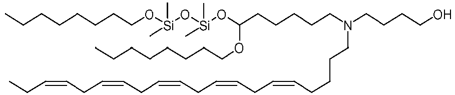 | 4-(((5Z,8Z,11Z,14Z,17Z)-Icosa-5,8,11,14,17-pentaen-1-yl)(6-(octyloxy)-6-((1,1,3,3-tetramethyl-3-(octyloxy)disiloxaneyl)oxy)hexyl)amino)butan-1-ol |
| 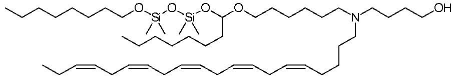 | 13-Heptyl-5-((5Z,8Z,11Z,14Z,11Z)-icosa-5,8,11,14,17-pentaen-1-yl)-15,15,17,17-tetramethyl-12,14,16,18-tetraoxa-5-aza-15,17-disilahexacosan-1-ol |
| 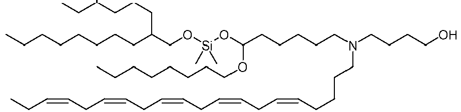 | 16-Hexyl-5-((5Z,8Z,11Z,14Z,17Z)-icosa-5,8,11,14,17-pentaen-1-yl)-13,13-dimethyl-11-(octyloxy)-12,14-dioxa-5-aza-13-silatetracosan-1-ol |
| 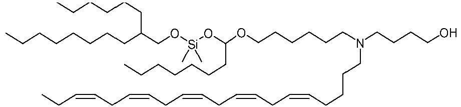 | 13-Heptyl-18-hexyl-5-((5Z,8Z,11Z,14Z,17Z)-icosa-5,8,11,14,17-pentaen-1-yl)-15,15-dimethyl-12,14,16-trioxa-5-aza-15-silahexacosan-1-ol |
| 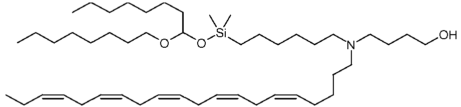 | 14-Heptyl-5-((5Z,8Z,11Z,14Z,17Z)-icosa-5,8,11,14,17-pentaen-1-yl)-12,12-dimethyl-13,15-dioxa-5-aza-12-silatricosan-1-ol |
| 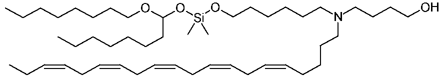 | 15-Heptyl-5-((5Z,8Z,11Z,14Z,17Z)-icosa-5,8,11,14,17-pentaen-1-yl)-13,13-dimethyl-12,14,16-trioxa-5-aza-13-silatetracosan-1-ol |
| 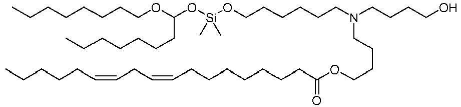 | 15-Heptyl-5-(4-hydroxybutyl)-13,13-dimethyl-12,14,16-trioxa-5-aza-13-silatetracosyl (9Z,12Z)-octadeca-9,12-dienoat |
| 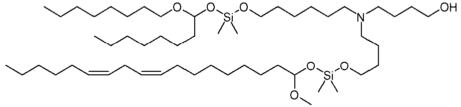 | 5-(3-((8Z,11Z)-Heptadeca-8,11-dien-1-yl)-5,5-dimethyl-2,4,6-trioxa-5-siladecan-10-yl)-15-heptyl-13,13-dimethyl-12,14,16-trioxa-5-aza-13-silatetracosan-1-ol |
| 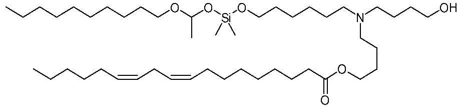 | 5-(4-Hydroxybutyl)-13,13,15-trimethyl-12,14,16-trioxa-5-aza-13-silahexacosyl (9Z,12Z)-octadeca-9,12-dienoat |
| 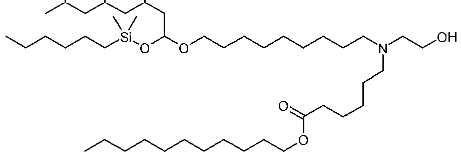 | Undecyl-9-heptyl-20-(2-hydroxyethyl)-7,7-dimethyl-8,10-dioxa-20-aza-7-silahexacosan-26-oat |
| 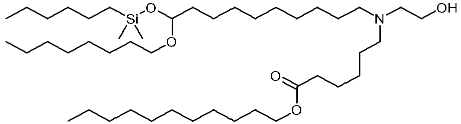 | Undecyl-6-((10-((hexyldimethylsilyl)oxy)-10-(octyloxy)decyl)(2-hydroxyethyl)amino)hexanoat |
| 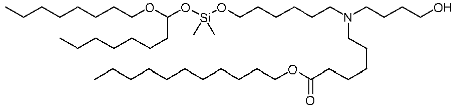 | Undecyl-17-heptyl-7-(4-hydroxybutyl)-15,15-dimethyl-14,16,18-trioxa-7-aza-15-silahexacosanoat |
| 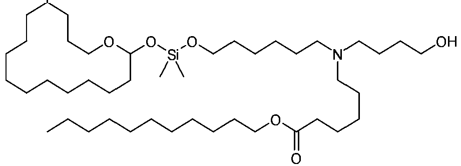 | Undecyl-6-((6-((dimethyl((oxacyclohexadecan-2-yl)oxy)silyl)oxy)hexyl)(4-hydroxybutyl)amino)hexanoat |
| 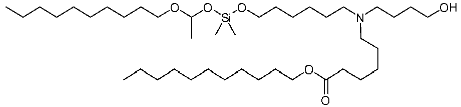 | Undecyl-7-(4-hydroxybutyl)-15,15,17-trimethyl-14,16,18-trioxa-7-aza-15-silaoctacosanoat |
| 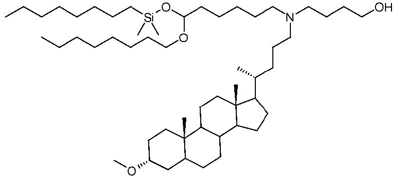 | 4-((6-((Dimethyl(octyl)silyl)oxy)-6-(octyloxy)hexyl)((4R)-4-((3R,10S,13R)-3-Methoxy-10,13-dimethylhexadecahydro-1H-cyclopenta[a]phenanthren-17-yl)pentyl)amino)butan-1-ol |
| 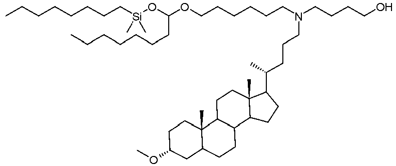 | 13-Heptyl-5-((4R)-4-((3R,10S,13R)-3-methoxy-10,13-dimethylhexadecahydro-1H-cyclopenta[a]phenanthren-17-yl)pentyl)-15,15-dimethyl-12,14-dioxa-5-aza-15-silatricosan-1-ol |
| 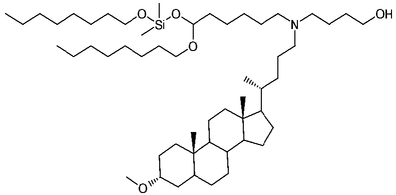 | 5-((4R)-4-((3R,10S,13R)-3-Methoxy-10,13-dimethylhexadecahydro-1H-cyclopenta[a]phenanthren-17-yl)pentyl)-13,13-dimethyl-11-(octyloxy)-12,14-dioxa-5-aza-13-siladocosan-1-ol |
| 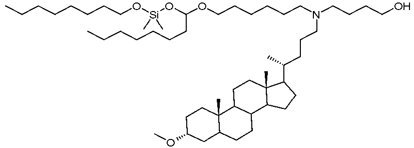 | 13-Heptyl-5-((4R)-4-((3R,10S,13R)-3-methoxy-10,13-dimethylhexadecahydro-1H-cyclopenta[a]phenanthren-17-yl)pentyl)-15,15-dimethyl-12,14,16-trioxa-5-aza-15-silatetracosan-1-ol |
| 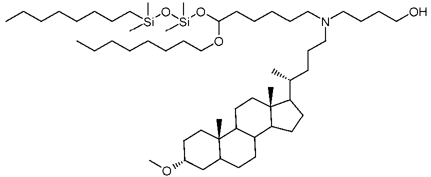 | 4-(((4R)-4-((3R,10S,13R)-3-Methoxy-10,13-dimethylhexadecahydro-1H-cyclopenta[a]phenanthren-17-yl)pentyl)(6-(octyloxy)-6-((1,1,3,3-Tetramethyl-3-octyldisiloxaneyl)oxy)hexyl)amino)butan-1-ol |
| 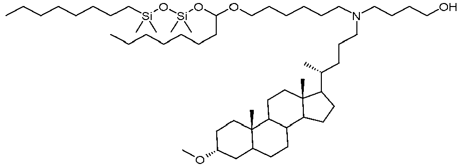 | 13-Heptyl-5-((4R)-4-((3R,10S,13R)-3-methoxy-10,13-dimethylhexadecahydro-1H-cyclopenta[a]phenanthren-17-yl)pentyl)-15,15,17,17-tetramethyl-12,14,16-trioxa-5-aza-15,17-disilapentacosan-1-ol |
| 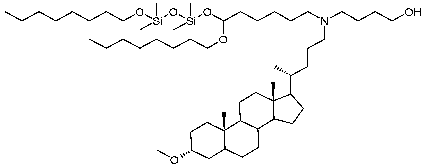 | 4-(((4R)-4-((3R,10S,13R)-3-Methoxy-10,13-dimethylhexadecahydro-1H-cyclopenta[a]phenanthren-17-yl)pentyl)(6-(octyloxy)-6-((1,1,3,3-Tetramethyl-3-(octyloxy)disiloxanyl)oxy)hexyl)amino)butan-1-ol |
| 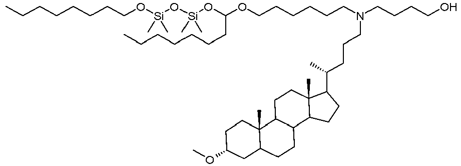 | 13-Heptyl-5-((4R)-4-((3R,10S,13R)-3-methoxy-10,13-dimethylhexadecahydro-1H-cyclopenta[a]phenanthren-17-yl)pentyl)-15,15,17,17-tetramethyl-12,14,16,18-tetraoxa-5-aza-15,17-disilahexacosan-1-ol |
| 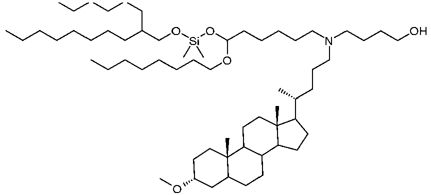 | 16-Hexyl-5-((4R)-4-((3R,10S,13R)-3-methoxy-10,13-dimethylhexadecahydro-1H-cyclopenta[a]phenanthren-17-yl)pentyl)-13,13-dimethyl-11-(octyloxy)-12,14-dioxa-5-aza-13-silatetracosan-1-ol |
| 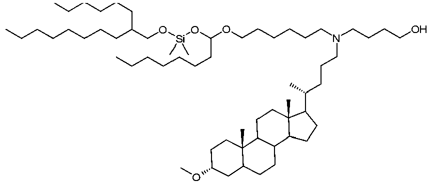 | 13-Heptyl-18-hexyl-5-((4R)-4-((3R,10S,13R)-3-methoxy-10,13-dimethylhexadecahydro-1H-cyclopenta[a]phenanthren-17-yl)pentyl)-15,15-dimethyl-12,14,16-trioxa-5-aza-15-silahexacosan-1-ol |
| 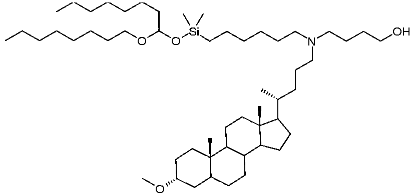 | 14-Heptyl-5-((4R)-4-((3R,10S,13R)-3-methoxy-10,13-dimethylhexadecahydro-1H-cyclopenta[a]phenanthren-17-yl)pentyl)-12,12-dimethyl-13,15-dioxa-5-aza-12-silatricosan-1-ol |
| 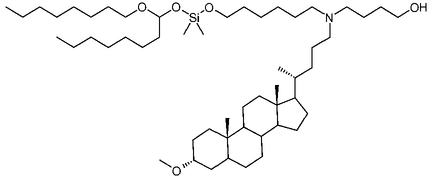 | 15-Heptyl-5-((4R)-4-((3R,10S,13R)-3-methoxy-10,13-dimethylhexadecahydro-1H-cyclopenta[a]phenanthren-17-yl)pentyl)-13,13-dimethyl-12,14,16-trioxa-5-aza-13-silatetracosan-1-ol |
| 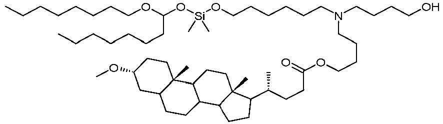 | 15-Heptyl-5-(4-hydroxybutyl)-13,13-dimethyl-12,14,16-trioxa-5-aza-13-silatetracosyl(4R)-4-((3R,10S,13R)-3-Methoxy-10,13-dimethylhexadecahydro-1H-cyclopenta[a]phenanthren-17-yl)pentanoat |
| 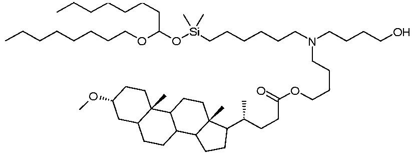 | 14-Heptyl-5-(4-hydroxybutyl)-12,12-dimethyl-13,15-dioxa-5-aza-12-silatricosyl(4R)-4-((3R,10S,13R)-3-Methoxy-10,13-dimethylhexadecahydro-1H-cyclopenta[a]phenanthren-17-yl)pentanoat |

21. Verbindung mit einer der folgenden Strukturen (Ia), (Ib), (Ic), 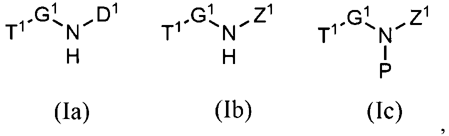 wobei D¹, G¹, T¹und P wie in Anspruch 1 definiert sind und 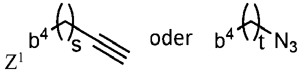 ist, wobei b⁴ eine Bindung an N darstellt
s eine ganze Zahl von 1 bis 6 ist
t eine ganze Zahl von 2 bis 6 ist.

22. Eine Verbindung mit der folgenden Struktur (II)) 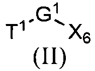 wobei G¹wie in Anspruch 1 definiert ist; 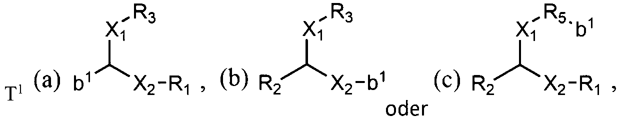 wobei b¹, X₁ und X₂, R₁ , R₂ , R₃ und R₅ wie in Anspruch 1 definiert sind; und X₆ aus Cl, Br, I, OMs, OTs, OTf ausgewählt ist.

23. Verbindung nach Anspruch 22, die eine der folgenden Strukturen aufweist:
| **Struktur** | **IUPAC-Bezeichnung** |
|---|---|
| 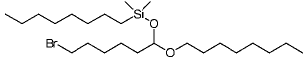 | ((6-Brom-1-(octyloxy)hexyl)oxy)dimethyl(octyl)silan |
| 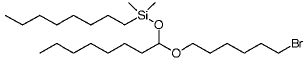 | ((1-((6-Bromhexyl)oxy)octyl)oxy)dimethyl(octyl)silan |
| 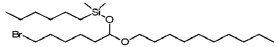 | ((6-Brom-1-(decyloxy)hexyl)oxy)(hexyl)dimethylsilan |
| 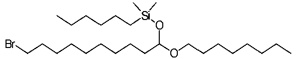 | ((10-Brom-1-(octyloxy)decyl)oxy)(hexyl)dimethylsilan |
| 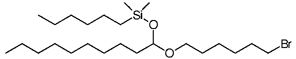 | ((1-((6-Bromhexyl)oxy)decyl)oxy)(hexyl)dimethylsilan |
| 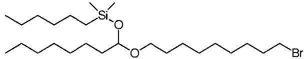 | ((1-((9-Bromnonyl)oxy)octyl)oxy)(hexyl)dimethylsilan |
| 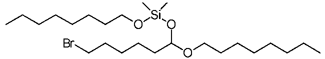 | 12-(5-Brompentyl)-10,10-dimethyl-9,11,13-trioxa-10-silahenicosan |
| 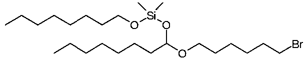 | 1-Brom-8-heptyl-10,10-dimethyl-7,9,11-trioxa-10-silanonadecan |
| 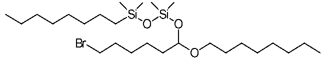 | 1-((6-Brom-1-(octyloxy)hexyl)oxy)-1,1,3,3-tetramethyl-3-octyldisiloxan |
| 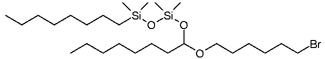 | 1-((1-((6-Bromhexyl)oxy)octyl)oxy)-1,1,3,3-tetramethyl-3-octyldisiloxan |
| 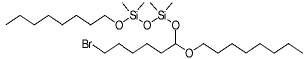 | 1-((6-Brom-1-(octyloxy)hexyl)oxy)-1,1,3,3-tetramethyl-3-(octyloxy)disiloxan |
| 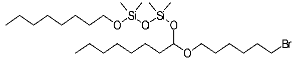 | 1-((1-((6-Bromhexyl)oxy)octyl)oxy)-1,1,3,3-tetramethyl-3-(octyloxy)disiloxan |
| 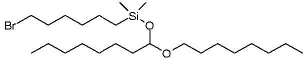 | (6-Bromhexyl)dimethyl((1-(octyloxy)octyl)oxy)silan |
| 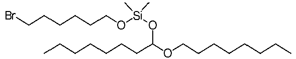 | 1-Brom-10-heptyl-8,8-dimethyl-7,9,11-trioxa-8-silanonadecan |
| 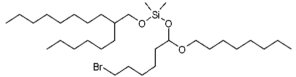 | 10-(5-Brompentyl)-15-hexyl-12,12-dimethyl-9,11,13-trioxa-12-silatricosan |
| 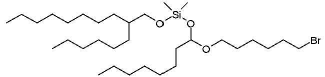 | 1-Brom-8-heptyl-13-hexyl-10,10-dimethyl-7,9,11-trioxa-10-silahenicosan |
| 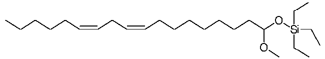 | Triethyl(((9Z,12Z)-1-methoxyoctadeca-9,12-dien-1-yl)oxy)silan |
| 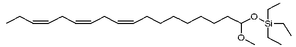 | Triethyl(((9Z,12Z,15Z)-1-methoxyoctadeca-9,12,15-trien-1-yl)oxy)silan |
| 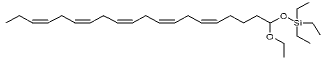 | (((5Z,8Z,11Z,14Z,17Z)-1-Ethoxyicosa-5,8,11,14,17-pentaen-1-yl)oxy)triethylsilan |
| 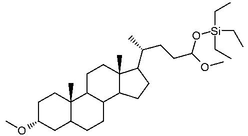 | Triethyl(((4R)-1-methoxy-4-((3R,10S,13R)-3-methoxy-10,13-dimethylhexadecahydro-1H-cyclopenta[a]phenanthren-17-yl)pentyl)oxy)silan |
| 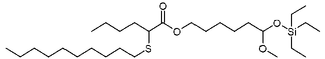 | 6-Methoxy-6-((triethylsilyl)oxy)hexyl-2-(decylthio)hexanoat |
| 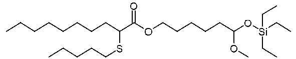 | 6-Methoxy-6-((triethylsilyl)oxy)hexyl-2-(pentylthio)decanoat |
| 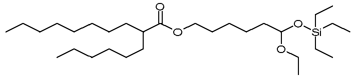 | 6-Ethoxy-6-((triethylsilyl)oxy)hexyl-2-hexyldecanoat |
| 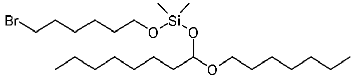 | 1-Brom-10-heptyl-8,8-dimethyl-7,9,11-trioxa-8-silaoctadecan |
| 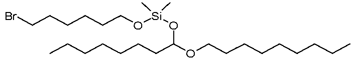 | 1-Brom-10-heptyl-8,8-dimethyl-7,9,11-trioxa-8-silaicosan |
| 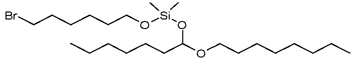 | 1-Brom-10-hexyl-8,8-dimethyl-7,9,11-trioxa-8-silanonadecan |
| 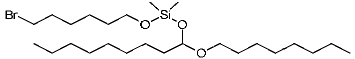 | 1-Brom-8,8-dimethyl-10-octyl-7,9,11-trioxa-8-silanonadecan |
| 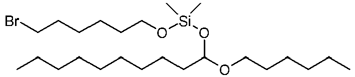 | 1-Brom-8,8-dimethyl-10-nonyl-7,9,11-trioxa-8-silaheptadecan |
| 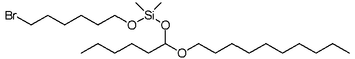 | 1-Brom-8,8-dimethyl-10-pentyl-7,9,11-trioxa-8-silahenicosan |
| 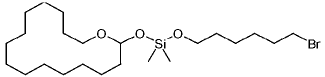 | ((6-Bromhexyl)oxy)dimethyl((oxacyclohexadecan-2-yl)oxy)silan |
| 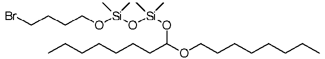 | 1-(4-Brombutoxy)-1,1,3,3-tetramethyl-3-((1-(octyloxy)octyl)oxy)disiloxan |
| 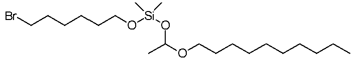 | 1-Brom-8,8,10-trimethyl-7,9,11-trioxa-8-silahenicosan |
| 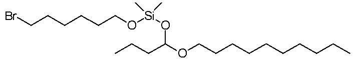 | 1-Brom-8,8-dimethyl-10-propyl-7,9,11-trioxa-8-silahenicosan |
| 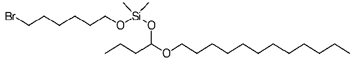 | 1-Brom-8,8-dimethyl-10-propyl-7,9,11-trioxa-8-silatricosan |
| 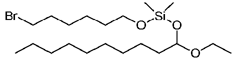 | 13-Brom-6,6-dimethyl-4-nonyl-3,5,7-trioxa-6-silatridecan |
| 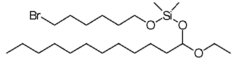 | 13-Brom-6,6-dimethyl-4-undecyl-3,5,7-trioxa-6-silatridecan |
| 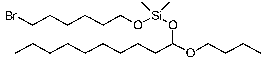 | 15-Brom-8,8-dimethyl-6-nonyl-5,7,9-trioxa-8-silapentadecan |
| 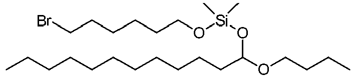 | 15-Brom-8,8-dimethyl-6-undecyl-5,7,9-trioxa-8-silapentadecan |
| 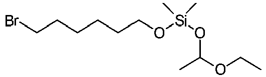 | 13-Brom-4,6,6-trimethyl-3,5,7-trioxa-6-silatridecan |
| 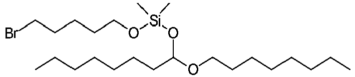 | 1-Brom-9-heptyl-7,7-dimethyl-6,8,10-trioxa-7-silaoctadecan |
| 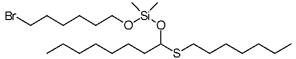 | 1-Brom-10-heptyl-8,8-dimethyl-7,9-dioxa-11-thia-8-silaoctadecan |
| 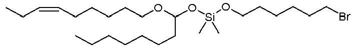 | (Z)-1-Brom-10-heptyl-8,8-dimethyl-7,9,11-trioxa-8-silaicos-17-en |
| 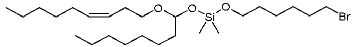 | (Z)-1-Brom-10-heptyl-8,8-dimethyl-7,9,11-trioxa-8-silaicos-14-en |
| 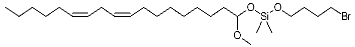 | 10-Brom-3-((8Z,11Z)-heptadeca-8,11-dien-1-yl)-5,5-dimethyl-2,4,6-trioxa-5-siladecan |

24. Lipidnanopartikel, umfassend ein therapeutisches Mittel und eine Verbindung gemäß einem der Ansprüche 1 bis 20, insbesondere eine Verbindung gemäß Anspruch 8, 9, 10, 11, 12 oder 20.

## Revendications

1. Composé répondant à la formule (I) 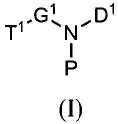
ou son sel ou son stéréoisomère,
dans laquelle :
G¹ est un alkylène en C₂-C₉ non substitué,
- (CH₂)ₓ-CH=CH-(CH₂)_{y}-, x étant un nombre entier choisi parmi 1 à 6, y étant un nombre entier choisi parmi 1 à 6, et la somme de x + y étant un nombre entier choisi parmi 2 à 7, ou
- (CH₂)_{w}-X-(CH₂)_{z}-, w étant un nombre entier choisi parmi 1 à 7, z étant un nombre entier choisi parmi 2 à 7, la somme de w + z étant un nombre entier choisi parmi 3 à 8, -(CH₂)_{z}- étant lié à N, et X étant choisi parmi O, S, SO et SO₂ ;
T¹ est 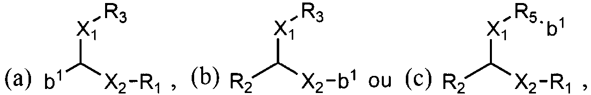
dans lesquels
b¹ est une liaison à G¹,
X₁ et X₂ sont identiques ou différents et représentent chacun indépendamment O ou S,
R₁ est un alkyle en C₁-C₁₇ linéaire, un alkyle en C₃-C₁₇ non linéaire, dans les deux cas contenant éventuellement un S, SO, SO₂, O ou Si(R^{a})₂, dans lequel R^{a} est un alkyle en C₁-C₆, à n'importe quelle position dans la chaîne carbonée à condition que l'hétéroatome ne soit pas dans la position alpha ou oméga de la chaîne carbonée, un alcényle en C₃-C₁₇ contenant une double liaison à condition qu'il y ait au moins un groupe -CH₂- entre la double liaison et X₂, ou un alcényle en C₈-C₂₀ polyinsaturé à condition qu'il y ait au moins un groupe -CH₂- entre la première double liaison et X₂, ou
R₁ est 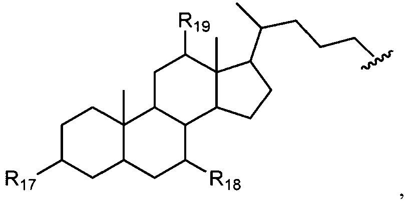 R₁₇, R₁₈ et R₁₉ étant identiques ou différents et pouvant être chacun indépendamment H, OH, un alcoxy en C₁-C₆ ou le fluor ;
R₂ est
un alkyle en C₁-C₁₇ linéaire, alkyle en C₃-C₁₇ non linéaire, dans les deux cas contenant éventuellement un S, SO, SO₂, O ou Si(R^{b} )₂, dans lequel R^{b} est un alkyle en C₁-C₆, à n'importe quelle position dans la chaîne carbonée à condition que l'hétéroatome ne soit pas dans la position alpha ou oméga de la chaîne carbonée, un alcényle en C₃-C₁₇ contenant une double liaison à condition qu'il y ait au moins un groupe -CH₂- entre la double liaison et le carbone reliant X₁ et X₂, ou un alcényle en C₈-C₂₀ polyinsaturé à condition qu'il y ait au moins un groupe -CH₂- entre la première double liaison et le carbone reliant X₁ et X₂, ou
R₂ est 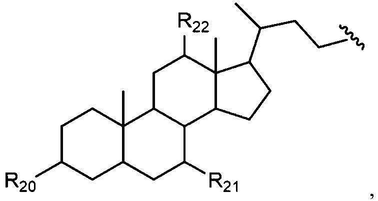 R₂₀, R₂₁ et R₂₂ étant identiques ou différents et pouvant être chacun indépendamment H, OH, -alcoxy en C₁-C₆ ou fluor,
à condition que R₁ et R₂ ne puissent pas être tous deux en même temps, 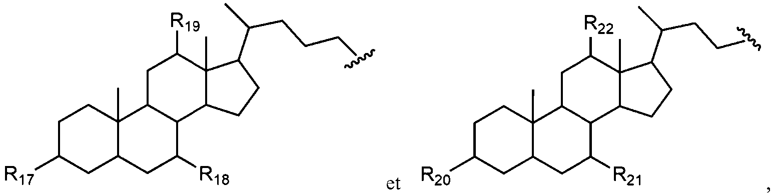
ou
R₁ est lié à R₃ par l'intermédiaire d'un alkylène ou d'un alcényle monoinsaturé formant un cycle à C₅ -C₃₀ chaînons, ou
R₂ est lié à R₃ par l'intermédiaire d'un alkylène ou d'un alcényle monoinsaturé formant un cycle à C₅-C₃₀ chaînons, dans lequel l'atome de carbone de R₂ en première ou deuxième position, numéroté à partir de l'atome de carbone lié à l'atome de carbone reliant X₁ et X₂, peut être éventuellement remplacé par un hétéroatome O ou S, ou
R₂ est lié à R₁ par l'intermédiaire d'un alkylène ou d'un alcényle monoinsaturé formant un cycle à C₅-C₃₀ chaînons, dans lequel l'atome de carbone de R₂ en première ou deuxième position, numéroté à partir de l'atome de carbone lié à l'atome de carbone reliant X₁ et X₂, peut être éventuellement remplacé par un hétéroatome O ou S ;
R₃ est 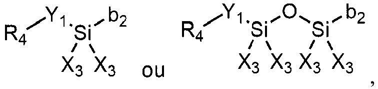
R₅ -b¹ est 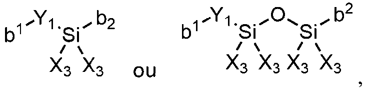
dans lesquels
b² est une liaison à X₁,
Y₁ est -O- ou -CH₂ - ou -O-CH₂-CH₂- dans lequel -CH₂- est lié à Si,
chaque X₃ est choisi indépendamment dans le groupe constitué par un alkyle en C₁-C₄ ou un alcoxy en C₁-C₄,
R₄ est un alkyle en C₂-C₁₇ linéaire, ou un alkyle en C₃-C₁₇ non linéaire, dans les deux cas contenant éventuellement un S, SO, SO₂, O ou Si(R^{c})₂, dans lequel R^{c} est un alkyle en C₁-C₆, à n'importe quelle position dans la chaîne carbonée à condition que l'hétéroatome ne soit pas dans la position alpha ou oméga de la chaîne carbonée, un alcényle en C₃-C₂₀ contenant une double liaison à condition qu'il y ait au moins un groupe -CH₂- entre la double liaison et Y₁, ou un alcényle en C₈-C₂₀ polyinsaturé à condition qu'il y ait au moins un groupe -CH₂- entre la première double liaison et Y₁ ou,
R⁴ est 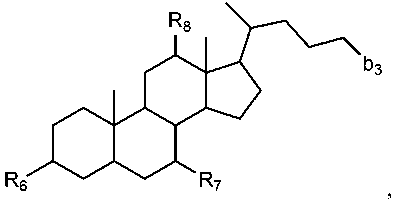
dans lequel b³ est une liaison à Y₁,
R₆, R₇ et R₈ sont identiques ou différents et peuvent être chacun indépendamment H, OH, un alcoxy en C₁-C₆ ou le fluor ;
D¹ est choisi dans le groupe constitué par 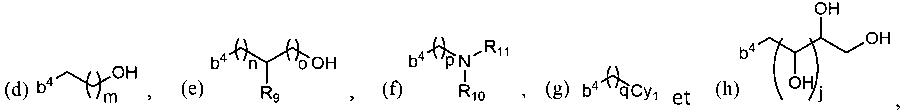 dans lesquels
b⁴ est une liaison à l'azote,
R₉ peut être un alkyle en C₁-C₆, cyclopentyle, cyclohexyle, hydroxyle, hydroxyméthyle, hydroxyéthyle, phényle, benzyle, 4-hydroxy benzyle, 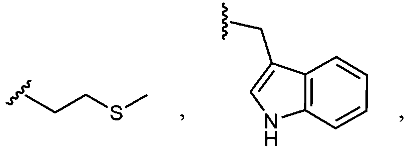
R₁₀ et R₁₁ sont choisis indépendamment parmi H et un alkyle en C₁-C₆,
m est un nombre entier choisi parmi 1 à 6,
n est un nombre entier choisi parmi 0 à 6,
o est un nombre entier choisi parmi 0 à 6,
p est un nombre entier choisi parmi 2 à 6,
q est un nombre entier choisi parmi 0 à 6,
j est un nombre entier choisi parmi 1 à 4,
Cy₁ est un cycloalkyle en C₃-C₆ éventuellement substitué par un ou plusieurs -OH, ou
Cy₁ est un cycle pyranose, furanose, qui peut être lié par l'intermédiaire de l'un quelconque de son groupe OH, le cycle pyranose ou furanose étant éventuellement substitué par un groupe -NH-CO-CH₃, l'adénine, la guanine, l'uracile, la cytosine, la thymine, un mono- ou oligosaccharide, ou par un cycle hétérocyclique à 4, 5, 6 ou 7 chaînons contenant 1, 2, 3 ou 4 hétéroatomes choisis parmi N, O ou S; ou
Cy₁ est un cycle hétérocyclique à 4, 5, 6 ou 7 chaînons contenant 1, 2, 3 ou 4 hétéroatomes choisis parmi O, N ou S, le cycle hétérocyclique étant éventuellement substitué par R₁₂,
dans lequel
R₁₂ représente un alkyle en C₁-C₆, un peptide contenant de l'arginine, un cycle pyranose ou furanose lié par l'un quelconque de ses atomes de carbone cycliques au cycle hétérocyclique à 4, 5, 6 ou 7 chaînons, le cycle pyranose ou furanose étant éventuellement substitué par un groupe -NH-CO-CH₃, l'adénine, la guanine, l'uracile, la cytosine, la thymine, un mono- ou oligosaccharide,
R₁₂ peut être un groupe choisi parmi (d), (e) ou (f), dans lesquels b⁴ est une liaison au cycle hétérocyclique, m, n, o, p, R₉, R₁₀ et R₁₁ sont tels que définis ci-dessus,
ou
R₁₂ peut être un groupe 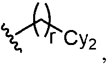 dans lequel r est un nombre entier choisi parmi 1 à 4 et Cy₂ est un cycle hétérocyclique à 4, 5, 6 ou 7 chaînons contenant 1, 2, 3 ou 4 hétéroatomes choisis parmi O, N et S, éventuellement substitué par un alkyle en C₁-C₆ ;
P est choisi parmi
(i) G²-T²
dans lequel
G² est défini comme G¹ ci-dessus, G¹ et G² pouvant être identiques ou différents ;
T² est défini comme T¹ ci-dessus, b¹ est une liaison à G², et T¹ et T² peuvent être identiques ou différents ; ou
(ii) G² -T³
dans lequel
G² est tel que défini ci-dessus ;
T³ est 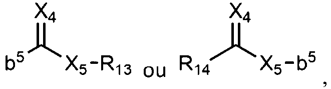
dans lesquels
b⁵ est une liaison à G²,
X₄ et X₅ peuvent être identiques ou différents et représentent chacun indépendamment O, S ou NR^{d}, R^{d} étant H, un alkyle en C₁-C₆, -OH ou alcoxy en C₁-C₆,
R₁₃ est un alkyle en C₂-C₁₇ linéaire, ou un alkyle en C₃-C₁₇ non linéaire, tous deux contenant éventuellement un S, SO, SO₂, O ou Si(R^{e})₂, dans lequel R^{e} est un alkyle en C₁-C₆, à n'importe quelle position dans la chaîne carbonée à condition que l'hétéroatome ne soit pas dans la position alpha ou oméga de la chaîne carbonée, un alcényle en C₃-C₂₀ contenant une double liaison à condition qu'il y ait au moins un groupe -CH₂- entre la double liaison et X₅, ou un alcényle en C₈-C₂₀ polyinsaturé à condition qu'il y ait au moins un groupe -CH₂- entre la première double liaison et X₅ ou,
R₁₃ est 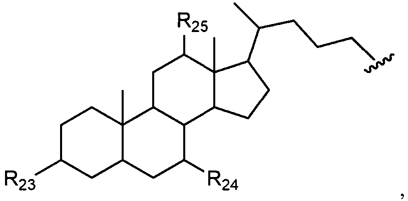 R₂₃, R₂₄ et R₂₅ étant identiques ou différents et pouvant être chacun indépendamment H, OH, un alcoxy en C₁-C₆ ou le fluor,
R₁₄ est un alkyle en C₁-C₁₇ linéaire, un alkyle en C₃-C₁₇ non linéaire, tous deux contenant éventuellement un S, SO, SO₂, O ou Si(R^{f})₂, dans lequel R^{f} est un alkyle en C₁-C₆, à n'importe quelle position dans la chaîne carbonée à condition que l'hétéroatome ne soit pas dans la position alpha ou oméga de la chaîne carbonée, un alcényle en C₃-C₂₀ contenant une double liaison à condition qu'il y ait au moins un groupe -CH₂- entre la double liaison et le carbone reliant X₄ et X₅, ou un alcényle en C₈-C₂₀ polyinsaturé à condition qu'il y ait au moins un groupe -CH₂- entre la première double liaison et le carbone reliant X₄ et X₅, ou
R₁₄ est 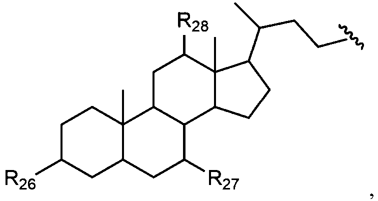 R₂₆, R₂₇, R₂₈ étant identiques ou différents et pouvant être chacun indépendamment H, OH, un alcoxy en C₁-C₆ ou le fluor ;
et (iii) T⁴, dans lequel
T⁴ est 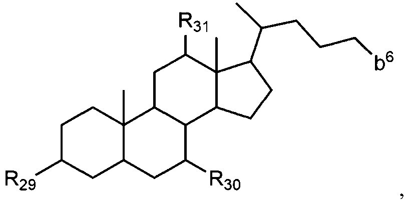 dans lequel b⁶ est une liaison à l'atome d'azote, R₂₉, R₃₀, R₃₁ sont identiques ou différents et peuvent chacun indépendamment représenter H, OH, un alcoxy en C₁-C₆ ou le fluor ;
un alcényle en C₄-C₂₀ contenant une double liaison, à condition qu'il y ait au moins un -CH₂-entre la double liaison et N, ou
un alcényle en C₈-C₂₀ polyinsaturé, à condition qu'il y ait au moins un -CH₂- entre la première double liaison et N.

2. Composé selon la revendication 1, dans lequel G¹ et, s'il est présent, G² sont identiques ou différents et chacun est indépendamment un alkylène en C₄-C₉ linéaire, de préférence un alkylène en C₃-C₉, ou à titre d'alternative chacun est indépendamment un alkylène en C₅, C₆, C₇ ou C₉ linéaire.

3. Composé selon la revendication 1 ou 2, dans lequel R₁ est un alkyle en C₁, C₂, C₄, C₆, C₇, C₈, C₉, C₁₀ ou C₁₂ linéaire, ou R₁ est un alcényle en C₉ linéaire, et/ou R₂ est un alkyle en C₁, C₃, C₅, C₆, C₇, C₈, C₉ ou C₁₁ linéaire, de préférence R₁ est un alkyle en C₂, C₈ ou C₁₀ linéaire, ou R₁ est un alcényle en C₉ linéaire et/ou R₂ est un alkyle en C₁, C₃, C₇ ou C₉ linéaire.

4. Composé selon la revendication 3, dans lequel T¹ est (c) et R₁ est un alkyle en C₂, C₄, C₆, C₇, C₈, C₉, C₁₀ ou C₁₂ linéaire, ou R₁ est un alcényle en C₉ linéaire et R₂ est un alkyle en C₁, C₃, C₅, C₆, C₇, C₈, C₉ ou C₁₁ linéaire, de préférence R₁ est un alkyle en C₂, C₈ ou C₁₀ linéaire, ou R₁ est un alcényle en C₉ linéaire et R₂ est un alkyle en C₁, C₃, C₇ ou C₉ linéaire.

5. Composé selon l'une quelconque des revendications 1 à 3, dans lequel P est G²-T².

6. Composé selon la revendication 5, dans lequel G¹-T¹ et G²-T² sont identiques.

7. Composé selon l'une quelconque des revendications 1 à 3, dans lequel P est G²-T³.

8. Composé selon la revendication 7, dans lequel T¹-G¹ présente l'une des structures suivantes

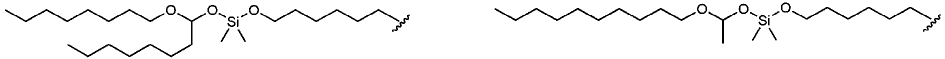

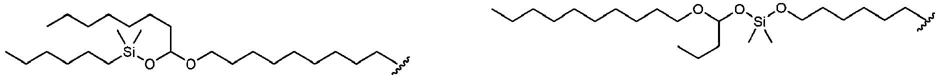

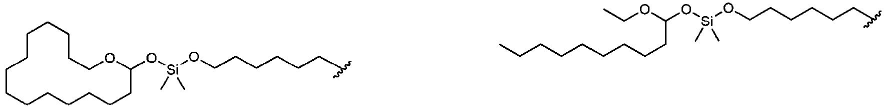

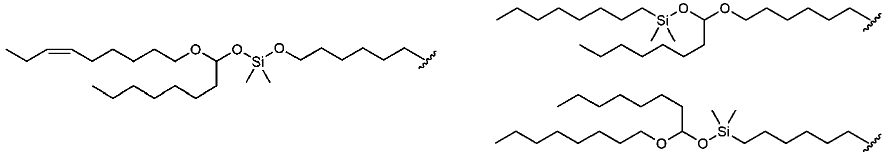

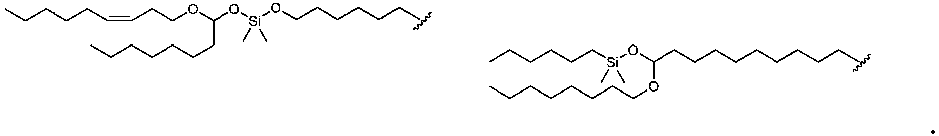

9. Composé selon la revendication 7 ou 8, dans lequel T³ présente la structure suivante 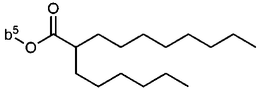 dans laquelle b⁵ est la liaison à G².

10. Composé selon la revendication 7 ou 8, dans lequel T³ présente l'une des structures suivantes 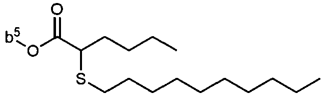 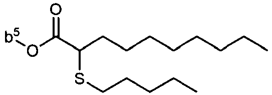 dans lesquelles b⁵ est la liaison à G².

11. Composé selon la revendication 7 ou 8, dans lequel T³ présente l'une des structures suivantes 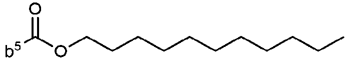 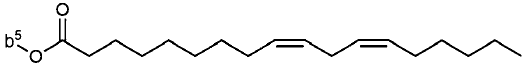 dans lesquelles b⁵ est la liaison à G².

12. Composé selon la revendication 7 ou 8, dans lequel T³ présente la structure suivante 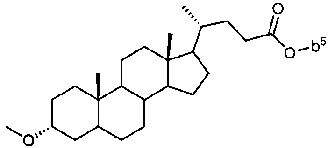 dans laquelle b⁵ est la liaison à G².

13. Composé selon l'une quelconque des revendications 1 à 3, dans lequel P est T⁴.

14. Composé selon la revendication 13, dans lequel T⁴ est 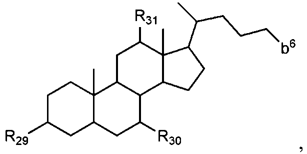
dans laquelle b⁶ est une liaison à l'atome d'azote, R₂₉, R₃₀, R₃₁ peuvent être identiques ou
différents et chacun peut être indépendamment H, OH, un alcoxy en C₁-C₆ ou le fluor ; ou
un alcényle en C₈-C₂₀ polyinsaturé, à condition qu'il y ait au moins un groupe -CH₂- entre la première double liaison et N.

15. Composé selon la revendication 14, dans lequel T¹ -G¹ présente l'une des structures suivantes

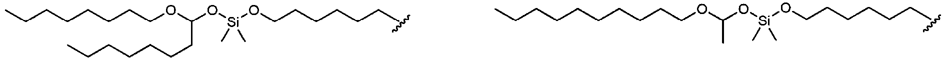

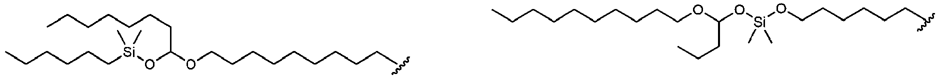

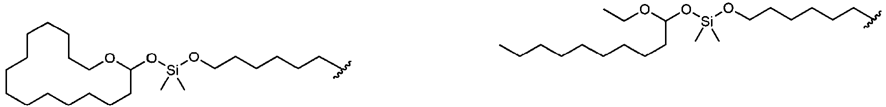

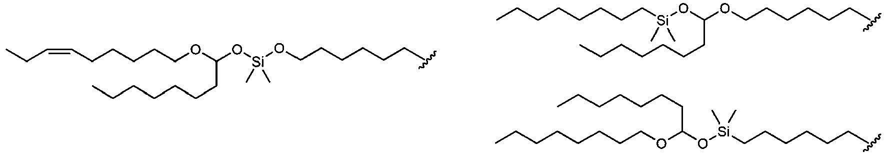

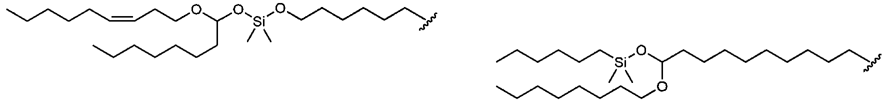

16. Composé selon la revendication 14 ou 15, dans lequel T⁴ présente la structure suivante 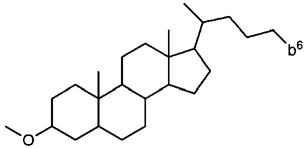 dans laquelle b⁶ est une liaison à l'atome d'azote.

17. Composé selon l'une quelconque des revendications 1 à 16, dans lequel D¹ présente l'une des structures suivantes :

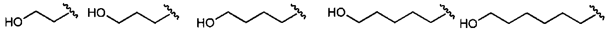

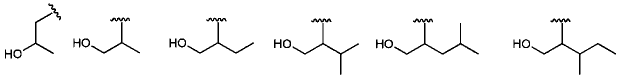

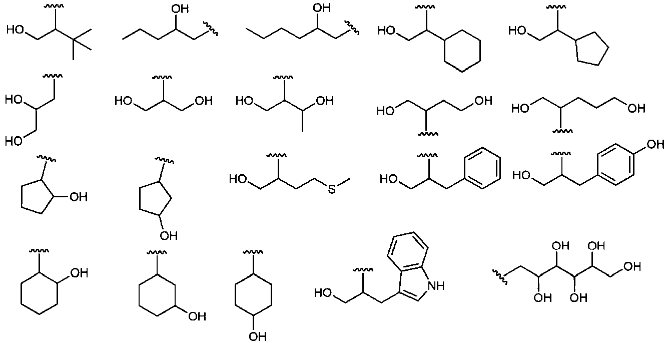

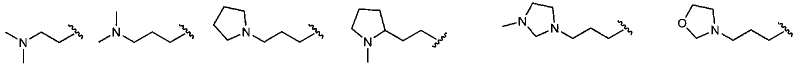

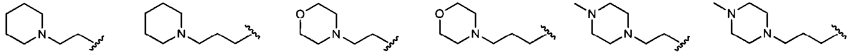

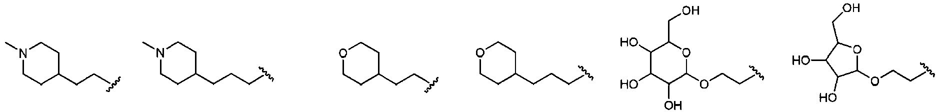

18. Composé selon l'une quelconque des revendications 1 à 16, dans lequel D¹ présente l'une des structures suivantes : 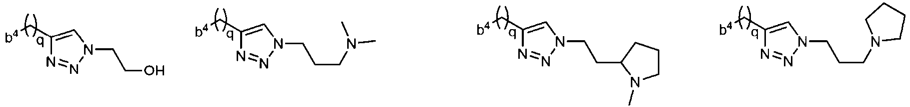 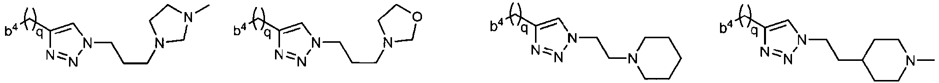 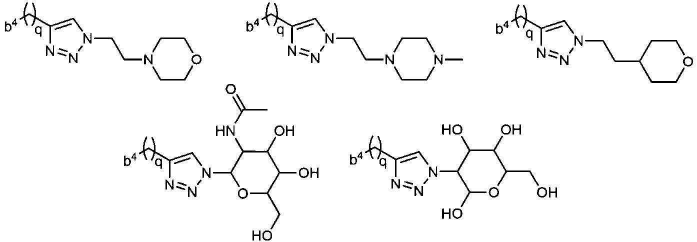 dans lesquelles q est un nombre entier choisi parmi 1 à 4 et b⁴ est une liaison à l'azote.

19. Composé selon l'une quelconque des revendications 1 à 16, dans lequel D¹ présente l'une des structures suivantes :

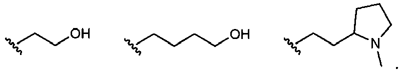

20. Composé selon la revendication 1, qui présente l'une des structures suivantes :
| **Structure** | **Nom IUPAC** |
|---|---|
| 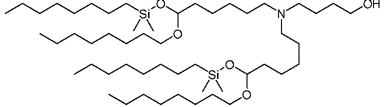 | 4-(bis(6-((diméthyl(octyl)silyl)oxy)-6-(octyloxy)hexyl)amino)butan-1-ol |
| 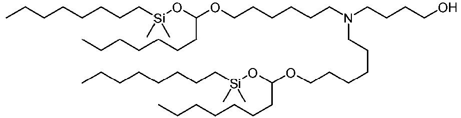 | 5-(6-((1-((dimethyl(octyl)silyl)oxy)octyl)oxy)hexyl)-13-heptyl-15,15-diméthyl-12,14-dioxa-5-aza-15-silatricosan-1-ol |
| 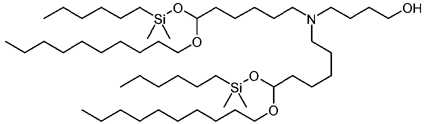 | 4-(bis(6-(décyloxy)-6-((hexyldiméthylsilyl)oxy)hexyl)amino)butan-1-ol |
| 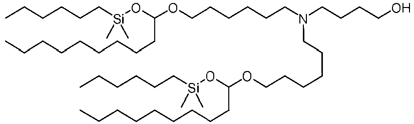 | 5-(6-((1-((hexyldiméthylsilyl)oxy)décyl)oxy)hexyl)-15,15-diméthyl-13-nonyl-12,14-dioxa-5-aza-15-silahénicosan-1-ol |
| 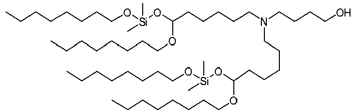 | 5-(6-((diméthyl(octyloxy)silyl)oxy)-6-(octyloxy)hexyl)-13,13-dimethyl-11-(octyloxy)-12,14-dioxa-5-aza-13-siladocosan-1-ol |
| 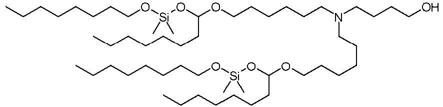 | 13-heptyl-5-(8-heptyl-10,10-diméthyl-7,9,11-trioxa-10-silanonadécyl)-15,15-diméthyl-12,14,16-trioxa-5-aza-15-silatétracosan-1-ol |
| 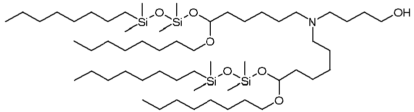 | 4-(bis(6-(octyloxy)-6-((1,1,3,3-tétraméthyl-3-octyldisiloxaneyl)oxy)hexyl)amino)butan-1-ol |
| 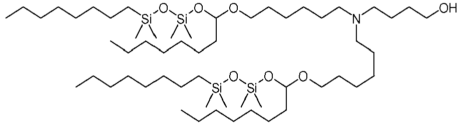 | 13-heptyl-15,15,17,17-tétraméthyl-5-(6-((1-((1,1,3,3-tétraméthyl-3-octyldisiloxaneyl)oxy)octyl)oxy)hexyl)-12,14,16-trioxa-5-aza-15,17-disilapentacosan-1-ol |
| 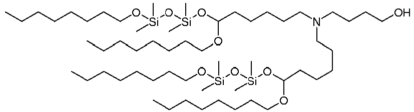 | 4-(bis(6-(octyloxy)-6-((1,1,3,3-tétraméthyl-3-(octyloxy)disiloxaneyl)oxy)hexyl)amino)butan-1-ol |
| 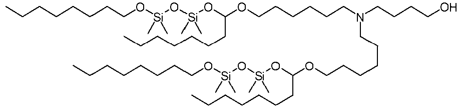 | 13-heptyl-15,15,17,17-tetramethyl-5-(6-((1-((1,1,3,3-tétraméthyl-3-(octyloxy)disiloxaneyl)oxy)octyl)oxy)hexyl)-12,14,16,18-tétraoxa-5-aza-15,17-disilahexacosan-1-ol |
| 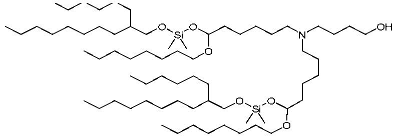 | 16-hexyl-5-(6-((((2-hexyldécyl)oxy)diméthylsilyl)oxy)-6-(octyloxy)hexyl)-13,13-diméthyl-11-(octyloxy)-12,14-dioxa-5-aza-13-silatetracosan-1-ol |
| 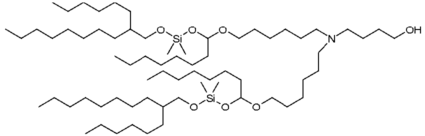 | 13-heptyl-5-(8-heptyl-13-hexyl-10,10-dimethyl-7,9,11-trioxa-10-silahénicosyl)-18-hexyl-15,15-diméthyl-12,14,16-trioxa-5-aza-15-silahexacosan-1-ol |
| 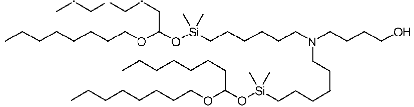 | 5-(6-(diméthyl((1-(octyloxy)octyl)oxy)silyl)hexyl)-14-heptyl-12,12-diméthyl-13,15-dioxa-5-aza-12-silatricosan-1-ol |
| 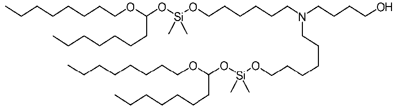 | 15-heptyl-5-(10-heptyl-8,8-diméthyl-7,9,11-trioxa-8-silanonadécyl)-13,13-diméthyl-12,14,16-trioxa-5-aza-13-silatétracosan-1-ol |
| 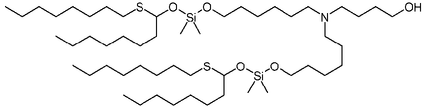 | 15-heptyl-5-(10-heptyl-8,8-dimethyl-7,9-dioxa-11-thia-8-silanonadécyl)-13,13-diméthyl-12,14-dioxa-16-thia-5-aza-13-silatétracosan-1-ol |
| 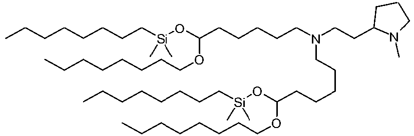 | 6-((diméthyl(octyl)silyl)oxy)-N-(6-((diméthyl(octyl)silyl)oxy)-6-(octyloxy)hexyl)-N-(2-(1-methylpyrrolidin-2-yl)ethyl)-6-(octyloxy)hexan-1-amine |
| 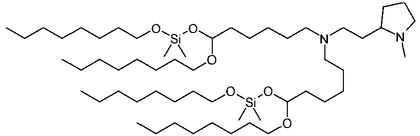 | 6-((diméthyl(octyloxy)silyl)oxy)-N-(6-((diméthyl(octyloxy)silyl)oxy)-6-(octyloxy)hexyl)-N-(2-(1-methylpyrrolidin-2-yl)éthyl)-6-(octyloxy)hexan-1-amine |
| 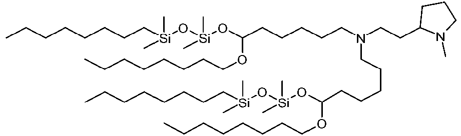 | N-(2-(1-methylpyrrolidin-2-yl)ethyl)-6-(octyloxy)-N-(6-(octyloxy)-6-((1,1,3,3-tétraméthyl-3-octyldisiloxaneyl)oxy)hexyl)-6-((1,1,3,3-tétraméthyl-3-octyldisiloxaneyl)oxy)hexan-1-amine |
| 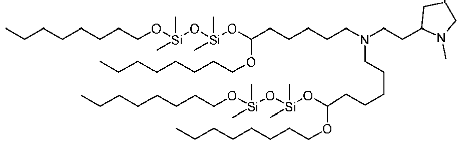 | N-(2-(1-méthylpyrrolidin-2-yl)éthyl)-6-(octyloxy)-N-(6-(octyloxy)-6-((1,1,3,3-tétraméthyl-3-(octyloxy)disiloxaneyl)oxy)hexyl)-6-((1,1,3,3-tétraméthyl-3-(octyloxy)disiloxaneyl)oxy)hexan-1-amine |
| 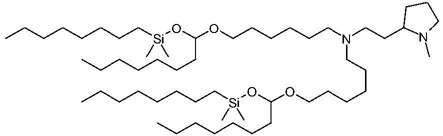 | 6-((1-((diméthyl(octyl)silyl)oxy)octyl)oxy)-N-(6-((1-((diméthyl(octyl)silyl)oxy)octyl)oxy)hexyl)-N-(2-(1-méthylpyrrolidin-2-yl)éthyl)hexan-1-amine |
| 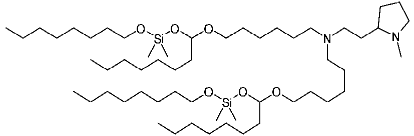 | 8-heptyl-N-(8-heptyl-10,10-diméthyl-7,9,11-trioxa-10-silanonadécyl)-10,10-diméthyl-N-(2-(1-méthylpyrrolidin-2-yl)éthyl)-7,9,11-trioxa-10-silanonadécan-1-amine |
| 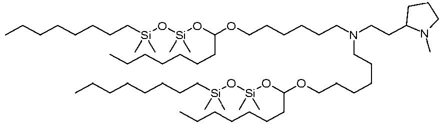 | N-(2-(1-méthylpyrrolidin-2-yl)éthyl)-6-((1-((1,1,3,3-tétraméthyl-3-octyldisiloxaneyl)oxy)octyl)oxy)-N-(6-((1-((1,1,3,3-tétraméthyl-3-octyldisiloxaneyl)oxy)octyl)oxy)hexyl)hexan-1-amine |
| 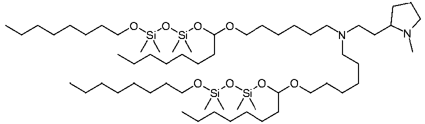 | N-(2-(1-méthylpyrrolidin-2-yl)éthyl)-6-((1-((1,1,3,3-tétraméthyl-3-(octyloxy)disiloxaneyl)oxy)octyl)oxy)-N-(6-((1-((1,1,3,3-tétraméthyl-3-(octyloxy)disiloxaneyl)oxy)octyl)oxy)hexyl)hex an-1-amine |
| 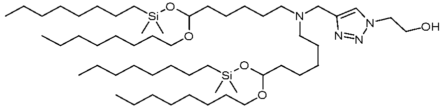 | 2-(4-((bis(6-((diméthyl(octyl)silyl)oxy)-6-(octyloxy)hexyl)amino)méthyl)-1H-1,2,3-triazol-1-yl)éthan-1-ol |
| 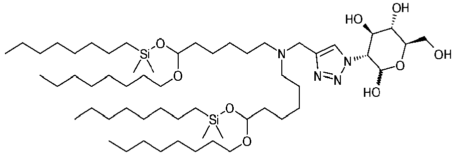 | (3R,4R,5S,6R)-3-(4-((bis(6-((diméthyl(octyl)silyl)oxy)-6-(octyloxy)hexyl)amino)méthyl)-1H-1,2,3-triazol-1-yl)-6-(hydroxyméthyl)tétrahydro-2H-pyran-2,4,5-triol |
| 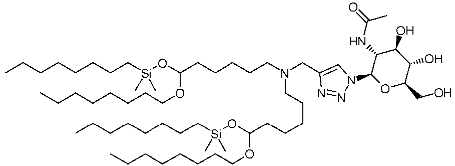 | N-((2R,3R,4R,5S,6R)-2-(4-((bis(6-((diméthyl(octyl)silyl)oxy)-6-(octyloxy)hexyl)amino)méthyl)-1H-1,2,3-triazol-1-yl)-4,5-dihydroxy-6-(hydroxyméthyl)tétrahydro-2H-pyran-3-yl)acétamide |
| 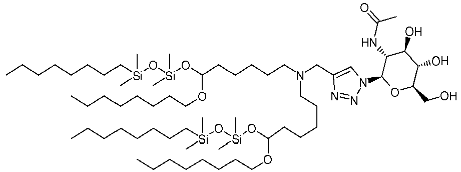 | N-((2R,3R,4R,5S,6R)-2-(4-((bis(6-(octyloxy)-6-((1,1,3,3-tétraméthyl-3-octyldisiloxaneyl)oxy)hexyl)amino)méthyl)-1H-1,2,3-triazol-1-yl)-4,5-dihydroxy-6-(hydroxyméthyl)tétrahydro-2H-pyran-3-yl)acétamide |
| 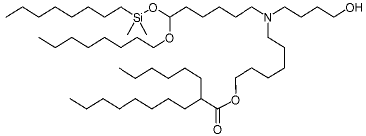 | 2-hexyldécanoate de 6-((6-((diméthyl(octyl)silyl)oxy)-6-(octyloxy)hexyl)(4-hydroxybutyl)amino)hexyle |
| 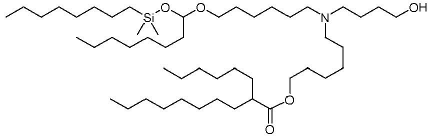 | 2-hexyldécanoate de 15-heptyl-7-(4-hydroxybutyl)-17,17-diméthyl-14,16-dioxa-7-aza-17-silapentacosyle |
| 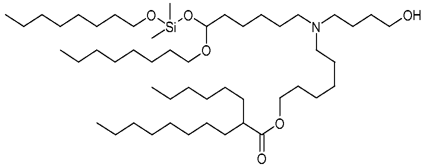 | 2-hexyldécanoate de 7-(4-hydroxybutyl)-15,15-diméthyl-13-(octyloxy)-14,16-dioxa-7-aza-15-silatétracosyle |
| 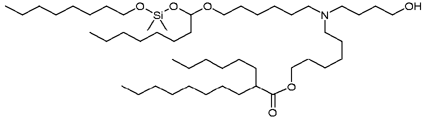 | 2-hexyldécanoate de 15-heptyl-7-(4-hydroxybutyl)-17,17-diméthyl-14,16,18-trioxa-7-aza-17-silahexacosyle |
| 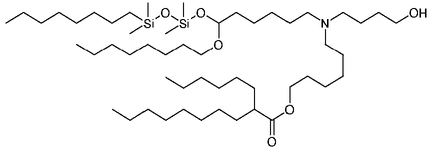 | 2-hexyldécanoate de 6-((4-hydroxybutyl)(6-(octyloxy)-6-((1,1,3,3-tétraméthyl-3-octyldisiloxaneyl)oxy)hexyl)amino)hexyle |
| 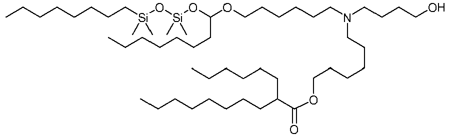 | 2-hexyldécanoate de 15-heptyl-7-(4-hydroxybutyl)-17,17,19,19-tétraméthyl-14,16,18-trioxa-7-aza-17,19-disilaheptacosyle |
| 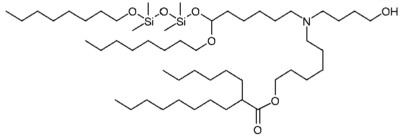 | 2-hexyldécanoate de 6-((4-hydroxybutyl)(6-(octyloxy)-6-((1,1,3,3-tétraméthyl-3-(octyloxy)disiloxaneyl)oxy)hexyl)amino)hexyle |
| 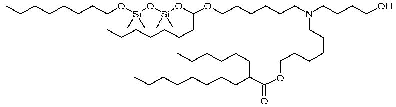 | 2-hexyldécanoate de 15-heptyl-7-(4-hydroxybutyl)-17,17,19,19-tétraméthyl-14,16,18,20-tétraoxa-7-aza-17,19-disilaoctacosyle |
| 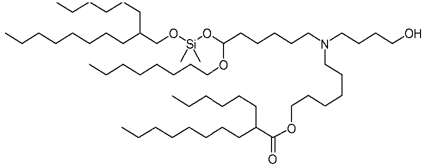 | 2-hexyldécanoate de 18-hexyl-7-(4-hydroxybutyl)-15,15-diméthyl-13-(octyloxy)-14,16-dioxa-7-aza-15-silahexacosyle |
| 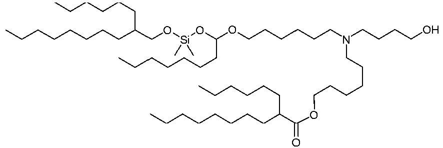 | 2-hexyldécanoate de 15-heptyl-20-hexyl-7-(4-hydroxybutyl)-17,17-diméthyl-14,16,18-trioxa-7-aza-17-silaoctacosyle |
| 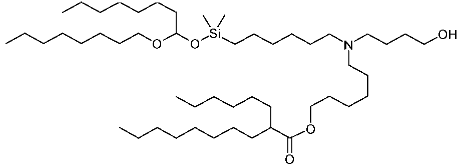 | 2-hexyldécanoate de 16-heptyl-7-(4-hydroxybutyl)-14,14-diméthyl-15,17-dioxa-7-aza-14-silapentacosyle |
| 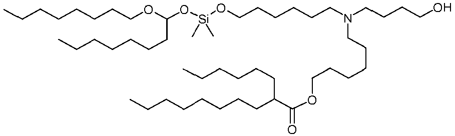 | 2-hexyldécanoate de 17-heptyl-7-(4-hydroxybutyl)-15,15-diméthyl-14,16,18-trioxa-7-aza-15-silahexacosyle |
| 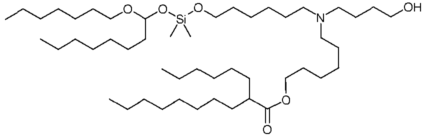 | 2-hexyldécanoate de 17-heptyl-7-(4-hydroxybutyl)-15,15-diméthyl-14,16,18-trioxa-7-aza-15-silapentacosyle |
| 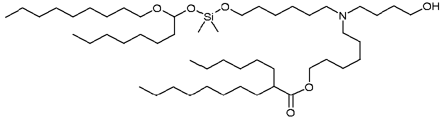 | 2-hexyldécanoate de 17-heptyl-7-(4-hydroxybutyl)-15,15-diméthyl-14,16,18-trioxa-7-aza-15-silaheptacosyle |
| 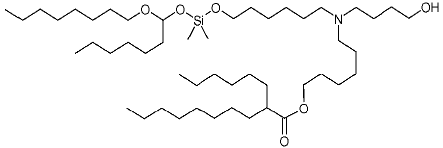 | 2-hexyldécanoate de 17-hexyl-7-(4-hydroxybutyl)-15,15-diméthyl-14,16,18-trioxa-7-aza-15-silahexacosyle |
| 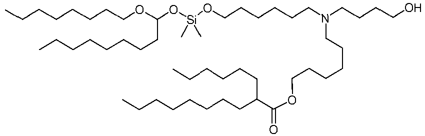 | 2-hexyldécanoate de 7-(4-hydroxybutyl)-15,15-diméthyl-17-octyl-14,16,18-trioxa-7-aza-15-silahexacosyle |
| 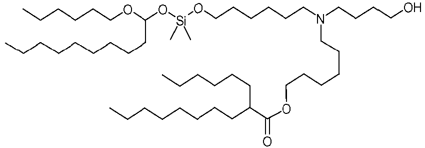 | 2-hexyldécanoate de 18-(4-hydroxybutyl)-10,10-diméthyl-8-nonyl-7,9,11-trioxa-18-aza-10-silatétracosan-24-yle |
| 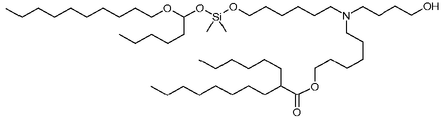 | 2-hexyldécanoate de 7-(4-hydroxybutyl)-15,15-diméthyl-17-pentyl-14,16,18-trioxa-7-aza-15-silaoctacosyle |
| 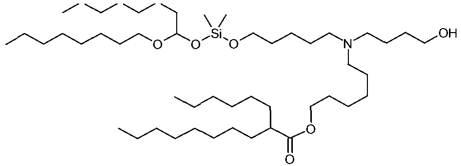 | 2-hexyldécanoate de 16-heptyl-7-(4-hydroxybutyl)-14,14-diméthyl-13,15,17-trioxa-7-aza-14-silapentacosyle |
| 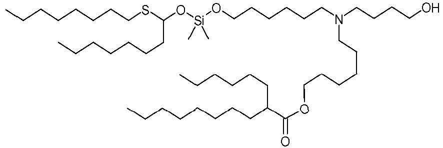 | 2-hexyldécanoate de 17-heptyl-7-(4-hydroxybutyl)-15,15-diméthyl-14,16-dioxa-18-thia-7-aza-15-silahexacosyle |
| 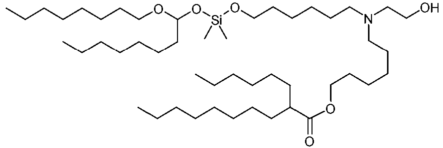 | 2-hexyldécanoate de 17-heptyl-7-(2-hydroxyéthyl)-15,15-diméthyl-14,16,18-trioxa-7-aza-15-silahexacosyle |
| 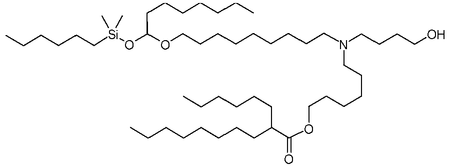 | 2-hexyldécanoate de 9-heptyl-20-(4-hydroxybutyl)-7,7-diméthyl-8,10-dioxa-20-aza-7-silahexacosan-26-yle |
| 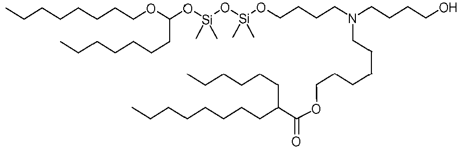 | 2-hexyldécanoate de 17-heptyl-7-(4-hydroxybutyl)-13,13,15,15-tétraméthyl-12,14,16,18-tétraoxa-7-aza-13,15-disilahexacosyle |
| 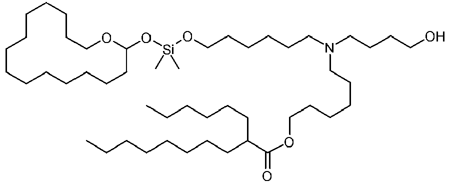 | 2-hexyldécanoate de 6-((6-((diméthyl((oxacyclohexadécan-2-yl)oxy)silyl)oxy)hexyl)(4-hydroxybutyl)amino)hexyle |
| 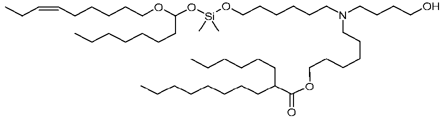 | 2-hexyldécanoate de (Z)-17-heptyl-7-(4-hydroxybutyl)-15,15-diméthyl-14,16,18-trioxa-7-aza-15-silaheptacos-24-ène-1-yle |
| 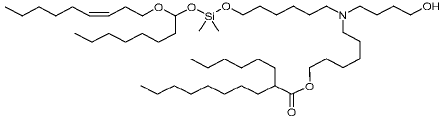 | 2-hexyldécanoate de (Z)-17-heptyl-7-(4-hydroxybutyl)-15,15-diméthyl-14,16,18-trioxa-7-aza-15-silaheptacos-21-ène-1-yle |
| 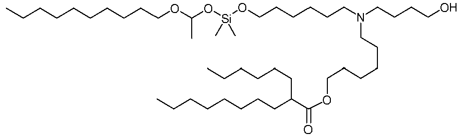 | 2-hexyldécanoate de 7-(4-hydroxybutyl)-15,15,17-triméthyl-14,16,18-trioxa-7-aza-15-silaoctacosyle |
| 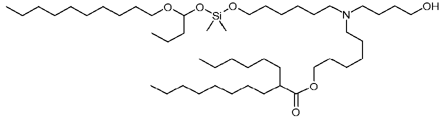 | 2-hexyldécanoate de 7-(4-hydroxybutyl)-15,15-diméthyl-17-propyl-14,16,18-trioxa-7-aza-15-silaoctacosyle |
| 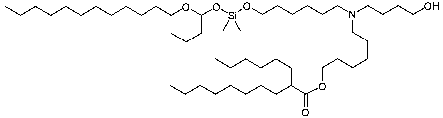 | 2-hexyldécanoate de 7-(4-hydroxybutyl)-15,15-diméthyl-17-propyl-14,16,18-trioxa-7-aza-15-silatriacontyle |
| 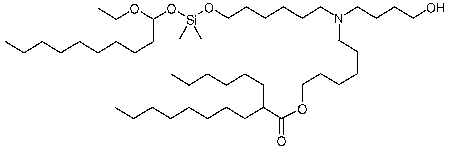 | 2-hexyldécanoate de 14-(4-hydroxybutyl)-6,6-diméthyl-4-nonyl-3,5,7-trioxa-14-aza-6-silaicosan-20-yle |
| 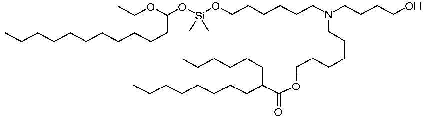 | 2-hexyldécanoate de 14-(4-hydroxybutyl)-6,6-diméthyl-4-undécyl-3,5,7-trioxa-14-aza-6-silaicosan-20-yle |
| 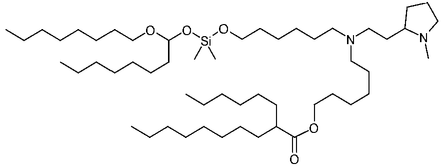 | 2-hexyldécanoate de 17-heptyl-15,15-diméthyl-7-(2-(1-méthylpyrrolidin-2-yl)éthyl)-14,16,18-trioxa-7-aza-15-silahexacosyle |
| 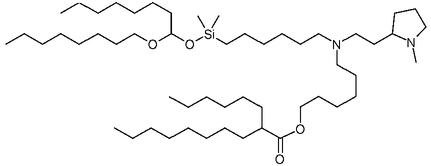 | 2-hexyldécanoate de 16-heptyl-14,14-diméthyl-7-(2-(1-méthylpyrrolidin-2-yl)éthyl)-15,17-dioxa-7-aza-14-silapentacosyle |
| 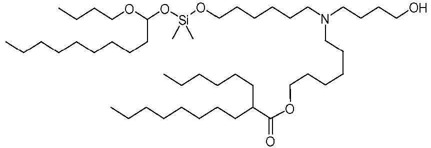 | 2-hexyldécanoate de 16-(4-hydroxybutyl)-8,8-diméthyl-6-nonyl-5,7,9-trioxa-16-aza-8-siladocosan-22-yle |
| 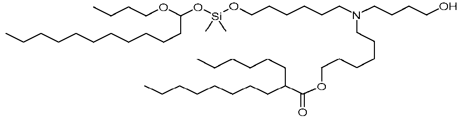 | 2-hexyldécanoate de 16-(4-hydroxybutyl)-8,8-diméthyl-6-undécyl-5,7,9-trioxa-16-aza-8-siladocosan-22-yle |
| 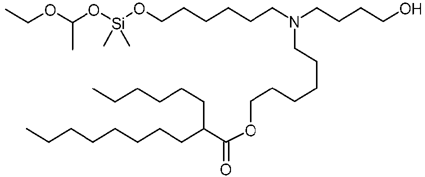 | 2-hexyldécanoate de 14-(4-hydroxybutyl)-4,6,6-triméthyl-3,5,7-trioxa-14-aza-6-silaicosan-20-yle |
| 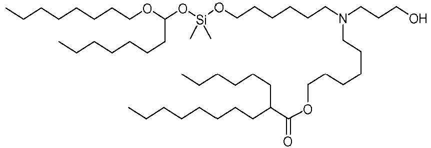 | 2-hexyldécanoate de 17-heptyl-7-(3-hydroxypropyl)-15,15-diméthyl-14,16,18-trioxa-7-aza-15-silahexacosyle |
| 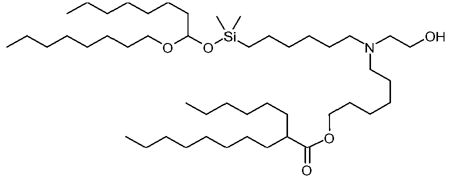 | 2-hexyldécanoate de 16-heptyl-7-(2-hydroxyéthyl)-14,14-diméthyl-15,17-dioxa-7-aza-14-silapentacosyle |
| 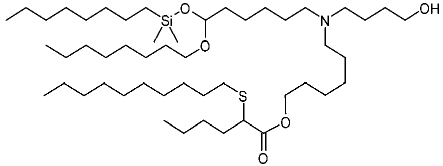 | 2-(décylthio)hexanoate de 6-((6-((diméthyl(octyl)silyl)oxy)-6-(octyloxy)hexyl)(4-hydroxybutyl)amino)hexyle |
| 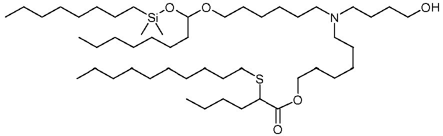 | 2-(décylthio)hexanoate de 15-heptyl-7-(4-hydroxybutyl)-17,17-diméthyl-14,16-dioxa-7-aza-17-silapentacosyle |
| 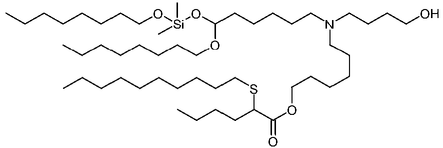 | 2-(décylthio)hexanoate de 7-(4-hydroxybutyl)-15,15-diméthyl-13-(octyloxy)-14,16-dioxa-7-aza-15-silatétracosyle |
| 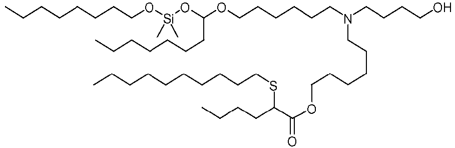 | 2-(décylthio)hexanoate de 15-heptyl-7-(4-hydroxybutyl)-17,17-diméthyl-14,16,18-trioxa-7-aza-17-silahexacosyle |
| 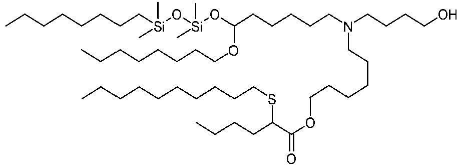 | 2-(décylthio)hexanoate de 6-((4-hydroxybutyl)(6-(octyloxy)-6-((1,1,3,3-tétraméthyl-3-octyldisiloxaneyl)oxy)hexyl)amino)hexyle |
| 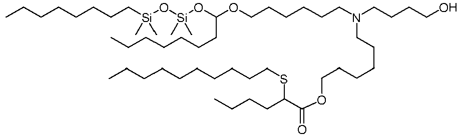 | 2-(décylthio)hexanoate de 15-heptyl-7-(4-hydroxybutyl)-17,17,19,19-tétraméthyl-14,16,18-trioxa-7-aza-17,19-disilaheptacosyle |
| 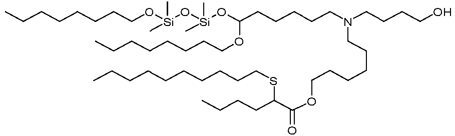 | 2-(décylthio)hexanoate de 6-((4-hydroxybutyl)(6-(octyloxy)-6-((1,1,3,3-tétraméthyl-3-(octyloxy)disiloxaneyl)oxy)hexyl)amino)hexyle |
| 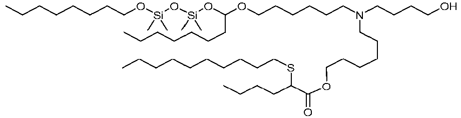 | 2-(décylthio)hexanoate de 15-heptyl-7-(4-hydroxybutyl)-17,17,19,19-tétraméthyl-14,16,18,20-tétraoxa-7-aza-17,19-disilaoctacosyle |
| 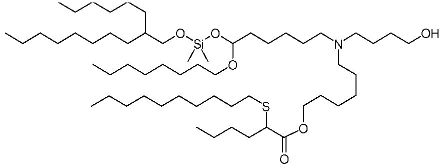 | 2-(décylthio)hexanoate de 18-hexyl-7-(4-hydroxybutyl)-15,15-diméthyl-13-(octyloxy)-14,16-dioxa-7-aza-15-silahexacosyle |
| 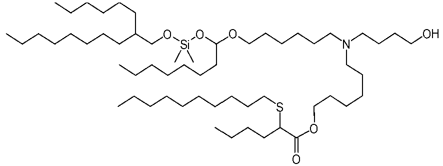 | 2-(décylthio)hexanoate de 15-heptyl-20-hexyl-7-(4-hydroxybutyl)-17,17-diméthyl-14,16,18-trioxa-7-aza-17-silaoctacosyle |
| 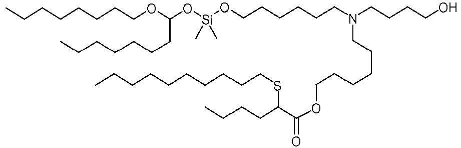 | 2-(décylthio)hexanoate de 17-heptyl-7-(4-hydroxybutyl)-15,15-diméthyl-14,16,18-trioxa-7-aza-15-silahexacosyle |
| 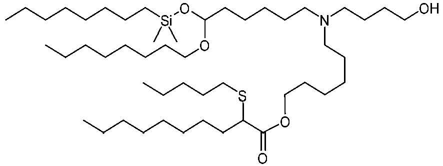 | 2-(pentylthio)décanoate de 6-((6-((diméthyl(octyl)silyl)oxy)-6-(octyloxy)hexyl)(4-hydroxybutyl)amino)hexyle |
| 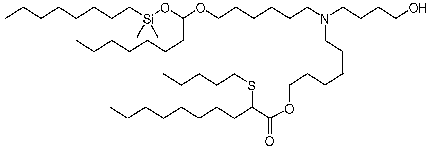 | 2-(pentylthio)décanoate de 15-heptyl-7-(4-hydroxybutyl)-17,17-diméthyl-14,16-dioxa-7-aza-17-silapentacosyle |
| 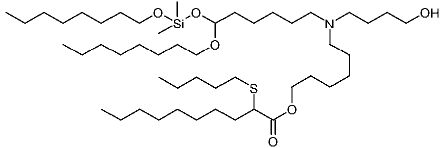 | 2-(pentylthio)décanoate de 7-(4-hydroxybutyl)-15,15-diméthyl-13-(octyloxy)-14,16-dioxa-7-aza-15-silatétracosyle |
| 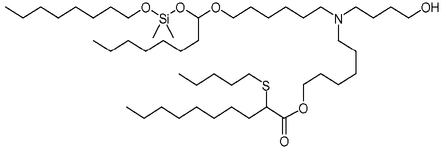 | 2-(pentylthio)décanoate de 15-heptyl-7-(4-hydroxybutyl)-17,17-diméthyl-14,16,18-trioxa-7-aza-17-silahexacosyle |
| 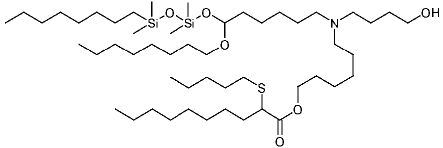 | 2-(pentylthio)décanoate de 6-((4-hydroxybutyl)(6-(octyloxy)-6-((1,1,3,3-tétraméthyl-3-octyldisiloxaneyl)oxy)hexyl)amino)hexyle |
| 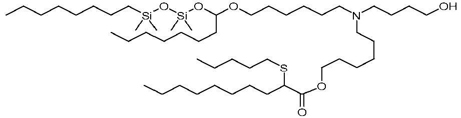 | 2-(pentylthio)décanoate de 15-heptyl-7-(4-hydroxybutyl)-17,17,19,19-tétraméthyl-14,16,18-trioxa-7-aza-17,19-disilaheptacosyle |
| 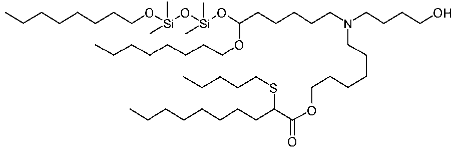 | 2-(pentylthio)décanoate de 6-((4-hydroxybutyl)(6-(octyloxy)-6-((1,1,3,3-tétraméthyl-3-(octyloxy)disiloxaneyl)oxy)hexyl)amino)hexyle |
| 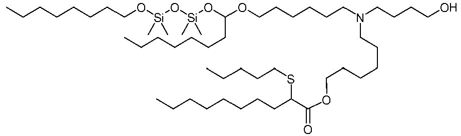 | 2-(pentylthio)décanoate de 15-heptyl-7-(4-hydroxybutyl)-17,17,19,19-tétraméthyl-14,16,18,20-tétraoxa-7-aza-17,19-disilaoctacosyle |
| 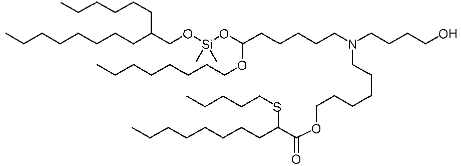 | 2-(pentylthio)décanoate de 18-hexyl-7-(4-hydroxybutyl)-15,15-diméthyl-13-(octyloxy)-14,16-dioxa-7-aza-15-silahexacosyle |
| 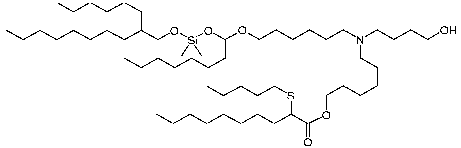 | 2-(pentylthio)décanoate de 15-heptyl-20-hexyl-7-(4-hydroxybutyl)-17,17-diméthyl-14,16,18-trioxa-7-aza-17-silaoctacosyle |
| 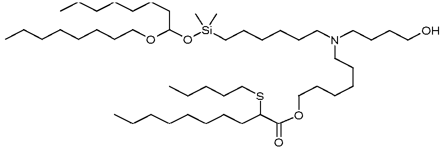 | 2-(pentylthio)décanoate de 16-heptyl-7-(4-hydroxybutyl)-14,14-diméthyl-15,17-dioxa-7-aza-14-silapentacosyle |
| 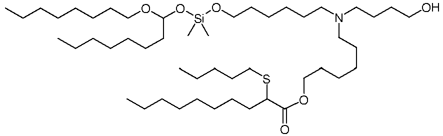 | 2-(pentylthio)décanoate de 17-heptyl-7-(4-hydroxybutyl)-15,15-diméthyl-14,16,18-trioxa-7-aza-15-silahexacosyle |
| 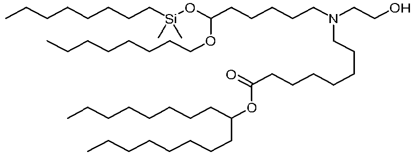 | 8-((6-((diméthyl(octyl)silyl)oxy)-6-(octyloxy)hexyl)(2-hydroxyéthyl)amino)octanoate d'heptadécan-9-yle |
| 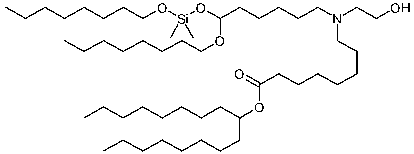 | 18-(2-hydroxyéthyl)-10,10-diméthyl-12-(octyloxy)-9,11-dioxa-18-aza-10-silahexacosan-26-oate d'heptadécan-9-yle |
| 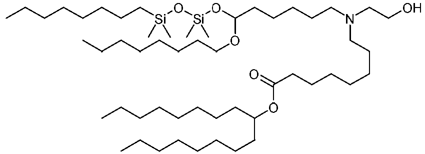 | 8-((2-hydroxyéthyl)(6-(octyloxy)-6-((1,1,3,3-tétraméthyl-3-octyldisiloxan-1-yl)oxy)hexyl)amino)octanoate d'heptadécan-9-yle |
| 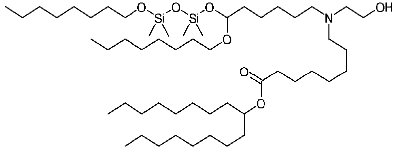 | 8-((2-hydroxyéthyl)(6-(octyloxy)-6-((1,1,3,3-tétraméthyl-3-(octyloxy)disiloxaneyl)oxy)hexyl)amino) octanoate d'heptadécan-9-yle |
| 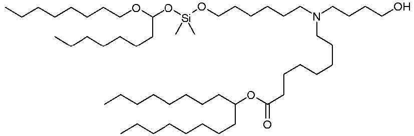 | 10-heptyl-20-(4-hydroxybutyl)-12,12-diméthyl-9,11,13-trioxa-20-aza-12-silaoctacosan-28-oate d'heptadécan-9-yle |
| 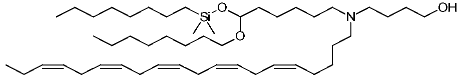 | 4-((6-((diméthyl(octyl)silyl)oxy)-6-(octyloxy)hexyl)((5Z,8Z,11Z,14Z,17Z)-icosa-5,8,11,14,17-pentaene-1-yl)amino)butan-1-ol |
| 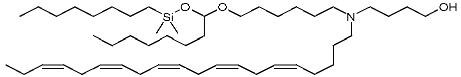 | 13-heptyl-5-((5Z,8Z,11Z,14Z,17Z)-icosa-5,8,11,14,17-pentaène-1-yl)-15,15-diméthyl-12,14-dioxa-5-aza-15-silatricosan-1-ol |
| 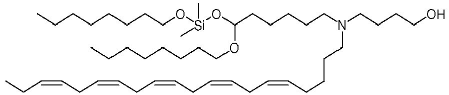 | 5-((5Z,8Z,11Z,14Z,17Z)-icosa-5,8,11,14,17-pentaène-1-yl)-13,13-diméthyl-11-(octyloxy)-12,14-dioxa-5-aza-13-siladocosan-1-ol |
| 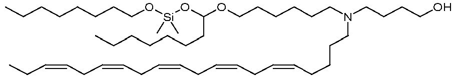 | 13-heptyl-5-((5Z,8Z,11Z,14Z,17Z)-icosa-5,8,11,14,17-pentaène-1-yl)-15,15-diméthyl-12,14,16-trioxa-5-aza-15-silatétracosan-1-ol |
| 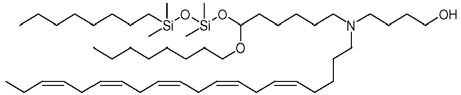 | 4-(((5Z,8Z,11Z,14Z,17Z)-icosa-5,8,11,14,17-pentaène-1-yl)(6-(octyloxy)-6-((1,1,3,3-tétraméthyl-3-octyldisiloxaneyl)oxy)hexyl)amino)butan-1-ol |
| 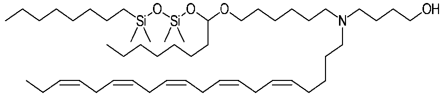 | 13-heptyl-5-((5Z,8Z,11Z,14Z,17Z)-icosa-5,8,11,14,17-pentaén-1-yl)-15,15,17,17-tétraméthyl-12,14,16-trioxa-5-aza-15,17-disilapentacosan-1-ol |
| 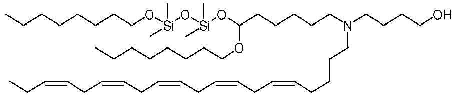 | 4-(((5Z,8Z,11Z,14Z,17Z)-icosa-5,8,11,14,17-pentaène-1-yl)(6-(octyloxy)-6-((1,1,3,3-tétraméthyl-3-(octyloxy)disiloxaneyl)oxy)hexyl)amino)butan-1-ol |
| 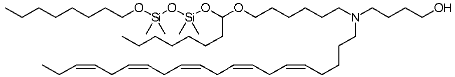 | 13-heptyl-5-((5Z,8Z,11Z,14Z,17Z)-icosa-5,8,11,14,17-pentaène-1-yl)-15,15,17,17-tétraméthyl-12,14,16,18-tétraoxa-5-aza-15,17-disilahexacosan-1-ol |
| 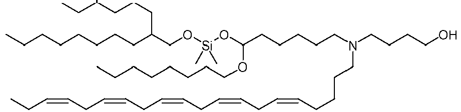 | 16-hexyl-5-((5Z,8Z,11Z,14Z,17Z)-icosa-5,8,11,14,17-pentaène-1-yl)-13,13-diméthyl-11-(octyloxy)-12,14-dioxa-5-aza-13-silatétracosan-1-ol |
| 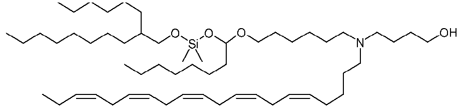 | 13-heptyl-18-hexyl-5-((5Z,8Z,11Z,14Z,17Z)-icosa-5,8,11,14,17-pentaène-1-yl)-15,15-diméthyl-12,14,16-trioxa-5-aza-15-silahexacosan-1-ol |
| 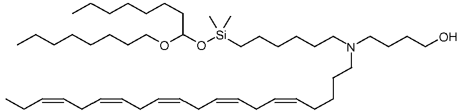 | 14-heptyl-5-((5Z,8Z,11Z,14Z,17Z)-icosa-5,8,11,14,17-pentaène-1-yl)-12,12-diméthyl-13,15-dioxa-5-aza-12-silatricosan-1-ol |
| 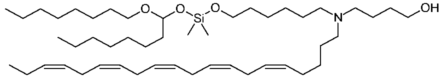 | 15-heptyl-5-((5Z,8Z,11Z,14Z,17Z)-icosa-5,8,11,14,17-pentaène-1-yl)-13,13-diméthyl-12,14,16-trioxa-5-aza-13-silatétracosan-1-ol |
| 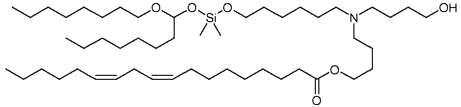 | (9Z,12Z)-octadéca-9,12-diénoate de 15-heptyl-5-(4-hydroxybutyl)-13,13-diméthyl-12,14,16-trioxa-5-aza-13-silatétracosyle |
| 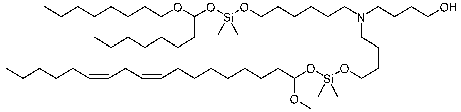 | 5-(3-((8Z,11Z)-heptadéca-8,11-diène-1-yl)-5,5-diméthyl-2,4,6-trioxa-5-siladécan-10-yl)-15-heptyl-13,13-diméthyl-12,14,16-trioxa-5-aza-13-silatétracosan-1-ol |
| 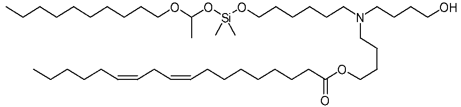 | (9Z,12Z)-octadéca-9,12-diénoate de 5-(4-hydroxybutyl)-13,13,15-triméthyl-12,14,16-trioxa-5-aza-13-silahexacosyle |
| 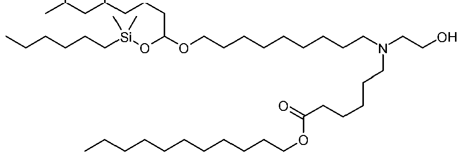 | 9-heptyl-20-(2-hydroxyéthyl)-7,7-diméthyl-8,10-dioxa-20-aza-7-silahexacosan-26-oate d'undécyle |
| 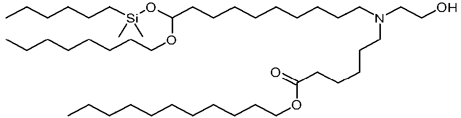 | 6-((10-((hexyldiméthylsilyl)oxy)-10-(octyloxy)décyle)(2-hydroxyéthyl)amino) hexanoate d'undécyle |
| 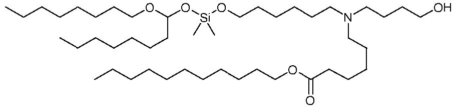 | 17-heptyl-7-(4-hydroxybutyl)-15,15-diméthyl-14,16,18-trioxa-7-aza-15-silahexacosanoate d' undécyle |
| 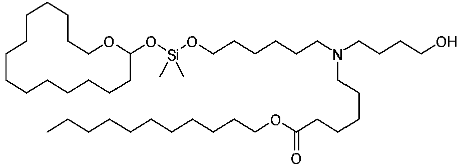 | 6-((6-((diméthyl((oxacyclohexadécan-2-yl)oxy)silyl)oxy)hexyle)(4-hydroxybutyle)amino)hexanoate d'undécyle |
| 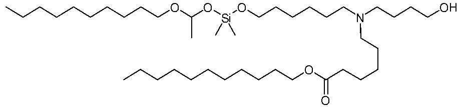 | 7-(4-hydroxybutyl)-15,15,17-triméthyl-14,16,18-trioxa-7-aza-15-silaoctacosanoate d' undécyle |
| 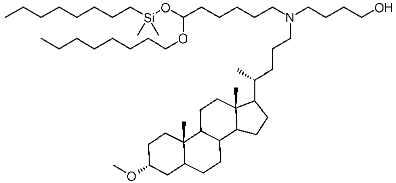 | 4-((6-((diméthyl(octyl)silyl)oxy)-6-(octyloxy)hexyl)((4R)-4-((3R,10S,13R)-3-méthoxy-10,13-diméthylhexadécahydro-1H-cyclopenta[a]phenanthrene-17-yl)pentyl)amino)butan-1-ol |
| 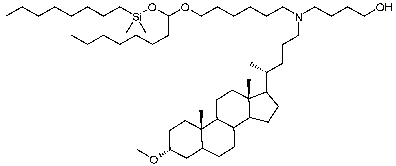 | 13-heptyl-5-((4R)-4-((3R,10S,13R)-3-méthoxy-10,13-diméthylhexadécahydro-1H-cyclopenta[a]phénanthrène-17-yl)pentyl)-15,15-diméthyl-12,14-dioxa-5-aza-15-silatricosan-1-ol |
| 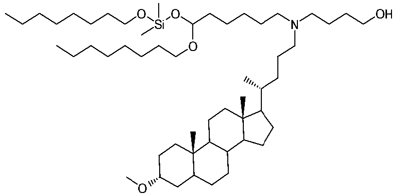 | 5-((4R)-4-((3R,10S,13R)-3-méthoxy-10,13-diméthylhexadécahydro-1H-cyclopenta[a]phénanthrène-17-yl)pentyl)-13,13-diméthyl-11-(octyloxy)-12,14-dioxa-5-aza-13-siladocosan-1-ol |
| 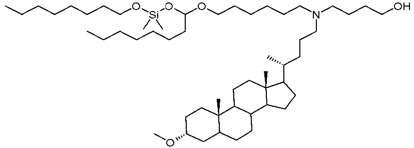 | 13-heptyl-5-((4R)-4-((3R,10S,13R)-3-méthoxy-10,13-diméthylhexadécahydro-1H-cyclopenta[a]phénanthrène-17-yl)pentyl)-15,15-diméthyl-12,14,16-trioxa-5-aza-15-silatétracosan-1-ol |
| 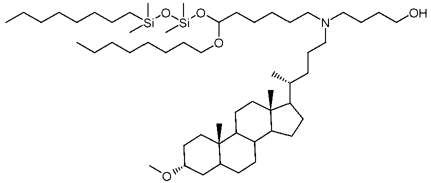 | 4-(((4R)-4-((3R,10S,13R)-3-méthoxy-10,13-diméthylhexadécahydro-1H-cyclopenta[a]phénanthrène-17-yl)pentyl)(6-(octyloxy)-6-((1,1,3,3-tétraméthyl-3-octyldisiloxaneyl)oxy)hexyl)amino)butan-1-ol |
| 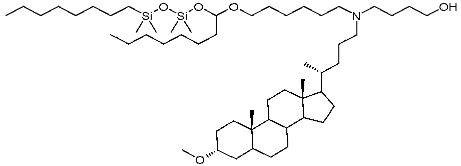 | 13-heptyl-5-((4R)-4-((3R,10S,13R)-3-méthoxy-10,13-diméthylhexadécahydro-1H-cyclopenta[a]phénanthrène-17-yl)pentyl)-15,15,17,17-tétraméthyl-12,14,16-trioxa-5-aza-15,17-disilapentacosan-1-ol |
| 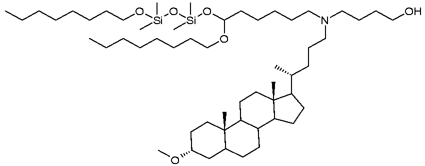 | 4-(((4R)-4-((3R,10S,13R)-3-méthoxy-10,13-diméthylhexadécahydro-1H-cyclopenta[a]phénanthrène-17-yl)pentyl)(6-(octyloxy)-6-((1,1,3,3-tétraméthyl-3-(octyloxy)disiloxaneyl)oxy)hexyl)amino)butan-1-ol |
| 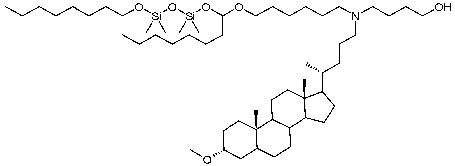 | 13-heptyl-5-((4R)-4-((3R,10S,13R)-3-méthoxy-10,13-diméthylhexadécahydro-1H-cyclopenta[a]phénanthrène-17-yl)pentyl)-15,15,17,17-tétraméthyl-12,14,16,18-tétraoxa-5-aza-15,17-disilahexacosan-1-ol |
| 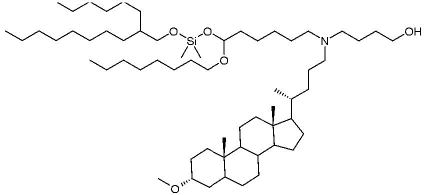 | 16-hexyl-5-((4R)-4-((3R,10S,13R)-3-méthoxy-10,13-diméthylhexadécahydro-1H-cyclopenta[a]phénanthrène-17-yl)pentyl)-13,13-diméthyl-11-(octyloxy)-12,14-dioxa-5-aza-13-silatétracosan-1-ol |
| 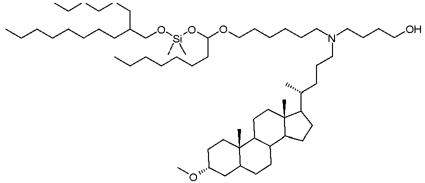 | 13-heptyl-18-hexyl-5-((4R)-4-((3R,10S,13R)-3-méthoxy-10,13-diméthylhexadécahydro-1H-cyclopenta[a]phénanthrène-17-yl)pentyl)-15,15-diméthyl-12,14,16-trioxa-5-aza-15-silahexacosan-1-ol |
| 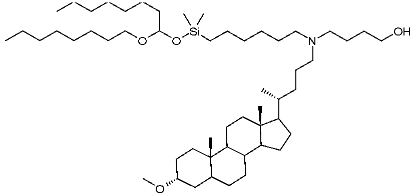 | 14-heptyl-5-((4R)-4-((3R,10S,13R)-3-méthoxy-10,13-diméthylhexadécahydro-1H-cyclopenta[a]phénanthrène-17-yl)pentyl)-12,12-diméthyl-13,15-dioxa-5-aza-12-silatricosan-1-ol |
| 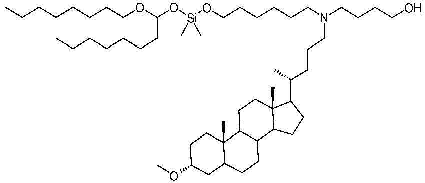 | 15-heptyl-5-((4R)-4-((3R,10S,13R)-3-méthoxy-10,13-diméthylhexadécahydro-1H-cyclopenta[a]phénanthrène-17-yl)pentyl)-13,13-diméthyl-12,14,16-trioxa-5-aza-13-silatétracosan-1-ol |
| 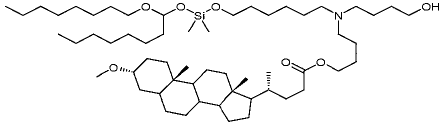 | (4R)-4-((3R,10S,13R)-3-méthoxy-10,13-diméthylhexadécahydro-1H-cyclopenta[a]phénanthrène-17-yl)pentanoate de 15-heptyl-5-(4-hydroxybutyl)-13,13-diméthyl-12,14,16-trioxa-5-aza-13-silatétracosyle |
| 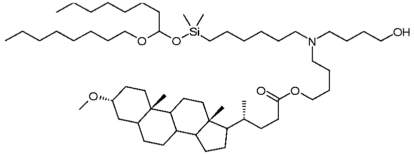 | (4R)-4-((3R,10S,13R)-3-méthoxy-10,13-diméthylhexadécahydro-1H-cyclopenta[a]phénanthrène-17-yl)pentanoate de 14-heptyl-5-(4-hydroxybutyl)-12,12-diméthyl-13,15-dioxa-5-aza-12-silatricosyle |

21. Composé présentant l'une quelconque des structures (Ia), (Ib), (Ic), 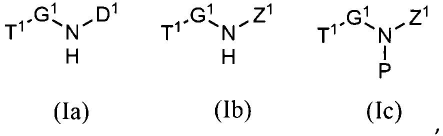 dans lesquelles D¹, G¹, T¹ et P sont tels que définis dans la revendication 1 et
Z¹ est un groupe 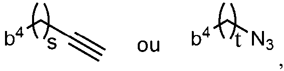 dans lesquels b⁴ est une liaison à N
s est un nombre entier compris entre 1 et 6
t est un nombre entier compris entre 2 et 6.

22. Composé ayant la structure (II) suivante 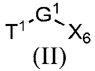 dans laquelle G¹est tel que défini dans la revendication 1 ;
T' est 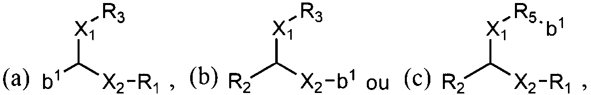 dans lesquels b¹, X₁ et X₂, R₁, R₂, R₃ et R₅ sont tels que définis dans la revendication 1 ; et
X₆ est choisi parmi Cl, Br, I, OMs, OTs, OTf.

23. Composé selon la revendication 22, qui présente l'une quelconque des structures suivantes :
| **Structure** | **Nom IUPAC** |
|---|---|
| 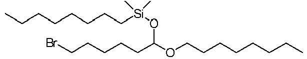 | ((6-bromo-1(octyloxy)hexyl)oxy)diméthyl(octyl)silane |
| 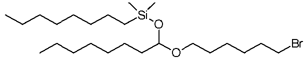 | ((1-((6-bromohexyl)oxy)octyl)oxy)diméthyl(octyl)silane |
| 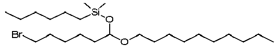 | ((6-bromo-1-(décyloxy)hexyl)oxy)(hexyl)diméthylsilane |
| 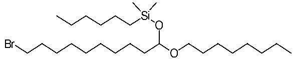 | ((10-bromo-1-(octyloxy)décyl)oxy)(hexyl)diméthylsilane |
| 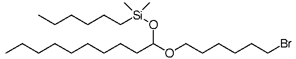 | ((1-((6-bromohexyl)oxy)décyl)oxy)(hexyl)diméthylsilane |
| 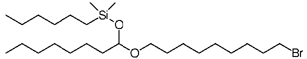 | ((1-((9-bromononyl)oxy)octyl)oxy)(hexyl)diméthylsilane |
| 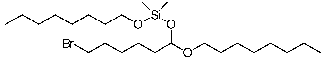 | 12-(5-bromopentyl)-10,10-diméthyl-9,11,13-trioxa-10-silahénicosane |
| 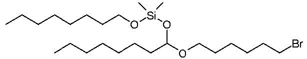 | 1-bromo-8-heptyl-10,10-diméthyl-7,9,11-trioxa-10-silanonadécane |
| 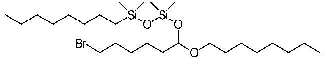 | 1-((6-bromo-1-(octyloxy)hexyl)oxy)-1,1,3,3-tétraméthyl-3-octyldisiloxane |
| 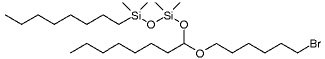 | 1-((1-((6-bromohexyl)oxy)octyl)oxy)-1,1,3,3-tétraméthyl-3-octyldisiloxane |
| 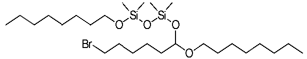 | 1-((6-bromo-1-(octyloxy)hexyl)oxy)-1,1,3,3-tétraméthyl-3-(octyloxy)disiloxane |
| 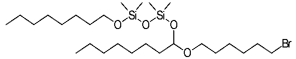 | 1-((1-((6-bromohexyl)oxy)octyl)oxy)-1,1,3,3-tétraméthyl-3-(octyloxy)disiloxane |
| 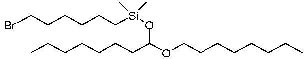 | (6-bromohexyl)diméthyl((1-(octyloxy)octyl)oxy)silane |
| 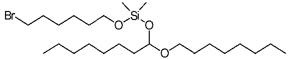 | 1-bromo-10-heptyl-8,8-diméthyl-7,9,11-trioxa-8-silanonadécane |
| 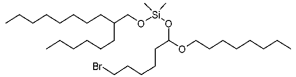 | 10-(5-bromopentyl)-15-hexyl-12,12-diméthyl-9,11,13-trioxa-12-silatricosane |
| 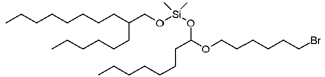 | 1-bromo-8-heptyl-13-hexyl-10,10-diméthyl-7,9,11-trioxa-10-silahénicosane |
| 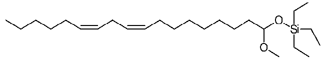 | triéthyl(((9Z,12Z)-1-méthoxyoctadéca-9,12-diène-1-yl)oxy)silane |
| 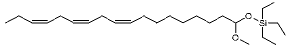 | triéthyl(((9Z,12Z,15Z)-1-méthoxyoctadéca-9,12,15-triène-1-yl)oxy)silane |
| 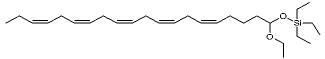 | (((5Z,8Z,11Z,14Z,17Z)-1-éthoxyicosa-5,8,11,14,17-pentaène-1-yl)oxy)triéthylsilane |
| 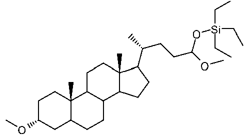 | triéthyl(((4R)-1-méthoxy-4-((3R,10S,13R)-3-méthoxy-10,13-diméthylhexadécahydro-1H-cyclopenta[a]phénanthrène-17-yl)pentyl)oxy)silane |
| 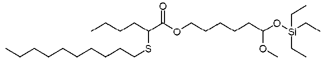 | 2-(décylthio)hexanoate de 6-méthoxy-6-((triéthylsilyl)oxy)hexyle |
| 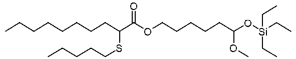 | 2-(pentylthio)décanoate de 6-méthoxy-6-((triéthylsilyl)oxy)hexyle |
| 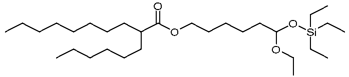 | 2-hexyldécanoate de 6-éthoxy-6-((triéthylsilyl)oxy)hexyle |
| 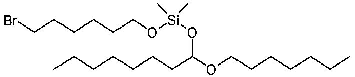 | 1-bromo-10-heptyl-8,8-diméthyl-7,9,11-trioxa-8-silaoctadécane |
| 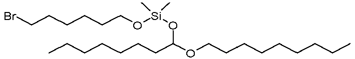 | 1-bromo-10-heptyl-8,8-diméthyl-7,9,11-trioxa-8-silaicosane |
| 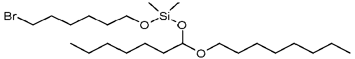 | 1-bromo-10-hexyl-8,8-diméthyl-7,9,11-trioxa-8-silanonadécane |
| 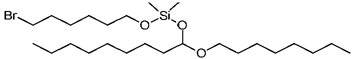 | 1-bromo-8,8-diméthyl-10-octyl-7,9,11-trioxa-8-silanonadécane |
| 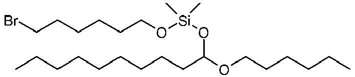 | 1-bromo-8,8-diméthyl-10-nonyl-7,9,11-trioxa-8-silaheptadécane |
| 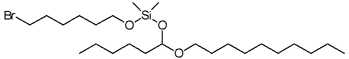 | 1-bromo-8,8-diméthyl-10-pentyl-7,9,11-trioxa-8-silahénicosane |
| 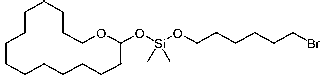 | ((6-bromohexyl)oxy)diméthyl((oxacyclohexadécan-2-yl)oxy)silane |
| 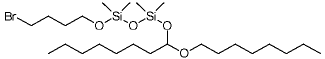 | 1-(4-bromobutoxy)-1,1,3,3-tétraméthyl-3-((1-(octyloxy)octyl)oxy)disiloxane |
| 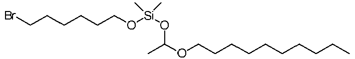 | 1-bromo-8,8,10-triméthyl-7,9,11-trioxa-8-silahénicosane |
| 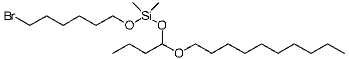 | 1-bromo-8,8-diméthyl-10-propyl-7,9,11-trioxa-8-silahénicosane |
| 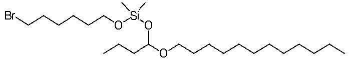 | 1-bromo-8,8-diméthyl-10-propyl-7,9,11-trioxa-8-silatricosane |
| 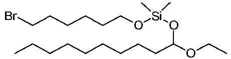 | 13-bromo-6,6-diméthyl-4-nonyl-3,5,7-trioxa-6-silatridécane |
| 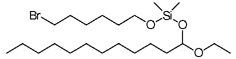 | 13-bromo-6,6-diméthyl-4-undécyl-3,5,7-trioxa-6-silatridécane |
| 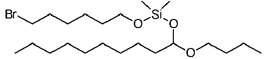 | 15-bromo-8,8-diméthyl-6-nonyl-5,7,9-trioxa-8-silapentadécane |
| 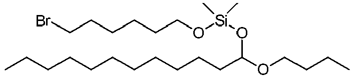 | 15-bromo-8,8-diméthyl-6-undécyl-5,7,9-trioxa-8-silapentadécane |
| 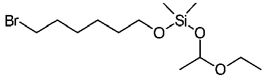 | 13-bromo-4,6,6-triméthyl-3,5,7-trioxa-6-silatridécane |
| 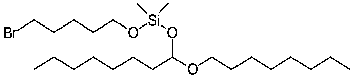 | 1-bromo-9-heptyl-7,7-diméthyl-6,8,10-trioxa-7-silaoctadécane |
| 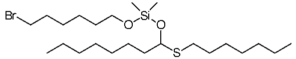 | 1-bromo-10-heptyl-8,8-diméthyl-7,9-dioxa-11-thia-8-silaoctadécane |
| 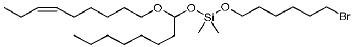 | (Z)-1-bromo-10-heptyl-8,8-diméthyl-7,9,11-trioxa-8-silaicos-17-ène |
| 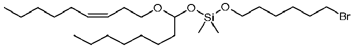 | (Z)-1-bromo-10-heptyl-8,8-diméthyl-7,9,11-trioxa-8-silaicos-14-ène |
| 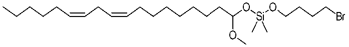 | 10-bromo-3-((8Z,11Z)-heptadéca-8,11-diène-1-yl)-5,5-diméthyl-2,4,6-trioxa-5-siladécane |

24. Nanoparticule lipidique comprenant un agent thérapeutique et un composé selon l'une quelconque des revendications 1 à 20, en particulier un composé selon la revendication 8, 9, 10, 11, 12 ou 20.
